# EUROPEAN PATENT SPECIFICATION

(11) **EP 1 385 885 B1**
(45) Date of publication and mention of the grant of the patent: **16.04.2008**
(21) Application number: 02754563.1
(22) Date of filing: 07.05.2002
(51) Int. Cl.: C07K 14/705

(54) **FRAGMENTS OF THE RETINOIC ACID-RELATED ORPHAN RECEPTOR (ROR) COMPRISING THE LIGAND BINDING DOMAIN (LBD), CRYSTAL STRUCTURE OF THE LBD OF ROR-BETA AND THEIR APPLICATIONS**
FRAGMENTE VON RETINOIC ACID-RELATED ORPHAN REZEPTOREN (ROR) WELCHE DIE LIGANDENBINDEDOMAINE (LBD) ENTHALTEN, KRISTALLSTRUKTUR DER LBD VON ROR-BETA UND DEREN VERWENDUNGEN
FRAGMENTS DU RECEPTEUR ORPHELIN APPARENTE AUX RECEPTEURS DE L'ACIDE RETINOIQUE (ROR) COMPORTANT LE DOMAINE DE LIASON AUX LIGANDS (LBD), LA STRUCTURE CRISTALLINE DU LBD DU ROR-BETA, ET LEURS APPLICATIONS

(30) Priority: 07.05.2001 EP 01401175
(43) Date of publication of application: 04.02.2004
(73) Proprietor: CENTRE NATIONAL DE LA RECHERCHE SCIENTIFIQUE (CNRS), 75794 Paris Cedex 16 (FR)
(72) Inventor: MORAS, Dino, F-67450 Lampertheim (FR); RENAUD, Jean-Paul, F-67540 Ostwald (FR); STEHLIN, Catherine, F-67000 Strasbourg (FR); WURTZ, Jean-Marie, F-67404 Drusenheim (FR); SCHUELE, Roland, 79367 Weisweil (DE); GREINER, Erich, Friedrich, 69120 Heidelberg (DE)
(74) Representative: Grosset-Fournier, Chantal Catherine
(86) International application number: PCT/EP2002/005024
(87) International publication number: WO 2003/000732

(56) References cited:
- WO-A-00/24757
- WO-A-01/26737
- WO-A-99/50660
- GREINER ERICH F ET AL: "Functional analysis of retinoid Z receptor beta, a brain-specific nuclear orphan receptor." PROCEEDINGS OF THE NATIONAL ACADEMY OF SCIENCES OF THE UNITED STATES, vol. 93, no. 19, 1996, pages 10105-10110, XP002194193 1996 ISSN: 0027-8424
- GREINER ERICH F ET AL: "Differential ligand-dependent protein-protein interactions between nuclear receptors and a neuronal-specific cofactor." PROCEEDINGS OF THE NATIONAL ACADEMY OF SCIENCES OF THE UNITED STATES, vol. 97, no. 13, 30 June 2000 (2000-06-30), pages 7160-7165, XP002194194 June 30, 2000 ISSN: 0027-8424
- DATABASE EMBL [Online] retrieved from EBI Database accession no. P35398; P51448; Q92753; P45446; P51449; P51450 XP002221987
- GILLILAND G L ET AL: "Crystallization of biological macromolecules for X-ray diffraction studies" CURRENT OPINION IN STRUCTURAL BIOLOGY, CURRENT BIOLOGY LTD., LONDON, GB, vol. 6, no. 5, October 1996 (1996-10), pages 595-603, XP002128062 ISSN: 0959-440X
- ABOLA ENRIQUE ET AL: "Automation of X-ray crystallography." NATURE STRUCTURAL BIOLOGY, vol. 7, no. Supplement, November 2000 (2000-11), pages 973-977, XP001062873 ISSN: 1072-8368
- BOURGUET WILLIAM ET AL: "Crystal structure of a heterodimeric complex of RAR and RXR ligand-binding domains." MOLECULAR CELL, vol. 5, no. 2, February 2000 (2000-02), pages 289-298, XP002194192 ISSN: 1097-2765
- NOLTE R T ET AL: "LIGAND BINDING AND CO-ACTIVATOR ASSEMBLY OF THE PEROXISOME PROLIFERATOR-ACTIVATED RECEPTOR-GAMMA" NATURE, MACMILLAN JOURNALS LTD. LONDON, GB, vol. 395, 10 September 1998 (1998-09-10), pages 137-143, XP002906503 ISSN: 0028-0836
- STEHLIN CATHERINE ET AL: "X-ray structure of the orphan nuclear receptor RORbeta ligand-binding domain in the active conformation." EMBO (EUROPEAN MOLECULAR BIOLOGY ORGANIZATION) JOURNAL, vol. 20, no. 21, 1 November 2001 (2001-11-01), pages 5822-5831, XP002221986 ISSN: 0261-4189

### SEQUENCE LISTING

<110> CNRS
<120> POLYPEPTIDES DERIVED FROM RETINOIC ACID-RELATED ORPHAN RECEPTOR (ROR), AND THEIR APPLICATIONS
<130> IOB 01 CNR RORB
<140>
   <141>
<160> 37
<170> PatentIn Ver. 2.1
<210> 1
   <211> 732
   <212> DNA
   <213> Artificial Sequence
<220>
   <223> Description of Artificial Sequence: nucleotide sequence coding for a fragment of rat RORβ
<220>
   <221> CDS
   <222> (1)..(732)
<400> 1
   atg tct gag atc gat cga att gca cag aac atc att aag tcc cat ttg      48
   Met Ser Glu Ile Asp Arg Ile Ala Gln Asn Ile Ile Lys Ser His Leu
     1               5                  10                  15
   gag acg tgc cag tac aca atg gaa gag ctc cat cag ctg gca tgg cag      96
   Glu Thr Cys Gln Tyr Thr Met Glu Glu Leu His Gln Leu Ala Trp Gln
                20                  25                  30
   acc cac acc tat gag gaa ata aag gca tat caa agc aag tcc agg gag     144
   Thr His Thr Tyr Glu Glu Ile Lys Ala Tyr Gln Ser Lys Ser Arg Glu
            35                  40                  45
   gct ctg tgg cag cag tgt gcc atc cag atc acc cac gcc atc caa tat     192
   Ala Leu Trp Gln Gln Cys Ala Ile Gln Ile Thr His Ala Ile Gln Tyr
        50                  55                  60
   gtg gtg gag ttc gca aag cgg ata aca ggc ttc atg gag ctg tgt cag     240
   Val Val Glu Phe Ala Lys Arg Ile Thr Gly Phe Met Glu Leu Cys Gln
    65                  70                  75                  80
   aac gat cag atc tta ctt ctg aag tca ggt tgc ttg gaa gtg gtt tta     288
   Asn Asp Gln Ile Leu Leu Leu Lys Ser Gly Cys Leu Glu Val Val Leu
                    85                  90                  95
   gtg aga atg tgc cgt gcc ttc aac cca tta aac aac act gtt ctg ttt     336
   Val Arg Met Cys Arg Ala Phe Asn Pro Leu Asn Asn Thr Val Leu Phe
               100                 105                 110
   gaa gga aaa tat gga gga atg caa atg ttc aaa gcc tta ggt tct gat     384
   Glu Gly Lys Tyr Gly Gly Met Gln Met Phe Lys Ala Leu Gly Ser Asp
           115                 120                 125
   gac cta gtg aat gaa gca ttt gac ttt gcg aag aat ctg tgt tcc ttg     432
   Asp Leu Val Asn Glu Ala Phe Asp Phe Ala Lys Asn Leu Cys Ser Leu
       130                 135                 140
   cag ctg acc gag gaa gag att gct ctg ttc tcc tct gct gtt ctg ata     480
   Gln Leu Thr Glu Glu Glu Ile Ala Leu Phe Ser Ser Ala Val Leu Ile
   145                 150                 155                 160
   tcc cca gac cga gcc tgg ctg tta gaa cca aga aaa gtc cag aag ctt     528
   Ser Pro Asp Arg Ala Trp Leu Leu Glu Pro Arg Lys Val Gln Lys Leu
                   165                 170                 175
   cag gaa aaa att tat ttt gca ctt caa cat gtg att cag aag aat cac     576
   Gln Glu Lys Ile Tyr Phe Ala Leu Gln His Val Ile Gln Lys Asn His
               180                 185                 190
   ctg gat gat gag acc ctg gca aag tta ata gcc aag ata cca act atc     624
   Leu Asp Asp Glu Thr Leu Ala Lys Leu Ile Ala Lys Ile Pro Thr Ile
           195                 200                 205
   acg gca gtc tgc aac ttg cat ggg gag aag cta cag gta ttt aag cag     672
   Thr Ala Val Cys Asn Leu His Gly Glu Lys Leu Gln Val Phe Lys Gln
       210                 215                 220
   tct cat cca gac ata gtg aat aca ctg ttt cct cca ttg tac aag gag     720
   Ser His Pro Asp Ile Val Asn Thr Leu Phe Pro Pro Leu Tyr Lys Glu
   225                 230                 235                 240
   ctc ttt aat cct                                                     732
   Leu Phe Asn Pro
<210> 2
   <211> 244
   <212> PRT
   <213> Artificial Sequence
   <223> Description of Artificial Sequence: nucleotide sequence coding for a fragment of rat RORβ
<400> 2
   Met Ser Glu Ile Asp Arg Ile Ala Gln Asn Ile Ile Lys Ser His Leu
   1 5 10 15
   Glu Thr Cys Gln Tyr Thr Met Glu Glu Leu His Gln Leu Ala Trp Gln
   20 25 30
   Thr His Thr Tyr Glu Glu Ile Lys Ala Tyr Gln Ser Lys Ser Arg Glu
   35 40 45
   Ala Leu Trp Gln Gln Cys Ala Ile Gln Ile Thr His Ala Ile Gln Tyr
   50 55 60
   Val Val Glu Phe Ala Lys Arg Ile Thr Gly Phe Met Glu Leu Cys Gln
   65 70 75 80
   Asn Asp Gln Ile Leu Leu Leu Lys Ser Gly Cys Leu Glu Val Val Leu
   85 90 95
   Val Arg Met Cys Arg Ala Phe Asn Pro Leu Asn Asn Thr Val Leu Phe
   100 105 110
   Glu Gly Lys Tyr Gly Gly Met Gln Met Phe Lys Ala Leu Gly Ser Asp
   115 120 125
   Asp Leu Val Asn Glu Ala Phe Asp Phe Ala Lys Asn Leu Cys Ser Leu
   130 135 140
   Gln Leu Thr Glu Glu Glu Ile Ala Leu Phe Ser Ser Ala Val Leu Ile
   145 150 155 160
   Ser Pro Asp Arg Ala Trp Leu Leu Glu Pro Arg Lys Val Gln Lys Leu
   165 170 175
   Gln Glu Lys Ile Tyr Phe Ala Leu Gln His Val Ile Gln Lys Asn His
   180 185 190
   Leu Asp Asp Glu Thr Leu Ala Lys Leu Ile Ala Lys Ile Pro Thr Ile
   195 200 205
   Thr Ala Val Cys Asn Leu His Gly Glu Lys Leu Gln Val Phe Lys Gln
   210 215 220
   Ser His Pro Asp Ile Val Asn Thr Leu Phe Pro Pro Leu Tyr Lys Glu
   225 230 235 240
   Leu Phe Asn Pro
<210> 3
   <211> 244
   <212> PRT
   <213> Artificial Sequence
<220>
   <223> Description of Artificial Sequence: fragment of human RORβ
<400> 3
   Met Thr Glu Ile Asp Arg Ile Ala Gln Asn Ile Ile Lys Ser His Leu
   1 5 10 15
   Glu Thr Cys Gln Tyr Thr Met Glu Glu Leu His Gln Leu Ala Trp Gln
   20 25 30
   Thr His Thr Tyr Glu Glu Ile Lys Ala Tyr Gln Ser Lys Ser Arg Glu
   35 40 45
   Ala Leu Trp Gln Gln Cys Ala Ile Gln Ile Thr His Ala Ile Gln Tyr
   50 55 60
   Val Val Glu Phe Ala Lys Arg Ile Thr Gly Phe Met Glu Leu Cys Gln
   65 70 75 80
   Asn Asp Gln Ile Leu Leu Leu Lys Ser Gly Cys Leu Glu Val Val Leu
   85 90 95
   Val Arg Met Cys Arg Ala Phe Asn Pro Leu Asn Asn Thr Val Leu Phe
   100 105 110
   Glu Gly Lys Tyr Gly Gly Met Gln Met Phe Lys Ala Leu Gly Ser Asp
   115 120 125
   Asp Leu Val Asn Glu Ala Phe Asp Phe Ala Lys Asn Leu Cys Ser Leu
   130 135 140
   Gln Leu Thr Glu Glu Glu Ile Ala Leu Phe Ser Ser Ala Val Leu Ile
   145 150 155 160
   Ser Pro Asp Arg Ala Trp Leu Ile Glu Pro Arg Lys Val Gln Lys Leu
   165 170 175
   Gln Glu Lys Ile Tyr Phe Ala Leu Gln His Val Ile Gln Lys Asn His
   180 185 190
   Leu Asp Asp Glu Thr Leu Ala Lys Leu Ile Ala Lys Ile Pro Thr Ile
   195 200 205
   Thr Ala Val Cys Asn Leu His Gly Glu Lys Leu Gln Val Phe Lys Gln
   210 215 220
   Ser His Pro Glu Ile Val Asn Thr Leu Phe Pro Pro Leu Tyr Lys Glu
   225 230 235 240
   Leu Phe Asn Pro
<210> 4
   <211> 242
   <212> PRT
   <213> Artificial Sequence
<220>
   <223> Description of Artificial Sequence: fragment of human RORγ
<400> 4
   Leu Thr Glu Ile Glu His Leu Val Gln Ser Val Cys Lys Ser Tyr Arg
    1               5                  10                  15
   Glu Thr Cys Gln Leu Arg Leu Glu Asp Leu Leu Arg Gln Arg Ser Asn
               20                  25                  30
   Ile Phe Ser Arg Glu Glu Val Thr Gly Tyr Gln Arg Lys Ser Met Trp
           35                  40                  45
   Glu Met Trp Glu Arg Cys Ala His His Leu Thr Glu Ala Ile Gln Tyr
       50                  55                  60
   Val Val Glu Phe Ala Lys Arg Leu Ser Gly Phe Met Glu Leu Cys Gln
   65                  70                  75                  80
   Asn Asp Gln Ile Val Leu Leu Lys Ala Gly Ala Met Glu Val Val Leu
                   85                  90                  95
   Val Arg Met Cys Arg Ala Tyr Asn Ala Asp Asn Arg Thr Val Phe Phe
               100                 105                 110
   Glu Gly Lys Tyr Gly Gly Met Glu Leu Phe Arg Ala Leu Gly Cys Ser
           115                 120                 125
   Glu Leu Ile Ser Ser Ile Phe Asp Phe Ser His Ser Leu Ser Ala Leu
       130                 135                 140
   His Phe Ser Glu Asp Glu Ile Ala Leu Tyr Thr Ala Leu Val Leu Ile
   145                 150                 155                 160
   Asn Ala His Arg Pro Gly Leu Gln Glu Lys Arg Lys Val Glu Gln Leu
                   165                 170                 175
   Gln Tyr Asn Leu Glu Leu Ala Phe His His His Leu Cys Lys Thr His
               180                 185                 190
   Arg Gln Ser Ile Leu Ala Lys Leu Pro Pro Lys Gly Lys Leu Arg Ser
           195                 200                 205
   Leu Cys Ser Gln His Val Glu Arg Leu Gln Ile Phe Gln His Leu His
       210                 215                 220
   Pro Ile Val Val Gln Ala Ala Phe Pro Pro Leu Tyr Lys Glu Leu Phe
   225                 230                 235                 240
   Ser Thr
<210> 5
   <211> 242
   <212> PRT
   <213> Artificial Sequence
<220>
   <223> Description of Artificial Sequence: fragment of murine RORγ
<400> 5
   Leu Thr Asp Ile Glu Tyr Leu Val Gln Asn Val Cys Lys Ser Phe Arg
   1               5                   10                  15
   Glu Thr Cys Gln Leu Arg Leu Glu Asp Leu Leu Arg Gln Arg Thr Asn
               20                  25                  30
   Leu Phe Ser Arg Glu Glu Val Thr Ser Tyr Gln Arg Lys Ser Met Trp
           35                  40                  45
   Glu Met Trp Glu Arg Cys Ala His His Leu Thr Glu Ala Ile Gln Tyr
       50                  55                  60
   Val Val Glu Phe Ala Lys Arg Leu Ser Gly Phe Met Glu Leu Cys Gln
   65                  70                  75                  80
   Asn Asp Gln Ile Ile Leu Leu Thr Ala Gly Ala Met Glu Val Val Leu
                   85                  90                  95
   Val Arg Met Cys Arg Ala Tyr Asn Ala Asn Asn His Thr Val Phe Phe
               100                 105                 110
   Glu Gly Lys Tyr Gly Gly Val Glu Leu Phe Arg Ala Leu Gly Cys Ser
           115                 120                 125
   Glu Leu Ile Ser Ser Ile Phe Asp Phe Ser His Phe Leu Ser Ala Leu
       130                 135                 140
   Cys Phe Ser Glu Asp Glu Ile Ala Leu Tyr Thr Ala Leu Val Leu Ile
   145                 150                 155                 160
   Asn Ala Asn Arg Pro Gly Leu Gln Glu Lys Arg Arg Val Glu His Leu
                   165                 170                 175
   Gln Tyr Asn Leu Glu Leu Ala Phe His His His Leu Cys Lys Thr His
               180                 185                 190
   Arg Gln Gly Leu Leu Ala Lys Leu Pro Pro Lys Gly Lys Leu Arg Ser
           195                 200                 205
   Leu Cys Ser Gln His Val Glu Lys Leu Gln Ile Phe Gln His Leu His
       210                 215                 220
   Pro Ile Val Val Gln Ala Ala Phe Pro Pro Leu Tyr Lys Glu Leu Phe
   225                 230                 235                 240
   Ser Thr
<210> 6
   <211> 244
   <212> PRT
   <213> Artificial Sequence
<220>
   <223> Description of Artificial Sequence: fragment of human RORα
<400> 6
   Met Ala Glu Leu Glu His Leu Ala Gln Asn Ile Ser Lys Ser His Leu
     1               5                  10                  15
   Glu Thr Cys Gln Tyr Leu Arg Glu Glu Leu Gln Gln Ile Thr Trp Gln
                20                  25                  30
   Thr Phe Leu Gln Glu Glu Ile Glu Asn Tyr Gln Asn Lys Gln Arg Glu
            35                  40                  45
   Val Met Trp Gln Leu Cys Ala Ile Lys Ile Thr Glu Ala Ile Gln Tyr
        50                  55                  60
   Val Val Glu Phe Ala Lys Arg Ile Asp Gly Phe Met Glu Leu Cys Gln
    65                  70                  75                  80
   Asn Asp Gln Ile Val Leu Leu Lys Ala Gly Ser Leu Glu Val Val Phe
                    85                  90                  95
   Ile Arg Met Cys Arg Ala Phe Asp Ser Gln Asn Asn Thr Val Tyr Phe
               100                 105                 110
   Asp Gly Lys Tyr Ala Ser Pro Asp Val Phe Lys Ser Leu Gly Cys Glu
           115                 120                 125
   Asp Phe Ile Ser Phe Val Phe Glu Phe Gly Lys Ser Leu Cys Ser Met
       130                 135                 140
   His Leu Thr Glu Asp Glu Ile Ala Leu Phe Ser Ala Phe Val Leu Met
   145                 150                 155                 160
   Ser Ala Asp Arg Ser Trp Leu Gln Glu Lys Val Lys Ile Glu Lys Leu
                   165                 170                 175
   Gln Gln Lys Ile Gln Leu Ala Leu Gln His Val Leu Gln Lys Asn His
               180                 185                 190
   Arg Glu Asp Gly Ile Leu Thr Lys Leu Ile Cys Lys Val Ser Thr Leu
           195                 200                 205
   Arg Ala Leu Cys Gly Arg His Thr Glu Lys Leu Met Ala Phe Lys Ala
       210                 215                 220
   Ile Tyr Pro Asp Ile Val Arg Leu His Phe Pro Pro Leu Tyr Lys Glu
   225                 230                 235                 240
   Leu Phe Thr Ser
<210> 7
   <211> 244
   <212> PRT
   <213> Artificial Sequence
<220>
   <223> Description of Artificial Sequence: fragment of murine RORα
<400> 13
<210> 8
   <211> 245
   <212> PRT
   <213> Artificial Sequence
   <223> Description of Artificial Sequence: nucleotide sequence coding for a fragment of rat RORβ
<400> 8
   Thr Met Ser Glu Ile Asp Arg Ile Ala Gln Asn Ile Ile Lys Ser His
   1               5                   10                  15
   Leu Glu Thr Cys Gln Tyr Thr Met Glu Glu Leu His Gln Leu Ala Trp
               20                  25                  30
   Gln Thr His Thr Tyr Glu Glu Ile Lys Ala Tyr Gln Ser Lys Ser Arg
           35                  40                  45
   Glu Ala Leu Trp Gln Gln Cys Ala Ile Gln Ile Thr His Ala Ile Gln
       50                  55                  60
   Tyr Val Val Glu Phe Ala Lys Arg Ile Thr Gly Phe Met Glu Leu Cys
   65                  70                  75                  80
   Gln Asn Asp Gln Ile Leu Leu Leu Lys Ser Gly Cys Leu Glu Val Val
                   85                  90                  95
   Leu Val Arg Met Cys Arg Ala Phe Asn Pro Leu Asn Asn Thr Val Leu
               100                 105                 110
   Phe Glu Gly Lys Tyr Gly Gly Met Gln Met Phe Lys Ala Leu Gly Ser
           115                 120                 125
   Asp Asp Leu Val Asn Glu Ala Phe Asp Phe Ala Lys Asn Leu Cys Ser
       130                 135                 140
   Leu Gln Leu Thr Glu Glu Glu Ile Ala Leu Phe Ser Ser Ala Val Leu
   145                 150                 155                 160
   Ile Ser Pro Asp Arg Ala Trp Leu Leu Glu Pro Arg Lys Val Gln Lys
                   165                 170                 175
   Leu Gln Glu Lys Ile Tyr Phe Ala Leu Gln His Val Ile Gln Lys Asn
               180                 185                 190
   His Leu Asp Asp Glu Thr Leu Ala Lys Leu Ile Ala Lys Ile Pro Thr
           195                 200                 205
   Ile Thr Ala Val Cys Asn Leu His Gly Glu Lys Leu Gln Val Phe Lys
       210                 215                 220
   Gln Ser His Pro Asp Ile Val Asn Thr Leu Phe Pro Pro Leu Tyr Lys
   225                 230                 235                 240
   Glu Leu Phe Asn Pro
<210> 9
   <211> 245
   <212> PRT
   <213> Artificial Sequence
<220>
   <223> Description of Artificial Sequence: fragment of human RORβ
<400> 9
   Thr Met Thr Glu Ile Asp Arg Ile Ala Gln Asn Ile Ile Lys Ser His
    1               5                  10                  15
   Leu Glu Thr Cys Gln Tyr Thr Met Glu Glu Leu His Gln Leu Ala Trp
               20                  25                  30
   Gln Thr His Thr Tyr Glu Glu Ile Lys Ala Tyr Gln Ser Lys Ser Arg
           35                  40                  45
   Glu Ala Leu Trp Gln Gln Cys Ala Ile Gln Ile Thr His Ala Ile Gln
       50                  55                  60
   Tyr Val Val Glu Phe Ala Lys Arg Ile Thr Gly Phe Met Glu Leu Cys
    65                 70                  75                      80
   Gln Asn Asp Gln Ile Leu Leu Leu Lys Ser Gly Cys Leu Glu Val Val
                   85                  90                  95
   Leu Val Arg Met Cys Arg Ala Phe Asn Pro Leu Asn Asn Thr Val Leu
               100                 105                 110
   Phe Glu Gly Lys Tyr Gly Gly Met Gln Met Phe Lys Ala Leu Gly Ser
           115                 120                 125
   Asp Asp Leu Val Asn Glu Ala Phe Asp Phe Ala Lys Asn Leu Cys Ser
       130                 135                 140
   Leu Gln Leu Thr Glu Glu Glu Ile Ala Leu Phe Ser Ser Ala Val Leu
   145                 150                 155                 160
   Ile Ser Pro Asp Arg Ala Trp Leu Ile Glu Pro Arg Lys Val Gln Lys
                   165                 170                 175
   Leu Gln Glu Lys Ile Tyr Phe Ala Leu Gln His Val Ile Gln Lys Asn
               180                 185                 190
   His Leu Asp Asp Glu Thr Leu Ala Lys Leu Ile Ala Lys Ile Pro Thr
           195                 200                 205
   Ile Thr Ala Val Cys Asn Leu His Gly Glu Lys Leu Gln Val Phe Lys
       210                 215                 220
   Gln Ser His Pro Glu Ile Val Asn Thr Leu Phe Pro Pro Leu Tyr Lys
   225                 230                 235                 240
   Glu Leu Phe Asn Pro
<210> 10
   <211> 243
   <212> PRT
   <213> Artificial Sequence
<220>
   <223> Description of Artificial Sequence: fragment of human RORγ
<400> 10
   Ser Leu Thr Glu Ile Glu His Leu Val Gln Ser Val Cys Lys Ser Tyr
    1               5                  10                  15
   Arg Glu Thr Cys Gln Leu Arg Leu Glu Asp Leu Leu Arg Gln Arg Ser
               20                  25                  30
   Asn Ile Phe Ser Arg Glu Glu Val Thr Gly Tyr Gln Arg Lys Ser Met
           35                  40                  45
   Trp Glu Met Trp Glu Arg Cys Ala His His Leu Thr Glu Ala Ile Gln
       50                  55                  60
   Tyr Val Val Glu Phe Ala Lys Arg Leu Ser Gly Phe Met Glu Leu Cys
   65                  70                  75                  80
   Gln Asn Asp Gln Ile Val Leu Leu Lys Ala Gly Ala Met Glu Val Val
                   85                  90                  95
   Leu Val Arg Met Cys Arg Ala Tyr Asn Ala Asp Asn Arg Thr Val Phe
               100                 105                 110
   Phe Glu Gly Lys Tyr Gly Gly Met Glu Leu Phe Arg Ala Leu Gly Cys
           115                 120                 125
   Ser Glu Leu Ile Ser Ser Ile Phe Asp Phe Ser His Ser Leu Ser Ala
       130                 135                 140
   Leu His Phe Ser Glu Asp Glu Ile Ala Leu Tyr Thr Ala Leu Val Leu
   145                 150                 155                 160
   Ile Asn Ala His Arg Pro Gly Leu Gln Glu Lys Arg Lys Val Glu Gln
                   165                 170                 175
   Leu Gln Tyr Asn Leu Glu Leu Ala Phe His His His Leu Cys Lys Thr
               180                 185                 190
   His Arg Gln Ser Ile Leu Ala Lys Leu Pro Pro Lys Gly Lys Leu Arg
           195                 200                 205
   Ser Leu Cys Ser Gln His Val Glu Arg Leu Gln Ile Phe Gln His Leu
       210                 215                 220
   His Pro Ile Val Val Gln Ala Ala Phe Pro Pro Leu Tyr Lys Glu Leu
   225                 230                 235                 240
   Phe Ser Thr
<210> 11
   <211> 243
   <212> PRT
   <213> Artificial Sequence
<220>
   <223> Description of Artificial Sequence: fragment of murine RORγ
<400> 11
   Ser Leu Thr Asp Ile Glu Tyr Leu Val Gln Asn Val Cys Lys Ser Phe
    1               5                  10                  15
   Arg Glu Thr Cys Gln Leu Arg Leu Glu Asp Leu Leu Arg Gln Arg Thr
               20                  25                  30
   Asn Leu Phe Ser Arg Glu Glu Val Thr Ser Tyr Gln Arg Lys Ser Met
           35                  40                  45
   Trp Glu Met Trp Glu Arg Cys Ala His His Leu Thr Glu Ala Ile Gln
       50                  55                  60
   Tyr Val Val Glu Phe Ala Lys Arg Leu Ser Gly Phe Met Glu Leu Cys
   65                  70                  75                  80
   Gln Asn Asp Gln Ile Ile Leu Leu Thr Ala Gly Ala Met Glu Val Val
                   85                  90                  95
   Leu Val Arg Met Cys Arg Ala Tyr Asn Ala Asn Asn His Thr Val Phe
               100                 105                 110
   Phe Glu Gly Lys Tyr Gly Gly Val Glu Leu Phe Arg Ala Leu Gly Cys
           115                 120                 125
   Ser Glu Leu Ile Ser Ser Ile Phe Asp Phe Ser His Phe Leu Ser Ala
       130                 135                 140
   Leu Cys Phe Ser Glu Asp Glu Ile Ala Leu Tyr Thr Ala Leu Val Leu
   145                 150                 155                 160
   Ile Asn Ala Asn Arg Pro Gly Leu Gln Glu Lys Arg Arg Val Glu His
                   165                 170                 175
   Leu Gln Tyr Asn Leu Glu Leu Ala Phe His His His Leu Cys Lys Thr
               180                 185                 190
   His Arg Gln Gly Leu Leu Ala Lys Leu Pro Pro Lys Gly Lys Leu Arg
           195                 200                 205
   Ser Leu Cys Ser Gln His Val Glu Lys Leu Gln Ile Phe Gln His Leu
       210                 215                 220
   His Pro Ile Val Val Gln Ala Ala Phe Pro Pro Leu Tyr Lys Glu Leu
   225                 230                 235                 240
   Phe Ser Thr
<210> 12
   <211> 245
   <212> PRT
   <213> Artificial Sequence
<220>
   <223> Description of Artificial Sequence: fragment of human RORα
<400> 12
   Ser Met Ala Glu Leu Glu His Leu Ala Gln Asn Ile Ser Lys Ser His
   1               5                   10                  15
   Leu Glu Thr Cys Gln Tyr Leu Arg Glu Glu Leu Gln Gln Ile Thr Trp
               20                  25                  30
   Gln Thr Phe Leu Gln Glu Glu Ile Glu Asn Tyr Gln Asn Lys Gln Arg
           35                  40                  45
   Glu Val Met Trp Gln Leu Cys Ala Ile Lys Ile Thr Glu Ala Ile Gln
       50                  55                  60
   Tyr Val Val Glu Phe Ala Lys Arg Ile Asp Gly Phe Met Glu Leu Cys
   65                  70                  75                  80
   Gln Asn Asp Gln Ile Val Leu Leu Lys Ala Gly Ser Leu Glu Val Val
                   85                  90                  95
   Phe Ile Arg Met Cys Arg Ala Phe Asp Ser Gln Asn Asn Thr Val Tyr
               100                 105                 110
   Phe Asp Gly Lys Tyr Ala Ser Pro Asp Val Phe Lys Ser Leu Gly Cys
           115                 120                 125
   Glu Asp Phe Ile Ser Phe Val Phe Glu Phe Gly Lys Ser Leu Cys Ser
       130                 135                 140
   Met His Leu Thr Glu Asp Glu Ile Ala Leu Phe Ser Ala Phe Val Leu
   145                 150                 155                 160
   Met Ser Ala Asp Arg Ser Trp Leu Gln Glu Lys Val Lys Ile Glu Lys
                   165                 170                 175
   Leu Gln Gln Lys Ile Gln Leu Ala Leu Gln His Val Leu Gln Lys Asn
               180                 185                 190
   His Arg Glu Asp Gly Ile Leu Thr Lys Leu Ile Cys Lys Val Ser Thr
           195                 200                 205
   Leu Arg Ala Leu Cys Gly Arg His Thr Glu Lys Leu Met Ala Phe Lys
       210                 215                 220
   Ala Ile Tyr Pro Asp Ile Val Arg Leu His Phe Pro Pro Leu Tyr Lys
   225                 230                 235                 240
   Glu Leu Phe Thr Ser
<210> 13
   <211> 245
   <212> PRT
   <213> Artificial Sequence
<220>
   <223> Description of Artificial Sequence: fragment of murine RORα
<400> 13
   Ser Met Ala Glu Leu Glu His Leu Ala Gln Asn Ile Ser Lys Ser His
   1               5                   10                  15
   Leu Glu Thr Cys Gln Tyr Leu Arg Glu Glu Leu Gln Gln Ile Thr Trp
               20                  25                  30
   Gln Thr Phe Leu Gln Glu Glu Ile Glu Asn Tyr Gln Asn Lys Gln Arg
           35                  40                  45
   Glu Val Met Trp Gln Leu Cys Ala Ile Lys Ile Thr Glu Ala Ile Gln
       50                  55                  60
   Tyr Val Val Glu Phe Ala Lys Arg Ile Asp Gly Phe Met Glu Leu Cys
   65                  70                  75                  80
   Gln Asn Asp Gln Ile Val Leu Leu Lys Ala Gly Ser Leu Glu Val Val
                   85                  90                  95
   Phe Ile Arg Met Cys Arg Ala Phe Asp Ser Gln Asn Asn Thr Val Tyr
               100                 105                 110
   Phe Asp Gly Lys Tyr Ala Ser Pro Asp Val Phe Lys Ser Leu Gly Cys
           115                 120                 125
   Glu Asp Phe Ile Ser Phe Val Phe Glu Phe Gly Lys Ser Leu Cys Ser
       130                 135                 140
   Met His Leu Thr Glu Asp Glu Ile Ala Leu Phe Ser Ala Phe Val Leu
   145                 150                 155                 160
   Met Ser Ala Asp Arg Ser Trp Leu Gln Glu Lys Val Lys Ile Glu Lys
                   165                 170                 175
   Leu Gln Gln Lys Ile Gln Leu Ala Leu Gln His Val Leu Gln Lys Asn
               180                 185                 190
   His Arg Glu Asp Gly Ile Leu Thr Lys Leu Ile Cys Lys Val Ser Thr
           195                 200                 205
   Leu Arg Ala Leu Cys Gly Arg His Thr Glu Lys Leu Met Ala Phe Lys
       210                 215                 220
   Ala Ile Tyr Pro Asp Ile Val Arg Leu His Phe Pro Pro Leu Tyr Lys
   225                 230                 235                 240
   Glu Leu Phe Thr Ser
                   245
<210> 14
   <211> 244
   <212> PRT
   <213> Artificial Sequence
   <223> Description of Artificial Sequence: nucleotide sequence coding for a fragment of rat RORβ
<400> 14
   Thr Met Ser Glu Ile Asp Arg Ile Ala Gln Asn Ile Ile Lys Ser His
   1               5                   10                  15
   Leu Glu Thr Cys Gln Tyr Thr Met Glu Glu Leu His Gln Leu Ala Trp
               20                  25                  30
   Gln Thr His Thr Tyr Glu Glu Ile Lys Ala Tyr Gln Ser Lys Ser Arg
           35                  40                  45
   Glu Ala Leu Trp Gln Gln Cys Ala Ile Gln Ile Thr His Ala Ile Gln
       50                  55                  60
   Tyr Val Val Glu Phe Ala Lys Arg Ile Thr Gly Phe Met Glu Leu Cys
   65                  70                  75                  80
   Gln Asn Asp Gln Ile Leu Leu Leu Lys Ser Gly Cys Leu Glu Val Val
                   85                  90                  95
   Leu Val Arg Met Cys Arg Ala Phe Asn Pro Leu Asn Asn Thr Val Leu
               100                 105                 110
   Phe Glu Gly Lys Tyr Gly Gly Met Gln Met Phe Lys Ala Leu Gly Ser
           115                 120                 125
   Asp Asp Leu Val Asn Glu Ala Phe Asp Phe Ala Lys Asn Leu Cys Ser
       130                 135                 140
   Leu Gln Leu Thr Glu Glu Glu Ile Ala Leu Phe Ser Ser Ala Val Leu
   145                 150                 155                 160
   Ile Ser Pro Asp Arg Ala Trp Leu Leu Glu Pro Arg Lys Val Gln Lys
                   165                 170                 175
   Leu Gln Glu Lys Ile Tyr Phe Ala Leu Gln His Val Ile Gln Lys Asn
               180                 185                 190
   His Leu Asp Asp Glu Thr Leu Ala Lys Leu Ile Ala Lys Ile Pro Thr
           195                 200                 205
   Ile Thr Ala Val Cys Asn Leu His Gly Glu Lys Leu Gln Val Phe Lys
       210                 215                 220
   Gln Ser His Pro Asp Ile Val Asn Thr Leu Phe Pro Pro Leu Tyr Lys
   225                 230                 235                 240
   Glu Leu Phe Asn
<210> 15
   <211> 244
   <212> PRT
   <213> Artificial Sequence
<220>
   <223> Description of Artificial Sequence: fragment of human RORβ
<400> 15
   Thr Met Thr Glu Ile Asp Arg Ile Ala Gln Asn Ile Ile Lys Ser His
   1               5                   10                  15
   Leu Glu Thr Cys Gln Tyr Thr Met Glu Glu Leu His Gln Leu Ala Trp
               20                  25                  30
   Gln Thr His Thr Tyr Glu Glu Ile Lys Ala Tyr Gln Ser Lys Ser Arg
           35                  40                  45
   Glu Ala Leu Trp Gln Gln Cys Ala Ile Gln Ile Thr His Ala Ile Gln
       50                  55                  60
   Tyr Val Val Glu Phe Ala Lys Arg Ile Thr Gly Phe Met Glu Leu Cys
   65                  70                  75                  80
   Gln Asn Asp Gln Ile Leu Leu Leu Lys Ser Gly Cys Leu Glu Val Val
                   85                  90                  95
   Leu Val Arg Met Cys Arg Ala Phe Asn Pro Leu Asn Asn Thr Val Leu
               100                 105                 110
   Phe Glu Gly Lys Tyr Gly Gly Met Gln Met Phe Lys Ala Leu Gly Ser
           115                 120                 125
   Asp Asp Leu Val Asn Glu Ala Phe Asp Phe Ala Lys Asn Leu Cys Ser
       130                 135                 140
   Leu Gln Leu Thr Glu Glu Glu Ile Ala Leu Phe Ser Ser Ala Val Leu
   145                 150                 155                 160
   Ile Ser Pro Asp Arg Ala Trp Leu Ile Glu Pro Arg Lys Val Gln Lys
                   165                 170                 175
   Leu Gln Glu Lys Ile Tyr Phe Ala Leu Gln His Val Ile Gln Lys Asn
               180                 185                 190
   His Leu Asp Asp Glu Thr Leu Ala Lys Leu Ile Ala Lys Ile Pro Thr
           195                 200                 205
   Ile Thr Ala Val Cys Asn Leu His Gly Glu Lys Leu Gln Val Phe Lys
       210                 215                 220
   Gln Ser His Pro Glu Ile Val Asn Thr Leu Phe Pro Pro Leu Tyr Lys
   225                 230                 235                 240
   Glu Leu Phe Asn
<210> 16
   <211> 242
   <212> PRT
   <213> Artificial Sequence
<220>
   <223> Description of Artificial Sequence: fragment of human RORγ
<400> 16
   Ser Leu Thr Glu Ile Glu His Leu Val Gln Ser Val Cys Lys Ser Tyr
    1               5                  10                  15
   Arg Glu Thr Cys Gln Leu Arg Leu Glu Asp Leu Leu Arg Gln Arg Ser
               20                  25                  30
   Asn Ile Phe Ser Arg Glu Glu Val Thr Gly Tyr Gln Arg Lys Ser Met
           35                  40                  45
   Trp Glu Met Trp Glu Arg Cys Ala His His Leu Thr Glu Ala Ile Gln
       50                  55                  60
   Tyr Val Val Glu Phe Ala Lys Arg Leu Ser Gly Phe Met Glu Leu Cys
   65                  70                  75                  80
   Gln Asn Asp Gln Ile Val Leu Leu Lys Ala Gly Ala Met Glu Val Val
                   85                  90                  95
   Leu Val Arg Met Cys Arg Ala Tyr Asn Ala Asp Asn Arg Thr Val Phe
               100                 105                 110
   Phe Glu Gly Lys Tyr Gly Gly Met Glu Leu Phe Arg Ala Leu Gly Cys
           115                 120                 125
   Ser Glu Leu Ile Ser Ser Ile Phe Asp Phe Ser His Ser Leu Ser Ala
       130                 135                 140
   Leu His Phe Ser Glu Asp Glu Ile Ala Leu Tyr Thr Ala Leu Val Leu
   145                 150                 155                 160
   Ile Asn Ala His Arg Pro Gly Leu Gln Glu Lys Arg Lys Val Glu Gln
                   165                 170                 175
   Leu Gln Tyr Asn Leu Glu Leu Ala Phe His His His Leu Cys Lys Thr
               180                 185                 190
   His Arg Gln Ser Ile Leu Ala Lys Leu Pro Pro Lys Gly Lys Leu Arg
           195                 200                 205
   Ser Leu Cys Ser Gln His Val Glu Arg Leu Gln Ile Phe Gln His Leu
       210                 215                 220
   His Pro Ile Val Val Gln Ala Ala Phe Pro Pro Leu Tyr Lys Glu Leu
   225                 230                 235                 240
   Phe Ser
<210> 17
   <211> 242
   <212> PRT
   <213> Artificial Sequence
<220>
   <223> Description of Artificial Sequence: fragment of murine RORγ
<400> 17
   Ser Leu Thr Asp Ile Glu Tyr Leu Val Gln Asn Val Cys Lys Ser Phe
    1               5                  10                  15
   Arg Glu Thr Cys Gln Leu Arg Leu Glu Asp Leu Leu Arg Gln Arg Thr
               20                  25                  30
   Asn Leu Phe Ser Arg Glu Glu Val Thr Ser Tyr Gln Arg Lys Ser Met
           35                  40                  45
   Trp Glu Met Trp Glu Arg Cys Ala His His Leu Thr Glu Ala Ile Gln
       50                  55                  60
   Tyr Val Val Glu Phe Ala Lys Arg Leu Ser Gly Phe Met Glu Leu Cys
    65                 70                  75                  80
   Gln Asn Asp Gln Ile Ile Leu Leu Thr Ala Gly Ala Met Glu Val Val
                   85                  90                  95
   Leu Val Arg Met Cys Arg Ala Tyr Asn Ala Asn Asn His Thr Val Phe
               100                 105                 110
   Phe Glu Gly Lys Tyr Gly Gly Val Glu Leu Phe Arg Ala Leu Gly Cys
           115                 120                 125
   Ser Glu Leu Ile Ser Ser Ile Phe Asp Phe Ser His Phe Leu Ser Ala
       130                 135                 140
   Leu Cys Phe Ser Glu Asp Glu Ile Ala Leu Tyr Thr Ala Leu Val Leu
   145                 150                 155                 160
   Ile Asn Ala Asn Arg Pro Gly Leu Gln Glu Lys Arg Arg Val Glu His
                   165                 170                 175
   Leu Gln Tyr Asn Leu Glu Leu Ala Phe His His His Leu Cys Lys Thr
               180                 185                 190
   His Arg Gln Gly Leu Leu Ala Lys Leu Pro Pro Lys Gly Lys Leu Arg
           195                 200                 205
   Ser Leu Cys Ser Gln His Val Glu Lys Leu Gln Ile Phe Gln His Leu
       210                 215                 220
   His Pro Ile Val Val Gln Ala Ala Phe Pro Pro Leu Tyr Lys Glu Leu
   225                 230                 235                 240
   Phe Ser
<210> 18
   <211> 244
   <212> PRT
   <213> Artificial Sequence
<220>
   <223> Description of Artificial Sequence: fragment of human RORα
<400> 18
   Ser Met Ala Glu Leu Glu His Leu Ala Gln Asn Ile Ser Lys Ser His
    1               5                  10                  15
   Leu Glu Thr Cys Gln Tyr Leu Arg Glu Glu Leu Gln Gln Ile Thr Trp
               20                  25                  30
   Gln Thr Phe Leu Gln Glu Glu Ile Glu Asn Tyr Gln Asn Lys Gln Arg
           35                  40                  45
   Glu Val Met Trp Gln Leu Cys Ala Ile Lys Ile Thr Glu Ala Ile Gln
       50                  55                  60
   Tyr Val Val Glu Phe Ala Lys Arg Ile Asp Gly Phe Met Glu Leu Cys
   65                  70                  75                  80
   Gln Asn Asp Gln Ile Val Leu Leu Lys Ala Gly Ser Leu Glu Val Val
                   85                  90                  95
   Phe Ile Arg Met Cys Arg Ala Phe Asp Ser Gln Asn Asn Thr Val Tyr
               100                 105                 110
   Phe Asp Gly Lys Tyr Ala Ser Pro Asp Val Phe Lys Ser Leu Gly Cys
           115                 120                 125
   Glu Asp Phe Ile Ser Phe Val Phe Glu Phe Gly Lys Ser Leu Cys Ser
       130                 135                 140
   Met His Leu Thr Glu Asp Glu Ile Ala Leu Phe Ser Ala Phe Val Leu
   145                 150                 155                 160
   Met Ser Ala Asp Arg Ser Trp Leu Gln Glu Lys Val Lys Ile Glu Lys
                   165                 170                 175
   Leu Gln Gln Lys Ile Gln Leu Ala Leu Gln His Val Leu Gln Lys Asn
               180                 185                 190
   His Arg Glu Asp Gly Ile Leu Thr Lys Leu Ile Cys Lys Val Ser Thr
           195                 200                 205
   Leu Arg Ala Leu Cys Gly Arg His Thr Glu Lys Leu Met Ala Phe Lys
       210                 215                 220
   Ala Ile Tyr Pro Asp Ile Val Arg Leu His Phe Pro Pro Leu Tyr Lys
   225                 230                 235                 240
   Glu Leu Phe Thr
<210> 19
   <211> 244
   <212> PRT
   <213> Artificial Sequence
<220>
   <223> Description of Artificial Sequence: fragment of murine RORα
<400> 19
   Ser Met Ala Glu Leu Glu His Leu Ala Gln Asn Ile Ser Lys Ser His
    1               5                  10                  15
   Leu Glu Thr Cys Gln Tyr Leu Arg Glu Glu Leu Gln Gln Ile Thr Trp
               20                  25                  30
   Gln Thr Phe Leu Gln Glu Glu Ile Glu Asn Tyr Gln Asn Lys Gln Arg
           35                  40                  45
   Glu Val Met Trp Gln Leu Cys Ala Ile Lys Ile Thr Glu Ala Ile Gln
       50                  55                  60
   Tyr Val Val Glu Phe Ala Lys Arg Ile Asp Gly Phe Met Glu Leu Cys
   65                  70                  75                  80
   Gln Asn Asp Gln Ile Val Leu Leu Lys Ala Gly Ser Leu Glu Val Val
                   85                  90                  95
   Phe Ile Arg Met Cys Arg Ala Phe Asp Ser Gln Asn Asn Thr Val Tyr
               100                 105                 110
   Phe Asp Gly Lys Tyr Ala Ser Pro Asp Val Phe Lys Ser Leu Gly Cys
           115                 120                 125
   Glu Asp Phe Ile Ser Phe Val Phe Glu Phe Gly Lys Ser Leu Cys Ser
       130                 135                 140
   Met His Leu Thr Glu Asp Glu Ile Ala Leu Phe Ser Ala Phe Val Leu
   145                 150                 155                 160
   Met Ser Ala Asp Arg Ser Trp Leu Gln Glu Lys Val Lys Ile Glu Lys
                   165                 170                 175
   Leu Gln Gln Lys Ile Gln Leu Ala Leu Gln His Val Leu Gln Lys Asn
               180                 185                 190
   His Arg Glu Asp Gly Ile Leu Thr Lys Leu Ile Cys Lys Val Ser Thr
           195                 200                 205
   Leu Arg Ala Leu Cys Gly Arg His Thr Glu Lys Leu Met Ala Phe Lys
       210                 215                 220
   Ala Ile Tyr Pro Asp Ile Val Arg Leu His Phe Pro Pro Leu Tyr Lys
   225                 230                 235                 240
   Glu Leu Phe Thr
<210> 20
   <211> 243
   <212> PRT
   <213> Artificial Sequence
   <223> Description of Artificial Sequence: nucleotide sequence coding for a fragment of rat RORβ
<400> 20
   Met Ser Glu Ile Asp Arg Ile Ala Gln Asn Ile Ile Lys Ser His Leu
    1               5                  10                  15
   Glu Thr Cys Gln Tyr Thr Met Glu Glu Leu His Gln Leu Ala Trp Gln
               20                  25                  30
   Thr His Thr Tyr Glu Glu Ile Lys Ala Tyr Gln Ser Lys Ser Arg Glu
           35                  40                  45
   Ala Leu Trp Gln Gln Cys Ala Ile Gln Ile Thr His Ala Ile Gln Tyr
       50                  55                  60
   Val Val Glu Phe Ala Lys Arg Ile Thr Gly Phe Met Glu Leu Cys Gln
   65                  70                  75                  80
   Asn Asp Gln Ile Leu Leu Leu Lys Ser Gly Cys Leu Glu Val Val Leu
                   85                  90                  95
   Val Arg Met Cys Arg Ala Phe Asn Pro Leu Asn Asn Thr Val Leu Phe
               100                 105                 110
   Glu Gly Lys Tyr Gly Gly Met Gln Met Phe Lys Ala Leu Gly Ser Asp
           115                 120                 125
   Asp Leu Val Asn Glu Ala Phe Asp Phe Ala Lys Asn Leu Cys Ser Leu
       130                 135                 140
   Gln Leu Thr Glu Glu Glu Ile Ala Leu Phe Ser Ser Ala Val Leu Ile
   145                 150                 155                 160
   Ser Pro Asp Arg Ala Trp Leu Leu Glu Pro Arg Lys Val Gln Lys Leu
                   165                 170                 175
   Gln Glu Lys Ile Tyr Phe Ala Leu Gln His Val Ile Gln Lys Asn His
               180                 185                 190
   Leu Asp Asp Glu Thr Leu Ala Lys Leu Ile Ala Lys Ile Pro Thr Ile
           195                 200                 205
   Thr Ala Val Cys Asn Leu His Gly Glu Lys Leu Gln Val Phe Lys Gln
       210                 215                 220
   Ser His Pro Asp Ile Val Asn Thr Leu Phe Pro Pro Leu Tyr Lys Glu
   225                 230                 235                 240
   Leu Phe Asn
<210> 21
   <211> 243
   <212> PRT
   <213> Artificial Sequence
<220>
   <223> Description of Artificial Sequence: fragment of human RORβ
<400> 21
   Met Thr Glu Ile Asp Arg Ile Ala Gln Asn Ile Ile Lys Ser His Leu
   1               5                   10                  15
   Glu Thr Cys Gln Tyr Thr Met Glu Glu Leu His Gln Leu Ala Trp Gln
               20                  25                  30
   Thr His Thr Tyr Glu Glu Ile Lys Ala Tyr Gln Ser Lys Ser Arg Glu
           35                  40                  45
   Ala Leu Trp Gln Gln Cys Ala Ile Gln Ile Thr His Ala Ile Gln Tyr
       50                  55                  60
   Val Val Glu Phe Ala Lys Arg Ile Thr Gly Phe Met Glu Leu Cys Gln
   65                  70                  75                  80
   Asn Asp Gln Ile Leu Leu Leu Lys Ser Gly Cys Leu Glu Val Val Leu
                   85                  90                  95
   Val Arg Met Cys Arg Ala Phe Asn Pro Leu Asn Asn Thr Val Leu Phe
               100                 105                 110
   Glu Gly Lys Tyr Gly Gly Met Gln Met Phe Lys Ala Leu Gly Ser Asp
           115                 120                 125
   Asp Leu Val Asn Glu Ala Phe Asp Phe Ala Lys Asn Leu Cys Ser Leu
       130                 135                 140
   Gln Leu Thr Glu Glu Glu Ile Ala Leu Phe Ser Ser Ala Val Leu Ile
   145                 150                 155                 160
   Ser Pro Asp Arg Ala Trp Leu Ile Glu Pro Arg Lys Val Gln Lys Leu
                   165                 170                 175
   Gln Glu Lys Ile Tyr Phe Ala Leu Gln His Val Ile Gln Lys Asn His
               180                 185                 190
   Leu Asp Asp Glu Thr Leu Ala Lys Leu Ile Ala Lys Ile Pro Thr Ile
           195                 200                 205
   Thr Ala Val Cys Asn Leu His Gly Glu Lys Leu Gln Val Phe Lys Gln
       210                 215                 220
   Ser His Pro Glu Ile Val Asn Thr Leu Phe Pro Pro Leu Tyr Lys Glu
   225                 230                 235                 240
   Leu Phe Asn
<210> 22
   <211> 241
   <212> PRT
   <213> Artificial Sequence
<220>
   <223> Description of Artificial Sequence: fragment of human RORγ
<400> 22
   Leu Thr Glu Ile Glu His Leu Val Gln Ser Val Cys Lys Ser Tyr Arg
   1               5                   10                  15
   Glu Thr Cys Gln Leu Arg Leu Glu Asp Leu Leu Arg Gln Arg Ser Asn
               20                  25                  30
   Ile Phe Ser Arg Glu Glu Val Thr Gly Tyr Gln Arg Lys Ser Met Trp
           35                  40                  45
   Glu Met Trp Glu Arg Cys Ala His His Leu Thr Glu Ala Ile Gln Tyr
       50                  55                  60
   Val Val Glu Phe Ala Lys Arg Leu Ser Gly Phe Met Glu Leu Cys Gln
   65                  70                  75                  80
   Asn Asp Gln Ile Val Leu Leu Lys Ala Gly Ala Met Glu Val Val Leu
                   85                  90                  95
   Val Arg Met Cys Arg Ala Tyr Asn Ala Asp Asn Arg Thr Val Phe Phe
               100                 105                 110
   Glu Gly Lys Tyr Gly Gly Met Glu Leu Phe Arg Ala Leu Gly Cys Ser
           115                 120                 125
   Glu Leu Ile Ser Ser Ile Phe Asp Phe Ser His Ser Leu Ser Ala Leu
       130                 135                 140
   His Phe Ser Glu Asp Glu Ile Ala Leu Tyr Thr Ala Leu Val Leu Ile
   145                 150                 155                 160
   Asn Ala His Arg Pro Gly Leu Gln Glu Lys Arg Lys Val Glu Gln Leu
                   165                 170                 175
   Gln Tyr Asn Leu Glu Leu Ala Phe His His His Leu Cys Lys Thr His
               180                 185                 190
   Arg Gln Ser Ile Leu Ala Lys Leu Pro Pro Lys Gly Lys Leu Arg Ser
           195                 200                 205
   Leu Cys Ser Gln His Val Glu Arg Leu Gln Ile Phe Gln His Leu His
       210                 215                 220
   Pro Ile Val Val Gln Ala Ala Phe Pro Pro Leu Tyr Lys Glu Leu Phe
   225                 230                 235                 240
   Ser
<210> 23
   <211> 241
   <212> PRT
   <213> Artificial Sequence
<220>
   <223> Description of Artificial Sequence: fragment of murine RORγ
<400> 23
   Leu Thr Asp Ile Glu Tyr Leu Val Gln Asn Val Cys Lys Ser Phe Arg
   1               5                   10                  15
   Glu Thr Cys Gln Leu Arg Leu Glu Asp Leu Leu Arg Gln Arg Thr Asn
               20                  25                  30
   Leu Phe Ser Arg Glu Glu Val Thr Ser Tyr Gln Arg Lys Ser Met Trp
           35                  40                  45
   Glu Met Trp Glu Arg Cys Ala His His Leu Thr Glu Ala Ile Gln Tyr
       50                  55                  60
   Val Val Glu Phe Ala Lys Arg Leu Ser Gly Phe Met Glu Leu Cys Gln
   65                  70                  75                  80
   Asn Asp Gln Ile Ile Leu Leu Thr Ala Gly Ala Met Glu Val Val Leu
                   85                  90                  95
   Val Arg Met Cys Arg Ala Tyr Asn Ala Asn Asn His Thr Val Phe Phe
               100                 105                 110
   Glu Gly Lys Tyr Gly Gly Val Glu Leu Phe Arg Ala Leu Gly Cys Ser
           115                 120                 125
   Glu Leu Ile Ser Ser Ile Phe Asp Phe Ser His Phe Leu Ser Ala Leu
       130                 135                 140
   Cys Phe Ser Glu Asp Glu Ile Ala Leu Tyr Thr Ala Leu Val Leu Ile
   145                 150                 155                 160
   Asn Ala Asn Arg Pro Gly Leu Gln Glu Lys Arg Arg Val Glu His Leu
                   165                 170                 175
   Gln Tyr Asn Leu Glu Leu Ala Phe His His His Leu Cys Lys Thr His
               180                 185                 190
   Arg Gln Gly Leu Leu Ala Lys Leu Pro Pro Lys Gly Lys Leu Arg Ser
           195                 200                 205
   Leu Cys Ser Gln His Val Glu Lys Leu Gln Ile Phe Gln His Leu His
       210                 215                 220
   Pro Ile Val Val Gln Ala Ala Phe Pro Pro Leu Tyr Lys Glu Leu Phe
   225                 230                 235                 240
   Ser
<210> 24
   <211> 243
   <212> PRT
   <213> Artificial Sequence
<220>
   <223> Description of Artificial Sequence: fragment of human RORα
<400> 24
   Met Ala Glu Leu Glu His Leu Ala Gln Asn Ile Ser Lys Ser His Leu
   1               5                   10                  15
   Glu Thr Cys Gln Tyr Leu Arg Glu Glu Leu Gln Gln Ile Thr Trp Gln
               20                  25                  30
   Thr Phe Leu Gln Glu Glu Ile Glu Asn Tyr Gln Asn Lys Gln Arg Glu
           35                  40                  45
   Val Met Trp Gln Leu Cys Ala Ile Lys Ile Thr Glu Ala Ile Gln Tyr
       50                  55                  60
   Val Val Glu Phe Ala Lys Arg Ile Asp Gly Phe Met Glu Leu Cys Gln
   65                  70                  75                  80
   Asn Asp Gln Ile Val Leu Leu Lys Ala Gly Ser Leu Glu Val Val Phe
                   85                  90                  95
   Ile Arg Met Cys Arg Ala Phe Asp Ser Gln Asn Asn Thr Val Tyr Phe
               100                 105                 110
   Asp Gly Lys Tyr Ala Ser Pro Asp Val Phe Lys Ser Leu Gly Cys Glu
           115                 120                 125
   Asp Phe Ile Ser Phe Val Phe Glu Phe Gly Lys Ser Leu Cys Ser Met
       130                 135                 140
   His Leu Thr Glu Asp Glu Ile Ala Leu Phe Ser Ala Phe Val Leu Met
   145                 150                 155                 160
   Ser Ala Asp Arg Ser Trp Leu Gln Glu Lys Val Lys Ile Glu Lys Leu
                   165                 170                 175
   Gln Gln Lys Ile Gln Leu Ala Leu Gln His Val Leu Gln Lys Asn His
               180                 185                 190
   Arg Glu Asp Gly Ile Leu Thr Lys Leu Ile Cys Lys Val Ser Thr Leu
           195                 200                 205
   Arg Ala Leu Cys Gly Arg His Thr Glu Lys Leu Met Ala Phe Lys Ala
       210                 215                 220
   Ile Tyr Pro Asp Ile Val Arg Leu His Phe Pro Pro Leu Tyr Lys Glu
   225                 230                 235                 240
   Leu Phe Thr
<210> 25
   <211> 243
   <212> PRT
   <213> Artificial Sequence
<220>
   <223> Description of Artificial Sequence: fragment of murine RORα
<400> 25
   Met Ala Glu Leu Glu His Leu Ala Gln Asn Ile Ser Lys Ser His Leu
    1               5                  10                  15
   Glu Thr Cys Gln Tyr Leu Arg Glu Glu Leu Gln Gln Ile Thr Trp Gln
               20                  25                  30
   Thr Phe Leu Gln Glu Glu Ile Glu Asn Tyr Gln Asn Lys Gln Arg Glu
           35                  40                  45
   Val Met Trp Gln Leu Cys Ala Ile Lys Ile Thr Glu Ala Ile Gln Tyr
       50                  55                  60
   Val Val Glu Phe Ala Lys Arg Ile Asp Gly Phe Met Glu Leu Cys Gln
   65                  70                  75                  80
   Asn Asp Gln Ile Val Leu Leu Lys Ala Gly Ser Leu Glu Val Val Phe
                   85                  90                  95
   Ile Arg Met Cys Arg Ala Phe Asp Ser Gln Asn Asn Thr Val Tyr Phe
               100                 105                 110
   Asp Gly Lys Tyr Ala Ser Pro Asp Val Phe Lys Ser Leu Gly Cys Glu
           115                 120                 125
   Asp Phe Ile Ser Phe Val Phe Glu Phe Gly Lys Ser Leu Cys Ser Met
       130                 135                 140
   His Leu Thr Glu Asp Glu Ile Ala Leu Phe Ser Ala Phe Val Leu Met
   145                 150                 155                 160
   Ser Ala Asp Arg Ser Trp Leu Gln Glu Lys Val Lys Ile Glu Lys Leu
                   165                 170                 175
   Gln Gln Lys Ile Gln Leu Ala Leu Gln His Val Leu Gln Lys Asn His
               180                 185                 190
   Arg Glu Asp Gly Ile Leu Thr Lys Leu Ile Cys Lys Val Ser Thr Leu
           195                 200                 205
   Arg Ala Leu Cys Gly Arg His Thr Glu Lys Leu Met Ala Phe Lys Ala
       210                 215                 220
   Ile Tyr Pro Asp Ile Val Arg Leu His Phe Pro Pro Leu Tyr Lys Glu
   225                 230                 235                 240
   Leu Phe Thr
<210> 26
   <211> 251
   <212> PRT
   <213> Artificial Sequence
   <223> Description of Artificial Sequence: nucleotide sequence coding for a fragment of rat RORβ
<400> 26
   Gly Gln Leu Ala Pro Gly Ile Thr Met Ser Glu Ile Asp Arg Ile Ala
   1 5 10 15
   Gln Asn Ile Ile Lys Ser His Leu Glu Thr Cys Gln Tyr Thr Met Glu
   20 25 30
   Glu Leu His Gln Leu Ala Trp Gln Thr His Thr Tyr Glu Glu Ile Lys
   35 40 45
   Ala Tyr Gln Ser Lys Ser Arg Glu Ala Leu Trp Gln Gln Cys Ala Ile
   50 55 60
   Gln Ile Thr His Ala Ile Gln Tyr Val Val Glu Phe Ala Lys Arg Ile
   65 70 75 80
   Thr Gly Phe Met Glu Leu Cys Gln Asn Asp Gln Ile Leu Leu Leu Lys
   85 90 95
   Ser Gly Cys Leu Glu Val Val Leu Val Arg Met Cys Arg Ala Phe Asn
   100 105 110
   Pro Leu Asn Asn Thr Val Leu Phe Glu Gly Lys Tyr Gly Gly Met Gln
   115 120 125
   Met Phe Lys Ala Leu Gly Ser Asp Asp Leu Val Asn Glu Ala Phe Asp
   130 135 140
   Phe Ala Lys Asn Leu Cys Ser Leu Gln Leu Thr Glu Glu Glu Ile Ala
   145 150 155 160
   Leu Phe Ser Ser Ala Val Leu Ile Ser Pro Asp Arg Ala Trp Leu Leu
   165 170 175
   Glu Pro Arg Lys Val Gln Lys Leu Gln Glu Lys Ile Tyr Phe Ala Leu
   180 185 190
   Gln His Val Ile Gln Lys Asn His Leu Asp Asp Glu Thr Leu Ala Lys
   195 200 205
   Leu Ile Ala Lys Ile Pro Thr Ile Thr Ala Val Cys Asn Leu His Gly
   210 215 220
   Glu Lys Leu Gln Val Phe Lys Gln Ser His Pro Asp Ile Val Asn Thr
   225 230 235 240
   Leu Phe Pro Pro Leu Tyr Lys Glu Leu Phe Asn
   245 250
<210> 27
   <211> 251
   <212> PRT
   <213> Artificial Sequence
<220>
   <223> Description of Artificial Sequence: fragment of human RORβ
<400> 27
   Gly Gln Leu Ala Pro Gly Ile Thr Met Thr Glu Ile Asp Arg Ile Ala
   1 5 10 15
   Gln Asn Ile Ile Lys Ser His Leu Glu Thr Cys Gln Tyr Thr Met Glu
   20 25 30
   Glu Leu His Gln Leu Ala Trp Gln Thr His Thr Tyr Glu Glu Ile Lys
   35 40 45
   Ala Tyr Gln Ser Lys Ser Arg Glu Ala Leu Trp Gln Gln Cys Ala Ile
   50 55 60
   Gln Ile Thr His Ala Ile Gln Tyr Val Val Glu Phe Ala Lys Arg Ile
   65 70 75 80
   Thr Gly Phe Met Glu Leu Cys Gln Asn Asp Gln Ile Leu Leu Leu Lys
   85 90 95
   Ser Gly Cys Leu Glu Val Val Leu Val Arg Met Cys Arg Ala Phe Asn
   100 105 110
   Pro Leu Asn Asn Thr Val Leu Phe Glu Gly Lys Tyr Gly Gly Met Gln
   115 120 125
   Met Phe Lys Ala Leu Gly Ser Asp Asp Leu Val Asn Glu Ala Phe Asp
   130 135 140
   Phe Ala Lys Asn Leu Cys Ser Leu Gln Leu Thr Glu Glu Glu Ile Ala
   145 150 155 160
   Leu Phe Ser Ser Ala Val Leu Ile Ser Pro Asp Arg Ala Trp Leu Ile
   165 170 175
   Glu Pro Arg Lys Val Gln Lys Leu Gln Glu Lys Ile Tyr Phe Ala Leu
   180 185 190
   Gln His Val Ile Gln Lys Asn His Leu Asp Asp Glu Thr Leu Ala Lys
   195 200 205
   Leu Ile Ala Lys Ile Pro Thr Ile Thr Ala Val Cys Asn Leu His Gly
   210 215 220
   Glu Lys Leu Gln Val Phe Lys Gln Ser His Pro Glu Ile Val Asn Thr
   225 230 235 240
   Leu Phe Pro Pro Leu Tyr Lys Glu Leu Phe Asn
   245 250
<210> 28
   <211> 249
   <212> PRT
   <213> Artificial Sequence
<220>
   <223> Description of Artificial Sequence: fragment of human RORγ
<400> 28
   Thr Pro Glu Ala Pro Tyr Ala Ser Leu Thr Glu Ile Glu His Leu Val
   1               5                   10                  15
   Gln Ser Val Cys Lys Ser Tyr Arg Glu Thr Cys Gln Leu Arg Leu Glu
               20                  25                  30
   Asp Leu Leu Arg Gln Arg Ser Asn Ile Phe Ser Arg Glu Glu Val Thr
           35                  40                  45
   Gly Tyr Gln Arg Lys Ser Met Trp Glu Met Trp Glu Arg Cys Ala His
       50                  55                  60
   His Leu Thr Glu Ala Ile Gln Tyr Val Val Glu Phe Ala Lys Arg Leu
   65                  70                  75                  80
   Ser Gly Phe Met Glu Leu Cys Gln Asn Asp Gln Ile Val Leu Leu Lys
                   85                  90                  95
   Ala Gly Ala Met Glu Val Val Leu Val Arg Met Cys Arg Ala Tyr Asn
               100                 105                 110
   Ala Asp Asn Arg Thr Val Phe Phe Glu Gly Lys Tyr Gly Gly Met Glu
           115                 120                 125
   Leu Phe Arg Ala Leu Gly Cys Ser Glu Leu Ile Ser Ser Ile Phe Asp
       130                 135                 140
   Phe Ser His Ser Leu Ser Ala Leu His Phe Ser Glu Asp Glu Ile Ala
   145                 150                 155                 160
   Leu Tyr Thr Ala Leu Val Leu Ile Asn Ala His Arg Pro Gly Leu Gln
                   165                 170                 175
   Glu Lys Arg Lys Val Glu Gln Leu Gln Tyr Asn Leu Glu Leu Ala Phe
               180                 185                 190
   His His His Leu Cys Lys Thr His Arg Gln Ser Ile Leu Ala Lys Leu
           195                 200                 205
   Pro Pro Lys Gly Lys Leu Arg Ser Leu Cys Ser Gln His Val Glu Arg
       210                 215                 220
   Leu Gln Ile Phe Gln His Leu His Pro Ile Val Val Gln Ala Ala Phe
   225                 230                 235                 240
   Pro Pro Leu Tyr Lys Glu Leu Phe Ser
                   245
<210> 29
   <211> 249
   <212> PRT
   <213> Artificial Sequence
<220>
   <223> Description of Artificial Sequence: fragment of murine RORγ
<400> 29
   Ala Pro Glu Val Pro Tyr Ala Ser Leu Thr Asp Ile Glu Tyr Leu Val
   1               5                   10                  15
   Gln Asn Val Cys Lys Ser Phe Arg Glu Thr Cys Gln Leu Arg Leu Glu
               20                  25                  30
   Asp Leu Leu Arg Gln Arg Thr Asn Leu Phe Ser Arg Glu Glu Val Thr
           35                  40                  45
   Ser Tyr Gln Arg Lys Ser Met Trp Glu Met Trp Glu Arg Cys Ala His
       50                  55                  60
   His Leu Thr Glu Ala Ile Gln Tyr Val Val Glu Phe Ala Lys Arg Leu
   65                  70                  75                  80
   Ser Gly Phe Met Glu Leu Cys Gln Asn Asp Gln Ile Ile Leu Leu Thr
                   85                  90                  95
   Ala Gly Ala Met Glu Val Val Leu Val Arg Met Cys Arg Ala Tyr Asn
               100                 105                 110
   Ala Asn Asn His Thr Val Phe Phe Glu Gly Lys Tyr Gly Gly Val Glu
           115                 120                 125
   Leu Phe Arg Ala Leu Gly Cys Ser Glu Leu Ile Ser Ser Ile Phe Asp
       130                 135                 140
   Phe Ser His Phe Leu Ser Ala Leu Cys Phe Ser Glu Asp Glu Ile Ala
   145                 150                 155                 160
   Leu Tyr Thr Ala Leu Val Leu Ile Asn Ala Asn Arg Pro Gly Leu Gln
                   165                 170                 175
   Glu Lys Arg Arg Val Glu His Leu Gln Tyr Asn Leu Glu Leu Ala Phe
               180                 185                 190
   His His His Leu Cys Lys Thr His Arg Gln Gly Leu Leu Ala Lys Leu
           195                 200                 205
   Pro Pro Lys Gly Lys Leu Arg Ser Leu Cys Ser Gln His Val Glu Lys
       210                 215                 220
   Leu Gln Ile Phe Gln His Leu His Pro Ile Val Val Gln Ala Ala Phe
   225                 230                 235                 240
   Pro Pro Leu Tyr Lys Glu Leu Phe Ser
                   245
<210> 30
   <211> 251
   <212> PRT
   <213> Artificial Sequence
<220>
   <223> Description of Artificial Sequence: fragment of human RORα
<400> 30
   Gly Glu Thr Ser Pro Thr Val Ser Met Ala Glu Leu Glu His Leu Ala
   1               5                   10                  15
   Gln Asn Ile Ser Lys Ser His Leu Glu Thr Cys Gln Tyr Leu Arg Glu
               20                  25                  30
   Glu Leu Gln Gln Ile Thr Trp Gln Thr Phe Leu Gln Glu Glu Ile Glu
           35                  40                  45
   Asn Tyr Gln Asn Lys Gln Arg Glu Val Met Trp Gln Leu Cys Ala Ile
       50                  55                  60
   Lys Ile Thr Glu Ala Ile Gln Tyr Val Val Glu Phe Ala Lys Arg Ile
   65                  70                  75                  80
   Asp Gly Phe Met Glu Leu Cys Gln Asn Asp Gln Ile Val Leu Leu Lys
                   85                  90                  95
   Ala Gly Ser Leu Glu Val Val Phe Ile Arg Met Cys Arg Ala Phe Asp
               100                 105                 110
   Ser Gln Asn Asn Thr Val Tyr Phe Asp Gly Lys Tyr Ala Ser Pro Asp
           115                 120                 125
   Val Phe Lys Ser Leu Gly Cys Glu Asp Phe Ile Ser Phe Val Phe Glu
       130                 135                 140
   Phe Gly Lys Ser Leu Cys Ser Met His Leu Thr Glu Asp Glu Ile Ala
   145                 150                 155                 160
   Leu Phe Ser Ala Phe Val Leu Met Ser Ala Asp Arg Ser Trp Leu Gln
                   165                 170                 175
   Glu Lys Val Lys Ile Glu Lys Leu Gln Gln Lys Ile Gln Leu Ala Leu
               180                 185                 190
   Gln His Val Leu Gln Lys Asn His Arg Glu Asp Gly Ile Leu Thr Lys
           195                 200                 205
   Leu Ile Cys Lys Val Ser Thr Leu Arg Ala Leu Cys Gly Arg His Thr
       210                 215                 220
   Glu Lys Leu Met Ala Phe Lys Ala Ile Tyr Pro Asp Ile Val Arg Leu
   225                 230                 235                 240
   His Phe Pro Pro Leu Tyr Lys Glu Leu Phe Thr
                   245                 250
<210> 31
   <211> 251
   <212> PRT
   <213> Artificial Sequence
<220>
   <223> Description of Artificial Sequence: fragment of murine RORα
<400> 31
   Gly Glu Thr Ser Pro Thr Val Ser Met Ala Glu Leu Glu His Leu Ala
    1               5                  10                  15
   Gln Asn Ile Ser Lys Ser His Leu Glu Thr Cys Gln Tyr Leu Arg Glu
               20                  25                  30
   Glu Leu Gln Gln Ile Thr Trp Gln Thr Phe Leu Gln Glu Glu Ile Glu
           35                  40                  45
   Asn Tyr Gln Asn Lys Gln Arg Glu Val Met Trp Gln Leu Cys Ala Ile
       50                  55                  60
   Lys Ile Thr Glu Ala Ile Gln Tyr Val Val Glu Phe Ala Lys Arg Ile
   65                  70                  75                  80
   Asp Gly Phe Met Glu Leu Cys Gln Asn Asp Gln Ile Val Leu Leu Lys
                   85                  90                  95
   Ala Gly Ser Leu Glu Val Val Phe Ile Arg Met Cys Arg Ala Phe Asp
               100                 105                 110
   Ser Gln Asn Asn Thr Val Tyr Phe Asp Gly Lys Tyr Ala Ser Pro Asp
           115                 120                 125
   Val Phe Lys Ser Leu Gly Cys Glu Asp Phe Ile Ser Phe Val Phe Glu
       130                 135                 140
   Phe Gly Lys Ser Leu Cys Ser Met His Leu Thr Glu Asp Glu Ile Ala
   145                 150                 155                 160
   Leu Phe Ser Ala Phe Val Leu Met Ser Ala Asp Arg Ser Trp Leu Gln
                   165                 170                 175
   Glu Lys Val Lys Ile Glu Lys Leu Gln Gln Lys Ile Gln Leu Ala Leu
               180                 185                 190
   Gln His Val Leu Gln Lys Asn His Arg Glu Asp Gly Ile Leu Thr Lys
           195                 200                 205
   Leu Ile Cys Lys Val Ser Thr Leu Arg Ala Leu Cys Gly Arg His Thr
       210                 215                 220
   Glu Lys Leu Met Ala Phe Lys Ala Ile Tyr Pro Asp Ile Val Arg Leu
   225                 230                 235                 240
   His Phe Pro Pro Leu Tyr Lys Glu Leu Phe Thr
                   245                 250
<210> 32
   <211> 252
   <212> PRT
   <213> Artificial Sequence
   <223> Description of Artificial Sequence: nucleotide sequence coding for a fragment of rat RORβ
<400> 32
   Gly Gln Leu Ala Pro Gly Ile Thr Met Ser Glu Ile Asp Arg Ile Ala
   1               5                   10                  15
   Gln Asn Ile Ile Lys Ser His Leu Glu Thr Cys Gln Tyr Thr Met Glu
               20                  25                  30
   Glu Leu His Gln Leu Ala Trp Gln Thr His Thr Tyr Glu Glu Ile Lys
           35                  40                  45
   Ala Tyr Gln Ser Lys Ser Arg Glu Ala Leu Trp Gln Gln Cys Ala Ile
       50                  55                  60
   Gln Ile Thr His Ala Ile Gln Tyr Val Val Glu Phe Ala Lys Arg Ile
   65                  70                  75                  80
   Thr Gly Phe Met Glu Leu Cys Gln Asn Asp Gln Ile Leu Leu Leu Lys
                   85                  90                  95
   Ser Gly Cys Leu Glu Val Val Leu Val Arg Met Cys Arg Ala Phe Asn
               100                 105                 110
   Pro Leu Asn Asn Thr Val Leu Phe Glu Gly Lys Tyr Gly Gly Met Gln
           115                 120                 125
   Met Phe Lys Ala Leu Gly Ser Asp Asp Leu Val Asn Glu Ala Phe Asp
       130                 135                 140
   Phe Ala Lys Asn Leu Cys Ser Leu Gln Leu Thr Glu Glu Glu Ile Ala
   145                 150                 155                 160
   Leu Phe Ser Ser Ala Val Leu Ile Ser Pro Asp Arg Ala Trp Leu Leu
                   165                 170                 175
   Glu Pro Arg Lys Val Gln Lys Leu Gln Glu Lys Ile Tyr Phe Ala Leu
               180                 185                 190
   Gln His Val Ile Gln Lys Asn His Leu Asp Asp Glu Thr Leu Ala Lys
           195                 200                 205
   Leu Ile Ala Lys Ile Pro Thr Ile Thr Ala Val Cys Asn Leu His Gly
       210                 215                 220
   Glu Lys Leu Gln Val Phe Lys Gln Ser His Pro Asp Ile Val Asn Thr
   225                 230                 235                 240
   Leu Phe Pro Pro Leu Tyr Lys Glu Leu Phe Asn Pro
                   245                 250
<210> 33
   <211> 252
   <212> PRT
   <213> Artificial Sequence
<220>
   <223> Description of Artificial Sequence: fragment of human RORβ
<400> 33
   Gly Gln Leu Ala Pro Gly Ile Thr Met Thr Glu Ile Asp Arg Ile Ala
   1               5                   10                  15
   Gln Asn Ile Ile Lys Ser His Leu Glu Thr Cys Gln Tyr Thr Met Glu
               20                  25                  30
   Glu Leu His Gln Leu Ala Trp Gln Thr His Thr Tyr Glu Glu Ile Lys
           35                  40                  45
   Ala Tyr Gln Ser Lys Ser Arg Glu Ala Leu Trp Gln Gln Cys Ala Ile
       50                  55                  60
   Gln Ile Thr His Ala Ile Gln Tyr Val Val Glu Phe Ala Lys Arg Ile
   65                  70                  75                  80
   Thr Gly Phe Met Glu Leu Cys Gln Asn Asp Gln Ile Leu Leu Leu Lys
                   85                  90                  95
   Ser Gly Cys Leu Glu Val Val Leu Val Arg Met Cys Arg Ala Phe Asn
               100                 105                 110
   Pro Leu Asn Asn Thr Val Leu Phe Glu Gly Lys Tyr Gly Gly Met Gln
           115                 120                 125
   Met Phe Lys Ala Leu Gly Ser Asp Asp Leu Val Asn Glu Ala Phe Asp
       130                 135                 140
   Phe Ala Lys Asn Leu Cys Ser Leu Gln Leu Thr Glu Glu Glu Ile Ala
   145                 150                 155                 160
   Leu Phe Ser Ser Ala Val Leu Ile Ser Pro Asp Arg Ala Trp Leu Ile
                   165                 170                 175
   Glu Pro Arg Lys Val Gln Lys Leu Gln Glu Lys Ile Tyr Phe Ala Leu
               180                 185                 190
   Gln His Val Ile Gln Lys Asn His Leu Asp Asp Glu Thr Leu Ala Lys
           195                 200                 205
   Leu Ile Ala Lys Ile Pro Thr Ile Thr Ala Val Cys Asn Leu His Gly
       210                 215                 220
   Glu Lys Leu Gln Val Phe Lys Gln Ser His Pro Glu Ile Val Asn Thr
   225                 230                 235                 240
   Leu Phe Pro Pro Leu Tyr Lys Glu Leu Phe Asn Pro
                   245                 250
<210> 34
   <211> 250
   <212> PRT
   <213> Artificial Sequence
<220>
   <223> Description of Artificial Sequence: fragment of human RORγ
<400> 34
   Thr Pro Glu Ala Pro Tyr Ala Ser Leu Thr Glu Ile Glu His Leu Val
   1               5                   10                  15
   Gln Ser Val Cys Lys Ser Tyr Arg Glu Thr Cys Gln Leu Arg Leu Glu
               20                  25                  30
   Asp Leu Leu Arg Gln Arg Ser Asn Ile Phe Ser Arg Glu Glu Val Thr
           35                  40                  45
   Gly Tyr Gln Arg Lys Ser Met Trp Glu Met Trp Glu Arg Cys Ala His
       50                  55                  60
   His Leu Thr Glu Ala Ile Gln Tyr Val Val Glu Phe Ala Lys Arg Leu
   65                  70                  75                  80
   Ser Gly Phe Met Glu Leu Cys Gln Asn Asp Gln Ile Val Leu Leu Lys
                   85                  90                  95
   Ala Gly Ala Met Glu Val Val Leu Val Arg Met Cys Arg Ala Tyr Asn
               100                 105                 110
   Ala Asp Asn Arg Thr Val Phe Phe Glu Gly Lys Tyr Gly Gly Met Glu
           115                 120                 125
   Leu Phe Arg Ala Leu Gly Cys Ser Glu Leu Ile Ser Ser Ile Phe Asp
       130                 135                 140
   Phe Ser His Ser Leu Ser Ala Leu His Phe Ser Glu Asp Glu Ile Ala
   145                 150                 155                 160
   Leu Tyr Thr Ala Leu Val Leu Ile Asn Ala His Arg Pro Gly Leu Gln
                   165                 170                 175
   Glu Lys Arg Lys Val Glu Gln Leu Gln Tyr Asn Leu Glu Leu Ala Phe
               180                 185                 190
   His His His Leu Cys Lys Thr His Arg Gln Ser Ile Leu Ala Lys Leu
           195                 200                 205
   Pro Pro Lys Gly Lys Leu Arg Ser Leu Cys Ser Gln His Val Glu Arg
       210                 215                 220
   Leu Gln Ile Phe Gln His Leu His Pro Ile Val Val Gln Ala Ala Phe
   225                 230                 235                 240
   Pro Pro Leu Tyr Lys Glu Leu Phe Ser Thr
                   245
<210> 35
   <211> 250
   <212> PRT
   <213> Artificial Sequence
<220>
   <223> Description of Artificial Sequence: fragment of murine RORγ
<400> 35
   Ala Pro Glu Val Pro Tyr Ala Ser Leu Thr Asp Ile Glu Tyr Leu Val
   1               5                   10                  15
   Gln Asn Val Cys Lys Ser Phe Arg Glu Thr Cys Gln Leu Arg Leu Glu
               20                  25                  30
   Asp Leu Leu Arg Gln Arg Thr Asn Leu Phe Ser Arg Glu Glu Val Thr
           35                  40                  45
   Ser Tyr Gln Arg Lys Ser Met Trp Glu Met Trp Glu Arg Cys Ala His
       50                  55                  60
   His Leu Thr Glu Ala Ile Gln Tyr Val Val Glu Phe Ala Lys Arg Leu
   65                  70                  75                  80
   Ser Gly Phe Met Glu Leu Cys Gln Asn Asp Gln Ile Ile Leu Leu Thr
                   85                  90                  95
   Ala Gly Ala Met Glu Val Val Leu Val Arg Met Cys Arg Ala Tyr Asn
               100                 105                 110
   Ala Asn Asn His Thr Val Phe Phe Glu Gly Lys Tyr Gly Gly Val Glu
           115                 120                 125
   Leu Phe Arg Ala Leu Gly Cys Ser Glu Leu Ile Ser Ser Ile Phe Asp
       130                 135                 140
   Phe Ser His Phe Leu Ser Ala Leu Cys Phe Ser Glu Asp Glu Ile Ala
   145                 150                 155                 160
   Leu Tyr Thr Ala Leu Val Leu Ile Asn Ala Asn Arg Pro Gly Leu Gln
                   165                 170                 175
   Glu Lys Arg Arg Val Glu His Leu Gln Tyr Asn Leu Glu Leu Ala Phe
               180                 185                 190
   His His His Leu Cys Lys Thr His Arg Gln Gly Leu Leu Ala Lys Leu
           195                 200                 205
   Pro Pro Lys Gly Lys Leu Arg Ser Leu Cys Ser Gln His Val Glu Lys
       210                 215                 220
   Leu Gln Ile Phe Gln His Leu His Pro Ile Val Val Gln Ala Ala Phe
   225                 230                 235                 240
   Pro Pro Leu Tyr Lys Glu Leu Phe Ser Thr
                   245
<210> 36
   <211> 252
   <212> PRT
   <213> Artificial Sequence
<220>
   <223> Description of Artificial Sequence: fragment of human RORα
<400> 36
   Gly Glu Thr Ser Pro Thr Val Ser Met Ala Glu Leu Glu His Leu Ala
   1               5                   10                  15
   Gln Asn Ile Ser Lys Ser His Leu Glu Thr Cys Gln Tyr Leu Arg Glu
               20                  25                  30
   Glu Leu Gln Gln Ile Thr Trp Gln Thr Phe Leu Gln Glu Glu Ile Glu
           35                  40                  45
   Asn Tyr Gln Asn Lys Gln Arg Glu Val Met Trp Gln Leu Cys Ala Ile
       50                  55                  60
   Lys Ile Thr Glu Ala Ile Gln Tyr Val Val Glu Phe Ala Lys Arg Ile
   65                  70                  75                  80
   Asp Gly Phe Met Glu Leu Cys Gln Asn Asp Gln Ile Val Leu Leu Lys
                   85                  90                  95
   Ala Gly Ser Leu Glu Val Val Phe Ile Arg Met Cys Arg Ala Phe Asp
               100                 105                 110
   Ser Gln Asn Asn Thr Val Tyr Phe Asp Gly Lys Tyr Ala Ser Pro Asp
           115                 120                 125
   Val Phe Lys Ser Leu Gly Cys Glu Asp Phe Ile Ser Phe Val Phe Glu
       130                 135                 140
   Phe Gly Lys Ser Leu Cys Ser Met His Leu Thr Glu Asp Glu Ile Ala
   145                 150                 155                 160
   Leu Phe Ser Ala Phe Val Leu Met Ser Ala Asp Arg Ser Trp Leu Gln
                   165                 170                 175
   Glu Lys Val Lys Ile Glu Lys Leu Gln Gln Lys Ile Gln Leu Ala Leu
               180                 185                 190
   Gln His Val Leu Gln Lys Asn His Arg Glu Asp Gly Ile Leu Thr Lys
           195                 200                 205
   Leu Ile Cys Lys Val Ser Thr Leu Arg Ala Leu Cys Gly Arg His Thr
       210                 215                 220
   Glu Lys Leu Met Ala Phe Lys Ala Ile Tyr Pro Asp Ile Val Arg Leu
   225                 230                 235                 240
   His Phe Pro Pro Leu Tyr Lys Glu Leu Phe Thr Ser
                   245                 250
<210> 37
   <211> 252
   <212> PRT
   <213> Artificial Sequence
<220>
   <223> Description of Artificial Sequence: fragment of murine RORα
<400> 37
   Gly Glu Thr Ser Pro Thr Val Ser Met Ala Glu Leu Glu His Leu Ala
   1               5                   10                  15
   Gln Asn Ile Ser Lys Ser His Leu Glu Thr Cys Gln Tyr Leu Arg Glu
               20                  25                  30
   Glu Leu Gln Gln Ile Thr Trp Gln Thr Phe Leu Gln Glu Glu Ile Glu
           35                  40                  45
   Asn Tyr Gln Asn Lys Gln Arg Glu Val Met Trp Gln Leu Cys Ala Ile
       50                  55                  60
   Lys Ile Thr Glu Ala Ile Gln Tyr Val Val Glu Phe Ala Lys Arg Ile
   65                  70                  75                  80
   Asp Gly Phe Met Glu Leu Cys Gln Asn Asp Gln Ile Val Leu Leu Lys
                   85                  90                  95
   Ala Gly Ser Leu Glu Val Val Phe Ile Arg Met Cys Arg Ala Phe Asp
               100                 105                 110
   Ser Gln Asn Asn Thr Val Tyr Phe Asp Gly Lys Tyr Ala Ser Pro Asp
           115                 120                 125
   Val Phe Lys Ser Leu Gly Cys Glu Asp Phe Ile Ser Phe Val Phe Glu
       130                 135                 140
   Phe Gly Lys Ser Leu Cys Ser Met His Leu Thr Glu Asp Glu Ile Ala
   145                 150                 155                 160
   Leu Phe Ser Ala Phe Val Leu Met Ser Ala Asp Arg Ser Trp Leu Gln
                   165                 170                 175
   Glu Lys Val Lys Ile Glu Lys Leu Gln Gln Lys Ile Gln Leu Ala Leu
               180                 185                 190
   Gln His Val Leu Gln Lys Asn His Arg Glu Asp Gly Ile Leu Thr Lys
           195                 200                 205
   Leu Ile Cys Lys Val Ser Thr Leu Arg Ala Leu Cys Gly Arg His Thr
       210                 215                 220
   Glu Lys Leu Met Ala Phe Lys Ala Ile Tyr Pro Asp Ile Val Arg Leu
   225                 230                 235                 240
   His Phe Pro Pro Leu Tyr Lys Glu Leu Phe Thr Ser
                   245                 250

## Description

### Field of Invention

An aspect of this invention is to obtain crystal structure from orphan receptors by using a heterologous expression system, which will not only produce high amount of the desired protein but may also furnish a pseudo-ligand. The presence of this fortuitous molecule is important to stabilize an active agonist conformation by adding concomitantly a co-activator peptide. These two elements avoid any other non-active alternative conformations.

This method is illustrated by crystals of brain specific retinoic acid-related orphan receptor ligand binding domain (RORβ-LBD) in complex with a co-activator peptide and a fortuitous fatty acid ligand. This invention also relates to methods of using DNA sequence or derived constructions to produce proteins in order either to find out the physiological ligand or to screen for synthetic analogues. This invention also relates to methods for designing and selecting ligands that bind to the RORβ and methods of using such ligands. It refers also to the use of DNA sequences of RORβ or derived sequences thereof in order to identify other proteins which interact with RORβ. Obviously the object of this invention is also the usage of the structure of similar or homologous proteins or protein complexes, particularly all these claims are applied to the two isotypes RORα and γ.

### Background of this Invention

The orphan retinoic acid-related orphan receptor β (RORβ), also called retinoid Z receptor β (RZRβ) (NR1F2) belongs to the nuclear receptor (NR) superfamily and is expressed in areas of the central nervous system. The ligand-dependent activity of the nuclear receptor makes them obvious targets for drug design in many therapeutic areas. However in the case of orphan receptors, the ligand is not known and even the existence of a ligand is not proven. RORβ was never shown to bind retinoic acid. RORβ regulates genes whose products play a role in the context of sensory input integration as well as in the context of the biological clock. A behavioral phenotype of RORβ -/- mice was observed and seems to be similar to the phenotype described > 40 years ago for a spontaneous mouse mutation called *vacillans* (Sirlin, 1956). These mice display a duck-like gait, transient male incapability to sexually reproduce and a severely disorganised retina that suffer from post-natal degeneration.

Two other closely nuclear receptors are RORα and RORγ. RORα presents 61% identity and 74% similarity with RORβ. RORα is rather ubiquitously expressed (Becker-André et al., 1993) and has been demonstrated to play important roles in cerebellum development and immune response (Matysiak-Scholze and Nehls, 1997; Koibuchi and Chin., 1998). *Staggerer* mice were found to carry a deletion within the RORα gene that prevents translation of the ligand-binding domain. They present a severe cerebellar ataxia related to a defect in developpement of Purkinje cells. Certain functions of the immune system are also affected (Hamilton & al., 1996).

Certain functions of the immune system are also affected (Hamilton & al., 1996). RORα is also constitutively expressed during myogenesis (Lau et al., 1999).

The expression of RORγ is found mainly in skeletal muscle (Hirose et al., 1994) and is induced in the middle stage of adipocyte differentiation (Kurebayashi S., and Hirose T, 1998).

As with the other members of the nuclear receptor family, RORβ has several functional domains including a DNA binding domain (DBD), and a 250 residue ligand-binding domain (LBD) which contains the ligand -binding site, and is responsible for switching on the ligand-binding function.

It would be advantageous to devise methods and compositions for reducing the time required to discover ligands to the RORβ, synthesize such compounds and administer such compounds to organisms to modulate physiological processes regulated by the RORβ receptor or any of its isotypes α or β.

There have been no crystals reported so far of any orphan receptor in the agonist bound conformation. The structure of USP, the insect ortholog of RXR, has been published recently. Though RXRs bind 9c-RA, USP fail to bind this ligand and must still be considered as an orphan receptor. Juvenile hormones have been proposed to be the natural ligands of USP but matter is still controversial. Nevertheless, the USP LBD structure is in an antagonist-like conformation. We report discovered the first crystal structure of the orphan receptor ligand-binding domain (RORβ-LBD) in the agonist bound conformation, which represents the transcriptionally active form of nuclear receptor.

### Summary of the Invention

Polypeptides derived from the retinoic acid-related orphan receptor (ROR) in mammals, characterized in that they comprise at least the amino-acid sequence delimited in its N-tenninal extremity by the first amino acid of the H1 helix, and in its C-tenninal extremity by the last amino acid of the H12 helix can be mentioned here.

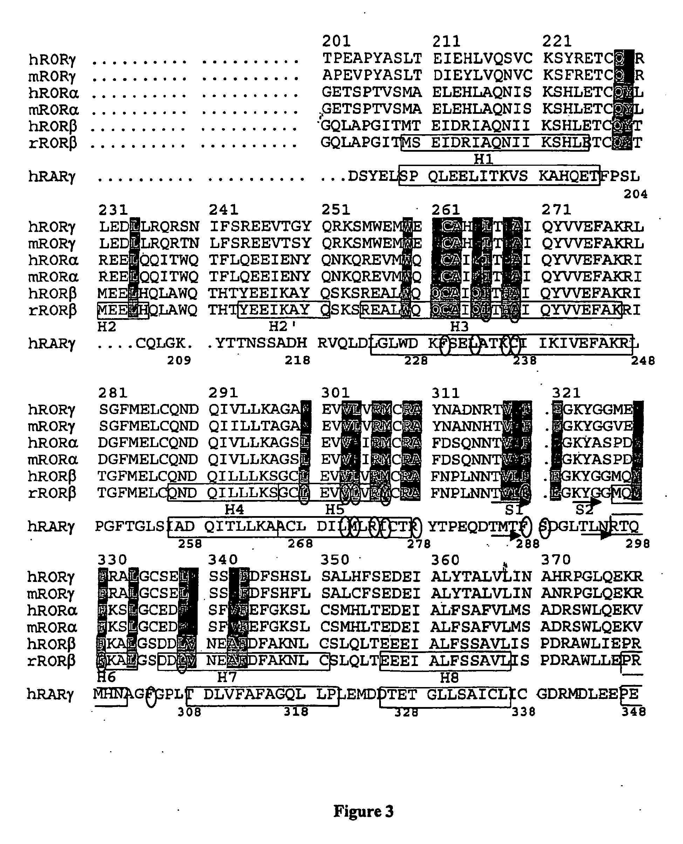

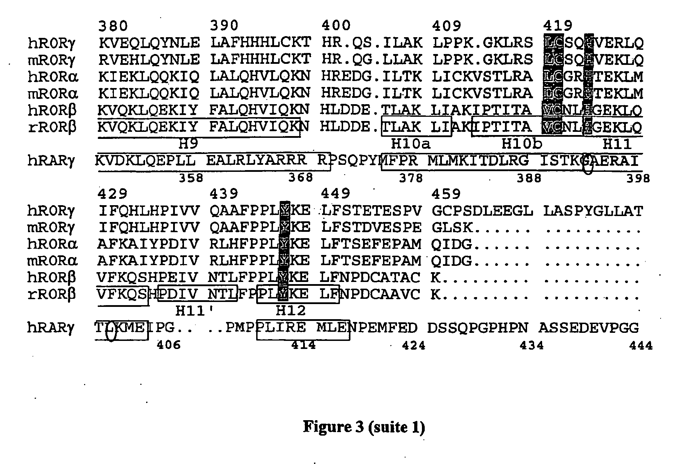

Polypeptides derived from the retinoic acid-related orphan receptor (ROR) in mammals, characterized in that they are delimited in their N-tenninal extremity by an amino-acid located between positions 1 to 209 of the rat, human, or murine RORβ, α, or γ, as represented on figure 3, or by an amino-acid located at corresponding positions in nuclear receptor ROR of other subtypes than α, β and γ, and/or of other mammals, and in their C-terminal extremity by an amino-acid located between positions 450 to 452 of the rat, human, or murine RORβ, α, or γ, as represented on figure 3, or by an amino-acid located at corresponding positions in nuclear receptor ROR of other subtypes than α, β and γ, and/or of other mammals can also be mentioned here.

Some polypeptides derived from the retinoic acid-related orphan receptor (ROR) in mammals are characterized in that they are delimited in their N-terminal extremity by an amino-acid located between positions 1 to 209 of the human or rat nuclear receptor RORβ, as represented on figure 3, or by an amino-acid located at corresponding positions in nuclear receptor ROR of other subtypes, such as RORα, and RORγ, as represented on figure 3, and/or of other mammals, and in their C-terminal extremity by an amino-acid located between positions 450 to 452 of the human or rat nuclear receptor RORβ, as represented on figure 3, or by an amino-acid located at corresponding positions in nuclear receptor ROR of other subtypes, such as RORα, and RORγ, and/or of other mammals.

Other polypeptides derived from the retinoic acid-related orphan receptor (ROR) in mammals are characterized in that they are delimited in their N-terminal extremity by the methionine in position 209 of the human or rat nuclear receptor RORβ, as represented on figure 3, or by the methionine or another amino-acid such as leucine located at a corresponding position in nuclear receptor ROR of other subtypes, such as RORα, and RORγ, and/or of other mammals, and in its C-terminal extremity by the phenylalanine in position 450 of the human or rat nuclear receptor RORβ, as represented on figure 3, or by the phenylalanine or another amino-acid located at a corresponding position in nuclear receptor ROR of other subtypes, such as RORα, and RORγ, and/or of other mammals.

Advantageously, polypeptides as defined above are characterized in that at least the approximately 100 to 200 first amino-acids of the N-terminal part of the sequence of said receptor is deleted.

Polypeptides defined above are more particularly characterized, in that they are polypeptides derived from the nuclear receptor ROR, wherein the binding properties of the ligand-binding domain, or LBD, of said receptor, are maintained.

Particular polypeptides derived from the nuclear receptor RORβ, of mammals, such as human or rat, comprise a polypeptide as defined above, such as the polypeptides delimited by the amino-acids located in positions 201 to 459 of the sequences of rat or human RORβ represented on figure 3, said polypeptides being characterized in that at least one of the cysteine in position 454 or in position 458 of the amino-acid sequence of said nuclear receptor RORβ, as represented on figure 3, is deleted or substituted by another amino-acid, natural or not, such as alanine or serine.

Polypeptides as defined above are more particularly characterized in that:
- the N-terminal sequence delimited by the amino-acids in position 1 to 200 of the receptor, is deleted,
- and the C-terminal sequence starting from the amino-acid in position 450 of the human or rat nuclear receptor RORβ represented on figure 3, or from the amino-acid located at a corresponding position in nuclear receptor ROR of other subtypes, such as RORα, and RORγ, as represented on figure 3, and/or of other mammals, and more preferably from the amino-acid in position 451, 452, or 453, is deleted.

The invention relates to polypeptides from the retinoic acid-related orphan receptor (ROR) in mammals characterized in that they are fragments of rat, human, or murine RORβ, α, or γ, delimited in their N-terminal extremity by the amino acid located in one of the positions 201 to 209 of the ROR sequences represented on figure 3, and in their C-terminal extremity by the amino acid located in one of the positions 451 or 452, of the ROR sequences represented on figure 3.

The invention concerns more particularly polypeptides as defined above, chosen among:
- the fragment delimited by the amino acids located in positions 209 to 452 of:
   - the sequence of the rat RORβ SEQ ID NO : 2,
   - the sequence of the human RORβ SEQ ID NO : 3,
   - the sequence of the human RORγ SEQ ID NO : 4,
   - the sequence of the murine RORγ SEQ ID NO : 5,
   - the sequence of the human RORα SEQ ID NO : 6,
   - the sequence of the murine RORα SEQ ID NO : 7,
- the fragment delimited by the amino acids located in positions 208 to 452 of :
   - the sequence of the rat RORβ SEQ ID NO : 8,
   - the sequence of the human RORβ SEQ ID NO : 9,
   - the sequence of the human RORγ SEQ ID NO : 10,
   - the sequence of the murine RORγ SEQ ID NO : 11,
   - the sequence of the human RORα SEQ ID NO : 12,
   - the sequence of the murine RORα SEQ ID NO : 13,
- the fragment delimited by the amino acids located in positions 208 to 451 of:
   - the sequence of the rat RORβ SEQ ID NO : 14,
   - the sequence of the human RORβ SEQ ID NO : 15,
   - the sequence of the human RORγ SEQ ID NO : 16,
   - the sequence of the murine RORγ SEQ ID NO : 17,
   - the sequence of the human RORα SEQ ID NO : 18,
   - the sequence of the murine RORα SEQ ID NO:19,
- the fragment delimited by the amino acids located in positions 209 to 451 of :
   - the sequence of the rat RORβ SEQ ID NO : 20,
   - the sequence of the human RORβ SEQ ID NO : 21,
   - the sequence of the human RORγ SEQ ID NO : 22,
   - the sequence of the murine RORγ SEQ ID NO : 23,
   - the sequence of the human RORα SEQ ID NO : 24,
   - the sequence of the murine RORα SEQ ID NO : 25,
- the fragment delimited by the amino acids located in positions 201 to 451 of:
   - the sequence of the rat RORβ SEQ ID NO : 26,
   - the sequence of the human RORβ SEQ ID NO : 27,
   - the sequence of the human RORγ SEQ ID NO : 28,
   - the sequence of the murine RORγ SEQ ID NO : 29,
   - the sequence of the human RORα SEQ ID NO : 30,
   - the sequence of the murine RORα SEQ ID NO : 31,
- the fragment delimited by the amino acids located in positions 201 to 452 of :
   - the sequence of the rat RORβ SEQ ID NO : 32,
   - the sequence of the human RORβ SEQ ID NO : 33,
   - the sequence of the human RORγ SEQ ID NO : 34,
   - the sequence of the murine RORγ SEQ ID NO : 35,
   - the sequence of the human RORα SEQ ID NO : 36,
   - the sequence of the murine RORα SEQ ID NO : 37.

Polypeptides as defined above according to the invention, are more particularly characterized by the following characteristics:
- they have the properties of binding a ligand and of transactivation of the LBD of the receptor ROR,
- they are soluble in aqueous solvents,
- they are crystallisable in aqueous solvents, especially by the hanging drop vapour diffusion method, more particularly at approximately 4°C,

The invention also concerns molecular complexes comprising a polypeptide as defined above, said polypeptide being in association with:
- a ROR-LBD ligand which is an agonist, such as stearic acid, or an antagonist of the ROR-LBD, such as retinoic acid,
- and/or with a co-peptide having a sequence of approximately 15-20 amino-acids and comprising the co-activator motif LXXLL or a co-repressor motif (I/L)XX(V/I)I or LXX(H/I)IXXX(I/L) wherein X represents any amino acid, natural or not, such as co-peptides chosen among fragments of co-activators of transcription, especially those of the p160 family, and more particularly among fragments of the co-activators SRC1, such as the fragment 686-700 of SRC1, or among fragments of co-repressors of transcription.

The invention also relates to nucleotide sequence coding for a polypeptide as defined above.

The invention also relates to nucleotide sequence as defined above, associated to elements necessary for the transcription of this sequence, particularly a promoter and a terminator of transcription.

The invention also concerns vector, particularly plasmid, comprising a nucleotide sequence as defined above.

The invention also relates to host cells, such as *E.coli,* transformed with a vector as defined above.

The invention also relates to a process for obtaining a polypeptide, or a molecular complex, as defined above, characterized in that it comprises :
- a step of transforming host cells with a nucleotide sequence as defined above, using a vector as defined above,
- a step of cultivating the transformed host cell as defined above thus obtained, in an appropriate culture medium,
- and the recovery, and if necessary, the purification of the recombinant polypeptide or molecular complex obtained.

The invention also relates to a crystal comprising a polypeptide according, or a molecular complex, as defined above.

Advantageously, a crystal as defined above, is characterized in that it diffracts to at least 3 angstrom resolution and has a crystal stability within 5% of its unit cell dimensions.

Preferred crystal as defined above, is such that the ROR-LBD has the following unit cell dimensions in angstroms : a= 52.302 Å, b= 58.490 Å and c= 106.036 Å, α=β=χ=90 °, and an orthorhombic space group P212121.

The invention also relates to a crystal as defined above, such as obtained by carrying out a process mentioned above, and comprising a step of crystallisation in aqueous solvents of the polypeptides, or the molecular complexes, as defined above, especially at 4°C by the hanging drop vapour diffusion method.

The invention also relates to the use of the polypeptides, or of the molecular complexes, or of the crystals, as defined above, for carrying out:
- a process for the screening of a ROR-LBD ligand which is an agonist, or an antagonist of said receptor, or for the screening of ligands that perturb the structure of the receptor and having an effect on the recruitment of cofactors (co-activators and co-repressors) and hence on gene regulation,
- or a process for the analysis of the tridimensional structure of the complexes formed with said polypeptides, molecular complexes or crystals and a particular compound.

The invention relates more particularly to the use mentioned above, for the screening of compounds acting as agonists or antagonists ofROR, said compounds being useful in the frame of the treatment of pathologies related to the central nervous system, the retinal organisation, the sensorial signal integration, the motricity, and sterility.

The invention also relates to a process for the screening of a ROR-LBD ligand which is an agonist, or an antagonist of said receptor, said process comprising the following steps :
- contacting a polypeptide, or a molecular complex, or a crystal according, as defined above, advantageously linked to a solid support, with the particular compound susceptible to be a ROR-LBD ligand, preferably one of the said polypeptide, or molecular complex, or crystal, or tested ligand, being labelled, such as with a fluorescent, radioactive or enzymatic label,
- detection of the possible association between the said polypeptide, or molecular complex, or crystal, and the tested ligand, by measuring the used label, especially after rinsing the support used in the preceding step, or by mass spectrometry under non denaturing conditions.

The invention also relates to a process for the analysis of the tridimensional structure of the complexes formed with a polypeptide, or a molecular complex, or a crystal, as defined above, and a particular compound susceptible to be a ROR-LBD ligand, said process comprising the following steps :
- contacting the said polypeptide, or molecular complex, or crystal, with said particular compound,
- crystallisation of the complex formed between the said polypeptide, or molecular complex, or crystal, and the tested ligand, especially with the vapour diffusion method, and tridimensional analysis of said complex, especially with the molecular replacement method,
- or tridimensional analysis of said complex in soluble state, by using an appropriate method such as NMR.

The present invention provides crystals of an RORβ-LBD bound to a ligand and to a coactivator peptide, i. e. an RORβ-LBD/ligand/peptide complex. The ligand is stearic acid.

The crystal diffracts to 1.9 Å resolution. The crystal of RORβ-LBD preferably has at least 243 amino acid and preferably comprises amino acid sequence 208 to 451 of rat RORβ. The present invention also provides the structure coordinates of the RORβ-LBD/ligand/peptide complex. The complete coordinates are listed in Table A.

The complete coordinates of crystals of an RORβ-LBD bound to a ligand and to a coactivator peptide, i. e. an RORβ-LBD/ligand/peptide complex, wherein the ligand is retinoic acid are listed in Table B.

The present invention also provides a method for determining at least a portion of the three-dimensional structure of molecules or molecular complexes which contain at least some structurally similar features to the RORβ-LBD. It is preferred that these molecules or molecular complexes_comprise at least a part of the ligand-binding site defined by structure coordinates of RORβ-LBD amino-acids Q228, Y229, L234, W259, Q261, C262, A263, Q265, I266, H268, A269, L299, V303, L304, R306, M307, R309, A310, V318, L319, F320, E321, M329, F330, L333, L338, 1339,_A342, F343, V419, C420, H423, and Y446 according to Table A or a mutant or homologue thereof.

The present invention also provides a computer comprising a computer readable form to the coordinates contained in Table A.

The present invention further provides a binding site in RORβ-LBD for_an RORβ-LBD agonist or antagonist ligand as well as methods for designing or selecting agonists, antagonist and/or a selective RORβ receptor modulator (SRORM) of ROR using information about the crystal structures disclosed herein.

The present invention also provides a method in order to crystallize orphan receptors, which permits the determination of the ligand-binding pocket, important for the discovery of agonists and antagonists.

### Brief description of the Drawing

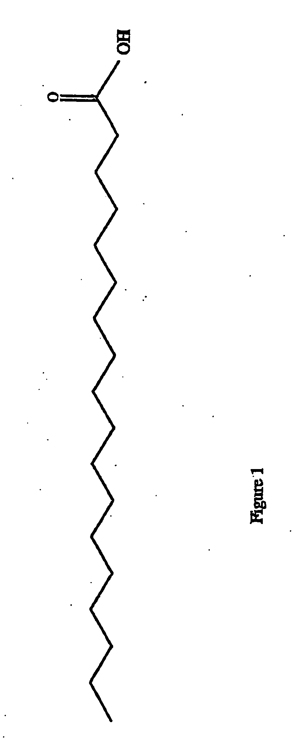

Figure 1-Stearate

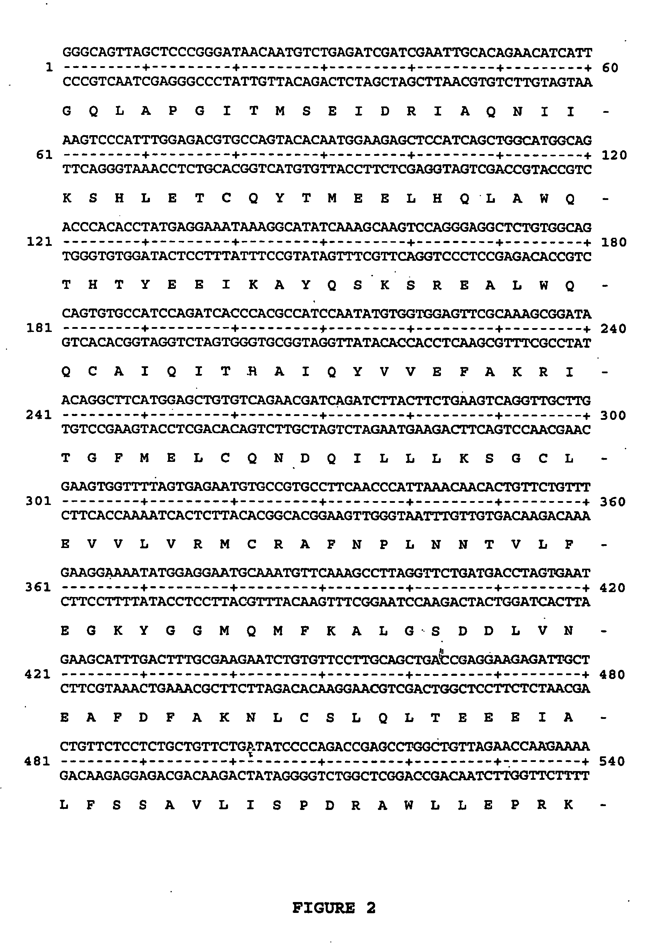

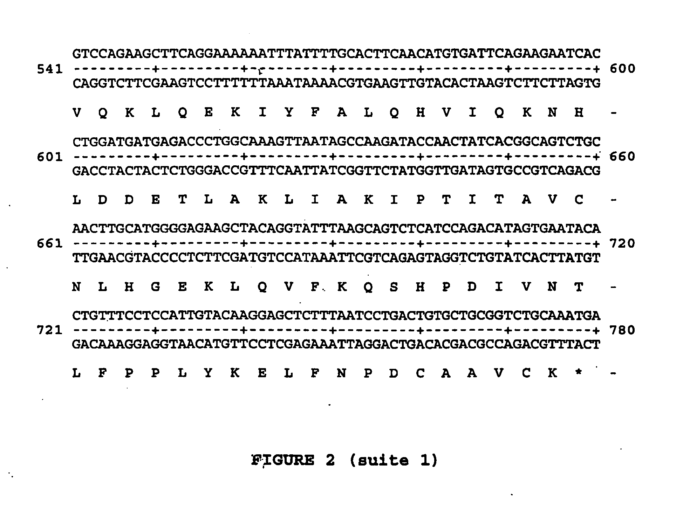

Figure 2- Nucleotide and polypeptide sequences of rat RORβ LBD.
Figure 3- Sequence of the rat RORβ LBD as cloned, with the secondary structural elements boxed (α helices) or drawn with an arrow (β strands). Sequences of human RORβ LBD, mouse RORα LBD, human RORα LBD, mouse RORγ LBD and human RORγ LBD are also given.
   For comparison, the aligned sequence of human RARγ LBD, which was used in order to solve the crystallographic structure is given. Residues involved in stearate binding in the case of RORβ or in trans retinoic acid in the case of human RARγ binding are in bold. Residues within a 4 Å cut-off are surrounded by a cercle.

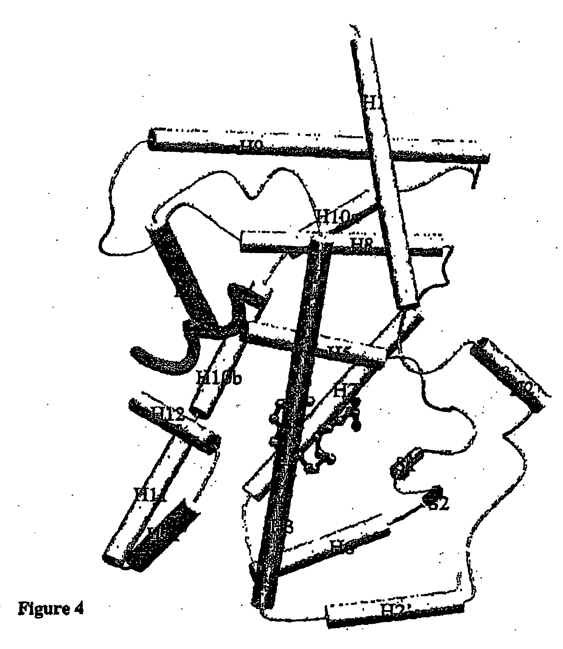

Figure 4- Ribbon style drawing of the RORβ LBD and the co-activator peptide. The ligand stearate is shown as a ball-and-stick figure

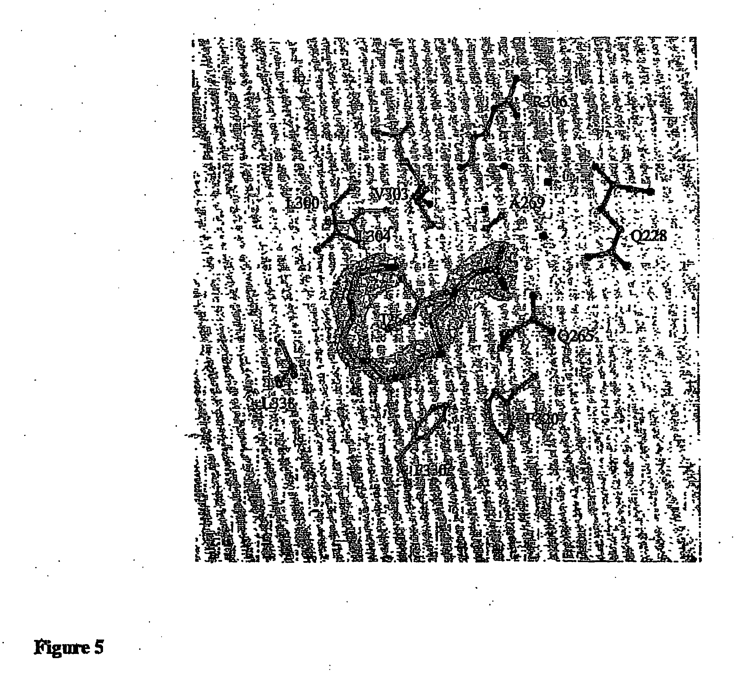

Figure 5- Difference (2Fo-Fc) electron density (1σ).

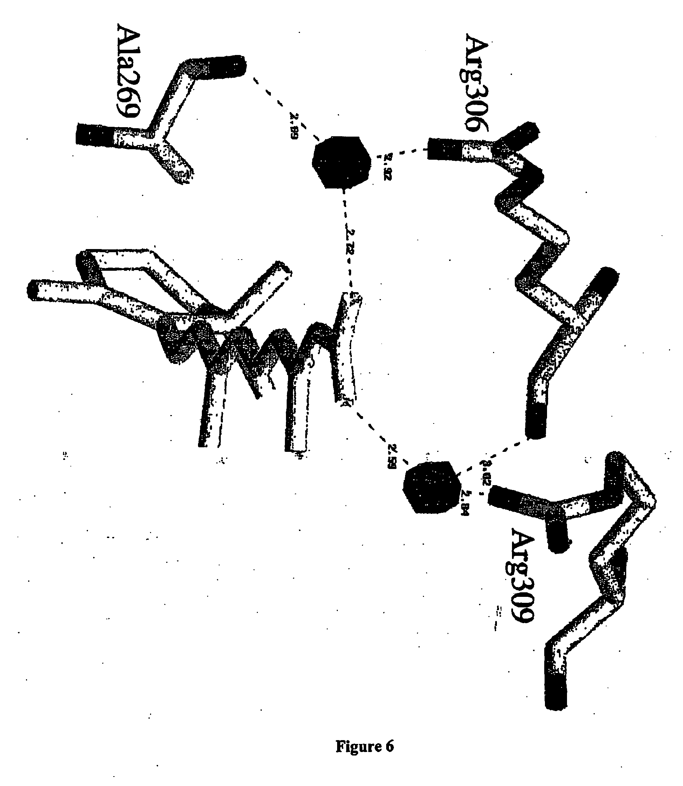

Figure 6: Detail of the hydrogen bond network formed with the ATRA carboxylate group.

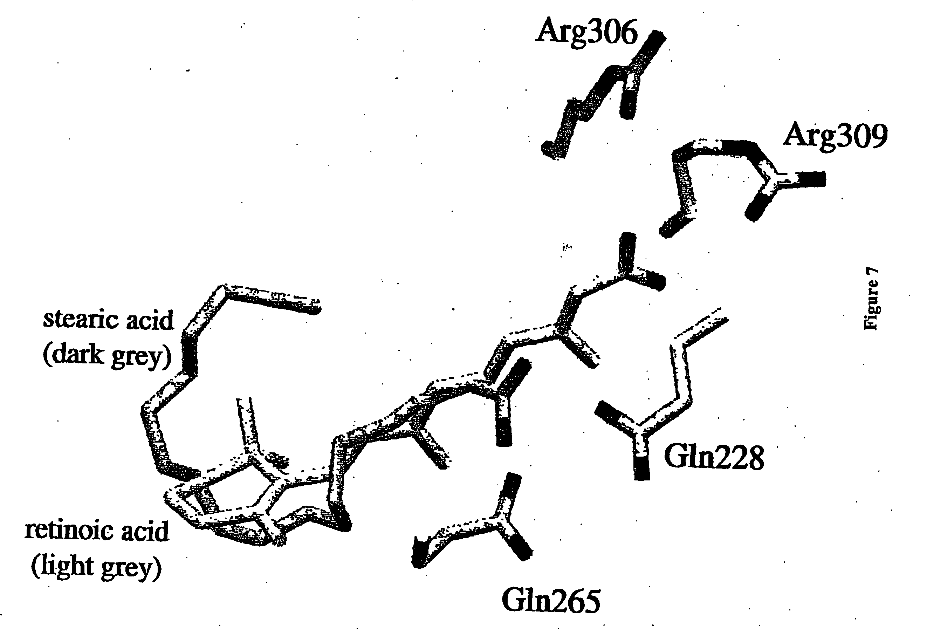

Figure 7: Superposition of stearate and ATRA in the RORβ LBD pocket.

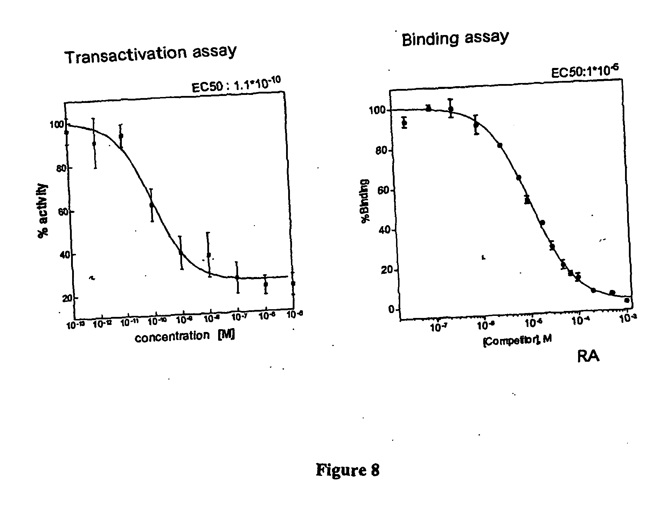

Figure 8: binding and transactivation assays for all-trans retinoic acid

### Detailed Description of the Invention

The first crystal structure of the RORβ ligand-binding domain (RORβ-LBD) has been determined to 1.9 Å resolution. Crystals of rat RORβ-LBD were grown from crystallizing solutions containing 0.1 M TrisHCl pH= 8.0 and PEG 6000 15%. X-ray diffraction patterns from the crystals have the symmetry and systematic absences of the orthorhombic space group P212121 with unit cell dimensions a= 52.302 Å b= 58.490 Å and c= 106.036 Å and one molecule per asymmetric unit (Mathews Volume = 2,57 Å³Da⁻¹). The structure was determined by the method of molecular replacement using the structure of the retinoic acid (RARγ-LBD) as the search model.

The complex of RORβ-LBD with sterarate and the co-activator peptide shows the mode of binding of the ligand to the orphan receptor in the agonist conformation.

The following abbreviations are used throughout the application:
A=Ala=Alanine
V=Val=Valine
L=Leu=Leucine
I=Ile=Isoleucine
P=Pro=Proline
F=Phe=Phenylalanine
W=Trp=Tryptophane
M=Met=Methionine
G=Gly=Glycine
S=Ser=Serine
T=Thr=Threonine
C=Cys=Cysteine
Y=Tyr=Tyrosine
N=Asn=Asparagine
Q=Gln=Glutamine
D=Asp=Aspartic acid
E=Glu=Glutamic acid
K=Lys=Lysine
R=Arg=Arginine
H=His=Histidine

"Atom type" refers to the element whose coordinates have been determined. Elements are defined by the first letter in the column.

"X, Y, Z" crystallographically define the atomic position determined for each atom.

" B" is a thermal factor that measures movement of the atom around its atomic center.

"Occ" is an occupancy factor that refers to the fraction of the molecules in which each atom occupies the position specified by the coordinates. A value of "1" indicates that each atom has the same conformation, i.e., the same position, in all molecules of the crystal.

Additional definitions are set forth in the specification where necessary.

The RORβ receptor described herein is intended to include any polypeptide which has the activity of the naturally occuring RORβ. The RORβ and RORβ-LBD contemplated herein includes all vertebrate and mammalian forms such as rat, mouse, pig, goat, horse, guinea pig, rabbit, monkey, orangutan and human. Such terms also include polypeptides that differ from naturally occuring forms of RORβ and RORβ-LBD by having amino acid deletions, substitutions, and additions, but which retain the activity of RORβ and RORβ-LBD, respectively. The crystal structure of the invention preferably contains at least 25%, more preferably at least 50%, more preferably at least 75%, more preferably at least 90%, more preferably at least 95%, more preferably at least 99%, and more preferably all of the coordinates listed in Table A. The crystal of the RORβ-LBD/RORβ-LBD-ligand/RORβ-LBD-ligand-peptide of the invention preferably has the following unit cell dimensions in angstroms: a= 52.302 Å b= 58.490 Å and c= 106.036 Å and an orthorhombic space group P212121.

This includes both agonists or activators and antagonist or inhibitors of the RORβ-LBD.

The peptides referred to herein (e.g., RORβ, RORβ-LBD, and the like) may be produced by any well-known method, including synthetic method, such as solid phase, liquid phase and combination solid/liquid phase syntheses; recombinant DNA methods, including cDNA cloning, optionally combined with site directed mutagenesis; and/or purification of the natural products, optionally combined with enzymatic cleavage methods to produce fragments of naturally occuring proteins.

Advantageously, the crystallizable compositions provided by this invention are amenable to x-ray crystallography. Thus, this invention also provides the three-dimensional structure of the RORβ-LBD/RORβ-LBD ligand peptide complex, particularly the complex of rat RORβ-LBD with stearic acid.

The three-dimensional structure of the RORβ-LBD/ligand complex of this invention is defined by a set of structure coordinates as set forth in Table A. The term "structure coordinates" refers to Cartesian coordinates derived from mathematical equations related to the patterns obtained on diffraction of a monochromatic beam of X-rays by the atoms (scattering centers) of a RORβ/stearate/peptide complex in crystal form. The diffraction data are used to calculate an electron density map of the repeating unit of the crystal. The electron density maps are then used to establish the positions of the individual atoms of the complex.

Those of skill in the art will understand that a set of structure coordinates for a receptor or receptor/ligand, or receptor/ligand/ peptide complex or a portion thereof, is a relative set of points that define a shape in three dimensions. Thus, it is possible that an entirely different set of coordinates could define a similar or identical shape. Moreover, slight variations in the individual coordinates will have little effect on overall shape.

The variations in coordinates discussed above may be generated because of mathematical manipulations of the structure coordinates. For example, the structure coordinates set forth in Table A could be manipulated by crystallographic permutations of the structure coordinates, fractionalization of the structure coordinates; integer additions or substractions to sets of the structure coordinates, inversion of the structure coordinates or any combination of the above.

Alternatively, modifications in the crystal structure due to mutations, additions, substitutions, and/or deletions of aminoacids, or other changes in any of the components that make up the crystal could also account for variations in structure coordinates. If such variations are within an acceptable standard error as compared to the original coordinates, the resulting three-dimensional shape is considered to be the same.

Various computational analyses are therefore necessary to determine whether a molecule or molecular complex or a portion thereof is sufficiently similar to all or parts of the RORβ receptor/stearate described above as to be considered the same. Such analyses may be carried out in current software applications, such as the Molecular Similarity application of Quanta (Molecular Simulations Inc., San Diego, CA) version 4.1, and as described in the accompanying User's Guide.

The Molecular Similarity application permits comparisons between different structures, different conformations of the same structure, and different parts of the same structure. The procedure used in Molecular Similarity to compare structures is divided into four steps: 1) load the structures to be compared.; 2) define the atom equivalences in these structures; 3) perform a fitting operation; and 4) analyze the results.

Each structure is identified by a name. One structure is identified as the target (i.e., the fixed structure); all remaining structures are working structures (i.e., moving structures). Since atom equivalency within QUANTA is defined by user input, for the purpose of this invention we will define equivalent atoms as protein backbone atoms (N, Cα, C and O) for all conserved residues between the two structures being compared. We will also consider only rigid fitting operations.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by QUANTA.

For the purpose of this invention, any molecule or molecular complex that has a root mean square deviation of conserved residue backbone atoms (N, Cα, C, O) of less than 1.5 Å when superimposed on the relevant backbone atoms described by structure coordinates listed in Table A are considered identical. More preferably, the root mean square deviation is less than 1 Å. In a preferred embodiment of the present invention , the molecule or molecular complex comprises at least a portion of the ligand binding site defined by structure coordinates of RORβ-LBD amino acids Q228, Y229, L234, W259, Q261, C262, A263, Q265, I266, H268, A269, L299, V303, L304, R306, M307, R309, A310, V318, L319, F320, E321, M329, F330, L333, L338, I339, A342, F343, V419, C420, H423, and Y446 according to Table A, or a mutant or homologue of said molecule or molecular complex. For purposes of the present invention , by " at least a portion of it" it is meant all or any parts of the ligand-binding site defined by these structure coordinates. More preferred are molecules or molecular complexes comprising all or any parts of the ligand-binding site defined by structure coordinates of RORβ-LBD amino acids Q228, Y229, L234, W259, Q261, C262, A263, Q265, 1266, H268, A269, L299, V303, L304, R306, M307, R309, A310, V318, L319, F320, E321, M329, F330, L333, L338,I339, A342, F343, V419, C420, H423, and Y446 according to Table A, or a mutant or homologue of said molecule or molecular complex. By mutant or homologue of the molecule or molecular complex it is meant a molecule or molecular complex having a binding pocket that has a root mean square deviation from the backbone atoms of said RORβ-LBD amino acids of not more than 1.5 Angstroms.

The term "root mean square deviation" means the square root of the arithmetic mean of the squares of deviations from the mean. It is a way to express the deviation or variation from a trend or object. For purposes of this invention, the "root mean square deviation" defines the variation in the backbone of a protein or protein complex from the relevant portion of the backbone of the RORβ portion of the complex as defined by the structure coordinates described herein. Once the structure coordinates of a protein crystal have been determined they are useful in solving the structures of other crystals or in modelling by homology other proteins particularly the two isotypes RORα and γ.

Thus, in accordance with the present invention, the structure coordinates of a RORβ/stearate/peptide complex, and in particular a complex, and portions thereof is stored in a machine-readable storage medium. Such data may be used for a variety of purposes, such as drug discovery and x-ray crystallographic analysis or protein crystal.

Accordingly, in one embodiment of this invention is provided a machine-readable data storage medium comprising a data storage material encoded with the structure coordinates set forth in Table A.

For the first time, the present invention permits the use of structure-based or rational drug design techniques to design, select, and synthesize chemical entities, including inhibitory and stimulatory compounds that are capable of binding to RORβ-LBD, or any portion thereof.

One particularly useful drug design technique enabled by this invention is iterative drug design. Iterative drug design is a method for optimizing associations between a protein and a compound by determining and evaluating the three-dimensional structures of successive sets of protein/compound complexes.

Those of skill in the art will realize that association of natural ligands or substrates with the binding pockets of their corresponding receptors or enzymes in the basis of many biological mechanisms of action. The term "binding pocket" as used herein, refers to a region of a molecule or molecular complex, that, as a result of its shape, favorably associates with another chemical entity or compound. Similarly, many drugs exert their biological effects through association with the binding pockets of receptors and enzymes. Such associations may occur with all or any parts of the binding pockets. An understanding of such associations will help lead to the design of drugs having more favorable associations with their target receptor, and thus, improved biological effects. Therefore, this information is valuable in designing potential ligands or inhibitors of receptors, such as inhibitors of RORβ.

The term "associating with" refers to a condition of proximity between chemical entities or compounds, or portions thereof. The association may be non-covalentwherein the juxtaposition is energetically favored by hydrogen bonding or van der Waals or electrostatic interactions -- or it may be covalent.

In iterative drug design, crystals of a series of protein/compound complexes are obtained and then the three-dimensional structures of each complex is solved. Such an approach provides insight into the association between the proteins and compounds of each complex. This is accomplished by selecting compounds with inhibitory activity, obtaining crystals of this new protein/compound complex, solving the three dimensional structure of the complex, and comparing the associations between the new protein/compound complex and previously solved protein/compound complexes. By observing how changes in the compound affected the protein/compound associations, these associations may be optimized.

In some cases, iterative drug design is carried out by forming successive proteincompound complexes and then crystallizing each new complex. Alternatively, a preformed protein crystal is soaked in the presence of an inhibitor, thereby forming a protein/ compound complex and obviating the need to crystallize each individual protein/compound complex.

As used herein, the term "soaked" refers to a process in which the crystal is transferred to a solution containing the compound of interest.

The structure coordinates set forth in Table A can also be used to aid in obtaining structural information about another crystallized molecule or molecular complex. This may be achieved by any of a number of well-known techniques, including molecular replacement.

The structure coordinates set forth in Table A can also be used for determining at least a portion of the three-dimensional structure of molecules or molecular complexes which contain at least some structurally similar features to RORβ. In particular, structural information about another crystallized molecule or molecular complex may be obtained. This may be achieved by any of a number of well-known techniques, including molecular replacement.

Therefore, in another embodiment this invention provides a method of utilizing molecular replacement to obtain structural information about a crystallized molecule or molecular complex whose structure is unknown comprising the steps of:
a) generating an X-ray diffraction pattern from said crystallized molecule or molecular complex,
b) applying at least a portion of the,structure coordinates set forth in Table A to the X-ray diffraction pattern to generate a three-dimensional electron density map of the molecule or molecular complex whose structure is unknown; and
c) using all or a portion of the structure coordinates set forth in Table A to generate homology models of RORβ-LBD or any other nuclear orphan or hormone receptor ligand-binding domain.

By using molecular replacement, all or part of the structure coordinates of the RORβ-LBD/RORβ-LBD-ligand/RORβ-LBD-ligand-peptide complex provided by this invention or molecular complex whose structure is unknown more quickly and efficiently than attempting to determine such information ab initio.

Molecular replacement provides an accurate estimation of the phases for an unknown structure. Phases are factors in equations used to solve crystal structures that cannot be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the RORβ-LBD/RORβ-LBD ligand complex according to Table A within the unit cell of the crystal of the unknown molecule or molecular complex so as best to account for the observed X-ray diffraction pattern of the cristal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex [E. Lattman, "Use of the Rotation and Translation Functions", in Meth. Enzymol., 115, pp55-77 (1985); M. G. Rossmann, ed., "The Molecular Replacement Method", Int. Sci. Rev. Set., No 13, Gordon & Breach, New York (1972)].

The structure of any portion of any crystallized molecule or molecular complex that is sufficiently homologous to any portion of the RORβ-LBD/RORβ-LBD ligand complex can be solved by this method.

The structure coordinates are also particularly useful to solve the structure of crystals of RORβ-LBD/RORβ-LBD ligand or RORβ-LBD ligand peptide co-complexed with a variety of chemical entities. This approach enables the determination of the optimal sites for interaction between chemical entities, including interaction of candidate RORβ inhibitors with the complex. For example, high resolution X-ray diffraction data collected from crystals exposed to different types of solvent allows the determination of where each type of solvent molecule resides. Small molecules that bind tightly to theses sites can then be designed and synthesized and tested for their RORβ inhibition activity.

All of the complexes referred to above may be studied using well-known X-ray difraction techniques and may be refined versus 1.5-3 Å resolution X-ray data to an R-value of about 0.20 or less using computer software, such as X-PLOR [Yale University, 1992, distributed by Molecular Simulations, Inc.; see, e.g., Blundell & Johnson, supra; Meth. Enzymol., vol. 114 & 115, H. W. Wyckoff et al., eds., Academic Press (1985)]. This information may thus be used to optimize known RORβ agonists/antagonists, and more importantly, to design new RORβ agonists/antagonists.

Accordingly, the present invention is also directed to a binding site in RORβ-LBD agonist or antagonist ligand in which a portion of RORβ-LBD ligand is in van der Waals contact or hydrogen bonding contact with at least one of the following residues: Q228, Y229, L234, W259, Q261, C262, A263, Q265, I266, H268, A269, L299, V303, L304, R306, M307, R309, A310, V318, L319, F320, E321, M329, F330, L333, L338, I339, A342, F343, V419, C420, H423, and Y446 of RORβ-LBD. For purposes of this invention, by RORβ-LBD binding site it is also meant to include mutants or homologues thererof. In a preferred embodiment, the mutants or homologues have at least 25% identity, more preferably 50% identity, more preferably 75% identity, and most preferably 95% identity to residues Q228, Y229, L234, W259, Q261, C262, A263, Q265, I266, H268, A269, L299, V303, L304, R306, M307, R309, A310, V318, L319, F320, E321, M329, F330, L333, L338, 1339, A342, F343, V419, C420, H423, and Y446 of RORβ-LBD binding sites.

The present invention is also directed to a machine readable data storage medium, comprising a data storage material encoded with machine readable data, wherein the data is defined by the structure coordinates of an RORβ-LBD/RORβ-LBD ligand according to Table A or a homologue of said complex, wherein said homologue comprises backbone atom that have a root mean square deviation from the backbone atoms of the complex of not more than 3.0 Å. Preferably, the machine readable data storage medium, according to the invention, is wherein said molecule or molecular complex is defined by the set of structure coordinates for RORβ-LBD/RORβ-LBD ligand according to Table A, or a homologue of said molecule or molecular complex, said homologue having a root mean square deviation from the backbone atoms of said aminoacids of not more than 2.0 Å. In a preferred embodiment the machine readable data storage medium comprises a data storage medium comprising a data storage material encoded with a first set of machine readable data comprising a Fourier transform of at least a portion of the structural coordinates for a RORβ-LBD/ RORβ-LBD ligand/ RORβ-LBD ligand peptide according to Table A; which, when combined with a second set of machine readable data comprising an X-ray diffraction pattern of a molecule or molecular complex of unknown structure, using a machine programmed with instructions for using said first set of data and said second set of data, can determine at least a portion of the structure coordinates corresponding to the second set of machine readable data, said first set of data and said second set of data.

The present invention also provides for computational methods using three dimensional models of the RORβ receptor that are based on crystals of RORβ-LBD/RORβ-LBD ligand complex. Generally the computational method of designing an RORβ ligand determines which amino acid or amino acids of the RORβ-LBD interact with a chemical moiety (at least one) of the ligand using a three dimensional model of a crystallized protein comprising the RORβ-LBD with a bound ligand, and selecting a chemical modification (at least one) of the chemical moiety to produce a second chemical moiety with a structure that either decreases or increases an interaction between the interacting amino acid and the second chemical moiety compared to the interaction between the interacting amino acid and the corresponding chemical moiety on the natural hormone.

The computational methods of the present invention are for designing RORβ synthetic ligands using such crystal and three dimensional structural information to generate synthetic ligands that modulate the conformational changes of the RORβ LBD. These computational methods are particularly useful in designing an antagonist or partial agonist to the RORβ, wherein the antagonist or partial agonist has an extended moiety that prevents any one of a number of ligand-induced molecular events that alter the receptor's influence on the regulation of gene expression, such as preventing the normal coordination of the activation domain observed for a naturally dccuring ligand or other ligands that mimic the naturally occuring ligand, such as an agonist. As described herein, synthetic ligands of the RORβ receptor will be useful in modulating RORβ activity in a variety of medical conditions. RORβ is known to comprise various domains as follows:
1) a variable amino-terminal domain;
2) a highly conserved DNA-binding domain (DBD); and
3) a less conserved carboxyl-terminal ligand-binding domain (LBD).

This modularity permits different domains of each protein to separately accomplish different functions, although the domains can influence each other. The separate function of a domain is usually preserved when a particular domain is isolated from the remainder of the protein. Using conventional protein chemistry techniques a modular domain can sometimes be separated from the parent protein. Using conventional molecular biology techniques each domain can usually be separately expressed with its original function intact or chimeraes of two different nuclear receptors can be constructed, wherein the chimerae retain the properties of the individual functional domains of the respective nuclear receptors from which the chimerae were generated.

### Amino-Terminal Domain

The amino-terminal domain is the least conserved of the three domains. This domain is involved in transcriptional activation and in some cases its uniqueness may dictate selective receptor-DNA binding and activation of target genes by specific receptors isoforms. This domain can display synergistic and antagonistic interactions with the domains of the LBD. For example, studies with mutated and/or deleted receptors show positive cooperativity of the amino and carboxy terminal domains.

In some cases, deletion of either of these domains will abolish the receptor's transcriptional activation functions.

### DNA-Binding Domain

The DBD is the most conserved domain. The DBD contains two perpendicularly oriented α-helices that extend from the base of the first and second zinc fingers. The two zinc fingers function in concert along with non-zinc finger residues to direct nuclear receptors to specific target sites on DNA and to align receptor homodimer to heterodimer interfaces. Various amino acids in DBD influence spacing between two half sites for receptor dimer binding.

### Ligand-binding domain

The LBD is the second most highly conserved domain. Whereas integrity of several different LBD sub-domains is important for ligand binding, truncated molecules containing only the LBD retain normal ligand-binding activity. This domain also participates in other functions, including dimerization, nuclear translocation and transcriptional activation. Importantly, this domain binds the ligand and undergoes ligand-induced conformational changes as detailed herein.

As described herein, the LBD of RORβ can be expressed, crystallized, its three dimensional structure determined with a ligand bound (either using crystal data from the same receptor or a different receptor or a combination thereof), and computational methods used to design ligands to its LBD, particularly ligands that contain an extension moiety that coordinates the activation domain of RORβ.

Once a computationaly designed ligand (CDL) is synthesized, it can be tested using assays to establish its activity as an agonist, partial agonist or antagonist, and affinity, as described herein. After such testing, the CDLs can be further refined by generating LBD crystals with a CDL bound to the LBD. The structure of the CDL can then be further refined using the chemical modification methods described herein for three dimensional models to improve the activity or affinity of the CDL and make a second generation CDLs with improved properties, such as that of a super agonist or antagonist.

Typically RORβ-LBD is purified to homogeneity for crystallisation. Purity of RORβ-LBD is measured with SDS-PAGE and mass spectrometry. The purified RORβ for crystallization should be at least 97.5% pure or 97.5% pure, preferably at least 99.0% pure or 99.0% pure, more preferably at least 99.5% pure or 99.5% pure.

Initially purification of the receptor can be obtained by conventional techniques, such as affinity chromatography and gel filtration chromatography.

To achieve higher purification for improved crystals of RORβ, it will be desirable to ligand shift purify the nuclear receptor using a column that separates the receptor according to charge, such as an ion exchange or hydrophobic interaction column, and then bind the eluted receptor with a ligand, especially an agonist. The ligand induces a change in the receptor's surface charge such that when rechromatographed on the same column, the receptor then elutes at the position of the liganded receptor and is removed by the original column run with the unliganded receptor. Usually saturating concentrations of ligand are used in the column and the protein can be preincubated with the ligand prior to passing it over the column.

Some developed methods involve engineering a "tag" such as with histidine placed on the end of the protein, such as on the amino terminus, and then using a cobalt chelation column for purification, Chaga, G., Biotech. Appl. Biochem. 29: 13811-13814 (1991) incorporated by reference.

To determine the three dimensional structure of a RORβ-LBD, it is desirable to co-crystallize the LBD with a corresponding LBD ligand.

Typically purified RORβ-LBD is equilibrated at a saturating concentration of ligand at a temperature that preserves the integrity of the protein. Ligand equilibration can be established between 2 and 37°C, although the receptor tends to be more stable in the 2-20°C range.

However if the ligand is unknown it is possible to co-crystallize the RORβ-LBD with a fortuitous ligand coming from the heterologous expression system i.e. *Escherichia coli* and by adding concomitantly a co-acdvator peptide.

Preferably crystals are made with the hanging drop methods. Regulated temperature control is desirable to improve crystal stability and quality. Temperatures between 4 and 25°C are generally used and it is often preferable to test crystallization over a range of temperatures. It is preferable to use crystallization temperatures from 18°C to 25°C, more preferably 20 to 23°C, and most preferably 22°C.

Ligands that interact with RORβ can act as agonists, antagonists and partial agonists based on what ligand-induced conformational changes take place.

Agonists induce changes in receptors that place them in an active conformation that allows them to influence transcription, either positively or negatively. There may be several different ligand induced changes in the receptor's conformation.

Antagonists, bind to receptors, but fail to induce conformational changes that leads to the receptor's transcriptionally active form or physiologically relevant conformations. Binding of an antagonist can also block the binding and therefore the actions of an agonist.

Partial agonists bind to receptors and induce only part of the changes in the receptors that are induced by agonists. The differences can be qualitative or quantitative. Thus, a partial agonist may induce some of the conformation changes induced by agonists, but not others, or it may only induce certain changes to a limited extent.

As described herein, the unliganded receptor is in a configuration that is either inactive, has some activity or has repressor activity. Binding of agonist ligands induces conformational changes in the receptor such that the receptor becomes more active, either to stimulate or repress the expression of the genes. The receptors may also have non-genomic actions, some of the known types of changes and/or the sequences of these are listed herein.

Ligand binding by the receptor is a dynamic process, which regulates receptor function by inducing an altered conformation.

The three-dimensional structure of the liganded RORβ receptor will greatly aid in the development of new RORβ synthetic ligands. In addition, RORβ is overall well suited to modern methods including three dimensional structure elucidation and combinatorial chemistry such as those disclosed in EP 335 628, U.S. patent 5, 463,564, which are incorporated herein by reference. Computer programs that use crystallographic data when practising the present invention will enable the rational design of ligand to RORβ.

Programs such as RASMOL can be used with the atomic coordinates from crystals generated by practicing the invention or used to practice the invention by generating three dimensional models and/or determining the structures involved in ligand binding. Computer program such as INSIGHT and GRASP allow further manipulation and the ability to introduce new structures. In addition, high throughput binding and bioactivity assays can be devised using purified recombinant protein and modern reporter gene transcription assays described herein and known in the art in order to refine the activity of a CDL.

Generally, the computational method of designing a RORβ synthetic ligand comprises two steps:
1) determining which amino acid or amino acids of RORβ-LBD interacts with a first chemical moiety (at least one) of the ligand using a three dimensional model of a crystallized protein comprising an RORβ-LBD with a bound ligand; and
2) selecting a chemical modifications (at least one) of the first chemical moiety to produce a second chemical moiety with a structure to either increase or decrease an interaction between the interacting amino acid and the second chemical moiety compared to the interaction between the interacting amino acid and the first chemical moiety.

Preferably the method is carried out wherein said three dimensional model is generated by comparing isomorphous ligand derivatives to produce improved phasing. Also preferred is wherein said selecting uses said first chemical moiety that interacts with at least one of the interacting amino acids_Q228, Y229, L234, W259, Q261, C262, A263, Q265, I266, H268, A269, L299, V303, L304, R306, M307, R309, A310, V318, L319, F320, E321, M329, F330, L333, L338,1339, A342, F343, V419, C420, H423 or Y446.

As shown herein, interacting amino acids form contacts with the ligand and the center of the atoms of the interacting amino acids are usually 2 to 4 angstroms away from the center of the atoms of the ligand. Generally these distances are determined by computer as discussed herein and in Mc Ree 1993, however distances can be determined manually once the three dimensional model is made. See also Renaud et al., Nature 378, 681-689 (1995) for stereochemical figures of three dimensional models.

More commonly, the atoms of the ligand and the atoms of interacting amino acids are 3 to 4 angstroms apart. The invention can be practiced by repeating step 1 and 2 to refine the fit of the ligand to the LBD and to determine a better ligand, such as an agonist. The three dimensional model of RORβ can be represented in two dimensions to determine which amino acids contact the ligand and to select a position on the ligand for chemical modification and changing the interaction with a particular amino acid compared to that before chemical modification. The chemical modification may be made using a computer, manually using a two dimensional representation of the three dimensional model or by chemically synthesizing the ligand. The ligand can also interact with distant amino acids after chemical modification of the ligand to create a new ligand. Distant amino acids are generally not in contact with the ligand before chemical modification. A chemical modification can change the structure of the ligand to make a new ligand that interacts with a distant amino acid usually at least 4.5 angstroms away from the ligand, preferably whereinsaid first chemical moiety is 6 to 12 angstroms away from a distant amino acid. Often distant amino acids will not line the surface of the binding activity for the ligand, they are too far away from the ligand to be part of a pocket or binding cavity. The interaction between a LBD amino acid and an atom of an LBD ligand can be made by any force or attraction described in nature. Usually the interaction between the atom of the amino acid and the ligand will be the result of a hydrogen bonding interaction , charge interaction, hydrophobic interaction, van der Waals interaction or dipole interaction. In the case of the hydrophobic interaction it is recognized that is not a per se interaction between the amino acid and ligand, but rather the usual result, in part, of the repulsion of water or other hydrophilic group from a hydrophobic surface. Reducing or enhancing the interaction of the LBD and a ligand can be measured by calculating or testing binding energies, computationally or using thermodynamic or kinetic methods as known in the art.

Chemical modifications will often enhance or reduce interactions of an atom of a LBD amino acid and an atom of an LBD ligand. Steric hindrance will be a common means of changing the interaction of the LBD cavity with the activation domain.

The present invention also provides methods for identifying compounds that modulates RORβ activity. Various methods or combination thereof can be used to identify these compounds. For example, test compounds can be modeled that fit spatially into the RORβ-LBD as defined by structure coordinates according to Table A, or using a three-dimensional structural model of RORβ-LBD, mutant RORβ-LBD, or RORβ-LBD homolog or portion thereof. Structure coordinates of the ligand binding site, in particular amino acids Q228, Y229, L234, W259, Q261, C262, A263, Q265, 1266, H268, A269, L299, V303, L304, R306, M307, R309, A310, V318, L319, F320, E321, M329, F330, L333, L338,1339, A342, F343, V419, C420, H423, or Y446 can also be used to identify structural and chemical features. Identified structural or chemical features can then be employed to design or select compounds as potential RORβ modulators. By structural and chemical features it is meant to include, but is not limited to, van der Waals interactions, hydrogen bonding interactions, charge interaction, hydrophobic bonding interaction, charge interaction, hydrophobic interaction and dipole interaction. Alternatively, or in conjunction, the three-dimensional structural model or the ligand binding site can be employed to design or select compounds as potential RORβ modulators. Compounds identified as potential RORβ modulators can then be synthesized and screened in an assay characterized by binding of a test compound to the RORβ-LBD. Examples of assays useful in screening of potential ROR modulators include, but are not limited to screening in silico, *in vitro* assays and high throughput assays. Finally, these methods may also involve modifying or replacing one or more amino acids from RORβ-LBD such as Q228, Y229, L234, W259, Q261, C262, A263, Q265, I266, H268, A269, L299, V303, L304, R306, M307, R309, A310, V318, L319, F320, E321, M329, F330, L333, L338, I339, A342, F343, V419, C420, H423, or Y446 of RORβ-LBD according to Table A.

A preferred method of the invention can be described as a computational method of designing an ROR antagonist from an ROR receptor agonist comprising:
1) determining a structure of a molecular recognition domain of said agonist using a three dimensional model of a crystallized protein comprising an RORLBD, and
2) selecting at least one chemical modification of said agonist that provides a ligand structure that extends beyond a binding site for said agonist and in the direction of at least one protein domain important in RORβ biological function.

Another preferred method of the invention can be described as a computational method of designing a selective RORβ receptor modulator such as a ROR receptor super agonist or antagonist comprising:
1) determining at least one interacting amino acid of an RORβ-LBD that interacts with at least one first chemical moiety of said ligand using a three dimensional model of a crystallized protein comprising RORβ-LBD with a bound ligand, and
2) selecting at least one chemical modification of said first chemical moiety to produce a second chemical moiety with a structure to reduce or enhance an interaction between said interacting amino acid and said second chemical moiety compared to said interaction between said interacting amino acid and said first chemical moiety.

However, as will be understood by those of skill in the art upon this disclosure, other structure based design methods can be used. Various computational structure based design methods have been disclosed in the art.

For example, a number computer modeling systems are available in which the sequence of the RORβ-LBD and the RORβ-LBD structure ( i.e., atomic coordinates of RORβ-LBD and/or the atomic coordinates of the ligand binding site, the bond and dihedral angles, and distances between atoms in the active site such as provided in Table A) can be input. This computer system then generates the structural details of the site in which a potential RORβ modulator binds so that complementary structural details of the potential modulators can be determined. Design in these modeling systems is generally based upon the compound being capable of physically and structurally associating with RORβ-LBD. In addition, the compound must be able to assume a conformation that allows it to associate with RORβ-LBD. Some modeling systems estimate the potential inhibitory or binding effect of a potential ROR modulator prior to actual synthesis and testing.

Methods for screening chemical entities or fragments for their ability to associate with RORβ-LBD are also well known. Often these methods begin by visual inspection of the active site on the computer screen. Selected fragments or chemical entities are then positioned with the RORβ-LBD. Docking is accomplished using software such as QUANTA and SYBYL, following by energy minimization and molecular dynamics with standard molecular mechanic forcefields such as CHARMM and AMBER. Examples of computer programs which assist in the selection of chemical fragment or chemical entities useful in the present invention include, but are not limited to, GRID (Goodford, P. J.J. Med. Chem. 1985 28: 849-857), AUTODOCK (Goodsell, D.S. and Olsen, A.J. Proteins, Structure, Functions, and Genetics 1990 8: 195-202), and DOCK (Kunts et al. J. Mol. Biol. 1982 161:269-288).

Upon selection of preferred chemical entities or fragments, their relationship to each other and RORβ-LBD can be visualized and then assembled into a single potential modulator. Programs useful in assembing the individual chemical entities include, but are not limited to CAVEAT (Bartlett et al. Molecular Recognition in Chemical and Biological Problems Special Publication, Royal Chem. Soc. 78, 00. 182-196 (1989) and 3D Database systems (Martin, Y.C. J. Med.Chem. 1992 35:2145-2154).

Alternatively, compounds may be designed de novo using either an empty active site or optionaly including some portion of a known inhibitor. Methods of this type of design include, but are not limited to LUDI (Bohm H-J, J. Comp. Aid. Molec. Design 1992 6:61-78) and LeapFrog (Tripos Associates, St. Louis MO).

The present invention is also directed to a RORβ-LBD selective RORβ modulator (SRORM), in particular an agonist or antagonist, identified by a computational process of the invention.
The present invention is further directed to a method for treating a ROR related disease comprising administering an effective amount of an antagonist identified by a computational process of the invention.

The present invention is also direct to a method for treating a ROR related disease comprising administering an effective amount of an agonist identified by a computational process of the invention.

Compounds identified as agonists, antagonists or SRORMs by the methods disclosed herein which are active when given orally can be formulated as liquids for example syrups, suspensions or emulsions, tablets, capsules and lozenges. A liquid composition will generally consist of a suspension or solution of the compound in a suitable liquid carrier(s), for example ethanol, glycerin, sorbitol, non-aqueous solvent such as polyethlene glycol, oils or water, with a suspending agent, preservative, surfactant, wetting agent, flavoring or coloring agent.

Alternatively, a liquid formulation can be prepared from a reconstitutable powder. For example a powder containing active compound, suspending agent, sucrose and a sweetener can be reconstituted with water to form a suspension; a syrup can be prepared from a powder containing active ingredient, sucrose and a sweetener. A composition in the form of a tablet can be prepared from a powder containing active ingredient, sucrose and a sweetener. A composition in the form of a tablet can be prepared using any suitable pharmaceutical carrier(s) routinely used for preparing solid compositions. Examples of such carriers include magnesium stearate, starch, lactose, sucrose, microcrystalline cellulose, binders, for example polyvinylpyrrolidone. The tablet can also be provided witha color film coating, or colorincluded as part of the carrier(s). In addition, active compound can be formulated in a controlled release dosage form as a tablet comprising a hydrophilic or hydrophobic matrix. A composition in the form of a capsule can be prepared using routine encapsulation procedures, for example by incorporation of active compound and excipients into a hard gelatin capsule. Alternatively, a semi-solid matrix of active compound and high molecular weight polyethylene glycol can be prepared and filled into a hard gelatin capsule; or a solution of active compound in polyethylene glycol or a suspension in edible oil, for example liquid paraffin or fractionated coconut oil can be prepared and filled into a soft gelatin capsule. Compounds identified by the processes described herein which are active when given parenterally can be formulated for intramuscular or intravenous administration. A typical composition for intra-muscular administration will consist of a suspension or solution of active ingredient in an oil, for example arachis oil or sesame oil. A typical composition for intravenous administration will consist of a sterile isotonic aqueous solution containing , for example active ingredient, dextrose, sodium chloride, a cosolvent, for example polyethylene glycol and, optionally, a chelating agent, for example, sodium metabisulphite.

Alternatively, the solution can be freeze dried and then reconstituted with a suitable solvent just prior to administration. Identified compounds which are active on rectal administration can be formulated as suppositories. A typical suppository formulation will generally consist of active ingredient with a binding and/or lubricating agent such as a gelatin or cocoa butter or other low melting vegetable or synthetic wax or fat. Identified compounds which are active on topical administration can be formulated as transdermal compositions. Such compositions include, for example, a backing, active compound reservoir, a control membrane, liner and contact adhesive. The typical daily dose of a varies according to individual needs, the condition to be treated and with the route of administration. Suitable doses are in the general range of 0.001 to 10mg/kg bodyweight of the recipient per day.

The following examples are to illustrate the invention, but should not be interpreted as a limitation thereon.

### Examples

### Cloning, Expression and Purification of the RORβ Ligand-Binding Domain

A cDNA for expression of the ligand-binding domain of the rat RORβ-LBD (RORβ-LBD) was constructed using the pet15b vector (Novagen) to include an N-terminal polyhistidine tag and a thrombin cleavage site. *E. coli* BL21 (DE3) cells were grown in LBM at 37 °C to an OD 0.6 and induced with 0.8mM IPTG. The incubation was maintained at 16 °C overnight. Cells were harvested ans stored at -20°C. A total of 6-9 mg of recombinant RORβ-LBD was isolated from a 6 gram cell pellet following sonication and chromatography on a cobalt-chelate resin. Polyhistidine-tagged RORβ-LBD of approximately 90% purity eluted in a gradient of 0 to 1M imidazole. Gel filtration was performed with a Superdex S-200 Hiload 16:60 from Pharmacia. Polyhistidine-tagged rRORβ-LBD of more than 95% purity and homogeneity as checked by SDS-PAGE was concentrated to 5.8 mg/ml in 20mM TrisHCl pH=8.5, 5mMDTT, 2 mM Chaps and 100mM NaCl.

### Crystallization

The RORβ-LBD stearate complex was crystallized at 22°C by vapor diffusion in the hanging-drop mode. In the crystallisation trials, the protein was used without further purification and co-crystallized with a 3-molar excess of SRC-1⁶⁸⁶⁻⁷⁰⁰ (RHKILHRLLQEGSPS) NR-interacting peptide co-activator sequence. Addition of the peptide was crucial to obtain crystals.In the initial trial to obtain crystallization conditions, a sparse matrix crystallization screen was done with a home screen. For each crystallization trial, a 4 µl drop was prepared by mixing 2 µl of purified protein (5.8 mg/ml) with an equal volume of reservoir solution. The reservoir contained 500 µl of the precipitating solution. A crystal measuring 110 x 60 x 30 mm at 22 °C in PEG 6000 15% and 100 mM Tris HCl at pH=8.0 grew within about 2 weeks. This crystal was used in a first data collection run ( as described below).

### Data Collection and Reduction

The crystals were cryoprotected by equilibration in 15 % PEG 6000 at pH 8.0 containing 15 % glycerol and then flash frozen in liquid ethane at liquid nitrogen temperature. X-ray diffraction data were collected at liquid nitrogen temperature from a single frozen crystal at the ID 14-3 beamline at the ESRF Grenoble, France. Crystals diffracted Xrays to a resolution limit of 1.9 Å. All data were integrated and scaled using DENZO and SCALEPACK ( Otwinowski and Minor, 1997). The data set between 30 and 3.4 Å shows a completeness of 88.9 % resolution with an Rsym (I) of 2.5. The completeness on high resolution ( between 3.4 and 1.9) was 99.8 % with an Rsym (I) of 3.3. The unit cell parameters were a= 52.302 Å b= 58.490 Å and c= 106.036 Å, a=b=c=90°. The crystal was composed by one monomer per asymetric unit, has a solvent content of 52%, and one molecule per asymmetric unit (Mathews Volume = 2.57 Å³ Da⁻¹) The estimated B factor by Wilson plot is 29. Inspection of systematic absences along each axis indicated that the space group was orthorhombic P212121.

**Table 1: Data Collection and Processing**

| | |
|---|---|
| Source | Grenoble ID14-3 |
| No of crytals | 1 |
| Wavelength | 0.93100 Å |
| Frames | 331 |
| ΔΦ | 1° |
| Crystal to plate distance | 260mm |
| Time/frame | 5sec(low res);20sec (high res) |
| Number of Observations | 28846 |
| Data Reduction Program | HKL |
| Unique reflections | 26336 |
| Reflections Used | 26331 |
| Resolution | 30.0-1.9 |
| Completeness | 100 |
| Multiplicity | 1 |
| Mosaicity | 0.5 |
| ^{a}Rsym | 3.6 % (15%) |
| Space group | P212121 |
| a | 52.302 Å |
| b | 58.490 Å |
| c | 106.036 Å |
| Wilson B-value | 28.78 Å² |

In the data collection, the last shell values are presented between parentheses.

### Structure Determination (Molecular Replacement)

The structure of the complex was solved by molecular replacement using the program AmoRe (Navaza, 1994) and the RARγ holo-LBD (Protein Data Bank accession code, 21bd) as a search model. The top solution had a correlation coefficient of 27.8 ( next highest solution 26.2) and an R factor of 52.7 after AMoRe rigid-body refinement. A solution could also be found with RAR anta as a search model according to the following values: correlation coefficient of 21.4 ( next highest solution 19.8) and an R factor of 55.9.

**Table 2: Molecular Replacement Statistics**

| | |
|---|---|
| Search Model: | holo-RARγ |
| | (PDB file 2LBD) |
| Program used | AMoRe |
| Resolution Range | 15-3.0 Å |
| Number of Reflections | 4338 |
| Number of Atoms | 2011 |
| RF Correlation (first solution) | 18.8 |
| TF Correlation (first solution) | 24.3 |
| TF R-factor (first solution) | 57.0 |
| Rigid Body Correlation | 26.0 |
| Rigid Body R-factor | 56.7 |

### Structure Refinement

The automated model building Arp/wARP (Perrakis A. et al., 1999) combined with iterative structure refinement was used and permit us to obtain 3 chains constructed which correspond to 243 residues and a connectivity index of 0.98. The calculated electron density maps 3Fo-2Fc delivered Rcryst= 0.2703 and a Rfree = 0.2644.
A partial model of the monomer was build using the graphic program O ( Jones et al., 1991) and subjected to alternating rounds of rigid body refinement with X-PLOR (Brünger, 1996) and manual building. The final steps, using cycles of positional refinement, manual rebuilding, the torsion slow-cool procedure from the program X-PLOR, and individual isotropic B factor refinement delivered Rcryst = 0.2238 and a Rfree =0.2494. The final model refined at 1.9 Å, comprises 244 residues, one ligand, a peptide of 10 amino-acid residues, and 146 water molecules. According to Procheck (Laskowski et al., 1993) 93.2% of all residues in the model are in the most favoured main chain torsion angles Ramachandran regions, 5.9% in addtional allowed regions, 0.4% in generously allowed regions and 0.4% are in disallowed regions. These last percentages corresponds to two residues (D403 and E404) situated in the loop 9-10, which did not present a well defined density.

**Table 3: Final Refinement Parameters**

| | | |
|---|---|---|
| Resolution Range | | 30.0-1.9 |
| Reflections | | 26331 |
| R-factor | | 22.4% |
| R-free | | 24.9% |
| # residues | | 201-452 (207) |
| # atoms | | 1977 |
| RMS deviations | | |
| | bond lengths | 0.008 |
| | bond angles | 1.282 |
| Average B-factors | | |
| Protein | | 29.5 Å² |
| Stearate | | 47.9 Å² |
| Water | | 39.8 Å² |

### Description of the Molecule

The structure of RORβ-LBD is complete from residues 208 through 451.
Analysis of the structure with program Procheck showed only minor exeptions to the allowed geometry. In the structure, the first seven residues of the chain (201-207) are not seen in the electron density map and are probably disordered. This leaves only one residue before the initial residue of the first α-helix (H1) in the wild-type structure. On the C-terminal end, only the last residue (452) is not seen in the electron density map. The loop between helices H9 and H10 (residues N399 and E405) is not well defined.

### Folding and packing

As expected, the RORβ-LBD has the same overall three-dimensional structure as those of the other nuclear hormone receptor LBDs. The molecule is folded into a "helical sandwich" consisting of 10 α-helices. There are two small pieces of beta strand, forming a short beta sheet located in the core of the molecule between helices 5 and 6 near the ligand binding site. Helix 12 is folded toward the ligand binding domain core. Its last turn comes in close contact to H4, H11 and the co-activator peptide. An interaction surface comprising residues from the H3-H4 region and H12 allows the co-activator peptide to bind.
The following sequence of the peptide is seen in the crystal structure: HKILHRLLQE. The LXXLL motif also called the NR-box is included in an amphipathic α-helix interacting with a hydrophobic cleft on the LBD surface. In particularly, the side-chains of Leu 693 and Leu 694 are part of an hydrophobic cluster composed also by Val 274 (H3), Ile 292 (H4), and Leu 295 (H4).
The γ carboxylate of E448 (H12) forms hydrogen bonds with the backbone amides of Leu 697 and Leu 698, residues of N-terminal turn of the peptide helix. This N-capping interaction was already described (Nolte & al., 1998, Shiau et al., 1998). This highly conserved glutamate residue is known to be important for transactivation. Another hydrogen bond requires Gin 288 (H4) OE2 with NE2 of His 695. The side chain of Glu 700 forms a water-mediated hydrogen bond to the carbonyl of residue Arg 696.

### Binding of stearate

The volume of the ligand binding pocket is 758 Å³, which is close to that of VDR (660 Å³) (Rochel et al., 2000). A fortuitous ligand, stearic acid, was found in the ligand binding pocket, which was previously characterized by mass spectrometry. Thus, it appears that E. coli-endogeneous stearic acid was co-purified and co-crystallised with the heterologously expressed RORβ LBD. The fatty acid (FA) is buried in a predominantly hydrophobic pocket formed by residues located in H3 (Gin 265, Ile 266, Ala269), H5 (Leu 300, Val303, Leu97), loop H5-H6 ( Phe 113), H6 (Phe 320), and H7 (Leu131, Val338). Most of these residues make van der Waals contact with the aliphatic chain of the FA (Figure 3a). The cavity contains also 11 ordered water molecules. One oxygen atom of the carboxylate group forms hydrogen bond with NE2 of Gln265. This residue varies among RORα and β. The other oxygen atom of the carboxylate group forms hydrogen bond with two ordered water molecules. These two molecules are part of a hydrogen bond network, which connects the carboxylate to other conserved residues among RORα and β of the LBP namely Gln228 and Arg306. Stearate adopts a U-shaped conformation upon binding.

**Table 4: Stearate contacts (3.5 Å)**

| Hydrogen bonds | | | | | | |
|---|---|---|---|---|---|---|
| 02 | Gln 265 Nε2 | 2.79Å | | | | |
| O1 | Wat 944 | | 2.54Å | O | Val 303 | 3.11Å |
| | | | | | | |
| Possible Close Contacts | | | | | | |
| O1 | Ala 269 Cβ | | 3.31Å | | | |
| 02 | Gln 265 Cγ | | 3.33Å | | | |
| 02 | Gln 265 Cδ | | 3.41Å | | | |
| 02 | Wat 946 | | 3.06 Å | | | |
| C3 | Wat 944 | | 3.43 Å | | | |
| C3 | Wat 946 | | 3.21 Å | | | |
| C4 | Wat 946 | | 3.30 Å | | | |
| C10 | Phe 123 CE1 | 3.43 Å | | | | |
| C11 | Leu 131 CD2 | 3.45 Å | | | | |

### crystallization and structure determination of the RORβ LBD/ATRA (all-trans retinoic acid) complex

As mentioned above, the RORβ LBD construct where the two C-terminal solvent-exposed cysteines have been removed by truncation of a 7-residue C-terminal segment has proved a valuable tool to get other crystal structures of the RORβ LBD in complex with other ligands. This is illustrated below with the description of the crystallization and the structure determination of the RORβ LBD/ATRA (all-*trans* retinoic acid) complex. This new structure reveals another mode of binding for the ligand and suggests that natural and synthetic retinoids are candidate ligands for RORβ. This family of compounds may thus be tested for binding to the RORβ LBD, for instance by mass spectrometry, and the crystallization may be tried in the positive cases. From the obtained structures, high-affinity ligands can be designed, synthetized, and tested *in vivo, in vitro,* as well as for crystallization. Even without the crystal structure of other complexes, filtering for ligand screening and/or design of better ligands can be achieved through docking studies *in computo.*

### Crystallization of the RORβ LBD/ATRA complex

The RORβ LBD/ATRA complex was crystallized by vapor diffusion in the hanging-drop mode. The protein (the RORβ LBD containing stearic acid, purified as previously) was co-crystallised with an excess ATRA and an excess SRC-1 (residues 686-700) under similar conditions as for the RORβ LBD/STE (stearic acid) complex. A 4 µl drop was prepared by mixing 2 µl of purified protein (5.8 mg/ml) with an equal volume of reservoir solution. The reservoir (500 µl) contained 18% PEG 6000 and 100 mM Tris HCl pH=8.0. A crystal measuring 300x 160 x 100 µm grew within 2 weeks at 22°C.

### Data Collection

The crystals were cryoprotected with a film of viscous paraffin oil and then flash frozen in liquid ethane at liquid nitrogen temperature. X-ray diffraction data were collected at liquid nitrogen temperature from a single frozen crystal at the BM14-CRG beamline at the ESRF Grenoble, France. Crystals diffracted X-rays to a resolution limit of 2.1 Å. All data were integrated and scaled using DENZO and SCALEPACK (Table 5). The data set between 20.0 and 2.1 Å shows a completeness of 100 % with an Rsym (I) of 4.5%. The completeness in the highest resolution shell (2.17-2.10 Å) was 100 % with an Rsym (I) of 17.5%. The unit cell parameters were a=52.199Å, b=58.125Å, and c=106.039Å, a=b=c=90°. The crystal contains one monomer per asymetric unit and a solvent content of 52%. The estimated B factor by Wilson plot is 32. Inspection of systematic absences along each axis indicated that the space group was P212121.

### Structure Determination and Refinement

The structure of the complex was solved by molecular replacement using the RORβ LBD/STE complex as a starting model. The all-*trans* retinoic acid was built using Quanta (MSI). The final model (Rcryst = 0.2180 and Rfree =0.2549), refined at 2.1 Å (Table 5), comprises 244 residues from the RORβ LBD, 10 residues from the peptide, one ligand, and 139 water molecules. According to Procheck, 91.2% of all residues in the model are in the most favoured main chain torsion angles Ramachandran regions, 5.9% in additional allowed regions, 2.1% in generously allowed regions and 0.0% in disallowed regions.

### Binding of ATRA (all-trans retinoic acid)

The protein-ligand contacts within 3.5 Å are listed in Table 6. The present structure reveals the binding site for the carboxylate group of ATRA, which is hydrogen-bonded to Arg 306 and Arg 309 through a water molecule in each case (Figure 6). The binding mode is different from that of stearate (Figure 7), which is hydrogen-bonded to Gln 265 directly and to Gln 228 through a water molecule. On the other hand, stearate makes more Van der Waals contacts with pocket residues thanks to its flexible chain which assumes a U shape probably in order to maximize the number of such contacts. ATRA is more rigid, allowing less Van der Waals contacts. Thus, there seems to be a delicate balance between Van der Waals contacts and hydrogen bonds ligand binding to the RORβ LBD.

**Table 5: Data collection and refinement statistics.**

| | |
|---|---|
| Source | ESRF BM14 |
| Wavelength | 0.976205 Å |
| Unique reflections | 19431 |
| Resolution range | 20.0- 2.1 |
| ^{a}Completeness | 100 % (100 %) |
| Multiplicity | 6.6 |
| Mosaicity | 0.75° |
| ^{a}Rsym | 4.5 % (17.5%) |
| Space group | P212121 |
| a | 52.199 Å |
| b | 58.125 Å |
| c | 106.039 Å |

| | |
|---|---|
| ^{a}The last shell values are presented between parentheses. | |

| | | |
|---|---|---|
| Resolution Range | | 20.0-2.1 |
| Reflections | | 17679 |
| R-factor | | 21.8% |
| R-free | | 25.5% |
| # visible residues | | 244 (residues 208-451) |
| # atoms | | 2219 |
| RMS deviations | | |
| | bond lengths | 0.007 |
| | bond angles | 1.129 |
| Average B-factors | | |
| Protein and peptide | | 33.6 Å² |
| All-trans retinoic acid | | 40.3 Å² |
| Water | | 43.2 Å² |
| Wilson B-factor | | 31.9 Å² |

**Table 6: RORβ LBD / ATRA (all-trans retinoic acid) contacts within 3.5 Å**

| Hydrogen bonds | | |
|---|---|---|
| O1 | N Gln 228 | 2.97 Å |
| O1 | Wat 802 | 2.72 Å |
| O2 | Wat 825 | 2.59 Å |
| NH1 | Arg 306 | 2.92 Å |
| NH1 | Arg 309 | 2.84 Å |
| | | |
| Van der Waals contacts | | |
| O1 | Arg 306 Cδ | 3.50 Å |
| 02 | Tyr 229 N | 3.42 Å |
| 02 | Gln 228 N | 3.42 Å |
| | | |
| C15 | Gin 228 N | 3.48 Å |
| C15 | Wat 825 | 3.39 Å |
| C18 | Cys 262 Cβ | 3.42 Å |
| C18 | Cys 262 Sγ | 3:44 Å |
| C19 | Leu 319 O | 3.34 Å |
| C20 | Wat 825 | 3.22 Å |

### Cell Culture and Transient Transfection Experiments.

HT22 were cultured in in Dulbecco's modified Eagle's medium (DMEM). The Medium was supplemented with 5% delipidated fetal calf serum, penicillin, streptomycin and glutamine. Transient transfection assays were carried out in 24-well plates (0.5 10⁵ cells per well) *N*-[1-(2,3-dioleoyloxy)propyl]-*N,N,N*-trimethylammoniummethylsulfate (DOTAP) lipofection (Roche Molecular Biochemicals) according to the manufacturer's protocol. Luciferase activity was assayed as recommended by the manufacturer (Promega) in a Microplate Luminometer (EG & G Berthold). Relative light units were normalized according to (Muller et al., 2002) and protein concentration was determined using the Bradford dye assay (Bio-Rad). All experiments were repeated at least five times.

**Ligands.** Purchased ligands include the following: *all*-*trans*-[20-methyl-3H]-retinoic acid (65 Ci/mmol) (NEN); *all*-*trans*-retinoic acid (Sigma)
**Recombinant plasmids.** *Reporter plasmids.* G5E1BTataLuc
*Expression vectors.* CMX-Gal, CMX-Gal-RORβ201-459, pGEX-RORβ201-459 described in (Greiner et al., 1996)

**Ligand binding assays.** Scintillation proximity assay were performed with purified bacterial expressed RORβ-LBD (stehlin et al 2001) (250 ng per well) and *all-trans*-[20-methyl-3H]-retinoic acid (60 Ci/mMol, NEN) in 96-well NiNTA-flash-plates (NEN) in a total volume of 100 µl. Binding buffer: 40 mM HEPES pH 7.6, 40 mM KCl, 0.2 % CHAPS, 0.1 mg/ml BSA. Binding was carried out for 1 hour at 4°C in 100 µl binding buffer. Radioligand was diluted in binding buffer to a final concentration of 5 nM. Unlabelled competing ligands were serially diluted in binding buffer and added at final concentrations ranging from 1 nM to 10 µM. Plates were shaken at 25°C for 2 hours. Then the radioactivity was measured with a Packard Topcount at 2 min per well. All concentrations were assayed in triplicate and the results were averaged. Values from wells void of competitor represented 100% binding. Saturation-binding experiments used the ligand concentrations indicated in the figure. Nonspecific binding was determined by including unlablled retinoic acid at 10⁻⁴ M and subtracted from total binding. Nonlinear regression analysis for the competition curves, saturation binding and Scratchard analysis to determine the Kd were performed using GRAPHPAD PRISM.

Greiner, E. F., Kirfel, J., Greschik, H., Dorflinger, U., Becker, P., Mercep, A., and Schule, R. (1996). Functional analysis of retinoid Z receptor beta, a brain-specific nuclear orphan receptor. Proc Natl Acad Sci U S A 93, 10105-10110. Muller, J. M., Metzger, E., Greschik, H., Bosserhoff, A. K., Mercep, L., Buettner, R., and Schule, R. (2002). The transcriptional coactivator FHL2 transmits Rho signals from the cell membrane into the nucleus. Embo J 21, 736-748.

**TABLE A: Crystallographic Coordinates of RORbeta LBD/stearic acid/SRC1 peptide complex**

| | | | | | | | | | |
|---|---|---|---|---|---|---|---|---|---|
| ATOM | 1 | CB | THR | 208 | 14.051 | -0.802 | 26.838 | 1.00 | 36.55 |
| ATOM | 2 | OG1 | THR | 208 | 15.478 | -0.898 | 26.824 | 1.00 | 35.48 |
| ATOM | 3 | CG2 | THR | 208 | 13.533 | -0.599 | 25.404 | 1.00 | 36.96 |
| ATOM | 4 | C | THR | 208 | 14.780 | 1.393 | 27.790 | 1.00 | 35.14 |
| ATOM | 5 | O | THR | 208 | 15.234 | 1.884 | 26.757 | 1.00 | 33.86 |
| ATOM | 6 | N | THR | 208 | 12.302 | 0.901 | 27.332 | 1.00 | 33.90 |
| ATOM | 7 | CA | THR | 208 | 13.631 | 0.366 | 27.747 | 1.00 | 34.84 |
| ATOM | 8 | N | MET | 209 | 15.261 | 1.697 | 28.997 | 1.00 | 35.20 |
| ATOM | 9 | CA | MET | 209 | 16.358 | 2.647 | 29.169 | 1.00 | 34.91 |
| ATOM | 10 | CB | MET | 209 | 16.717 | 2.770 | 30.658 | 1.00 | 34.61 |
| ATOM | 11 | CG | MET | 209 | 17.482 | 4.050 | 31.032 | 1.00 | 35.43 |
| ATOM | 12 | SD | MET | 209 | 16.609 | 5.601 | 30.589 | 1.00 | 35.84 |
| ATOM | 13 | CE | MET | 209 | 15.558 | 5.830 | 32.022 | 1.00 | 33.07 |
| ATOM | 14 | C | MET | 209 | 17.598 | 2.235 | 28.346 | 1.00 | 34.50 |
| ATOM | 15 | O | MET | 209 | 18.313 | 3.100 | 27.813 | 1.00 | 34.32 |
| ATOM | 16 | N | SER | 210 | 17.846 | 0.930 | 28.225 | 1.00 | 32.92 |
| ATOM | 17 | CA | SER | 210 | 18.999 | 0.456 | 27.461 | 1.00 | 33.75 |
| ATOM | 18 | CB | SER | 210 | 19.181 | -1.064 | 27.604 | 1.00 | 34.40 |
| ATOM | 19 | OG | SER | 210 | 18.057 | -1.784 | 27.107 | 1.00 | 37.86 |
| ATOM | 20 | C | SER | 210 | 18.886 | 0.821 | 25.983 | 1.00 | 32.54 |
| ATOM | 21 | O | SER | 210 | 19.888 | 1.145 | 25.345 | 1.00 | 32.59 |
| ATOM | 22 | N | GLU | 211 | 17.684 | 0.746 | 25.422 | 1.00 | 31.57 |
| ATOM | 23 | CA | GLU | 211 | 17.525 | 1.121 | 24.020 | 1.00 | 30.40 |
| ATOM | 24 | CB | GLU | 211 | 16.125 | 0.782 | 23.484 | 1.00 | 30.37 |
| ATOM | 25 | CG | GLU | 211 | 15.813 | 1.507 | 22.160 | 1.00 | 32.71 |
| ATOM | 26 | CD | GLU | 211 | 14.495 | 1.097 | 21.511 | 1.00 | 34.29 |
| ATOM | 27 | OE1 | GLU | 211 | 13.563 | 0.690 | 22.233 | 1.00 | 34.92 |
| ATOM | 28 | OE2 | GLU | 211 | 14.384 | 1.205 | 20.267 | 1.00 | 34.73 |
| ATOM | 29 | C | GLU | 211 | 17.752 | 2.625 | 23.883 | 1.00 | 28.59 |
| ATOM | 30 | O | GLU | 211 | 18.351 | 3.080 | 22.913 | 1.00 | 27.98 |
| ATOM | 31 | N | ILE | 212 | 17.264 | 3.396 | 24.848 | 1.00 | 27.40 |
| ATOM | 32 | CA | ILE | 212 | 17.424 | 4.845 | 24.781 | 1.00 | 27.79 |
| ATOM | 33 | CB | ILE | 212 | 16.764 | 5.571 | 25.978 | 1.00 | 26.35 |
| ATOM | 34 | CG2 | ILE | 212 | 17.010 | 7.069 | 25.860 | 1.00 | 27.12 |
| ATOM | 35 | CG1 | ILE | 212 | 15.257 | 5.276 | 26.029 | 1.00 | 26.58 |
| ATOM | 36 | CD1 | ILE | 212 | 14.503 | 5.643 | 24.770 | 1.00 | 24.95 |
| ATOM | 37 | C | ILE | 212 | 18.895 | 5.226 | 24.757 | 1.00 | 27.70 |
| ATOM | 38 | O | ILE | 212 | 19.302 | 6.124 | 24.021 | 1.00 | 26.95 |
| ATOM | 39 | N | ASP | 213 | 19.691 | 4.530 | 25.563 | 1.00 | 29.11 |
| ATOM | 40 | CA | ASP | 213 | 21.122 | 4.800 | 25.653 | 1.00 | 29.22 |
| ATOM | 41 | CB | ASP | 213 | 21.699 | 4.045 | 26.853 | 1.00 | 32.21 |
| ATOM | 42 | CG | ASP | 213 | 23.083 | 4.517 | 27.233 | 1.00 | 34.57 |
| ATOM | 43 | OD1 | ASP | 213 | 23.255 | 5.721 | 27.529 | 1.00 | 36.05 |
| ATOM | 44 | OD2 | ASP | 213 | 24.004 | 3.674 | 27.248 | 1.00 | 37.52 |
| ATOM | 45 | C | ASP | 213 | 21.873 | 4.430 | 24.365 | 1.00 | 28.75 |
| ATOM | 46 | O | ASP | 213 | 22.804 | 5.137 | 23.954 | 1.00 | 27.10 |
| ATOM | 47 | N | ARG | 214 | 21.475 | 3.329 | 23.732 | 1.00 | 28.19 |
| ATOM | 48 | CA | ARG | 214 | 22.108 | 2.908 | 22.480 | 1.00 | 29.03 |
| ATOM | 49 | CB | ARG | 214 | 21.597 | 1.535 | 22.047 | 1.00 | 32.44 |
| ATOM | 50 | CG | ARG | 214 | 21.849 | 0.417 | 23.050 | 1.00 | 38.05 |
| ATOM | 51 | CD | ARG | 214 | 21.039 | -0.810 | 22.667 | 1.00 | 41.57 |
| ATOM | 52 | NE | ARG | 214 | 20.958 | -1.777 | 23.759 | 1.00 | 46.23 |
| ATOM | 53 | CZ | ARG | 214 | 20.771 | -3.082 | 23.582 | 1.00 | 49.23 |
| ATOM | 54 | NH1 | ARG | 214 | 20.644 | -3.577 | 22.346 | 1.00 | 50.85 |
| ATOM | 55 | NH2 | ARG | 214 | 20.720 | -3.894 | 24.632 | 1.00 | 50.33 |
| ATOM | 56 | C | ARG | 214 | 21.785 | 3.928 | 21.383 | 1.00 | 27.33 |
| ATOM | 57 | O | ARG | 214 | 22.624 | 4.236 | 20.543 | 1.00 | 26.60 |
| ATOM | 58 | N | ILE | 215 | 20.559 | 4.446 | 21.391 | 1.00 | 26.45 |
| ATOM | 59 | CA | ILE | 215 | 20.150 | 5.432 | 20.395 | 1.00 | 24.84 |
| ATOM | 60 | CB | ILE | 215 | 18.629 | 5.699 | 20.472 | 1.00 | 26.20 |
| ATOM | 61 | CG2 | ILE | 215 | 18.280 | 7.043 | 19.821 | 1.00 | 25.14 |
| ATOM | 62 | CG1 | ILE | 215 | 17.881 | 4.554 | 19.781 | 1.00 | 25.45 |
| ATOM | 63 | CD1 | ILE | 215 | 16.422 | 4.458 | 20.176 | 1.00 | 25.36 |
| ATOM | 64 | C | ILE | 215 | 20.929 | 6.723 | 20.629 | 1.00 | 24.62 |
| ATOM | 65 | O | ILE | 215 | 21.426 | 7.338 | 19.686 | 1.00 | 23.32 |
| ATOM | 66 | N | ALA | 216 | 21.050 | 7.116 | 21.893 | 1.00 | 24.39 |
| ATOM | 67 | CA | ALA | 216 | 21.782 | 8.323 | 22.242 | 1.00 | 25.02 |
| ATOM | 68 | CB | ALA | 216 | 21.677 | 8.589 | 23.740 | 1.00 | 24.99 |
| ATOM | 69 | C | ALA | 216 | 23.249 | 8.232 | 21.829 | 1.00 | 25.36 |
| ATOM | 70 | O | ALA | 216 | 23.771 | 9.151 | 21.193 | 1.00 | 25.93 |
| ATOM | 71 | N | GLN | 217 | 23.922 | 7.137 | 22.177 | 1.00 | 25.23 |
| ATOM | 72 | CA | GLN | 217 | 25.332 | 6.999 | 21.819 | 1.00 | 26.24 |
| ATOM | 73 | CB | GLN | 217 | 25.917 | 5.705 | 22.397 | 1.00 | 29.19 |
| ATOM | 74 | CG | GLN | 217 | 26.090 | 5.734 | 23.917 | 1.00 | 35.04 |
| ATOM | 75 | CD | GLN | 217 | 27.010 | 4.630 | 24.420 | 1.00 | 38.21 |
| ATOM | 76 | OE1 | GLN | 217 | 26.714 | 3.443 | 24.278 | 1.00 | 40.35 |
| ATOM | 77 | NE2 | GLN | 217 | 28.140 | 5.022 | 25.006 | 1.00 | 41.77 |
| ATOM | 78 | C | GLN | 217 | 25.556 | 7.041 | 20.305 | 1.00 | 25.19 |
| ATOM | 79 | O | GLN | 217 | 26.518 | 7.651 | 19.823 | 1.00 | 24.32 |
| ATOM | 80 | N | ASN | 218 | 24.659 | 6.392 | 19.568 | 1.00 | 23.86 |
| ATOM | 81 | CA | ASN | 218 | 24.725 | 6.341 | 18.113 | 1.00 | 23.77 |
| ATOM | 82 | CB | ASN | 218 | 23.580 | 5.472 | 17.591 | 1.00 | 23.58 |
| ATOM | 83 | CG | ASN | 218 | 23.554 | 5.381 | 16.084 | 1.00 | 24.49 |
| ATOM | 84 | OD1 | ASN | 218 | 22.492 | 5.461 | 15.480 | 1.00 | 24.92 |
| ATOM | 85 | ND2 | ASN | 218 | 24.723 | 5.194 | 15.466 | 1.00 | 26.45 |
| ATOM | 86 | C | ASN | 218 | 24.632 | 7.746 | 17.508 | 1.00 | 23.84 |
| ATOM | 87 | O | ASN | 218 | 25.430 | 8.122 | 16.642 | 1.00 | 23.34 |
| ATOM | 88 | N | ILE | 219 | 23.654 | 8.518 | 17.967 | 1.00 | 23.13 |
| ATOM | 89 | CA | ILE | 219 | 23.464 | 9.872 | 17.462 | 1.00 | 22.12 |
| ATOM | 90 | CB | ILE | 219 | 22.116 | 10.448 | 17.948 | 1.00 | 20.95 |
| ATOM | 91 | CG2 | ILE | 219 | 22.033 | 11.927 | 17.630 | 1.00 | 18.97 |
| ATOM | 92 | CG1 | ILE | 219 | 20.975 | 9.653 | 17.300 | 1.00 | 20.42 |
| ATOM | 93 | CD1 | ILE | 219 | 19.602 | 9.904 | 17.878 | 1.00 | 21.20 |
| ATOM | 94 | C | ILE | 219 | 24.615 | 10.784 | 17.874 | 1.00 | 22.86 |
| ATOM | 95 | O | ILE | 219 | 25.132 | 11.552 | 17.063 | 1.00 | 21.51 |
| ATOM | 96 | N | ILE | 220 | 25.020 | 10.693 | 19.132 | 1.00 | 22.66 |
| ATOM | 97 | CA | ILE | 220 | 26.128 | 11.503 | 19.616 | 1.00 | 24.02 |
| ATOM | 98 | CB | ILE | 220 | 26.418 | 11.199 | 21.098 | 1.00 | 23.77 |
| ATOM | 99 | CG2 | ILE | 220 | 27.801 | 11.712 | 21.479 | 1.00 | 25.51 |
| ATOM | 100 | CG1 | ILE | 220 | 25.323 | 11.824 | 21.970 | 1.00 | 24.25 |
| ATOM | 101 | CD1 | ILE | 220 | 25.371 | 11.402 | 23.442 | 1.00 | 25.63 |
| ATOM | 102 | C | ILE | 220 | 27.379 | 11.217 | 18.783 | 1.00 | 24.51 |
| ATOM | 103 | O | ILE | 220 | 28.076 | 12.133 | 18.338 | 1.00 | 24.01 |
| ATOM | 104 | N | LYS | 221 | 27.655 | 9.937 | 18.563 | 1.00 | 25.74 |
| ATOM | 105 | CA | LYS | 221 | 28.826 | 9.538 | 17.796 | 1.00 | 26.09 |
| ATOM | 106 | CB | LYS | 221 | 28.987 | 8.017 | 17.845 | 1.00 | 27.05 |
| ATOM | 107 | CG | LYS | 221 | 30.069 | 7.447 | 16.940 | 1.00 | 30.22 |
| ATOM | 108 | CD | LYS | 221 | 30.218 | 5.948 | 17.211 | 1.00 | 33.17 |
| ATOM | 109 | CE | LYS | 221 | 30.998 | 5.233 | 16.112 | 1.00 | 35.08 |
| ATOM | 110 | NZ | LYS | 221 | 32.350 | 5.814 | 15.948 | 1.00 | 37.04 |
| ATOM | 111 | C | LYS | 221 | 28.724 | 10.027 | 16.352 | 1.00 | 26.19 |
| ATOM | 112 | O | LYS | 221 | 29.710 | 10.504 | 15.783 | 1.00 | 26.37 |
| ATOM | 113 | N | SER | 222 | 27.538 | 9.932 | 15.765 | 1.00 | 24.31 |
| ATOM | 114 | CA | SER | 222 | 27.365 | 10.388 | 14.389 | 1.00 | 24.58 |
| ATOM | 115 | CB | SER | 222 | 25.947 | 10.105 | 13.894 | 1.00 | 25.00 |
| ATOM | 116 | OG | SER | 222 | 25.818 | 10.539 | 12.544 | 1.00 | 27.88 |
| ATOM | 117 | C | SER | 222 | 27.654 | 11.882 | 14.278 | 1.00 | 23.15 |
| ATOM | 118 | O | SER | 222 | 28.312 | 12.332 | 13.340 | 1.00 | 20.13 |
| ATOM | 119 | N | HIS | 223 | 27.160 | 12.640 | 15.254 | 1.00 | 22.79 |
| ATOM | 120 | CA | HIS | 223 | 27.369 | 14.081 | 15.292 | 1.00 | 23.51 |
| ATOM | 121 | CB | HIS | 223 | 26.694 | 14.672 | 16.530 | 1.00 | 22.71 |
| ATOM | 122 | CG | HIS | 223 | 27.071 | 16.095 | 16.803 | 1.00 | 22.35 |
| ATOM | 123 | CD2 | HIS | 223 | 27.948 | 16.627 | 17.686 | 1.00 | 23.97 |
| ATOM | 124 | ND1 | HIS | 223 | 26.531 | 17.158 | 16.114 | 1.00 | 23.67 |
| ATOM | 125 | CE1 | HIS | 223 | 27.055 | 18.284 | 16.561 | 1.00 | 22.61 |
| ATOM | 126 | NE2 | HIS | 223 | 27.918 | 17.990 | 17.516 | 1.00 | 24.30 |
| ATOM | 127 | C | HIS | 223 | 28.860 | 14.408 | 15.334 | 1.00 | 24.83 |
| ATOM | 128 | O | HIS | 223 | 29.348 | 15.233 | 14.570 | 1.00 | 23.29 |
| ATOM | 129 | N | LEU | 224 | 29.581 | 13.758 | 16.239 | 1.00 | 26.00 |
| ATOM | 130 | CA | LEU | 224 | 31.005 | 14.012 | 16.378 | 1.00 | 27.42 |
| ATOM | 131 | CB | LEU | 224 | 31.065 | 13.241 | 17.579 | 1.00 | 28.17 |
| ATOM | 132 | CG | LEU | 224 | 31.564 | 13.762 | 18.936 | 1.00 | 29.44 |
| ATOM | 133 | CD1 | LEU | 224 | 31.630 | 12.901 | 20.062 | 1.00 | 30.81 |
| ATOM | 134 | CD2 | LEU | 224 | 31.482 | 15.226 | 19.118 | 1.00 | 31.36 |
| ATOM | 135 | C | LEU | 224 | 31.791 | 13.686 | 15.116 | 1.00 | 27.94 |
| ATOM | 136 | O | LEU | 224 | 32.777 | 14.358 | 14.823 | 1.00 | 28.46 |
| ATOM | 137 | N | GLU | 225 | 31.352 | 12.676 | 14.366 | 1.00 | 26.53 |
| ATOM | 138 | CA | GLU | 225 | 32.042 | 12.289 | 13.138 | 1.00 | 28.08 |
| ATOM | 139 | CB | GLU | 225 | 31.897 | 10.785 | 12.867 | 1.00 | 29.02 |
| ATOM | 140 | CG | GLU | 225 | 31.994 | 9.881 | 14.083 | 1.00 | 32.55 |
| ATOM | 141 | CD | GLU | 225 | 31.914 | 8.410 | 13.714 | 1.00 | 34.16 |
| ATOM | 142 | OE1 | GLU | 225 | 31.093 | 8.046 | 12.822 | 1.00 | 35.71 |
| ATOM | 143 | OE2 | GLU | 225 | 32.666 | 7.615 | 14.322 | 1.00 | 33.88 |
| ATOM | 144 | C | GLU | 225 | 31.540 | 13.028 | 11.897 | 1.00 | 26.89 |
| ATOM | 145 | O | GLU | 225 | 32.105 | 12.862 | 10.820 | 1.00 | 27.36 |
| ATOM | 146 | N | THR | 226 | 30.499 | 13.845 | 12.032 | 1.00 | 26.42 |
| ATOM | 147 | CA | THR | 226 | 29.959 | 14.540 | 10.860 | 1.00 | 25.86 |
| ATOM | 148 | CB | THR | 226 | 28.574 | 13.972 | 10.486 | 1.00 | 24.69 |
| ATOM | 149 | OG1 | THR | 226 | 27.670 | 14.165 | 11.579 | 1.00 | 21.37 |
| ATOM | 150 | CG2 | THR | 226 | 28.684 | 12.478 | 10.162 | 1.00 | 25.22 |
| ATOM | 151 | C | THR | 226 | 29.850 | 16.067 | 10.890 | 1.00 | 26.84 |
| ATOM | 152 | O | THR | 226 | 29.016 | 16.645 | 10.196 | 1.00 | 25.61 |
| ATOM | 153 | N | CYS | 227 | 30.670 | 16.719 | 11.698 | 1.00 | 27.47 |
| ATOM | 154 | CA | CYS | 227 | 30.677 | 18.177 | 11.733 | 1.00 | 29.95 |
| ATOM | 155 | CB | CYS | 227 | 30.725 | 18.694 | 13.160 | 1.00 | 30.26 |
| ATOM | 156 | SG | CYS | 227 | 29.129 | 18.666 | 13.964 | 1.00 | 31.37 |
| ATOM | 157 | C | CYS | 227 | 31.927 | 18.614 | 10.993 | 1.00 | 31.15 |
| ATOM | 158 | O | CYS | 227 | 32.949 | 17.926 | 11.044 | 1.00 | 32.10 |
| ATOM | 159 | N | GLN | 228 | 31.856 | 19.737 | 10.291 | 1.00 | 32.99 |
| ATOM | 160 | CA | GLN | 228 | 33.028 | 20.217 | 9.559 | 1.00 | 34.05 |
| ATOM | 161 | CB | GLN | 228 | 32.700 | 21.472 | 8.746 | 1.00 | 34.74 |
| ATOM | 162 | CG | GLN | 228 | 33.975 | 22.087 | 8.166 | 1.00 | 36.23 |
| ATOM | 163 | CD | GLN | 228 | 33.732 | 23.051 | 7.040 | 1.00 | 36.08 |
| ATOM | 164 | OE1 | GLN | 228 | 34.591 | 23.216 | 6.172 | 1.00 | 37.80 |
| ATOM | 165 | NE2 | GLN | 228 | 32.579 | 23.710 | 7.049 | 1.00 | 35.92 |
| ATOM | 166 | C | GLN | 228 | 34.163 | 20.543 | 10.527 | 1.00 | 34.44 |
| ATOM | 167 | O | GLN | 228 | 35.345 | 20.276 | 10.254 | 1.00 | 35.97 |
| ATOM | 168 | N | TYR | 229 | 33.794 | 21.134 | 11.656 | 1.00 | 33.42 |
| ATOM | 169 | CA | TYR | 229 | 34.762 | 21.508 | 12.664 | 1.00 | 34.12 |
| ATOM | 170 | CB | TYR | 229 | 34.804 | 23.025 | 12.814 | 1.00 | 34.64 |
| ATOM | 171 | CG | TYR | 229 | 35.252 | 23.753 | 11.578 | 1.00 | 35.84 |
| ATOM | 172 | CD1 | TYR | 229 | 36.530 | 23.558 | 11.052 | 1.00 | 37.22 |
| ATOM | 173 | CE1 | TYR | 229 | 36.960 | 24.278 | 9.925 | 1.00 | 37.82 |
| ATOM | 174 | CD2 | TYR | 229 | 34.412 | 24.667 | 10.953 | 1.00 | 36.84 |
| ATOM | 175 | CE2 | TYR | 229 | 34.823 | 25.380 | 9.841 | 1.00 | 37.23 |
| ATOM | 176 | CZ | TYR | 229 | 36.093 | 25.191 | 9.333 | 1.00 | 38.42 |
| ATOM | 177 | OH | TYR | 229 | 36.496 | 25.957 | 8.260 | 1.00 | 40.27 |
| ATOM | 178 | C | TYR | 229 | 34.420 | 20.895 | 14.004 | 1.00 | 34.39 |
| ATOM | 179 | O | TYR | 229 | 33.292 | 21.045 | 14.489 | 1.00 | 33.47 |
| ATOM | 180 | N | THR | 230 | 35.385 | 20.202 | 14.601 | 1.00 | 35.45 |
| ATOM | 181 | CA | THR | 230 | 35.151 | 19.622 | 15.913 | 1.00 | 37.95 |
| ATOM | 182 | CB | THR | 230 | 36.301 | 18.697 | 16.373 | 1.00 | 38.23 |
| ATOM | 183 | OG1 | THR | 230 | 37.502 | 19.463 | 16.518 | 1.00 | 40.39 |
| ATOM | 184 | CG2 | THR | 230 | 36.522 | 17.593 | 15.370 | 1.00 | 39.49 |
| ATOM | 185 | C | THR | 230 | 35.074 | 20.807 | 16.851 | 1.00 | 38.40 |
| ATOM | 186 | O | THR | 230 | 35.466 | 21.927 | 16.488 | 1.00 | 37.62 |
| ATOM | 187 | N | MET | 231 | 34.555 | 20.567 | 18.049 | 1.00 | 40.09 |
| ATOM | 188 | CA | MET | 231 | 34.421 | 21.614 | 19.055 | 1.00 | 42.13 |
| ATOM | 189 | CB | MET | 231 | 33.812 | 21.031 | 20.319 | 1.00 | 42.86 |
| ATOM | 190 | CG | MET | 231 | 32.379 | 21.368 | 20.493 | 1.00 | 44.14 |
| ATOM | 191 | SD | MET | 231 | 32.214 | 22.979 | 21.200 | 1.00 | 48.47 |
| ATOM | 192 | CE | MET | 231 | 31.477 | 23.899 | 19.813 | 1.00 | 44.81 |
| ATOM | 193 | C | MET | 231 | 35.761 | 22.239 | 19.392 | 1.00 | 42.66 |
| ATOM | 194 | O | MET | 231 | 35.833 | 23.413 | 19.754 | 1.00 | 42.23 |
| ATOM | 195 | N | GLU | 232 | 36.817 | 21.435 | 19.287 | 1.00 | 44.27 |
| ATOM | 196 | CA | GLU | 232 | 38.176 | 21.900 | 19.564 | 1.00 | 46.03 |
| ATOM | 197 | CB | GLU | 232 | 39.150 | 20.718 | 19.589 | 1.00 | 48.16 |
| ATOM | 198 | CG | GLU | 232 | 39.075 | 19.847 | 20.834 | 1.00 | 52.20 |
| ATOM | 199 | CD | GLU | 232 | 39.978 | 18.626 | 20.745 | 1.00 | 54.53 |
| ATOM | 200 | OE1 | GLU | 232 | 41.211 | 18.807 | 20.581 | 1.00 | 55.69 |
| ATOM | 201 | OE2 | GLU | 232 | 39.452 | 17.483 | 20.831 | 1.00 | 55.83 |
| ATOM | 202 | C | GLU | 232 | 38.629 | 22.893 | 18.484 | 1.00 | 45.35 |
| ATOM | 203 | O | GLU | 232 | 39.137 | 23.971 | 18.785 | 1.00 | 45.45 |
| ATOM | 204 | N | GLU | 233 | 38.449 | 22.506 | 17.227 | 1.00 | 44.58 |
| ATOM | 205 | CA | GLU | 233 | 38.837 | 23.341 | 16.099 | 1.00 | 43.02 |
| ATOM | 206 | CB | GLU | 233 | 38.530 | 22.625 | 14.780 | 1.00 | 43.47 |
| ATOM | 207 | CG | GLU | 233 | 39.477 | 21.486 | 14.414 | 1.00 | 43.69 |
| ATOM | 208 | CD | GLU | 233 | 39.053 | 20.792 | 13.134 | 1.00 | 44.50 |
| ATOM | 209 | OE1 | GLU | 233 | 37.937 | 20.228 | 13.113 | 1.00 | 42.94 |
| ATOM | 210 | OE2 | GLU | 233 | 39.826 | 20.819 | 12.144 | 1.00 | 45.82 |
| ATOM | 211 | C | GLU | 233 | 38.130 | 24.693 | 16.128 | 1.00 | 42.63 |
| ATOM | 212 | O | GLU | 233 | 38.664 | 25.689 | 15.629 | 1.00 | 41.56 |
| ATOM | 213 | N | LEU | 234 | 36.927 | 24.716 | 16.702 | 1.00 | 41.55 |
| ATOM | 214 | CA | LEU | 234 | 36.130 | 25.929 | 16.800 | 1.00 | 41.68 |
| ATOM | 215 | CB | LEU | 234 | 34.662 | 25.569 | 17.081 | 1.00 | 38.99 |
| ATOM | 216 | CG | LEU | 234 | 33.595 | 25.745 | 15.984 | 1.00 | 37.42 |
| ATOM | 217 | CD1 | LEU | 234 | 34.202 | 25.811 | 14.598 | 1.00 | 36.02 |
| ATOM | 218 | CD2 | LEU | 234 | 32.598 | 24.602 | 16.068 | 1.00 | 35.04 |
| ATOM | 219 | C | LEU | 234 | 36.659 | 26.884 | 17.874 | 1.00 | 43.02 |
| ATOM | 220 | O | LEU | 234 | 36.689 | 28.097 | 17.665 | 1.00 | 42.82 |
| ATOM | 221 | N | HIS | 235 | 37.063 | 26.347 | 19.024 | 1.00 | 44.76 |
| ATOM | 222 | CA | HIS | 235 | 37.609 | 27.183 | 20.100 | 1.00 | 46.16 |
| ATOM | 223 | CB | HIS | 235 | 37.997 | 26.304 | 21.303 | 1.00 | 47.16 |
| ATOM | 224 | CG | HIS | 235 | 36.823 | 25.826 | 22.102 | 1.00 | 47.88 |
| ATOM | 225 | CD2 | HIS | 235 | 35.624 | 26.405 | 22.363 | 1.00 | 48.26 |
| ATOM | 226 | ND1 | HIS | 235 | 36.816 | 24.615 | 22.765 | 1.00 | 48.61 |
| ATOM | 227 | CE1 | HIS | 235 | 35.664 | 24.465 | 23.398 | 1.00 | 48.18 |
| ATOM | 228 | NE2 | HIS | 235 | 34.922 | 25.536 | 23.169 | 1.00 | 48.08 |
| ATOM | 229 | C | HIS | 235 | 38.833 | 27.943 | 19.580 | 1.00 | 46.16 |
| ATOM | 230 | O | HIS | 235 | 39.093 | 29.086 | 19.972 | 1.00 | 46.99 |
| ATOM | 231 | N | GLN | 236 | 39.570 | 27.304 | 18.676 | 1.00 | 46.28 |
| ATOM | 232 | CA | GLN | 236 | 40.763 | 27.888 | 18.078 | 1.00 | 46.35 |
| ATOM | 233 | CB | GLN | 236 | 41.709 | 26.769 | 17.647 | 1.00 | 48.10 |
| ATOM | 234 | CG | GLN | 236 | 42.314 | 26.013 | 18.819 | 1.00 | 50.88 |
| ATOM | 235 | CD | GLN | 236 | 43.066 | 24.776 | 18.378 | 1.00 | 53.03 |
| ATOM | 236 | OE1 | GLN | 236 | 42.483 | 23.846 | 17.799 | 1.00 | 54.30 |
| ATOM | 237 | NE2 | GLN | 236 | 44.368 | 24.750 | 18.651 | 1.00 | 53.30 |
| ATOM | 238 | C | GLN | 236 | 40.469 | 28.804 | 16.882 | 1.00 | 45.38 |
| ATOM | 239 | O | GLN | 236 | 41.358 | 29.510 | 16.401 | 1.00 | 45.98 |
| ATOM | 240 | N | LEU | 237 | 39.231 | 28.793 | 16.396 | 1.00 | 43.26 |
| ATOM | 241 | CA | LEU | 237 | 38.864 | 29.650 | 15.276 | 1.00 | 41.13 |
| ATOM | 242 | CB | LEU | 237 | 38.032 | 28.877 | 14.249 | 1.00 | 40.63 |
| ATOM | 243 | CG | LEU | 237 | 38.733 | 27.918 | 13.291 | 1.00 | 40.27 |
| ATOM | 244 | CD1 | LEU | 237 | 37.700 | 27.272 | 12.379 | 1.00 | 39.92 |
| ATOM | 245 | CD2 | LEU | 237 | 39.777 | 28.674 | 12.485 | 1.00 | 40.45 |
| ATOM | 246 | C | LEU | 237 | 38.081 | 30.878 | 15.727 | 1.00 | 39.51 |
| ATOM | 247 | O | LEU | 237 | 38.086 | 31.903 | 15.047 | 1.00 | 38.80 |
| ATOM | 248 | N | ALA | 238 | 37.427 | 30.778 | 16.880 | 1.00 | 39.51 |
| ATOM | 249 | CA | ALA | 238 | 36.618 | 31.871 | 17.406 | 1.00 | 38.91 |
| ATOM | 250 | CB | ALA | 238 | 35.908 | 31.423 | 18.694 | 1.00 | 39.73 |
| ATOM | 251 | C | ALA | 238 | 37.408 | 33.152 | 17.656 | 1.00 | 38.89 |
| ATOM | 252 | O | ALA | 238 | 36.832 | 34.239 | 17.716 | 1.00 | 38.65 |
| ATOM | 253 | N | TRP | 239 | 38.725 | 33.041 | 17.794 | 1.00 | 39.18 |
| ATOM | 254 | CA | TRP | 239 | 39.524 | 34.239 | 18.022 | 1.00 | 39.10 |
| ATOM | 255 | CB | TRP | 239 | 40.771 | 33.912 | 18.844 | 1.00 | 39.97 |
| ATOM | 256 | CG | TRP | 239 | 40.439 | 33.575 | 20.265 | 1.00 | 41.05 |
| ATOM | 257 | CD2 | TRP | 239 | 40.329 | 34.502 | 21.357 | 1.00 | 41.89 |
| ATOM | 258 | CE2 | TRP | 239 | 39.925 | 33.764 | 22.496 | 1.00 | 42.28 |
| ATOM | 259 | CE3 | TRP | 239 | 40.529 | 35.889 | 21.481 | 1.00 | 42.04 |
| ATOM | 260 | CD1 | TRP | 239 | 40.111 | 32.345 | 20.768 | 1.00 | 41.20 |
| ATOM | 261 | NE1 | TRP | 239 | 39.800 | 32.451 | 22.113 | 1.00 | 41.62 |
| ATOM | 262 | CZ2 | TRP | 239 | 39.718 | 34.369 | 23.749 | 1.00 | 42.42 |
| ATOM | 263 | CZ3 | TRP | 239 | 40.318 | 36.495 | 22.734 | 1.00 | 41.71 |
| ATOM | 264 | CH2 | TRP | 239 | 39.917 | 35.732 | 23.846 | 1.00 | 41.59 |
| ATOM | 265 | C | TRP | 239 | 39.904 | 34.917 | 16.715 | 1.00 | 38.66 |
| ATOM | 266 | O | TRP | 239 | 40.315 | 36.081 | 16.708 | 1.00 | 39.17 |
| ATOM | 267 | N | GLN | 240 | 39.750 | 34.191 | 15.613 | 1.00 | 37.88 |
| ATOM | 268 | CA | GLN | 240 | 40.052 | 34.715 | 14.281 | 1.00 | 38.38 |
| ATOM | 269 | CB | GLN | 240 | 40.402 | 33.569 | 13.325 | 1.00 | 39.13 |
| ATOM | 270 | CG | GLN | 240 | 41.815 | 33.009 | 13.448 | 1.00 | 43.03 |
| ATOM | 271 | CD | GLN | 240 | 42.865 | 33.999 | 12.962 | 1.00 | 45.47 |
| ATOM | 272 | OE1 | GLN | 240 | 42.697 | 34.640 | 11.913 | 1.00 | 46.02 |
| ATOM | 273 | NE2 | GLN | 240 | 43.962 | 34.121 | 13.712 | 1.00 | 45.97 |
| ATOM | 274 | C | GLN | 240 | 38.841 | 35.473 | 13.726 | 1.00 | 38.04 |
| ATOM | 275 | O | GLN | 240 | 37.750 | 34.900 | 13.590 | 1.00 | 36.35 |
| ATOM | 276 | N | THR | 241 | 39.029 | 36.752 | 13.406 | 1.00 | 37.28 |
| ATOM | 277 | CA | THR | 241 | 37.938 | 37.554 | 12.865 | 1.00 | 37.13 |
| ATOM | 278 | CB | THR | 241 | 37.417 | 38.596 | 13.901 | 1.00 | 38.89 |
| ATOM | 279 | OG1 | THR | 241 | 38.436 | 39.565 | 14.186 | 1.00 | 40.81 |
| ATOM | 280 | CG2 | THR | 241 | 37.028 | 37.907 | 15.208 | 1.00 | 39.48 |
| ATOM | 281 | C | THR | 241 | 38.347 | 38.279 | 11.580 | 1.00 | 36.22 |
| ATOM | 282 | O | THR | 241 | 39.485 | 38.758 | 11.451 | 1.00 | 36.33 |
| ATOM | 283 | N | HIS | 242 | 37.430 | 38.324 | 10.613 | 1.00 | 34.05 |
| ATOM | 284 | CA | HIS | 242 | 37.695 | 39.012 | 9.354 | 1.00 | 31.44 |
| ATOM | 285 | CB | HIS | 242 | 36.472 | 38.974 | 8.436 | 1.00 | 29.25 |
| ATOM | 286 | CG | HIS | 242 | 36.149 | 37.615 | 7.904 | 1.00 | 27.92 |
| ATOM | 287 | CD2 | HIS | 242 | 36.719 | 36.887 | 6.915 | 1.00 | 26.55 |
| ATOM | 288 | ND1 | HIS | 242 | 35.111 | 36.853 | 8.393 | 1.00 | 27.30 |
| ATOM | 289 | CE1 | HIS | 242 | 35.055 | 35.715 | 7.727 | 1.00 | 26.17 |
| ATOM | 290 | NE2 | HIS | 242 | 36.020 | 35.710 | 6.826 | 1.00 | 27.22 |
| ATOM | 291 | C | HIS | 242 | 38.020 | 40.465 | 9.668 | 1.00 | 30.68 |
| ATOM | 292 | O | HIS | 242 | 37.358 | 41.096 | 10.490 | 1.00 | 30.78 |
| ATOM | 293 | N | THR | 243 | 39.038 | 40.991 | 9.004 | 1.00 | 31.03 |
| ATOM | 294 | CA | THR | 243 | 39.460 | 42.372 | 9.199 | 1.00 | 30.79 |
| ATOM | 295 | CB | THR | 243 | 40.822 | 42.612 | 8.543 | 1.00 | 30.73 |
| ATOM | 296 | OG1 | THR | 243 | 40.717 | 42.383 | 7.126 | 1.00 | 31.15 |
| ATOM | 297 | CG2 | THR | 243 | 41.864 | 41.669 | 9.140 | 1.00 | 29.69 |
| ATOM | 298 | C | THR | 243 | 38.470 | 43.363 | 8.595 | 1.00 | 31.03 |
| ATOM | 299 | O | THR | 243 | 37.534 | 42.970 | 7.901 | 1.00 | 29.40 |
| ATOM | 300 | N | TYR | 244 | 38.684 | 44.648 | 8.868 | 1.00 | 31.19 |
| ATOM | 301 | CA | TYR | 244 | 37.834 | 45.702 | 8.324 | 1.00 | 31.95 |
| ATOM | 302 | CB | TYR | 244 | 38.297 | 47.095 | 8.802 | 1.00 | 33.18 |
| ATOM | 303 | CG | TYR | 244 | 37.829 | 47.457 | 10.204 | 1.00 | 33.91 |
| ATOM | 304 | CD1 | TYR | 244 | 38.719 | 47.484 | 11.289 | 1.00 | 35.00 |
| ATOM | 305 | CE1 | TYR | 244 | 38.268 | 47.784 | 12.599 | 1.00 | 35.72 |
| ATOM | 306 | CD2 | TYR | 244 | 36.486 | 47.736 | 10.450 | 1.00 | 35.64 |
| ATOM | 307 | CE2 | TYR | 244 | 36.025 | 48.032 | 11.740 | 1.00 | 35.81 |
| ATOM | 308 | CZ | TYR | 244 | 36.915 | 48.054 | 12.809 | 1.00 | 36.54 |
| ATOM | 309 | OH | TYR | 244 | 36.440 | 48.350 | 14.072 | 1.00 | 36.95 |
| ATOM | 310 | C | TYR | 244 | 37.894 | 45.641 | 6.805 | 1.00 | 31.62 |
| ATOM | 311 | O | TYR | 244 | 36.879 | 45.842 | 6.123 | 1.00 | 31.91 |
| ATOM | 312 | N | GLU | 245 | 39.085 | 45.363 | 6.280 | 1.00 | 30.27 |
| ATOM | 313 | CA | GLU | 245 | 39.281 | 45.271 | 4.841 | 1.00 | 30.86 |
| ATOM | 314 | CB | GLU | 245 | 40.764 | 45.070 | 4.502 | 1.00 | 32.11 |
| ATOM | 315 | CG | GLU | 245 | 41.664 | 46.274 | 4.773 | 1.00 | 34.84 |
| ATOM | 316 | CD | GLU | 245 | 41.999 | 46.477 | 6.248 | 1.00 | 36.73 |
| ATOM | 317 | OE1 | GLU | 245 | 42.570 | 47.548 | 6.573 | 1.00 | 37.44 |
| ATOM | 318 | OE2 | GLU | 245 | 41.710 | 45.578 | 7.080 | 1.00 | 36.51 |
| ATOM | 319 | C | GLU | 245 | 38.476 | 44.105 | 4.283 | 1.00 | 29.31 |
| ATOM | 320 | O | GLU | 245 | 37.722 | 44.262 | 3.325 | 1.00 | 28.06 |
| ATOM | 321 | N | GLU | 246 | 36.632 | 42.939 | 4.904 | 1.00 | 28.59 |
| ATOM | 322 | CA | GLU | 246 | 37.921 | 41.740 | 4.458 | 1.00 | 28.73 |
| ATOM | 323 | CB | GLU | 246 | 38.360 | 40.539 | 5.298 | 1.00 | 28.89 |
| ATOM | 324 | CG | GLU | 246 | 39.841 | 40.230 | 5.106 | 1.00 | 33.43 |
| ATOM | 325 | CD | GLU | 246 | 40.377 | 39.192 | 6.065 | 1.00 | 34.90 |
| ATOM | 326 | OE1 | GLU | 246 | 40.008 | 39.213 | 7.259 | 1.00 | 36.44 |
| ATOM | 327 | OE2 | GLU | 246 | 41.196 | 38.363 | 5.625 | 1.00 | 37.65 |
| ATOM | 328 | C | GLU | 246 | 36.407 | 41.941 | 4.515 | 1.00 | 26.90 |
| ATOM | 329 | O | GLU | 246 | 35.690 | 41.539 | 3.604 | 1.00 | 26.66 |
| ATOM | 330 | N | ILE | 247 | 35.921 | 42.580 | 5.575 | 1.00 | 27.96 |
| ATOM | 331 | CA | ILE | 247 | 34.488 | 42.838 | 5.702 | 1.00 | 26.28 |
| ATOM | 332 | CB | ILE | 247 | 34.155 | 43.539 | 7.048 | 1.00 | 26.69 |
| ATOM | 333 | CG2 | ILE | 247 | 32.729 | 44.062 | 7.031 | 1.00 | 25.56 |
| ATOM | 334 | CG1 | ILE | 247 | 34.360 | 42.570 | 8.222 | 1.00 | 25.48 |
| ATOM | 335 | CD1 | ILE | 247 | 33.500 | 41.318 | 8.148 | 1.00 | 26.47 |
| ATOM | 336 | C | ILE | 247 | 34.043 | 43.741 | 4.546 | 1.00 | 27.42 |
| ATOM | 337 | O | ILE | 247 | 32.977 | 43.544 | 3.954 | 1.00 | 25.31 |
| ATOM | 338 | N | LYS | 248 | 34.876 | 44.723 | 4.214 | 1.00 | 27.71 |
| ATOM | 339 | CA | LYS | 248 | 34.538 | 45.638 | 3.135 | 1.00 | 28.31 |
| ATOM | 340 | CB | LYS | 248 | 35.547 | 46.786 | 3.059 | 1.00 | 31.00 |
| ATOM | 341 | CG | LYS | 248 | 34.912 | 48.072 | 2.556 | 1.00 | 36.20 |
| ATOM | 342 | CD | LYS | 248 | 34.261 | 48.862 | 3.708 | 1.00 | 37.72 |
| ATOM | 343 | CE | LYS | 248 | 33.507 | 47.965 | 4.691 | 1.00 | 39.77 |
| ATOM | 344 | NZ | LYS | 248 | 32.922 | 48.683 | 5.861 | 1.00 | 40.50 |
| ATOM | 345 | C | LYS | 248 | 34.482 | 44.914 | 1.802 | 1.00 | 27.15 |
| ATOM | 346 | O | LYS | 248 | 33.618 | 45.191 | 0.972 | 1.00 | 25.35 |
| ATOM | 347 | N | ALA | 249 | 35.404 | 43.976 | 1.612 | 1.00 | 27.48 |
| ATOM | 348 | CA | ALA | 249 | 35.462 | 43.197 | 0.380 | 1.00 | 27.44 |
| ATOM | 349 | CB | ALA | 249 | 36.639 | 42.235 | 0.423 | 1.00 | 28.05 |
| ATOM | 350 | C | ALA | 249 | 34.151 | 42.427 | 0.213 | 1.00 | 27.33 |
| ATOM | 351 | O | ALA | 249 | 33.576 | 42.386 | -0.882 | 1.00 | 26.70 |
| ATOM | 352 | N | TYR | 250 | 33.681 | 41.820 | 1.303 | 1.00 | 26.77 |
| ATOM | 353 | CA | TYR | 250 | 32.428 | 41.072 | 1.272 | 1.00 | 26.29 |
| ATOM | 354 | CB | TYR | 250 | 32.131 | 40.421 | 2.628 | 1.00 | 26.30 |
| ATOM | 355 | CG | TYR | 250 | 32.869 | 39.129 | 2.881 | 1.00 | 26.73 |
| ATOM | 356 | CD1 | TYR | 250 | 32.749 | 38.051 | 2.008 | 1.00 | 26.93 |
| ATOM | 357 | CE1 | TYR | 250 | 33.416 | 36.854 | 2.252 | 1.00 | 27.90 |
| ATOM | 358 | CD2 | TYR | 250 | 33.674 | 38.981 | 4.007 | 1.00 | 27.33 |
| ATOM | 359 | CE2 | TYR | 250 | 34.342 | 37.788 | 4.262 | 1.00 | 27.78 |
| ATOM | 360 | CZ | TYR | 250 | 34.210 | 36.731 | 3.385 | 1.00 | 27.25 |
| ATOM | 361 | OH | TYR | 250 | 34.878 | 35.556 | 3.650 | 1.00 | 28.52 |
| ATOM | 362 | C | TYR | 250 | 31.276 | 41.997 | 0.932 | 1.00 | 25.31 |
| ATOM | 363 | O | TYR | 250 | 30.352 | 41.612 | 0.230 | 1.00 | 24.79 |
| ATOM | 364 | N | GLN | 251 | 31.324 | 43.213 | 1.456 | 1.00 | 26.05 |
| ATOM | 365 | CA | GLN | 251 | 30.261 | 44.169 | 1.198 | 1.00 | 27.36 |
| ATOM | 366 | CB | GLN | 251 | 30.311 | 45.309 | 2.208 | 1.00 | 28.26 |
| ATOM | 367 | CG | GLN | 251 | 30.146 | 44.860 | 3.644 | 1.00 | 29.79 |
| ATOM | 368 | CD | GLN | 251 | 30.114 | 46.024 | 4.597 | 1.00 | 32.00 |
| ATOM | 369 | OE1 | GLN | 251 | 30.908 | 46.963 | 4.465 | 1.00 | 33.43 |
| ATOM | 370 | NE2 | GLN | 251 | 29.208 | 45.972 | 5.577 | 1.00 | 30.11 |
| ATOM | 371 | C | GLN | 251 | 30.333 | 44.739 | -0.207 | 1.00 | 28.15 |
| ATOM | 372 | O | GLN | 251 | 29.348 | 45.278 | -0.714 | 1.00 | 28.35 |
| ATOM | 373 | N | SER | 252 | 31.498 | 44.629 | -0.835 | 1.00 | 28.46 |
| ATOM | 374 | CA | SER | 252 | 31.670 | 45.138 | -2.195 | 1.00 | 29.58 |
| ATOM | 375 | CB | SER | 252 | 33.115 | 45.586 | -2.408 | 1.00 | 29.66 |
| ATOM | 376 | OG | SER | 252 | 33.399 | 46.707 | -1.587 | 1.00 | 33.07 |
| ATOM | 377 | C | SER | 252 | 31.287 | 44.087 | -3.225 | 1.00 | 30.02 |
| ATOM | 378 | O | SER | 252 | 31.319 | 44.333 | -4.432 | 1.00 | 30.26 |
| ATOM | 379 | N | LYS | 253 | 30.917 | 42.908 | -2.742 | 1.00 | 29.52 |
| ATOM | 380 | CA | LYS | 253 | 30.512 | 41.827 | -3.630 | 1.00 | 30.07 |
| ATOM | 381 | CB | LYS | 253 | 30.421 | 40.509 | -2.854 | 1.00 | 30.99 |
| ATOM | 382 | CG | LYS | 253 | 31.759 | 39.923 | -2.457 | 1.00 | 33.63 |
| ATOM | 383 | CD | LYS | 253 | 32.609 | 39.683 | -3.697 | 1.00 | 35.38 |
| ATOM | 384 | CE | LYS | 253 | 33.918 | 38.993 | -3.352 | 1.00 | 38.01 |
| ATOM | 385 | NZ | LYS | 253 | 33.690 | 37.648 | -2.761 | 1.00 | 36.66 |
| ATOM | 386 | C | LYS | 253 | 29.156 | 42.120 | -4.250 | 1.00 | 29.23 |
| ATOM | 387 | O | LYS | 253 | 28.333 | 42.815 | -3.663 | 1.00 | 29.23 |
| ATOM | 388 | N | SER | 254 | 28.922 | 41.596 | -5.446 | 1.00 | 29.17 |
| ATOM | 389 | CA | SER | 254 | 27.631 | 41.786 | -6.080 | 1.00 | 29.19 |
| ATOM | 390 | CB | SER | 254 | 27.667 | 41.371 | -7.545 | 1.00 | 29.22 |
| ATOM | 391 | OG | SER | 254 | 27.816 | 39.968 | -7.640 | 1.00 | 25.88 |
| ATOM | 392 | C | SER | 254 | 26.710 | 40.830 | -5.341 | 1.00 | 29.60 |
| ATOM | 393 | O | SER | 254 | 27.171 | 39.862 | -4.739 | 1.00 | 29.62 |
| ATOM | 394 | N | ARG | 255 | 25.416 | 41.097 | -5.404 | 1.00 | 28.75 |
| ATOM | 395 | CA | ARG | 255 | 24.430 | 40.257 | -4.749 | 1.00 | 29.78 |
| ATOM | 396 | CB | ARG | 255 | 23.034 | 40.853 | -4.969 | 1.00 | 31.17 |
| ATOM | 397 | CG | ARG | 255 | 21.941 | 40.222 | -4.139 | 1.00 | 34.14 |
| ATOM | 398 | CD | ARG | 255 | 20.773 | 41.191 | -3.965 | 1.00 | 37.49 |
| ATOM | 399 | NE | ARG | 255 | 19.500 | 40.485 | -3.868 | 1.00 | 40.31 |
| ATOM | 400 | CZ | ARG | 255 | 19.100 | 39.578 | -4.755 | 1.00 | 41.60 |
| ATOM | 401 | NH1 | ARG | 255 | 19.890 | 39.287 | -5.782 | 1.00 | 44.13 |
| ATOM | 402 | NH2 | ARG | 255 | 17.924 | 38.971 | -4.631 | 1.00 | 39.96 |
| ATOM | 403 | C | ARG | 255 | 24.508 | 38.809 | -5.255 | 1.00 | 27.97 |
| ATOM | 404 | O | ARG | 255 | 24.376 | 37.867 | -4.473 | 1.00 | 27.13 |
| ATOM | 405 | N | GLU | 256 | 24.748 | 38.628 | -6.549 | 1.00 | 27.81 |
| ATOM | 406 | CA | GLU | 256 | 24.854 | 37.284 | -7.115 | 1.00 | 28.27 |
| ATOM | 407 | CB | GLU | 256 | 24.876 | 37.331 | -8.647 | 1.00 | 29.71 |
| ATOM | 408 | CG | GLU | 256 | 24.028 | 38.425 | -9.243 | 1.00 | 34.67 |
| ATOM | 409 | CD | GLU | 256 | 24.701 | 39.784 | -9.149 | 1.00 | 34.34 |
| ATOM | 410 | OE1 | GLU | 256 | 25.746 | 39.971 | -9.808 | 1.00 | 37.48 |
| ATOM | 411 | OE2 | GLU | 256 | 24.193 | 40.652 | -8.417 | 1.00 | 34.93 |
| ATOM | 412 | C | GLU | 256 | 26.121 | 36.577 | -6.639 | 1.00 | 27.13 |
| ATOM | 413 | O | GLU | 256 | 26.106 | 35.379 | -6.379 | 1.00 | 26.34 |
| ATOM | 414 | N | ALA | 257 | 27.221 | 37.322 | -6.543 | 1.00 | 26.59 |
| ATOM | 415 | CA | ALA | 257 | 28.497 | 36.747 | -6.108 | 1.00 | 24.46 |
| ATOM | 416 | CB | ALA | 257 | 29.593 | 37.789 | -6.190 | 1.00 | 23.06 |
| ATOM | 417 | C | ALA | 257 | 28.406 | 36.210 | -4.685 | 1.00 | 23.27 |
| ATOM | 418 | O | ALA | 257 | 28.828 | 35.084 | -4.413 | 1.00 | 22.83 |
| ATOM | 419 | N | LEU | 258 | 27.855 | 37.015 | -3.780 | 1.00 | 22.28 |
| ATOM | 420 | CA | LEU | 258 | 27.743 | 36.602 | -2.386 | 1.00 | 22.36 |
| ATOM | 421 | CB | LEU | 258 | 27.382 | 37.788 | -1.480 | 1.00 | 21.42 |
| ATOM | 422 | CG | LEU | 258 | 27.623 | 37.516 | 0.012 | 1.00 | 21.16 |
| ATOM | 423 | CD1 | LEU | 258 | 29.104 | 37.335 | 0.264 | 1.00 | 22.80 |
| ATOM | 424 | CD2 | LEU | 258 | 27.098 | 38.656 | 0.857 | 1.00 | 22.10 |
| ATOM | 425 | C | LEU | 258 | 26.712 | 35.487 | -2.253 | 1.00 | 22.29 |
| ATOM | 426 | O | LEU | 258 | 26.921 | 34.537 | -1.498 | 1.00 | 21.82 |
| ATOM | 427 | N | TRP | 259 | 25.608 | 35.580 | -2.990 | 1.00 | 21.90 |
| ATOM | 428 | CA | TRP | 259 | 24.617 | 34.514 | -2.921 | 1.00 | 23.48 |
| ATOM | 429 | CB | TRP | 259 | 23.405 | 34.801 | -3.823 | 1.00 | 24.84 |
| ATOM | 430 | CG | TRP | 259 | 22.260 | 35.370 | -3.052 | 1.00 | 28.57 |
| ATOM | 431 | CD2 | TRP | 259 | 21.325 | 34.637 | -2.259 | 1.00 | 30.31 |
| ATOM | 432 | CE2 | TRP | 259 | 20.509 | 35.580 | -1.594 | 1.00 | 31.88 |
| ATOM | 433 | CB3 | TRP | 259 | 21.101 | 33.269 | -2.039 | 1.00 | 31.88 |
| ATOM | 434 | CD1 | TRP | 259 | 21.974 | 36.692 | -2.853 | 1.00 | 29.73 |
| ATOM | 435 | NE1 | TRP | 259 | 20.926 | 36.826 | -1.977 | 1.00 | 31.45 |
| ATOM | 436 | CZ2 | TRP | 259 | 19.484 | 35.202 | -0.721 | 1.00 | 33.37 |
| ATOM | 437 | CZ3 | TRP | 259 | 20.084 | 32.893 | -1.171 | 1.00 | 32.58 |
| ATOM | 438 | CH2 | TRP | 259 | 19.288 | 33.858 | -0.520 | 1.00 | 33.41 |
| ATOM | 439 | C | TRP | 259 | 25.279 | 33.203 | -3.328 | 1.00 | 23.16 |
| ATOM | 440 | O | TRP | 259 | 25.066 | 32.176 | -2.695 | 1.00 | 23.39 |
| ATOM | 441 | N | GLN | 260 | 26.094 | 33.238 | -4.378 | 1.00 | 24.68 |
| ATOM | 442 | CA | GLN | 260 | 26.793 | 32.037 | -4.824 | 1.00 | 24.94 |
| ATOM | 443 | CB | GLN | 260 | 27.666 | 32.352 | -6.046 | 1.00 | 28.06 |
| ATOM | 444 | CG | GLN | 260 | 28.451 | 31.142 | -6.568 | 1.00 | 34.11 |
| ATOM | 445 | CD | GLN | 260 | 29.968 | 31.291 | -6.467 | 1.00 | 38.33 |
| ATOM | 446 | OE1 | GLN | 260 | 30.524 | 31.565 | -5.390 | 1.00 | 39.00 |
| ATOM | 447 | NE2 | GLN | 260 | 30.650 | 31.084 | -7.594 | 1.00 | 40.25 |
| ATOM | 448 | C | GLN | 260 | 27.671 | 31.473 | -3.695 | 1.00 | 24.10 |
| ATOM | 449 | O | GLN | 260 | 27.671 | 30.269 | -3.435 | 1.00 | 23.36 |
| ATOM | 450 | N | GLN | 261 | 28.429 | 32.342 | -3.037 | 1.00 | 22.77 |
| ATOM | 451 | CA | GLN | 261 | 29.302 | 31.926 | -1.940 | 1.00 | 23.59 |
| ATOM | 452 | CB | GLN | 261 | 30.089 | 33.124 | -1.395 | 1.00 | 25.53 |
| ATOM | 453 | CG | GLN | 261 | 31.165 | 33.672 | -2.321 | 1.00 | 31.43 |
| ATOM | 454 | CD | GLN | 261 | 31.847 | 34.924 | -1.754 | 1.00 | 35.45 |
| ATOM | 455 | OE1 | GLN | 261 | 31.616 | 36.053 | -2.226 | 1.00 | 38.71 |
| ATOM | 456 | NE2 | GLN | 261 | 32.678 | 34.732 | -0.733 | 1.00 | 33.46 |
| ATOM | 457 | C | GLN | 261 | 28.508 | 31.295 | -0.796 | 1.00 | 22.79 |
| ATOM | 458 | O | GLN | 261 | 28.893 | 30.254 | -0.255 | 1.00 | 21.63 |
| ATOM | 459 | N | CYS | 262 | 27.404 | 31.940 | -0.425 | 1.00 | 21.36 |
| ATOM | 460 | CA | CYS | 262 | 26.558 | 31.437 | 0.651 | 1.00 | 21.18 |
| ATOM | 461 | CB | CYS | 262 | 25.483 | 32.453 | 1.003 | 1.00 | 20.56 |
| ATOM | 462 | SG | CYS | 262 | 26.149 | 33.886 | 1.830 | 1.00 | 23.42 |
| ATOM | 463 | C | CYS | 262 | 25.907 | 30.110 | 0.287 | 1.00 | 20.19 |
| ATOM | 464 | O | CYS | 262 | 25.800 | 29.216 | 1.128 | 1.00 | 20.82 |
| ATOM | 465 | N | ALA | 263 | 25.475 | 29.977 | -0.962 | 1.00 | 19.51 |
| ATOM | 466 | CA | ALA | 263 | 24.855 | 28.731 | -1.391 | 1.00 | 20.19 |
| ATOM | 467 | CB | ALA | 263 | 24.323 | 28.875 | -2.790 | 1.00 | 19.19 |
| ATOM | 468 | C | ALA | 263 | 25.871 | 27.582 | -1.319 | 1.00 | 20.32 |
| ATOM | 469 | O | ALA | 263 | 25.530 | 26.455 | -0.946 | 1.00 | 20.47 |
| ATOM | 470 | N | ILE | 264 | 27.120 | 27.869 | -1.666 | 1.00 | 19.64 |
| ATOM | 471 | CA | ILE | 264 | 28.163 | 26.844 | -1.613 | 1.00 | 20.56 |
| ATOM | 472 | CB | ILE | 264 | 29.479 | 27.352 | -2.233 | 1.00 | 21.64 |
| ATOM | 473 | CG2 | ILE | 264 | 30.599 | 26.336 | -2.003 | 1.00 | 24.40 |
| ATOM | 474 | CG1 | ILE | 264 | 29.276 | 27.584 | -3.733 | 1.00 | 22.79 |
| ATOM | 475 | CD1 | ILE | 264 | 30.506 | 28.182 | -4.453 | 1.00 | 23.68 |
| ATOM | 476 | C | ILE | 264 | 28.411 | 26.426 | -0.169 | 1.00 | 19.92 |
| ATOM | 477 | O | ILE | 264 | 28.490 | 25.235 | 0.142 | 1.00 | 20.57 |
| ATOM | 478 | N | GLN | 265 | 28.518 | 27.408 | 0.720 | 1.00 | 20.33 |
| ATOM | 479 | CA | GLN | 265 | 28.749 | 27.108 | 2.122 | 1.00 | 20.30 |
| ATOM | 480 | CB | GLN | 265 | 28.961 | 28.392 | 2.919 | 1.00 | 22.53 |
| ATOM | 481 | CG | GLN | 265 | 29.345 | 28.128 | 4.349 | 1.00 | 27.06 |
| ATOM | 482 | CD | GLN | 265 | 30.391 | 27.024 | 4.480 | 1.00 | 30.59 |
| ATOM | 483 | OE1 | GLN | 265 | 31.470 | 27.086 | 3.866 | 1.00 | 31.46 |
| ATOM | 484 | NE2 | GLN | 265 | 30.075 | 26.003 | 5.286 | 1.00 | 31.02 |
| ATOM | 485 | C | GLN | 265 | 27.584 | 26.313 | 2.722 | 1.00 | 19.48 |
| ATOM | 486 | O | GLN | 265 | 27.800 | 25.335 | 3.435 | 1.00 | 18.61 |
| ATOM | 487 | N | ILE | 266 | 26.357 | 26.734 | 2.427 | 1.00 | 18.19 |
| ATOM | 488 | CA | ILE | 266 | 25.167 | 26.049 | 2.933 | 1.00 | 19.33 |
| ATOM | 489 | CB | ILE | 266 | 23.877 | 26.775 | 2.494 | 1.00 | 19.84 |
| ATOM | 490 | CG2 | ILE | 266 | 22.652 | 25.925 | 2.813 | 1.00 | 17.56 |
| ATOM | 491 | CG1 | ILE | 266 | 23.797 | 28.134 | 3.179 | 1.00 | 20.35 |
| ATOM | 492 | CD1 | ILE | 266 | 22.643 | 28.987 | 2.693 | 1.00 | 21.92 |
| ATOM | 493 | C | ILE | 266 | 25.137 | 24.619 | 2.406 | 1.00 | 19.49 |
| ATOM | 494 | O | ILE | 266 | 24.860 | 23.681 | 3.151 | 1.00 | 19.71 |
| ATOM | 495 | N | THR | 267 | 25.427 | 24.452 | 1.120 | 1.00 | 19.07 |
| ATOM | 496 | CA | THR | 267 | 25.426 | 23.117 | 0.529 | 1.00 | 20.39 |
| ATOM | 497 | CB | THR | 267 | 25.732 | 23.173 | -0.973 | 1.00 | 19.62 |
| ATOM | 498 | OG1 | THR | 267 | 24.727 | 23.949 | -1.624 | 1.00 | 18.77 |
| ATOM | 499 | CG2 | THR | 267 | 25.747 | 21.767 | -1.577 | 1.00 | 19.77 |
| ATOM | 500 | C | THR | 267 | 26.476 | 22.239 | 1.204 | 1.00 | 19.70 |
| ATOM | 501 | O | THR | 267 | 26.240 | 21.063 | 1.484 | 1.00 | 19.00 |
| ATOM | 502 | N | HIS | 268 | 27.642 | 22.823 | 1.449 | 1.00 | 18.99 |
| ATOM | 503 | CA | HIS | 268 | 28.734 | 22.113 | 2.098 | 1.00 | 19.17 |
| ATOM | 504 | CB | HIS | 268 | 29.926 | 23.063 | 2.248 | 1.00 | 20.12 |
| ATOM | 505 | CG | HIS | 268 | 31.167 | 22.409 | 2.764 | 1.00 | 22.30 |
| ATOM | 506 | CD2 | HIS | 268 | 32.052 | 22.803 | 3.707 | 1.00 | 24.34 |
| ATOM | 507 | ND1 | HIS | 268 | 31.634 | 21.207 | 2.279 | 1.00 | 24.49 |
| ATOM | 508 | CE1 | HIS | 268 | 32.752 | 20.887 | 2.903 | 1.00 | 23.24 |
| ATOM | 509 | NE2 | HIS | 268 | 33.029 | 21.838 | 3.775 | 1.00 | 23.14 |
| ATOM | 510 | C | HIS | 268 | 28.237 | 21.616 | 3.462 | 1.00 | 19.37 |
| ATOM | 511 | O | HIS | 268 | 28.415 | 20.449 | 3.811 | 1.00 | 19.15 |
| ATOM | 512 | N | ALA | 269 | 27.587 | 22.501 | 4.215 | 1.00 | 17.47 |
| ATOM | 513 | CA | ALA | 269 | 27.050 | 22.139 | 5.525 | 1.00 | 18.95 |
| ATOM | 514 | CB | ALA | 269 | 26.522 | 23.380 | 6.237 | 1.00 | 18.60 |
| ATOM | 515 | C | ALA | 269 | 25.938 | 21.090 | 5.406 | 1.00 | 18.49 |
| ATOM | 516 | O | ALA | 269 | 25.838 | 20.181 | 6.229 | 1.00 | 19.32 |
| ATOM | 517 | N | ILE | 270 | 25.104 | 21.221 | 4.377 | 1.00 | 17.85 |
| ATOM | 518 | CA | ILE | 270 | 24.017 | 20.277 | 4.165 | 1.00 | 17.94 |
| ATOM | 519 | CB | ILE | 270 | 23.113 | 20.735 | 2.999 | 1.00 | 17.72 |
| ATOM | 520 | CG2 | ILE | 270 | 22.238 | 19.569 | 2.502 | 1.00 | 15.68 |
| ATOM | 521 | CG1 | ILE | 270 | 22.256 | 21.914 | 3.476 | 1.00 | 17.69 |
| ATOM | 522 | CD1 | ILE | 270 | 21.442 | 22.593 | 2.381 | 1.00 | 19.17 |
| ATOM | 523 | C | ILE | 270 | 24.571 | 18.883 | 3.889 | 1.00 | 19.48 |
| ATOM | 524 | O | ILE | 270 | 24.049 | 17.881 | 4.377 | 1.00 | 17.18 |
| ATOM | 525 | N | GLN | 271 | 25.644 | 18.815 | 3.114 | 1.00 | 19.72 |
| ATOM | 526 | CA | GLN | 271 | 26.229 | 17.519 | 2.826 | 1.00 | 20.49 |
| ATOM | 527 | CB | GLN | 271 | 27.376 | 17.687 | 1.827 | 1.00 | 21.65 |
| ATOM | 528 | CG | GLN | 271 | 26.832 | 18.047 | 0.439 | 1.00 | 22.49 |
| ATOM | 529 | CD | GLN | 271 | 27.895 | 18.166 | -0.646 | 1.00 | 23.92 |
| ATOM | 530 | OE1 | GLN | 271 | 27.588 | 18.037 | -1.831 | 1.00 | 26.25 |
| ATOM | 531 | NE2 | GLN | 271 | 29.129 | 18.429 | -0.252 | 1.00 | 23.47 |
| ATOM | 532 | C | GLN | 271 | 26.663 | 16.804 | 4.118 | 1.00 | 20.60 |
| ATOM | 533 | O | GLN | 271 | 26.516 | 15.586 | 4.236 | 1.00 | 20.59 |
| ATOM | 534 | N | TYR | 272 | 27.159 | 17.552 | 5.098 | 1.00 | 20.89 |
| ATOM | 535 | CA | TYR | 272 | 27.547 | 16.940 | 6.368 | 1.00 | 21.15 |
| ATOM | 536 | CB | TYR | 272 | 28.329 | 17.933 | 7.231 | 1.00 | 23.11 |
| ATOM | 537 | CG | TYR | 272 | 29.801 | 18.001 | 6.871 | 1.00 | 26.45 |
| ATOM | 538 | CD1 | TYR | 272 | 30.637 | 16.898 | 7.065 | 1.00 | 29.06 |
| ATOM | 539 | CE1 | TYR | 272 | 31.989 | 16.937 | 6.702 | 1.00 | 31.21 |
| ATOM | 540 | CD2 | TYR | 272 | 30.351 | 19.153 | 6.306 | 1.00 | 28.01 |
| ATOM | 541 | CE2 | TYR | 272 | 31.705 | 19.203 | 5.938 | 1.00 | 30.00 |
| ATOM | 542 | CZ | TYR | 272 | 32.513 | 18.091 | 6.140 | 1.00 | 31.38 |
| ATOM | 543 | OH | TYR | 272 | 33.846 | 18.137 | 5.786 | 1.00 | 34.21 |
| ATOM | 544 | C | TYR | 272 | 26.312 | 16.426 | 7.120 | 1.00 | 21.57 |
| ATOM | 545 | O | TYR | 272 | 26.378 | 15.401 | 7.810 | 1.00 | 20.19 |
| ATOM | 546 | N | VAL | 273 | 25.185 | 17.122 | 6.973 | 1.00 | 18.53 |
| ATOM | 547 | CA | VAL | 273 | 23.950 | 16.687 | 7.623 | 1.00 | 18.96 |
| ATOM | 548 | CB | VAL | 273 | 22.832 | 17.740 | 7.496 | 1.00 | 18.38 |
| ATOM | 549 | CG1 | VAL | 273 | 21.529 | 17.186 | 8.052 | 1.00 | 15.65 |
| ATOM | 550 | CG2 | VAL | 273 | 23.229 | 19.004 | 8.247 | 1.00 | 17.10 |
| ATOM | 551 | C | VAL | 273 | 23.474 | 15.365 | 7.007 | 1.00 | 19.57 |
| ATOM | 552 | O | VAL | 273 | 22.881 | 14.526 | 7.687 | 1.00 | 20.30 |
| ATOM | 553 | N | VAL | 274 | 23.731 | 15.181 | 5.718 | 1.00 | 19.26 |
| ATOM | 554 | CA | VAL | 274 | 23.352 | 13.934 | 5.065 | 1.00 | 21.47 |
| ATOM | 555 | CB | VAL | 274 | 23.595 | 13.997 | 3.538 | 1.00 | 19.96 |
| ATOM | 556 | CG1 | VAL | 274 | 23.323 | 12.640 | 2.914 | 1.00 | 22.18 |
| ATOM | 557 | CG2 | VAL | 274 | 22.678 | 15.052 | 2.905 | 1.00 | 20.72 |
| ATOM | 558 | C | VAL | 274 | 24.191 | 12.808 | 5.685 | 1.00 | 21.11 |
| ATOM | 559 | O | VAL | 274 | 23.668 | 11.746 | 6.021 | 1.00 | 22.06 |
| ATOM | 560 | N | GLU | 275 | 25.487 | 13.062 | 5.854 | 1.00 | 22.26 |
| ATOM | 561 | CA | GLU | 275 | 26.401 | 12.088 | 6.446 | 1.00 | 23.73 |
| ATOM | 562 | CB | GLU | 275 | 27.836 | 12.632 | 6.449 | 1.00 | 26.76 |
| ATOM | 563 | CG | GLU | 275 | 28.448 | 12.827 | 5.058 | 1.00 | 30.97 |
| ATOM | 564 | CD | GLU | 275 | 29.000 | 11.534 | 4.446 | 1.00 | 34.75 |
| ATOM | 565 | OE1 | GLU | 275 | 28.264 | 10.512 | 4.404 | 1.00 | 34.78 |
| ATOM | 566 | OE2 | GLU | 275 | 30.180 | 11.553 | 3.995 | 1.00 | 37.39 |
| ATOM | 567 | C | GLU | 275 | 25.954 | 11.803 | 7.875 | 1.00 | 22.91 |
| ATOM | 568 | O | GLU | 275 | 26.046 | 10.674 | 8.354 | 1.00 | 21.80 |
| ATOM | 569 | N | PHE | 276 | 25.468 | 12.842 | 8.547 | 1.00 | 21.94 |
| ATOM | 570 | CA | PHE | 276 | 24.978 | 12.725 | 9.915 | 1.00 | 21.89 |
| ATOM | 571 | CB | PHE | 276 | 24.512 | 14.101 | 10.412 | 1.00 | 20.02 |
| ATOM | 572 | CG | PHE | 276 | 23.891 | 14.091 | 11.790 | 1.00 | 20.99 |
| ATOM | 573 | CD1 | PHE | 276 | 24.507 | 13.431 | 12.850 | 1.00 | 20.12 |
| ATOM | 574 | CD2 | PHE | 276 | 22.722 | 14.817 | 12.040 | 1.00 | 20.12 |
| ATOM | 575 | CE1 | PHE | 276 | 23.975 | 13.502 | 14.144 | 1.00 | 21.36 |
| ATOM | 576 | CE2 | PHE | 276 | 22.182 | 14.895 | 13.325 | 1.00 | 20.21 |
| ATOM | 577 | CZ | PHE | 276 | 22.808 | 14.239 | 14.381 | 1.00 | 19.91 |
| ATOM | 578 | C | PHE | 276 | 23.809 | 11.741 | 9.935 | 1.00 | 21.78 |
| ATOM | 579 | O | PRE | 276 | 23.815 | 10.771 | 10.688 | 1.00 | 22.96 |
| ATOM | 580 | N | ALA | 277 | 22.813 | 11.997 | 9.091 | 1.00 | 21.26 |
| ATOM | 581 | CA | ALA | 277 | 21.626 | 11.148 | 9.012 | 1.00 | 21.02 |
| ATOM | 582 | CB | ALA | 277 | 20.650 | 11.705 | 7.979 | 1.00 | 19.08 |
| ATOM | 583 | C | ALA | 277 | 21.968 | 9.700 | 8.671 | 1.00 | 21.78 |
| ATOM | 584 | O | ALA | 277 | 21.450 | 8.774 | 9.294 | 1.00 | 22.63 |
| ATOM | 585 | N | LYS | 278 | 22.836 | 9.511 | 7.683 | 1.00 | 23.13 |
| ATOM | 586 | CA | LYS | 278 | 23.240 | 8.173 | 7.256 | 1.00 | 25.28 |
| ATOM | 587 | CB | LYS | 278 | 24.209 | 8.275 | 6.078 | 1.00 | 24.01 |
| ATOM | 588 | CG | LYS | 278 | 23.561 | 8.837 | 4.803 | 1.00 | 26.41 |
| ATOM | 589 | CD | LYS | 278 | 24.575 | 8.997 | 3.689 | 1.00 | 24.85 |
| ATOM | 590 | CE | LYS | 278 | 25.265 | 7.676 | 3.395 | 1.00 | 25.80 |
| ATOM | 591 | NZ | LYS | 278 | 26.214 | 7.801 | 2.259 | 1.00 | 28.08 |
| ATOM | 592 | C | LYS | 278 | 23.862 | 7.317 | 8.361 | 1.00 | 25.78 |
| ATOM | 593 | O | LYS | 278 | 23.866 | 6.091 | 8.271 | 1.00 | 26.96 |
| ATOM | 594 | N | ARG | 279 | 24.388 | 7.950 | 9.400 | 1.00 | 26.39 |
| ATOM | 595 | CA | ARG | 279 | 24.999 | 7.195 | 10.482 | 1.00 | 27.59 |
| ATOM | 596 | CB | ARG | 279 | 26.266 | 7.893 | 10.953 | 1.00 | 28.22 |
| ATOM | 597 | CG | ARG | 279 | 27.190 | 8.219 | 9.794 | 1.00 | 31.75 |
| ATOM | 598 | CD | ARG | 279 | 28.645 | 8.302 | 10.202 | 1.00 | 33.61 |
| ATOM | 599 | NE | ARG | 279 | 29.490 | 8.575 | 9.047 | 1.00 | 35.68 |
| ATOM | 600 | CZ | ARG | 279 | 30.818 | 8.479 | 9.035 | 1.00 | 38.22 |
| ATOM | 601 | NH1 | ARG | 279 | 31.478 | 8.108 | 10.128 | 1.00 | 38.46 |
| ATOM | 602 | NH2 | ARG | 279 | 31.488 | 8.760 | 7.920 | 1.00 | 37.61 |
| ATOM | 603 | C | ARG | 279 | 24.053 | 6.936 | 11.652 | 1.00 | 27.34 |
| ATOM | 604 | O | ARG | 279 | 24.397 | 6.217 | 12.589 | 1.00 | 28.95 |
| ATOM | 605 | N | ILE | 280 | 22.854 | 7.503 | 11.596 | 1.00 | 25.28 |
| ATOM | 606 | CA | ILE | 280 | 21.886 | 7.270 | 12.661 | 1.00 | 24.59 |
| ATOM | 607 | CB | ILE | 280 | 20.922 | 8.464 | 12.827 | 1.00 | 22.88 |
| ATOM | 608 | CG2 | ILE | 280 | 19.884 | 8.152 | 13.912 | 1.00 | 21.69 |
| ATOM | 609 | CG1 | ILE | 280 | 21.708 | 9.722 | 13.219 | 1.00 | 21.57 |
| ATOM | 610 | CD1 | ILE | 280 | 20.835 | 10.965 | 13.380 | 1.00 | 20.98 |
| ATOM | 611 | C | ILE | 280 | 21.085 | 6.014 | 12.315 | 1.00 | 24.77 |
| ATOM | 612 | O | ILE | 280 | 20.181 | 6.051 | 11.481 | 1.00 | 23.84 |
| ATOM | 613 | N | THR | 281 | 21.421 | 4.902 | 12.967 | 1.00 | 25.98 |
| ATOM | 614 | CA | THR | 281 | 20.754 | 3.628 | 12.706 | 1.00 | 27.40 |
| ATOM | 615 | CB | THR | 281 | 21.064 | 2.608 | 13.805 | 1.00 | 28.22 |
| ATOM | 616 | OG1 | THR | 281 | 22.485 | 2.514 | 13.970 | 1.00 | 32.17 |
| ATOM | 617 | CG2 | THR | 281 | 20.518 | 1.237 | 13.422 | 1.00 | 30.45 |
| ATOM | 618 | C | THR | 281 | 19.236 | 3.720 | 12.547 | 1.00 | 26.89 |
| ATOM | 619 | O | THR | 281 | 18.685 | 3.173 | 11.596 | 1.00 | 27.85 |
| ATOM | 620 | N | GLY | 282 | 18.568 | 4.405 | 13.468 | 1.00 | 25.97 |
| ATOM | 621 | CA | GLY | 282 | 17.120 | 4.536 | 13.395 | 1.00 | 24.93 |
| ATOM | 622 | C | GLY | 282 | 16.605 | 5.255 | 12.156 | 1.00 | 25.15 |
| ATOM | 623 | O | GLY | 282 | 15.502 | 4.981 | 11.680 | 1.00 | 23.70 |
| ATOM | 624 | N | PHE | 283 | 17.400 | 6.187 | 11.639 | 1.00 | 24.97 |
| ATOM | 625 | CA | PHE | 283 | 17.035 | 6.948 | 10.439 | 1.00 | 25.33 |
| ATOM | 626 | CB | PHE | 283 | 17.958 | 8.169 | 10.295 | 1.00 | 23.28 |
| ATOM | 627 | CG | PHE | 283 | 17.757 | 8.945 | 9.017 | 1.00 | 23.75 |
| ATOM | 628 | CD1 | PHE | 283 | 18.377 | 8.548 | 7.837 | 1.00 | 23.86 |
| ATOM | 629 | CD2 | PHE | 283 | 16.940 | 10.069 | 8.994 | 1.00 | 22.16 |
| ATOM | 630 | CE1 | PHE | 283 | 18.185 | 9.261 | 6.654 | 1.00 | 23.03 |
| ATOM | 631 | CE2 | PHE | 283 | 16.743 | 10.784 | 7.820 | 1.00 | 23.21 |
| ATOM | 632 | CZ | PHE | 283 | 17.367 | 10.382 | 6.648 | 1.00 | 20.80 |
| ATOM | 633 | C | PHE | 283 | 17.141 | 6.072 | 9.185 | 1.00 | 25.72 |
| ATOM | 634 | O | PHE | 283 | 16.287 | 6.128 | 8.295 | 1.00 | 26.38 |
| ATOM | 635 | N | MET | 284 | 18.194 | 5.265 | 9.115 | 1.00 | 26.64 |
| ATOM | 636 | CA | MET | 284 | 18.384 | 4.398 | 7.960 | 1.00 | 28.17 |
| ATOM | 637 | CB | MET | 284 | 19.825 | 3.887 | 7.916 | 1.00 | 29.11 |
| ATOM | 638 | CG | MET | 284 | 20.836 | 4.994 | 7.618 | 1.00 | 30.38 |
| ATOM | 639 | SD | MET | 284 | 20.370 | 6.064 | 6.205 | 1.00 | 33.12 |
| ATOM | 640 | CE | MET | 284 | 20.937 | 5.078 | 4.772 | 1.00 | 34.35 |
| ATOM | 641 | C | MET | 284 | 17.395 | 3.235 | 7.917 | 1.00 | 29.78 |
| ATOM | 642 | O | MET | 284 | 17.313 | 2.516 | 6.917 | 1.00 | 28.85 |
| ATOM | 643 | N | GLU | 285 | 16.629 | 3.068 | 8.991 | 1.00 | 31.01 |
| ATOM | 644 | CA | GLU | 285 | 15.638 | 1.999 | 9.060 | 1.00 | 31.97 |
| ATOM | 645 | CB | GLU | 285 | 15.439 | 1.536 | 10.507 | 1.00 | 33.56 |
| ATOM | 646 | CG | GLU | 285 | 16.658 | 0.814 | 11.078 | 1.00 | 37.02 |
| ATOM | 647 | CD | GLU | 285 | 16.417 | 0.259 | 12.469 | 1.00 | 38.77 |
| ATOM | 648 | OE1 | GLU | 285 | 15.390 | 0.617 | 13.090 | 1.00 | 39.52 |
| ATOM | 649 | OE2 | GLU | 285 | 17.265 | -0.529 | 12.942 | 1.00 | 40.58 |
| ATOM | 650 | C | GLU | 285 | 14.306 | 2.444 | 8.479 | 1.00 | 31.93 |
| ATOM | 651 | O | GLU | 285 | 13.430 | 1.619 | 8.211 | 1.00 | 32.23 |
| ATOM | 652 | N | LEU | 286 | 14.153 | 3.754 | 8.289 | 1.00 | 30.77 |
| ATOM | 653 | CA | LEU | 286 | 12.935 | 4.315 | 7.715 | 1.00 | 30.76 |
| ATOM | 654 | CB | LEU | 286 | 12.890 | 5.835 | 7.931 | 1.00 | 29.72 |
| ATOM | 655 | CG | LEU | 286 | 13.009 | 6.367 | 9.364 | 1.00 | 29.96 |
| ATOM | 656 | CD1 | LEU | 286 | 12.937 | 7.884 | 9.339 | 1.00 | 28.70 |
| ATOM | 657 | CD2 | LEU | 286 | 11.890 | 5.805 | 10.235 | 1.00 | 29.71 |
| ATOM | 658 | C | LEU | 286 | 12.960 | 4.011 | 6.220 | 1.00 | 29.96 |
| ATOM | 659 | O | LEU | 286 | 14.021 | 3.715 | 5.666 | 1.00 | 30.54 |
| ATOM | 660 | N | CYS | 287 | 11.814 | 4.075 | 5.555 | 1.00 | 30.89 |
| ATOM | 661 | CA | CYS | 287 | 11.821 | 3.791 | 4.123 | 1.00 | 31.57 |
| ATOM | 662 | CB | CYS | 287 | 10.400 | 3.677 | 3.555 | 1.00 | 32.51 |
| ATOM | 663 | SG | CYS | 287 | 9.395 | 5.168 | 3.569 | 1.00 | 35.47 |
| ATOM | 664 | C | CYS | 287 | 12.579 | 4.921 | 3.457 | 1.00 | 31.76 |
| ATOM | 665 | O | CYS | 287 | 12.504 | 6.071 | 3.891 | 1.00 | 30.50 |
| ATOM | 666 | N | GLN | 288 | 13.327 | 4.591 | 2.411 | 1.00 | 32.56 |
| ATOM | 667 | CA | GLN | 288 | 14.134 | 5.591 | 1.723 | 1.00 | 32.18 |
| ATOM | 668 | CB | GLN | 288 | 14.818 | 4.960 | 0.519 | 1.00 | 34.37 |
| ATOM | 669 | CG | GLN | 288 | 16.070 | 5.697 | 0.077 | 1.00 | 37.44 |
| ATOM | 670 | CD | GLN | 288 | 16.766 | 4.982 | -1.045 | 1.00 | 38.80 |
| ATOM | 671 | OE1 | GLN | 288 | 16.178 | 4.741 | -2.095 | 1.00 | 40.25 |
| ATOM | 672 | NE2 | GLN | 288 | 18.023 | 4.626 | -0.832 | 1.00 | 41.12 |
| ATOM | 673 | C | GLN | 288 | 13.304 | 6.800 | 1.305 | 1.00 | 31.30 |
| ATOM | 674 | O | GLN | 288 | 13.809 | 7.918 | 1.213 | 1.00 | 29.76 |
| ATOM | 675 | N | ASN | 289 | 12.019 | 6.586 | 1.063 | 1.00 | 29.60 |
| ATOM | 676 | CA | ASN | 289 | 11.180 | 7.701 | 0.685 | 1.00 | 30.09 |
| ATOM, | 677 | CB | ASN | 289 | 9.789 | 7.222 | 0.320 | 1.00 | 32.43 |
| ATOM | 678 | CG | ASN | 289 | 8.831 | 8.363 | 0.186 | 1.00 | 34.94 |
| ATOM | 679 | OD1 | ASN | 289 | 8.262 | 8.837 | 1.177 | 1.00 | 37.17 |
| ATOM | 680 | ND2 | ASN | 289 | 8.670 | 8.850 | -1.038 | 1.00 | 37.46 |
| ATOM | 681 | C | ASN | 289 | 11.065 | 8.737 | 1.803 | 1.00 | 28.55 |
| ATOM | 682 | O | ASN | 289 | 11.120 | 9.954 | 1.561 | 1.00 | 28.23 |
| ATOM | 683 | N | ASP | 290 | 10.880 | 8.253 | 3.027 | 1.00 | 27.44 |
| ATOM | 684 | CA | ASP | 290 | 10.750 | 9.146 | 4.166 | 1.00 | 25.42 |
| ATOM | 685 | CB | ASP | 290 | 10.100 | 8.423 | 5.351 | 1.00 | 25.58 |
| ATOM | 686 | CG | ASP | 290 | 8.593 | 8.245 | 5.159 | 1.00 | 25.63 |
| ATOM | 687 | OD1 | ASP | 290 | 8.033 | 8.887 | 4.247 | 1.00 | 25.74 |
| ATOM | 688 | OD2 | ASP | 290 | 7.966 | 7.479 | 5.918 | 1.00 | 24.50 |
| ATOM | 689 | C | ASP | 290 | 12.116 | 9.712 | 4.541 | 1.00 | 24.26 |
| ATOM | 690 | O | ASP | 290 | 12.205 | 10.793 | 5.113 | 1.00 | 23.09 |
| ATOM | 691 | N | GLN | 291 | 13.176 | 8.984 | 4.203 | 1.00 | 24.37 |
| ATOM | 692 | CA | GLN | 291 | 14.525 | 9.453 | 4.482 | 1.00 | 23.42 |
| ATOM | 693 | CB | GLN | 291 | 15.562 | 8.400 | 4.092 | 1.00 | 23.59 |
| ATOM | 694 | CG | GLN | 291 | 15.608 | 7.164 | 4.965 | 1.00 | 22.66 |
| ATOM | 695 | CD | GLN | 291 | 16.639 | 6.173 | 4.464 | 1.00 | 22.68 |
| ATOM | 696 | OE1 | GLN | 291 | 17.532 | 6.527 | 3.694 | 1.00 | 24.72 |
| ATOM | 697 | NE2 | GLN | 291 | 16.532 | 4.930 | 4.908 | 1.00 | 24.25 |
| ATOM | 698 | C | GLN | 291 | 14.773 | 10.704 | 3.649 | 1.00 | 23.72 |
| ATOM | 699 | O | GLN | 291 | 15.328 | 11.688 | 4.134 | 1.00 | 21.67 |
| ATOM | 700 | N | ILE | 292 | 14.358 | 10.648 | 2.384 | 1.00 | 23.54 |
| ATOM | 701 | CA | ILE | 292 | 14.541 | 11.765 | 1.462 | 1.00 | 23.40 |
| ATOM | 702 | CB | ILE | 292 | 14.166 | 11.363 | -0.003 | 1.00 | 24.40 |
| ATOM | 703 | CG2 | ILE | 292 | 14.176 | 12.592 | -0.913 | 1.00 | 24.00 |
| ATOM | 704 | CG1 | ILB | 292 | 15.160 | 10.326 | -0.535 | 1.00 | 25.84 |
| ATOM | 705 | CD1 | ILE | 292 | 16.582 | 10.825 | -0.654 | 1.00 | 28.34 |
| ATOM | 706 | C | ILE | 292 | 13.681 | 12.938 | 1.903 | 1.00 | 22.88 |
| ATOM | 707 | O | ILE | 292 | 14.148 | 14.078 | 1.952 | 1.00 | 22.08 |
| ATOM | 708 | N | LEU | 293 | 12.430 | 12.649 | 2.245 | 1.00 | 21.21 |
| ATOM | 709 | CA | LEU | 293 | 11.513 | 13.679 | 2.684 | 1.00 | 22.20 |
| ATOM | 710 | CB | LEU | 293 | 10.128 | 13.077 | 2.945 | 1.00 | 22.87 |
| ATOM | 711 | CG | LEU | 293 | 9.337 | 12.783 | 1.668 | 1.00 | 26.65 |
| ATOM | 712 | CD1 | LEU | 293 | 8.100 | 11.943 | 1.981 | 1.00 | 27.63 |
| ATOM | 713 | CD2 | LEU | 293 | 8.947 | 14.115 | 1.009 | 1.00 | 27.30 |
| ATOM | 714 | C | LEU | 293 | 12.041 | 14.366 | 3.936 | 1.00 | 21.47 |
| ATOM | 715 | O | LEU | 293 | 12.013 | 15.588 | 4.034 | 1.00 | 20.74 |
| ATOM | 716 | N | LEU | 294 | 12.532 | 13.578 | 4.887 | 1.00 | 22.09 |
| ATOM | 717 | CA | LEU | 294 | 13.053 | 14.158 | 6.111 | 1.00 | 21.85 |
| ATOM | 718 | CB | LEU | 294 | 13.410 | 13.062 | 7.120 | 1.00 | 22.49 |
| ATOM | 719 | CG | LEU | 294 | 12.210 | 12.367 | 7.770 | 1.00 | 23.61 |
| ATOM | 720 | CD1 | LEU | 294 | 12.693 | 11.448 | 8.877 | 1.00 | 23.93 |
| ATOM | 721 | CD2 | LEU | 294 | 11.254 | 13.400 | 8.339 | 1.00 | 23.56 |
| ATOM | 722 | C | LEU | 294 | 14.262 | 15.034 | 5.818 | 1.00 | 20.92 |
| ATOM | 723 | O | LEU | 294 | 14.371 | 16.138 | 6.335 | 1.00 | 20.70 |
| ATOM | 724 | N | LEU | 295 | 15.170 | 14.549 | 4.979 | 1.00 | 21.44 |
| ATOM | 725 | CA | LEU | 295 | 16.352 | 15.334 | 4.636 | 1.00 | 21.46 |
| ATOM | 726 | CB | LEU | 295 | 17.325 | 14.481 | 3.836 | 1.00 | 21.73 |
| ATOM | 727 | CG | LEU | 295 | 18.087 | 13.499 | 4.725 | 1.00 | 24.02 |
| ATOM | 728 | CD1 | LEU | 295 | 18.824 | 12.477 | 3.879 | 1.00 | 23.40 |
| ATOM | 729 | CD2 | LEU | 295 | 19.053 | 14.292 | 5.602 | 1.00 | 25.66 |
| ATOM | 730 | C | LEU | 295 | 16.020 | 16.605 | 3.857 | 1.00 | 21.70 |
| ATOM | 731 | O | LEU | 295 | 16.537 | 17.682 | 4.158 | 1.00 | 19.82 |
| ATOM | 732 | N | LYS | 296 | 15.158 | 16.473 | 2.855 | 1.00 | 21.79 |
| ATOM | 733 | CA | LYS | 296 | 14.769 | 17.605 | 2.029 | 1.00 | 25.11 |
| ATOM | 734 | CB | LYS | 296 | 13.821 | 17.139 | 0.919 | 1.00 | 27.17 |
| ATOM | 735 | CG | LYS | 296 | 13.241 | 18.257 | 0.084 | 1.00 | 30.32 |
| ATOM | 736 | CD | LYS | 296 | 12.261 | 17.694 | -0.938 | 1.00 | 33.40 |
| ATOM | 737 | CE | LYS | 296 | 11.800 | 18.766 | -1.903 | 1.00 | 35.42 |
| ATOM | 738 | NZ | LYS | 296 | 12.910 | 19.218 | -2.791 | 1.00 | 37.44 |
| ATOM | 739 | C | LYS | 296 | 14.109 | 18.714 | 2.831 | 1.00 | 23.87 |
| ATOM | 740 | O | LYS | 296 | 14.348 | 19.902 | 2.582 | 1.00 | 23.29 |
| ATOM | 741 | N | SER | 297 | 13.287 | 18.326 | 3.798 | 1.00 | 23.52 |
| ATOM | 742 | CA | SER | 297 | 12.587 | 19.294 | 4.628 | 1.00 | 24.66 |
| ATOM | 743 | CB | SER | 297 | 11.243 | 18.707 | 5.086 | 1.00 | 25.85 |
| ATOM | 744 | OG | SER | 297 | 11.426 | 17.494 | 5.798 | 1.00 | 25.78 |
| ATOM | 745 | C | SER | 297 | 13.374 | 19.791 | 5.852 | 1.00 | 24.58 |
| ATOM | 746 | O | SER | 297 | 13.123 | 20.898 | 6.338 | 1.00 | 25.12 |
| ATOM | 747 | N | GLY | 298 | 14.333 | 19.002 | 6.328 | 1.00 | 22.15 |
| ATOM | 748 | CA | GLY | 298 | 15.068 | 19.406 | 7.511 | 1.00 | 22.02 |
| ATOM | 749 | C | GLY | 298 | 16.570 | 19.616 | 7.445 | 1.00 | 20.47 |
| ATOM | 750 | O | GLY | 298 | 17.160 | 20.017 | 8.437 | 1.00 | 20.04 |
| ATOM | 751 | N | CYS | 299 | 17.203 | 19.363 | 6.306 | 1.00 | 20.94 |
| ATOM | 752 | CA | CYS | 299 | 18.649 | 19.543 | 6.237 | 1.00 | 21.39 |
| ATOM | 753 | CB | CYS | 299 | 19.182 | 19.250 | 4.827 | 1.00 | 23.06 |
| ATOM | 754 | SG | CYS | 299 | 18.429 | 20.203 | 3.482 | 1.00 | 29.00 |
| ATOM | 755 | C | CYS | 299 | 19.039 | 20.951 | 6.658 | 1.00 | 20.07 |
| ATOM | 756 | O | CYS | 299 | 19.907 | 21.121 | 7.506 | 1.00 | 18.32 |
| ATOM | 757 | N | LEU | 300 | 18.379 | 21.958 | 6.097 | 1.00 | 18.43 |
| ATOM | 758 | CA | LEU | 300 | 18.704 | 23.335 | 6.443 | 1.00 | 18.89 |
| ATOM | 759 | CB | LEU | 300 | 17.942 | 24.303 | 5.540 | 1.00 | 17.66 |
| ATOM | 760 | CG | LEU | 300 | 18.466 | 25.744 | 5.496 | 1.00 | 18.57 |
| ATOM | 761 | CD1 | LEU | 300 | 19.961 | 25.753 | 5.152 | 1.00 | 18.04 |
| ATOM | 762 | CD2 | LEU | 300 | 17.670 | 26.535 | 4.469 | 1.00 | 18.71 |
| ATOM | 763 | C | LEU | 300 | 18.424 | 23.657 | 7.913 | 1.00 | 18.49 |
| ATOM | 764 | O | LEU | 300 | 19.151 | 24.435 | 8.535 | 1.00 | 18.49 |
| ATOM | 765 | N | GLU | 301 | 17.368 | 23.061 | 8.462 | 1.00 | 18.69 |
| ATOM | 766 | CA | GLU | 301 | 17.020 | 23.278 | 9.858 | 1.00 | 18.42 |
| ATOM | 767 | CB | GLU | 301 | 15.680 | 22.604 | 10.181 | 1.00 | 18.90 |
| ATOM | 768 | CG | GLU | 301 | 14.505 | 23.235 | 9.443 | 1.00 | 21.00 |
| ATOM | 769 | CD | GLU | 301 | 13.163 | 22.710 | 9.897 | 1.00 | 22.11 |
| ATOM | 770 | OE1 | GLU | 301 | 13.145 | 21.861 | 10.809 | 1.00 | 22.99 |
| ATOM | 771 | OE2 | GLU | 301 | 12.128 | 23.156 | 9.342 | 1.00 | 23.74 |
| ATOM | 772 | C | GLU | 301 | 18.137 | 22.729 | 10.744 | 1.00 | 18.45 |
| ATOM | 773 | O | GLU | 301 | 18.488 | 23.327 | 11.764 | 1.00 | 17.98 |
| ATOM | 774 | N | VAL | 302 | 18.705 | 21.594 | 10.354 | 1.00 | 18.55 |
| ATOM | 775 | CA | VAL | 302 | 19.804 | 21.013 | 11.126 | 1.00 | 18.30 |
| ATOM | 776 | CB | VAL | 302 | 20.165 | 19.597 | 10.631 | 1.00 | 17.49 |
| ATOM | 777 | CG1 | VAL | 302 | 21.417 | 19.094 | 11.337 | 1.00 | 17.23 |
| ATOM | 778 | CG2 | VAL | 302 | 19.003 | 18.660 | 10.888 | 1.00 | 18.62 |
| ATOM | 779 | C | VAL | 302 | 21.037 | 21.913 | 10.991 | 1.00 | 18.44 |
| ATOM | 780 | O | VAL | 302 | 21.762 | 22.142 | 11.959 | 1.00 | 18.67 |
| ATOM | 781 | N | VAL | 303 | 21.274 | 22.413 | 9.782 | 1.00 | 17.76 |
| ATOM | 782 | CA | VAL | 303 | 22.406 | 23.296 | 9.546 | 1.00 | 17.41 |
| ATOM | 783 | CB | VAL | 303 | 22.461 | 23.762 | 8.074 | 1.00 | 17.57 |
| ATOM | 784 | CG1 | VAL | 303 | 23.544 | 24.829 | 7.906 | 1.00 | 16.57 |
| ATOM | 785 | CG2 | VAL | 303 | 22.737 | 22.574 | 7.156 | 1.00 | 18.12 |
| ATOM | 786 | C | VAL | 303 | 22.260 | 24.528 | 10.435 | 1.00 | 18.40 |
| ATOM | 787 | O | VAL | 303 | 23.219 | 24.985 | 11.051 | 1.00 | 18.46 |
| ATOM | 788 | N | LEU | 304 | 21.043 | 25.057 | 10.495 | 1.00 | 17.81 |
| ATOM | 789 | CA | LEU | 304 | 20.771 | 26.237 | 11.296 | 1.00 | 18.40 |
| ATOM | 790 | CB | LEU | 304 | 19.310 | 26.664 | 11.145 | 1.00 | 18.67 |
| ATOM | 791 | CG | LEU | 304 | 18.905 | 27.901 | 11.954 | 1.00 | 18.74 |
| ATOM | 792 | CD1 | LEU | 304 | 19.798 | 29.072 | 11.572 | 1.00 | 19.24 |
| ATOM | 793 | CD2 | LEU | 304 | 17.439 | 28.224 | 11.690 | 1.00 | 19.46 |
| ATOM | 794 | C | LEU | 304 | 21.089 | 25.994 | 12.762 | 1.00 | 19.34 |
| ATOM | 795 | O | LEU | 304 | 21.642 | 26.873 | 13.422 | 1.00 | 18.80 |
| ATOM | 796 | N | VAL | 305 | 20.720 | 24.811 | 13.265 | 1.00 | 19.25 |
| ATOM | 797 | CA | VAL | 305 | 20.984 | 24.439 | 14.655 | 1.00 | 19.61 |
| ATOM | 798 | CB | VAL | 305 | 20.342 | 23.072 | 15.031 | 1.00 | 20.39 |
| ATOM | 799 | CG1 | VAL | 305 | 20.877 | 22.596 | 16.381 | 1.00 | 18.01 |
| ATOM | 800 | CG2 | VAL | 305 | 18.819 | 23.192 | 15.081 | 1.00 | 18.19 |
| ATOM | 801 | C | VAL | 305 | 22.495 | 24.320 | 14.828 | 1.00 | 20.18 |
| ATOM | 802 | O | VAL | 305 | 23.059 | 24.849 | 15.779 | 1.00 | 20.30 |
| ATOM | 803 | N | ARG | 306 | 23.142 | 23.628 | 13.896 | 1.00 | 19.99 |
| ATOM | 804 | CA | ARG | 306 | 24.587 | 23.456 | 13.957 | 1.00 | 21.89 |
| ATOM | 805 | CB | ARG | 306 | 25.063 | 22.541 | 12.836 | 1.00 | 21.78 |
| ATOM | 806 | CG | ARG | 306 | 24.838 | 21.076 | 13.112 | 1.00 | 19.29 |
| ATOM | 807 | CD | ARG | 306 | 25.401 | 20.245 | 11.990 | 1.00 | 20.34 |
| ATOM | 808 | NE | ARG | 306 | 25.516 | 18.853 | 12.393 | 1.00 | 22.00 |
| ATOM | 809 | CZ | ARG | 306 | 26.166 | 17.928 | 11.702 | 1.00 | 22.25 |
| ATOM | 810 | NH1 | ARG | 306 | 26.760 | 18.247 | 10.558 | 1.00 | 20.55 |
| ATOM | 811 | NH2 | ARG | 306 | 26.241 | 16.692 | 12.173 | 1.00 | 22.86 |
| ATOM | 812 | C | ARG | 306 | 25.373 | 24.763 | 13.908 | 1.00 | 22.45 |
| ATOM | 813 | O | ARG | 306 | 26.474 | 24.840 | 14.445 | 1.00 | 22.73 |
| ATOM | 814 | N | MET | 307 | 24.805 | 25.785 | 13.277 | 1.00 | 24.18 |
| ATOM | 815 | CA | MET | 307 | 25.470 | 27.084 | 13.169 | 1.00 | 26.27 |
| ATOM | 816 | CB | MET | 307 | 24.608 | 28.046 | 12.353 | 1.00 | 27.38 |
| ATOM | 817 | CG | MET | 307 | 25.231 | 29.412 | 12.170 | 1.00 | 28.81 |
| ATOM | 818 | SD | MET | 307 | 24.099 | 30.460 | 11.288 | 1.00 | 30.68 |
| ATOM | 819 | CE | MET | 307 | 23.147 | 31.073 | 12.604 | 1.00 | 27.74 |
| ATOM | 820 | C | MET | 307 | 25.763 | 27.708 | 14.532 | 1.00 | 25.58 |
| ATOM | 821 | O | MET | 307 | 26.731 | 28.453 | 14.701 | 1.00 | 25.50 |
| ATOM | 822 | N | CYS | 308 | 24.921 | 27.400 | 15.505 | 1.00 | 26.62 |
| ATOM | 823 | CA | CYS | 308 | 25.087 | 27.935 | 16.842 | 1.00 | 25.94 |
| ATOM | 824 | CB | CYS | 308 | 23.901 | 27.512 | 17.693 | 1.00 | 27.50 |
| ATOM | 825 | SG | CYS | 308 | 22.349 | 27.999 | 16.879 | 1.00 | 32.50 |
| ATOM | 826 | C | CYS | 308 | 26.411 | 27.483 | 17.451 | 1.00 | 25.08 |
| ATOM | 827 | O | CYS | 308 | 26.951 | 28.139 | 18.337 | 1.00 | 26.40 |
| ATOM | 828 | N | ARG | 309 | 26.932 | 26.367 | 16.958 | 1.00 | 24.15 |
| ATOM | 829 | CA | ARG | 309 | 28.212 | 25.836 | 17.426 | 1.00 | 23.77 |
| ATOM | 830 | CB | ARG | 309 | 28.514 | 24.490 | 16.763 | 1.00 | 22.37 |
| ATOM | 831 | CG | ARG | 309 | 27.526 | 23.377 | 17.070 | 1.00 | 23.29 |
| ATOM | 832 | CD | ARG | 309 | 27.900 | 22.123 | 16.296 | 1.00 | 22.66 |
| ATOM | 833 | NE | ARG | 309 | 29.133 | 21.499 | 16.778 | 1.00 | 23.68 |
| ATOM | 834 | CZ | ARG | 309 | 30.260 | 21.397 | 16.079 | 1.00 | 22.00 |
| ATOM | 835 | NH1 | ARG | 309 | 30.337 | 21.884 | 14.850 | 1.00 | 20.45 |
| ATOM | 836 | NH2 | ARG | 309 | 31.309 | 20.777 | 16.612 | 1.00 | 21.98 |
| ATOM | 837 | C | ARG | 309 | 29.337 | 26.792 | 17.043 | 1.00 | 23.79 |
| ATOM | 838 | O | ARG | 309 | 30.334 | 26.918 | 17.758 | 1.00 | 23.81 |
| ATOM | 839 | N | ALA | 310 | 29.167 | 27.438 | 15.892 | 1.00 | 23.32 |
| ATOM | 840 | CA | ALA | 310 | 30.167 | 28.354 | 15.344 | 1.00 | 22.67 |
| ATOM | 841 | CB | ALA | 310 | 30.501 | 27.953 | 13.903 | 1.00 | 23.22 |
| ATOM | 842 | C | ALA | 310 | 29.690 | 29.796 | 15.376 | 1.00 | 22.59 |
| ATOM | 843 | O | ALA | 310 | 29.895 | 30.549 | 14.424 | 1.00 | 22.73 |
| ATOM | 844 | N | PHE | 311 | 29.047 | 30.170 | 16.471 | 1.00 | 22.48 |
| ATOM | 845 | CA | PHE | 311 | 28.552 | 31.524 | 16.639 | 1.00 | 23.10 |
| ATOM | 846 | CB | PHE | 311 | 27.025 | 31.516 | 16.677 | 1.00 | 22.61 |
| ATOM | 847 | CG | PHE | 311 | 26.419 | 32.879 | 16.811 | 1.00 | 24.45 |
| ATOM | 848 | CD1 | PRE | 311 | 26.166 | 33.422 | 18.066 | 1.00 | 25.03 |
| ATOM | 849 | CD2 | PHE | 311 | 26.111 | 33.625 | 15.677 | 1.00 | 24.86 |
| ATOM | 850 | CE1 | PRE | 311 | 25.610 | 34.697 | 18.189 | 1.00 | 26.19 |
| ATOM | 851 | CE2 | PHE | 311 | 25.555 | 34.904 | 15.787 | 1.00 | 25.65 |
| ATOM | 852 | CZ | PHE | 311 | 25.303 | 35.440 | 17.044 | 1.00 | 25.80 |
| ATOM | 853 | C | PHE | 311 | 29.118 | 32.064 | 17.950 | 1.00 | 23.95 |
| ATOM | 854 | O | PHE | 311 | 29.225 | 31.332 | 18.926 | 1.00 | 24.13 |
| ATOM | 855 | N | ASN | 312 | 29.496 | 33.336 | 17.965 | 1.00 | 23.37 |
| ATOM | 856 | CA | ASN | 312 | 30.041 | 33.928 | 19.170 | 1.00 | 23.53 |
| ATOM | 857 | CB | ASN | 312 | 31.413 | 34.524 | 18.884 | 1.00 | 24.46 |
| ATOM | 858 | CG | ASN | 312 | 32.066 | 35.059 | 20.125 | 1.00 | 25.90 |
| ATOM | 859 | OD1 | ASN | 312 | 31.439 | 35.112 | 21.187 | 1.00 | 28.29 |
| ATOM | 860 | ND2 | ASN | 312 | 33.326 | 35.465 | 20.009 | 1.00 | 25.97 |
| ATOM | 861 | C | ASN | 312 | 29.091 | 35.015 | 19.670 | 1.00 | 23.70 |
| ATOM | 862 | O | ASN | 312 | 29.051 | 36.121 | 19.127 | 1.00 | 22.70 |
| ATOM | 863 | N | PRO | 313 | 28.309 | 34.707 | 20.715 | 1.00 | 23.93 |
| ATOM | 864 | CD | PRO | 313 | 28.291 | 33.413 | 21.415 | 1.00 | 24.49 |
| ATOM | 865 | CA | PRO | 313 | 27.340 | 35.635 | 21.307 | 1.00 | 25.73 |
| ATOM | 866 | CB | PRO | 313 | 26.561 | 34.748 | 22.283 | 1.00 | 25.50 |
| ATOM | 867 | CG | PRO | 313 | 27.557 | 33.738 | 22.691 | 1.00 | 25.23 |
| ATOM | 868 | C | PRO | 313 | 27.955 | 36.856 | 21.976 | 1.00 | 26.34 |
| ATOM | 869 | O | PRO | 313 | 27.271 | 37.852 | 22.209 | 1.00 | 27.02 |
| ATOM | 870 | N | LEU | 314 | 29.243 | 36.782 | 22.283 | 1.00 | 27.08 |
| ATOM | 871 | CA | LEU | 314 | 29.933 | 37.908 | 22.900 | 1.00 | 28.42 |
| ATOM | 872 | CB | LEU | 314 | 31.384 | 37.525 | 23.202 | 1.00 | 29.58 |
| ATOM | 873 | CG | LEU | 314 | 31.588 | 36.580 | 24.390 | 1.00 | 30.01 |
| ATOM | 874 | CD1 | LEU | 314 | 32.985 | 35.966 | 24.353 | 1.00 | 30.50 |
| ATOM | 875 | CD2 | LEU | 314 | 31.366 | 37.344 | 25.685 | 1.00 | 31.31 |
| ATOM | 876 | C | LEU | 314 | 29.881 | 39.134 | 21.980 | 1.00 | 29.42 |
| ATOM | 877 | O | LEU | 314 | 29.618 | 40.247 | 22.438 | 1.00 | 29.09 |
| ATOM | 878 | N | ASN | 315 | 30.119 | 38.918 | 20.685 | 1.00 | 28.55 |
| ATOM | 879 | CA | ASN | 315 | 30.104 | 39.995 | 19.699 | 1.00 | 28.31 |
| ATOM | 880 | CB | ASN | 315 | 31.520 | 40.220 | 19.135 | 1.00 | 28.09 |
| ATOM | 881 | CG | ASN | 315 | 32.114 | 38.966 | 18.488 | 1.00 | 28.61 |
| ATOM | 882 | OD1 | ASN | 315 | 31.459 | 37.935 | 18.375 | 1.00 | 27.11 |
| ATOM | 883 | ND2 | ASN | 315 | 33.368 | 39.065 | 18.053 | 1.00 | 29.33 |
| ATOM | 884 | C | ASN | 315 | 29.123 | 39.771 | 18.536 | 1.00 | 28.16 |
| ATOM | 885 | O | ASN | 315 | 29.115 | 40.534 | 17.566 | 1.00 | 28.31 |
| ATOM | 886 | N | ASN | 316 | 28.289 | 38.742 | 18.637 | 1.00 | 27.03 |
| ATOM | 887 | CA | ASN | 316 | 27.322 | 38.430 | 17.582 | 1.00 | 26.93 |
| ATOM | 888 | CB | ASN | 316 | 26.246 | 39.522 | 17.467 | 1.00 | 27.40 |
| ATOM | 889 | CG | ASN | 316 | 25.247 | 39.491 | 18.621 | 1.00 | 29.23 |
| ATOM | 890 | OD1 | ASN | 316 | 24.845 | 38.423 | 19.079 | 1.00 | 29.19 |
| ATOM | 891 | ND2 | ASN | 316 | 24.828 | 40.673 | 19.081 | 1.00 | 29.80 |
| ATOM | 892 | C | ASN | 316 | 28.002 | 38.253 | 16.226 | 1.00 | 25.60 |
| ATOM | 893 | O | ASN | 316 | 27.688 | 38.956 | 15.264 | 1.00 | 25.51 |
| ATOM | 894 | N | THR | 317 | 28.947 | 37.322 | 16.162 | 1.00 | 24.54 |
| ATOM | 895 | CA | THR | 317 | 29.635 | 37.031 | 14.917 | 1.00 | 24.24 |
| ATOM | 896 | CB | THR | 317 | 31.124 | 37.428 | 14.970 | 1.00 | 24.58 |
| ATOM | 897 | OG1 | THR | 317 | 31.780 | 36.721 | 16.029 | 1.00 | 25.19 |
| ATOM | 898 | CG2 | THR | 317 | 31.255 | 38.924 | 15.194 | 1.00 | 24.84 |
| ATOM | 899 | C | THR | 317 | 29.516 | 35.532 | 14.648 | 1.00 | 22.99 |
| ATOM | 900 | O | THR | 317 | 29.262 | 34.741 | 15.559 | 1.00 | 22.92 |
| ATOM | 901 | N | VAL | 318 | 29.674 | 35.154 | 13.388 | 1.00 | 22.45 |
| ATOM | 902 | CA | VAL | 318 | 29.577 | 33.761 | 13.004 | 1.00 | 22.06 |
| ATOM | 903 | CB | VAL | 318 | 28.239 | 33.460 | 12.281 | 1.00 | 21.83 |
| ATOM | 904 | CG1 | VAL | 318 | 28.127 | 34.294 | 11.017 | 1.00 | 21.77 |
| ATOM | 905 | CG2 | VAL | 318 | 28.155 | 31.983 | 11.950 | 1.00 | 23.58 |
| ATOM | 906 | C | VAL | 318 | 30.719 | 33.402 | 12.081 | 1.00 | 21.76 |
| ATOM | 907 | O | VAL | 318 | 31.245 | 34.256 | 11.366 | 1.00 | 21.99 |
| ATOM | 908 | N | LEU | 319 | 31.109 | 32.132 | 12.122 | 1.00 | 21.37 |
| ATOM | 909 | CA | LEU | 319 | 32.177 | 31.626 | 11.278 | 1.00 | 22.24 |
| ATOM | 910 | CB | LEU | 319 | 32.619 | 30.243 | 11.767 | 1.00 | 22.86 |
| ATOM | 911 | CG | LEU | 319 | 33.660 | 29.513 | 10.921 | 1.00 | 23.05 |
| ATOM | 912 | CD1 | LEU | 319 | 34.955 | 30.318 | 10.928 | 1.00 | 25.19 |
| ATOM | 913 | CD2 | LEU | 319 | 33.896 | 28.116 | 11.475 | 1.00 | 24.34 |
| ATOM | 914 | C | LEU | 319 | 31.684 | 31.528 | 9.833 | 1.00 | 23.30 |
| ATOM | 915 | O | LEU | 319 | 30.687 | 30.870 | 9.551 | 1.00 | 22.87 |
| ATOM | 916 | N | PHE | 320 | 32.389 | 32.194 | 8.925 | 1.00 | 23.64 |
| ATOM | 917 | CA | PHE | 320 | 32.037 | 32.176 | 7.508 | 1.00 | 25.23 |
| ATOM | 918 | CB | PRE | 320 | 31.108 | 33.346 | 7.172 | 1.00 | 24.27 |
| ATOM | 919 | CG | PHE | 320 | 30.699 | 33.397 | 5.729 | 1.00 | 25.29 |
| ATOM | 920 | CD1 | PHE | 320 | 29.735 | 32.522 | 5.229 | 1.00 | 25.59 |
| ATOM | 921 | CD2 | PHE | 320 | 31.269 | 34.325 | 4.865 | 1.00 | 24.72 |
| ATOM | 922 | CE1 | PHE | 320 | 29.347 | 32.576 | 3.894 | 1.00 | 25.95 |
| ATOM | 923 | CE2 | PHE | 320 | 30.886 | 34.383 | 3.525 | 1.00 | 25.91 |
| ATOM | 924 | CZ | PHE | 320 | 29.924 | 33.509 | 3.040 | 1.00 | 25.81 |
| ATOM | 925 | C | PHE | 320 | 33.327 | 32.290 | 6.702 | 1.00 | 26.25 |
| ATOM | 926 | O | PHE | 320 | 34.045 | 33.284 | 6.787 | 1.00 | 26.22 |
| ATOM | 927 | N | GLU | 321 | 33.621 | 31.255 | 5.929 | 1.00 | 28.45 |
| ATOM | 928 | CA | GLU | 321 | 34.831 | 31.210 | 5.125 | 1.00 | 29.34 |
| ATOM | 929 | CB | GLU | 321 | 34.779 | 32.272 | 4.006 | 1.00 | 29.96 |
| ATOM | 930 | CG | GLU | 321 | 33.650 | 32.017 | 2.999 | 1.00 | 31.85 |
| ATOM | 931 | CD | GLU | 321 | 33.659 | 32.933 | 1.774 | 1.00 | 32.92 |
| ATOM | 932 | OE1 | GLU | 321 | 34.446 | 33.910 | 1.724 | 1.00 | 35.63 |
| ATOM | 933 | OE2 | GLU | 321 | 32.860 | 32.667 | 0.855 | 1.00 | 31.62 |
| ATOM | 934 | C | GLU | 321 | 36.114 | 31.357 | 5.953 | 1.00 | 29.82 |
| ATOM | 935 | O | GLU | 321 | 36.994 | 32.128 | 5.602 | 1.00 | 29.97 |
| ATOM | 936 | N | GLY | 322 | 36.202 | 30.635 | 7.067 | 1.00 | 29.47 |
| ATOM | 937 | CA | GLY | 322 | 37.411 | 30.658 | 7.880 | 1.00 | 28.49 |
| ATOM | 938 | C | GLY | 322 | 37.607 | 31.596 | 9.062 | 1.00 | 27.90 |
| ATOM | 939 | O | GLY | 322 | 38.501 | 31.362 | 9.882 | 1.00 | 28.38 |
| ATOM | 940 | N | LYS | 323 | 36.824 | 32.663 | 9.160 | 1.00 | 26.69 |
| ATOM | 941 | CA | LYS | 323 | 36.979 | 33.591 | 10.280 | 1.00 | 26.84 |
| ATOM | 942 | CB | LYS | 323 | 37.836 | 34.801 | 9.878 | 1.00 | 28.64 |
| ATOM | 943 | CG | LYS | 323 | 39.232 | 34.483 | 9.329 | 1.00 | 31.90 |
| ATOM | 944 | CD | LYS | 323 | 39.949 | 35.778 | 8.935 | 1.00 | 34.33 |
| ATOM | 945 | CE | LYS | 323 | 41.320 | 35.534 | 8.325 | 1.00 | 36.36 |
| ATOM | 946 | NZ | LYS | 323 | 42.359 | 35.109 | 9.323 | 1.00 | 38.14 |
| ATOM | 947 | C | LYS | 323 | 35.608 | 34.087 | 10.707 | 1.00 | 25.87 |
| ATOM | 948 | O | LYS | 323 | 34.638 | 33.952 | 9.961 | 1.00 | 23.69 |
| ATOM | 949 | N | TYR | 324 | 35.526 | 34.672 | 11.899 | 1.00 | 25.68 |
| ATOM | 950 | CA | TYR | 324 | 34.249 | 35.186 | 12.378 | 1.00 | 25.45 |
| ATOM | 951 | CB | TYR | 324 | 34.205 | 35.174 | 13.903 | 1.00 | 26.32 |
| ATOM | 952 | CG | TYR | 324 | 33.996 | 33.794 | 14.474 | 1.00 | 26.94 |
| ATOM | 953 | CD1 | TYR | 324 | 34.918 | 32.773 | 14.237 | 1.00 | 27.29 |
| ATOM | 954 | CE1 | TYR | 324 | 34.705 | 31.486 | 14.722 | 1.00 | 28.49 |
| ATOM | 955 | CD2 | TYR | 324 | 32.858 | 33.491 | 15.215 | 1.00 | 25.92 |
| ATOM | 956 | CE2 | TYR | 324 | 32.640 | 32.210 | 15.702 | 1.00 | 26.59 |
| ATOM | 957 | CZ | TYR | 324 | 33.564 | 31.211 | 15.451 | 1.00 | 27.01 |
| ATOM | 958 | OH | TYR | 324 | 33.339 | 29.930 | 15.920 | 1.00 | 28.62 |
| ATOM | 959 | C | TYR | 324 | 33.965 | 36.585 | 11.863 | 1.00 | 25.14 |
| ATOM | 960 | O | TYR | 324 | 34.864 | 37.422 | 11.781 | 1.00 | 25.62 |
| ATOM | 961 | N | GLY | 325 | 32.705 | 36.829 | 11.507 | 1.00 | 23.90 |
| ATOM | 962 | CA | GLY | 325 | 32.310 | 38.127 | 11.001 | 1.00 | 23.79 |
| ATOM | 963 | C | GLY | 325 | 30.897 | 38.461 | 11.441 | 1.00 | 25.33 |
| ATOM | 964 | O | GLY | 325 | 30.079 | 37.561 | 11.652 | 1.00 | 24.38 |
| ATOM | 965 | N | GLY | 326 | 30.610 | 39.752 | 11.589 | 1.00 | 24.25 |
| ATOM | 966 | CA | GLY | 326 | 29.282 | 40.168 | 12.004 | 1.00 | 24.94 |
| ATOM | 967 | C | GLY | 326 | 28.261 | 40.090 | 10.883 | 1.00 | 25.23 |
| ATOM | 968 | O | GLY | 326 | 28.598 | 39.728 | 9.751 | 1.00 | 25.79 |
| ATOM | 969 | N | MET | 327 | 27.014 | 40.445 | 11.185 | 1.00 | 26.02 |
| ATOM | 970 | CA | MET | 327 | 25.953 | 40.401 | 10.190 | 1.00 | 27.32 |
| ATOM | 971 | CB | MET | 327 | 24.595 | 40.646 | 10.864 | 1.00 | 29.29 |
| ATOM | 972 | CG | MET | 327 | 24.389 | 42.052 | 11.416 | 1.00 | 32.37 |
| ATOM | 973 | SD | MET | 327 | 23.965 | 43.257 | 10.146 | 1.00 | 37.07 |
| ATOM | 974 | CE | MET | 327 | 22.345 | 42.637 | 9.596 | 1.00 | 35.38 |
| ATOM | 975 | C | MET | 327 | 26.152 | 41.373 | 9.017 | 1.00 | 27.13 |
| ATOM | 976 | O | MET | 327 | 25.592 | 41.172 | 7.950 | 1.00 | 25.51 |
| ATOM | 977 | N | GLN | 328 | 26.950 | 42.423 | 9.211 | 1.00 | 27.79 |
| ATOM | 978 | CA | GLN | 328 | 27.198 | 43.392 | 8.138 | 1.00 | 29.04 |
| ATOM | 979 | CB | GLN | 328 | 28.025 | 44.571 | 8.662 | 1.00 | 31.55 |
| ATOM | 980 | CG | GLN | 328 | 29.460 | 44.204 | 8.990 | 1.00 | 33.92 |
| ATOM | 981 | CD | GLN | 328 | 29.682 | 43.901 | 10.462 | 1.00 | 36.11 |
| ATOM | 982 | OE1 | GLN | 328 | 28.873 | 43.216 | 11.102 | 1.00 | 35.48 |
| ATOM | 983 | NE2 | GLN | 328 | 30.802 | 44.407 | 11.011 | 1.00 | 36.91 |
| ATOM | 984 | C | GLN | 328 | 27.959 | 42.718 | 6.993 | 1.00 | 28.92 |
| ATOM | 985 | O | GLN | 328 | 27.970 | 43.193 | 5.864 | 1.00 | 28.52 |
| ATOM | 986 | N | MET | 329 | 28.604 | 41.604 | 7.317 | 1.00 | 28.72 |
| ATOM | 987 | CA | MET | 329 | 29.368 | 40.813 | 6.366 | 1.00 | 27.66 |
| ATOM | 988 | CB | MET | 329 | 29.998 | 39.633 | 7.124 | 1.00 | 29.67 |
| ATOM | 989 | CG | MET | 329 | 30.698 | 38.584 | 6.294 | 1.00 | 30.31 |
| ATOM | 990 | SD | MET | 329 | 31.675 | 37.438 | 7.333 | 1.00 | 30.76 |
| ATOM | 991 | CE | MET | 329 | 30.395 | 36.763 | 8.395 | 1.00 | 27.38 |
| ATOM | 992 | C | MET | 329 | 28.462 | 40.322 | 5.228 | 1.00 | 27.08 |
| ATOM | 993 | O | MET | 329 | 28.927 | 40.093 | 4.115 | 1.00 | 25.67 |
| ATOM | 994 | N | PHE | 330 | 27.166 | 40.197 | 5.505 | 1.00 | 25.56 |
| ATOM | 995 | CA | PHE | 330 | 26.202 | 39.717 | 4.510 | 1.00 | 25.27 |
| ATOM | 996 | CB | PHE | 330 | 25.258 | 38.717 | 5.164 | 1.00 | 26.21 |
| ATOM | 997 | CG | PHE | 330 | 25.960 | 37.546 | 5.779 | 1.00 | 26.31 |
| ATOM | 998 | CD1 | PHE | 330 | 26.573 | 36.591 | 4.979 | 1.00 | 26.75 |
| ATOM | 999 | CD2 | PHE | 330 | 26.037 | 37.413 | 7.160 | 1.00 | 27.20 |
| ATOM | 1000 | CE1 | PHE | 330 | 27.263 | 35.514 | 5.547 | 1.00 | 28.46 |
| ATOM | 1001 | CE2 | PHE | 330 | 26.722 | 36.342 | 7.737 | 1.00 | 28.83 |
| ATOM | 1002 | CZ | PHE | 330 | 27.337 | 35.391 | 6.928 | 1.00 | 27.19 |
| ATOM | 1003 | C | PHE | 330 | 25.368 | 40.812 | 3.858 | 1.00 | 24.89 |
| ATOM | 1004 | O | PHE | 330 | 24.351 | 40.536 | 3.226 | 1.00 | 24.95 |
| ATOM | 1005 | N | LYS | 331 | 25.803 | 42.051 | 4.007 | 1.00 | 24.84 |
| ATOM | 1006 | CA | LYS | 331 | 25.081 | 43.181 | 3.452 | 1.00 | 25.68 |
| ATOM | 1007 | CB | LYS | 331 | 25.923 | 44.442 | 3.602 | 1.00 | 27.69 |
| ATOM | 1008 | CG | LYS | 331 | 25.100 | 45.699 | 3.629 | 1.00 | 30.20 |
| ATOM | 1009 | CD | LYS | 331 | 24.105 | 45.611 | 4.781 | 1.00 | 34.11 |
| ATOM | 1010 | CE | LYS | 331 | 23.238 | 46.853 | 4.879 | 1.00 | 35.87 |
| ATOM | 1011 | NZ | LYS | 331 | 22.167 | 46.684 | 5.903 | 1.00 | 37.65 |
| ATOM | 1012 | C | LYS | 331 | 24.679 | 43.007 | 1.990 | 1.00 | 26.00 |
| ATOM | 1013 | O | LYS | 331 | 23.551 | 43.317 | 1.609 | 1.00 | 26.68 |
| ATOM | 1014 | N | ALA | 332 | 25.594 | 42.493 | 1.182 | 1.00 | 26.27 |
| ATOM | 1015 | CA | ALA | 332 | 25.357 | 42.299 | -0.242 | 1.00 | 26.94 |
| ATOM | 1016 | CB | ALA | 332 | 26.645 | 41.847 | -0.917 | 1.00 | 27.13 |
| ATOM | 1017 | C | ALA | 332 | 24.211 | 41.348 | -0.588 | 1.00 | 27.68 |
| ATOM | 1018 | O | ALA | 332 | 23.687 | 41.398 | -1.705 | 1.00 | 28.00 |
| ATOM | 1019 | N | LEU | 333 | 23.817 | 40.492 | 0.356 | 1.00 | 26.04 |
| ATOM | 1020 | CA | LEU | 333 | 22.709 | 39.566 | 0.119 | 1.00 | 25.55 |
| ATOM | 1021 | CB | LEU | 333 | 22.548 | 38.586 | 1.279 | 1.00 | 22.80 |
| ATOM | 1022 | CG | LEU | 333 | 23.589 | 37.495 | 1.501 | 1.00 | 23.44 |
| ATOM | 1023 | CD1 | LEU | 333 | 23.127 | 36.616 | 2.659 | 1.00 | 21.98 |
| ATOM | 1024 | CD2 | LEU | 333 | 23.766 | 36.673 | 0.241 | 1.00 | 24.51 |
| ATOM | 1025 | C | LEU | 333 | 21.391 | 40.318 | -0.048 | 1.00 | 25.80 |
| ATOM | 1026 | O | LEU | 333 | 20.454 | 39.807 | -0.644 | 1.00 | 26.35 |
| ATOM | 1027 | N | GLY | 334 | 21.330 | 41.533 | 0.480 | 1.00 | 26.06 |
| ATOM | 1028 | CA | GLY | 334 | 20.111 | 42.307 | 0.387 | 1.00 | 27.61 |
| ATOM | 1029 | C | GLY | 334 | 18.980 | 41.627 | 1.127 | 1.00 | 28.38 |
| ATOM | 1030 | O | GLY | 334 | 17.821 | 41.762 | 0.743 | 1.00 | 29.21 |
| ATOM | 1031 | N | SER | 335 | 19.313 | 40.898 | 2.191 | 1.00 | 28.07 |
| ATOM | 1032 | CA | SER | 335 | 18.310 | 40.190 | 2.984 | 1.00 | 28.11 |
| ATOM | 1033 | CB | SER | 335 | 18.251 | 38.723 | 2.551 | 1.00 | 29.24 |
| ATOM | 1034 | OG | SER | 335 | 18.153 | 38.604 | 1.143 | 1.00 | 31.20 |
| ATOM | 1035 | C | SER | 335 | 18.648 | 40.266 | 4.474 | 1.00 | 28.25 |
| ATOM | 1036 | O | SER | 335 | 18.816 | 39.235 | 5.134 | 1.00 | 27.31 |
| ATOM | 1037 | N | ASP | 336 | 18.751 | 41.482 | 5.004 | 1.00 | 27.15 |
| ATOM | 1038 | CA | ASP | 336 | 19.080 | 41.661 | 6.411 | 1.00 | 27.95 |
| ATOM | 1039 | CB | ASP | 336 | 19.182 | 43.146 | 6.758 | 1.00 | 28.40 |
| ATOM | 1040 | CG | ASP | 336 | 20.462 | 43.776 | 6.256 | 1.00 | 31.17 |
| ATOM | 1041 | OD1 | ASP | 336 | 21.300 | 43.061 | 5.671 | 1.00 | 30.76 |
| ATOM | 1042 | OD2 | ASP | 336 | 20.630 | 45.000 | 6.450 | 1.00 | 33.65 |
| ATOM | 1043 | C | ASP | 336 | 18.054 | 41.000 | 7.321 | 1.00 | 27.32 |
| ATOM | 1044 | O | ASP | 336 | 18.393 | 40.523 | 8.406 | 1.00 | 27.74 |
| ATOM | 1045 | N | ASP | 337 | 16.799 | 40.985 | 6.887 | 1.00 | 26.73 |
| ATOM | 1046 | CA | ASP | 337 | 15.748 | 40.364 | 7.678 | 1.00 | 27.00 |
| ATOM | 1047 | CB | ASP | 337 | 14.383 | 40.532 | 6.982 | 1.00 | 29.56 |
| ATOM | 1048 | CG | ASP | 337 | 14.362 | 39.994 | 5.564 | 1.00 | 30.90 |
| ATOM | 1049 | OD1 | ASP | 337 | 15.434 | 39.888 | 4.931 | 1.00 | 32.90 |
| ATOM | 1050 | OD2 | ASP | 337 | 13.251 | 39.693 | 5.066 | 1.00 | 32.99 |
| ATOM | 1051 | C | ASP | 337 | 16.082 | 38.886 | 7.917 | 1.00 | 25.07 |
| ATOM | 1052 | O | ASP | 337 | 15.948 | 38.375 | 9.031 | 1.00 | 23.90 |
| ATOM | 1053 | N | LEU | 338 | 16.537 | 38.210 | 6.873 | 1.00 | 23.72 |
| ATOM | 1054 | CA | LEU | 338 | 16.904 | 36.809 | 6.997 | 1.00 | 23.15 |
| ATOM | 1055 | CB | LEU | 338 | 17.174 | 36.212 | 5.618 | 1.00 | 21.77 |
| ATOM | 1056 | CG | LEU | 338 | 17.719 | 34.783 | 5.629 | 1.00 | 22.22 |
| ATOM | 1057 | CD1 | LEU | 338 | 16.705 | 33.837 | 6.276 | 1.00 | 22.75 |
| ATOM | 1058 | CD2 | LEU | 338 | 18.021 | 34.355 | 4.206 | 1.00 | 20.63 |
| ATOM | 1059 | C | LEU | 338 | 18.144 | 36.668 | 7.880 | 1.00 | 22.07 |
| ATOM | 1060 | O | LEU | 338 | 18.176 | 35.856 | 8.799 | 1.00 | 22.27 |
| ATOM | 1061 | N | VAL | 339 | 19.165 | 37.474 | 7.610 | 1.00 | 22.77 |
| ATOM | 1062 | CA | VAL | 339 | 20.388 | 37.406 | 8.403 | 1.00 | 23.12 |
| ATOM | 1063 | CB | VAL | 339 | 21.448 | 38.408 | 7.893 | 1.00 | 23.99 |
| ATOM | 1064 | CG1 | VAL | 339 | 22.718 | 38.275 | 8.717 | 1.00 | 23.37 |
| ATOM | 1065 | CG2 | VAL | 339 | 21.732 | 38.163 | 6.411 | 1.00 | 24.06 |
| ATOM | 1066 | C | VAL | 339 | 20.108 | 37.690 | 9.884 | 1.00 | 23.24 |
| ATOM | 1067 | O | VAL | 339 | 20.611 | 36.998 | 10.770 | 1.00 | 22.39 |
| ATOM | 1068 | N | ASN | 340 | 19.296 | 38.708 | 10.150 | 1.00 | 24.30 |
| ATOM | 1069 | CA | ASN | 340 | 18.973 | 39.065 | 11.522 | 1.00 | 25.14 |
| ATOM | 1070 | CB | ASN | 340 | 18.205 | 40.391 | 11.561 | 1.00 | 28.38 |
| ATOM | 1071 | CG | ASN | 340 | 19.134 | 41.592 | 11.610 | 1.00 | 30.44 |
| ATOM | 1072 | OD1 | ASN | 340 | 18.844 | 42.649 | 11.042 | 1.00 | 33.87 |
| ATOM | 1073 | ND2 | ASN | 340 | 20.258 | 41.440 | 12.303 | 1.00 | 31.74 |
| ATOM | 1074 | C | ASN | 340 | 18.189 | 37.970 | 12.229 | 1.00 | 24.36 |
| ATOM | 1075 | O | ASN | 340 | 18.392 | 37.737 | 13.415 | 1.00 | 23.30 |
| ATOM | 1076 | N | GLU | 341 | 17.294 | 37.298 | 11.514 | 1.00 | 24.45 |
| ATOM | 1077 | CA | GLU | 341 | 16.527 | 36.224 | 12.139 | 1.00 | 24.75 |
| ATOM | 1078 | CB | GLU | 341 | 15.350 | 35.814 | 11.254 | 1.00 | 26.89 |
| ATOM | 1079 | CG | GLU | 341 | 14.179 | 36.773 | 11.358 | 1.00 | 31.50 |
| ATOM | 1080 | CD | GLU | 341 | 12.945 | 36.239 | 10.681 | 1.00 | 34.84 |
| ATOM | 1081 | OE1 | GLU | 341 | 12.720 | 35.013 | 10.750 | 1.00 | 37.24 |
| ATOM | 1082 | OE2 | GLU | 341 | 12.187 | 37.038 | 10.096 | 1.00 | 37.28 |
| ATOM | 1083 | C | GLU | 341 | 17.432 | 35.027 | 12.417 | 1.00 | 23.12 |
| ATOM | 1084 | O | GLU | 341 | 17.290 | 34.356 | 13.448 | 1.00 | 22.72 |
| ATOM | 1085 | N | ALA | 342 | 18.365 | 34.766 | 11.506 | 1.00 | 21.14 |
| ATOM | 1086 | CA | ALA | 342 | 19.304 | 33.664 | 11.684 | 1.00 | 20.58 |
| ATOM | 1087 | CB | ALA | 342 | 20.158 | 33.498 | 10.442 | 1.00 | 19.08 |
| ATOM | 1088 | C | ALA | 342 | 20.193 | 33.943 | 12.906 | 1.00 | 21.25 |
| ATOM | 1089 | O | ALA | 342 | 20.386 | 33.074 | 13.764 | 1.00 | 21.78 |
| ATOM | 1090 | N | PHE | 343 | 20.720 | 35.164 | 12.987 | 1.00 | 21.28 |
| ATOM | 1091 | CA | PHE | 343 | 21.581 | 35.548 | 14.105 | 1.00 | 23.79 |
| ATOM | 1092 | CB | PHE | 343 | 22.201 | 36.933 | 13.864 | 1.00 | 22.95 |
| ATOM | 1093 | CG | PHE | 343 | 23.487 | 36.906 | 13.083 | 1.00 | 22.10 |
| ATOM | 1094 | CD1 | PHE | 343 | 23.540 | 36.348 | 11.814 | 1.00 | 21.63 |
| ATOM | 1095 | CD2 | PHE | 343 | 24.644 | 37.480 | 13.607 | 1.00 | 22.10 |
| ATOM | 1096 | CE1 | PHE | 343 | 24.724 | 36.366 | 11.079 | 1.00 | 22.66 |
| ATOM | 1097 | CE2 | PHE | 343 | 25.831 | 37.503 | 12.880 | 1.00 | 20.80 |
| ATOM | 1098 | CZ | PHE | 343 | 25.871 | 36.945 | 11.614 | 1.00 | 21.73 |
| ATOM | 1099 | C | PHE | 343 | 20.820 | 35.563 | 15.437 | 1.00 | 24.28 |
| ATOM | 1100 | O | PRE | 343 | 21.348 | 35.143 | 16.466 | 1.00 | 25.24 |
| ATOM | 1101 | N | ASP | 344 | 19.581 | 36.042 | 15.422 | 1.00 | 25.30 |
| ATOM | 1102 | CA | ASP | 344 | 18.807 | 36.088 | 16.655 | 1.00 | 27.20 |
| ATOM | 1103 | CB | ASP | 344 | 17.453 | 36.754 | 16.425 | 1.00 | 30.33 |
| ATOM | 1104 | CG | ASP | 344 | 17.581 | 38.215 | 16.084 | 1.00 | 34.37 |
| ATOM | 1105 | OD1 | ASP | 344 | 18.728 | 38.719 | 16.068 | 1.00 | 36.85 |
| ATOM | 1106 | OD2 | ASP | 344 | 16.529 | 38.853 | 15.835 | 1.00 | 36.92 |
| ATOM | 1107 | C | ASP | 344 | 18.600 | 34.674 | 17.165 | 1.00 | 25.45 |
| ATOM | 1108 | O | ASP | 344 | 18.728 | 34.400 | 18.364 | 1.00 | 25.36 |
| ATOM | 1109 | N | PHE | 345 | 18.274 | 33.770 | 16.250 | 1.00 | 24.47 |
| ATOM | 1110 | CA | PHE | 345 | 18.072 | 32.393 | 16.647 | 1.00 | 23.12 |
| ATOM | 1111 | CB | PHE | 345 | 17.703 | 31.513 | 15.457 | 1.00 | 23.28 |
| ATOM | 1112 | CG | PHE | 345 | 17.733 | 30.058 | 15.784 | 1.00 | 22.38 |
| ATOM | 1113 | CD1 | PHE | 345 | 16.777 | 29.512 | 16.633 | 1.00 | 22.93 |
| ATOM | 1114 | CD2 | PHE | 345 | 18.773 | 29.250 | 15.329 | 1.00 | 22.45 |
| ATOM | 1115 | CE1 | PHE | 345 | 16.856 | 28.183 | 17.036 | 1.00 | 21.83 |
| ATOM | 1116 | CE2 | PHE | 345 | 18.861 | 27.919 | 15.724 | 1.00 | 22.15 |
| ATOM | 1117 | CZ | PRE | 345 | 17.901 | 27.385 | 16.582 | 1.00 | 23.05 |
| ATOM | 1118 | C | PHE | 345 | 19.333 | 31.825 | 17.281 | 1.00 | 22.93 |
| ATOM | 1119 | O | PHE | 345 | 19.286 | 31.241 | 18.360 | 1.00 | 22.27 |
| ATOM | 1120 | N | ALA | 346 | 20.459 | 31.982 | 16.592 | 1.00 | 23.57 |
| ATOM | 1121 | CA | ALA | 346 | 21.712 | 31.457 | 17.099 | 1.00 | 25.20 |
| ATOM | 1122 | CB | ALA | 346 | 22.839 | 31.734 | 16.127 | 1.00 | 26.42 |
| ATOM | 1123 | C | ALA | 346 | 22.038 | 32.040 | 18.459 | 1.00 | 27.60 |
| ATOM | 1124 | O | ALA | 346 | 22.459 | 31.314 | 19.349 | 1.00 | 25.96 |
| ATOM | 1125 | N | LYS | 347 | 21.843 | 33.348 | 18.620 | 1.00 | 28.41 |
| ATOM | 1126 | CA | LYS | 347 | 22.127 | 33.985 | 19.901 | 1.00 | 30.85 |
| ATOM | 1127 | CB | LYS | 347 | 21.838 | 35.485 | 19.841 | 1.00 | 32.36 |
| ATOM | 1128 | CG | LYS | 347 | 21.860 | 36.147 | 21.202 | 1.00 | 36.99 |
| ATOM | 1129 | CD | LYS | 347 | 21.431 | 37.613 | 21.140 | 1.00 | 39.60 |
| ATOM | 1130 | CE | LYS | 347 | 21.227 | 38.185 | 22.561 | 1.00 | 42.60 |
| ATOM | 1131 | NZ | LYS | 347 | 22.459 | 38.130 | 23.428 | 1.00 | 42.88 |
| ATOM | 1132 | C | LYS | 347 | 21.298 | 33.355 | 21.011 | 1.00 | 30.95 |
| ATOM | 1133 | O | LYS | 347 | 21.847 | 32.949 | 22.039 | 1.00 | 31.24 |
| ATOM | 1134 | N | ASN | 348 | 19.986 | 33.259 | 20.807 | 1.00 | 30.97 |
| ATOM | 1135 | CA | ASN | 348 | 19.121 | 32.676 | 21.826 | 1.00 | 32.18 |
| ATOM | 1136 | CB | ASN | 348 | 17.645 | 32.804 | 21.435 | 1.00 | 34.34 |
| ATOM | 1137 | CG | ASN | 348 | 17.173 | 34.246 | 21.450 | 1.00 | 38.72 |
| ATOM | 1138 | OD1 | ASN | 348 | 17.656 | 35.057 | 22.247 | 1.00 | 40.24 |
| ATOM | 1139 | ND2 | ASN | 348 | 16.222 | 34.579 | 20.574 | 1.00 | 39.22 |
| ATOM | 1140 | C | ASN | 348 | 19.457 | 31.227 | 22.149 | 1.00 | 30.79 |
| ATOM | 1141 | O | ASN | 348 | 19.442 | 30.838 | 23.316 | 1.00 | 30.95 |
| ATOM | 1142 | N | LEU | 349 | 19.776 | 30.425 | 21.138 | 1.00 | 29.90 |
| ATOM | 1143 | CA | LEU | 349 | 20.122 | 29.033 | 21.409 | 1.00 | 29.04 |
| ATOM | 1144 | CB | LEU | 349 | 20.298 | 28.238 | 20.108 | 1.00 | 29.41 |
| ATOM | 1145 | CG | LEU | 349 | 20.380 | 26.738 | 20.388 | 1.00 | 28.84 |
| ATOM | 1146 | CD1 | LEU | 349 | 19.012 | 26.269 | 20.904 | 1.00 | 29.78 |
| ATOM | 1147 | CD2 | LEU | 349 | 20.756 | 25.976 | 19.136 | 1.00 | 30.12 |
| ATOM | 1148 | C | LEU | 349 | 21.419 | 28.977 | 22.212 | 1.00 | 28.27 |
| ATOM | 1149 | O | LEU | 349 | 21.527 | 28.229 | 23.181 | 1.00 | 28.13 |
| ATOM | 1150 | N | CYS | 350 | 22.405 | 29.768 | 21.802 | 1.00 | 27.19 |
| ATOM | 1151 | CA | CYS | 350 | 23.685 | 29.804 | 22.498 | 1.00 | 27.50 |
| ATOM | 1152 | CB | CYS | 350 | 24.610 | 30.856 | 21.875 | 1.00 | 27.40 |
| ATOM | 1153 | SG | CYS | 350 | 25.370 | 30.336 | 20.331 | 1.00 | 30.05 |
| ATOM | 1154 | C | CYS | 350 | 23.530 | 30.112 | 23.979 | 1.00 | 27.44 |
| ATOM | 1155 | O | CYS | 350 | 24.280 | 29.596 | 24.806 | 1.00 | 25.65 |
| ATOM | 1156 | N | SER | 351 | 22.551 | 30.944 | 24.316 | 1.00 | 28.30 |
| ATOM | 1157 | CA | SER | 351 | 22.341 | 31.321 | 25.716 | 1.00 | 29.76 |
| ATOM | 1158 | CB | SER | 351 | 21.239 | 32.377 | 25.835 | 1.00 | 27.97 |
| ATOM | 1159 | OG | SER | 351 | 19.972 | 31.819 | 25.545 | 1.00 | 27.29 |
| ATOM | 1160 | C | SER | 351 | 21.998 | 30.143 | 26.618 | 1.00 | 30.56 |
| ATOM | 1161 | O | SER | 351 | 22.104 | 30.253 | 27.835 | 1.00 | 30.76 |
| ATOM | 1162 | N | LEU | 352 | 21.582 | 29.027 | 26.030 | 1.00 | 30.81 |
| ATOM | 1163 | CA | LEU | 352 | 21.232 | 27.848 | 26.815 | 1.00 | 30.42 |
| ATOM | 1164 | CB | LEU | 352 | 20.275 | 26.948 | 26.035 | 1.00 | 31.12 |
| ATOM | 1165 | CG | LEU | 352 | 19.035 | 27.615 | 25.410 | 1.00 | 31.69 |
| ATOM | 1166 | CD1 | LEU | 352 | 18.055 | 26.553 | 24.966 | 1.00 | 31.69 |
| ATOM | 1167 | CD2 | LEU | 352 | 18.377 | 28.538 | 26.411 | 1.00 | 33.44 |
| ATOM | 1168 | C | LEU | 352 | 22.479 | 27.071 | 27.206 | 1.00 | 30.67 |
| ATOM | 1169 | O | LEU | 352 | 22.429 | 26.195 | 28.070 | 1.00 | 31.08 |
| ATOM | 1170 | N | GLN | 353 | 23.600 | 27.389 | 26.565 | 1.00 | 30.60 |
| ATOM | 1171 | CA | GLN | 353 | 24.871 | 26.733 | 26.869 | 1.00 | 31.59 |
| ATOM | 1172 | CB | GLN | 353 | 25.265 | 27.050 | 28.315 | 1.00 | 34.06 |
| ATOM | 1173 | CG | GLN | 353 | 25.542 | 28.519 | 28.575 | 1.00 | 37.01 |
| ATOM | 1174 | CD | GLN | 353 | 26.850 | 28.959 | 27.955 | 1.00 | 39.88 |
| ATOM | 1175 | OE1 | GLN | 353 | 27.890 | 28.318 | 28.156 | 1.00 | 43.06 |
| ATOM | 1176 | NE2 | GLN | 353 | 26.818 | 30.061 | 27.209 | 1.00 | 41.20 |
| ATOM | 1177 | C | GLN | 353 | 24.865 | 25.220 | 26.661 | 1.00 | 30.89 |
| ATOM | 1178 | O | GLN | 353 | 25.382 | 24.469 | 27.486 | 1.00 | 30.05 |
| ATOM | 1179 | N | LEU | 354 | 24.294 | 24.772 | 25.550 | 1.00 | 28.93 |
| ATOM | 1180 | CA | LEU | 354 | 24.229 | 23.349 | 25.268 | 1.00 | 27.99 |
| ATOM | 1181 | CB | LEU | 354 | 23.373 | 23.082 | 24.026 | 1.00 | 28.43 |
| ATOM | 1182 | CG | LEU | 354 | 21.881 | 23.406 | 24.046 | 1.00 | 30.02 |
| ATOM | 1183 | CD1 | LEU | 354 | 21.316 | 23.088 | 22.659 | 1.00 | 30.63 |
| ATOM | 1184 | CD2 | LEU | 354 | 21.156 | 22.608 | 25.114 | 1.00 | 29.61 |
| ATOM | 1185 | C | LEU | 354 | 25.604 | 22.736 | 25.039 | 1.00 | 27.41 |
| ATOM | 1186 | O | LEU | 354 | 26.540 | 23.415 | 24.604 | 1.00 | 27.14 |
| ATOM | 1187 | N | THR | 355 | 25.711 | 21.452 | 25.359 | 1.00 | 25.55 |
| ATOM | 1188 | CA | THR | 355 | 26.933 | 20.693 | 25.147 | 1.00 | 24.53 |
| ATOM | 1189 | CB | THR | 355 | 27.121 | 19.573 | 26.199 | 1.00 | 24.00 |
| ATOM | 1190 | OG1 | THR | 355 | 26.116 | 18.564 | 26.015 | 1.00 | 23.40 |
| ATOM | 1191 | CG2 | THR | 355 | 27.017 | 20.136 | 27.603 | 1.00 | 24.13 |
| ATOM | 1192 | C | THR | 355 | 26.750 | 20.026 | 23.785 | 1.00 | 24.40 |
| ATOM | 1193 | O | THR | 355 | 25.650 | 20.047 | 23.226 | 1.00 | 23.30 |
| ATOM | 1194 | N | GLU | 356 | 27.816 | 19.427 | 23.263 | 1.00 | 22.46 |
| ATOM | 1195 | CA | GLU | 356 | 27.754 | 18.756 | 21.971 | 1.00 | 24.63 |
| ATOM | 1196 | CB | GLU | 356 | 29.141 | 18.242 | 21.582 | 1.00 | 24.58 |
| ATOM | 1197 | CG | GLU | 356 | 30.075 | 19.358 | 21.170 | 1.00 | 26.29 |
| ATOM | 1198 | CD | GLU | 356 | 29.831 | 19.832 | 19.745 | 1.00 | 23.74 |
| ATOM | 1199 | OE1 | GLU | 356 | 30.390 | 19.223 | 18.820 | 1.00 | 26.81 |
| ATOM | 1200 | OE2 | GLU | 356 | 29.078 | 20.806 | 19.548 | 1.00 | 25.33 |
| ATOM | 1201 | C | GLU | 356 | 26.752 | 17.610 | 21.942 | 1.00 | 24.03 |
| ATOM | 1202 | O | GLU | 356 | 26.081 | 17.400 | 20.933 | 1.00 | 23.02 |
| ATOM | 1203 | N | GLU | 357 | 26.657 | 16.865 | 23.042 | 1.00 | 25.24 |
| ATOM | 1204 | CA | GLU | 357 | 25.721 | 15.746 | 23.122 | 1.00 | 25.24 |
| ATOM | 1205 | CB | GLU | 357 | 25.870 | 15.011 | 24.460 | 1.00 | 25.58 |
| ATOM | 1206 | CG | GLU | 357 | 27.261 | 14.435 | 24.698 | 1.00 | 28.22 |
| ATOM | 1207 | CD | GLU | 357 | 27.358 | 13.628 | 25.991 | 1.00 | 29.57 |
| ATOM | 1208 | OE1 | GLU | 357 | 26.600 | 13.913 | 26.936 | 1.00 | 28.40 |
| ATOM | 1209 | OE2 | GLU | 357 | 28.210 | 12.712 | 26.064 | 1.00 | 31.71 |
| ATOM | 1210 | C | GLU | 357 | 24.288 | 16.244 | 22.979 | 1.00 | 24.57 |
| ATOM | 1211 | O | GLU | 357 | 23.472 | 15.633 | 22.296 | 1.00 | 24.04 |
| ATOM | 1212 | N | GLU | 358 | 23.989 | 17.359 | 23.628 | 1.00 | 25.23 |
| ATOM | 1213 | CA | GLU | 358 | 22.655 | 17.933 | 23.560 | 1.00 | 25.61 |
| ATOM | 1214 | CB | GLU | 358 | 22.508 | 18.998 | 24.639 | 1.00 | 27.12 |
| ATOM | 1215 | CG | GLU | 358 | 22.943 | 18.437 | 25.982 | 1.00 | 29.78 |
| ATOM | 1216 | CD | GLU | 358 | 22.986 | 19.447 | 27.078 | 1.00 | 31.57 |
| ATOM | 1217 | OB1 | GLU | 358 | 23.395 | 20.605 | 26.822 | 1.00 | 32.27 |
| ATOM | 1218 | OE2 | GLU | 358 | 22.632 | 19.067 | 28.215 | 1.00 | 32.12 |
| ATOM | 1219 | C | GLU | 358 | 22.371 | 18.491 | 22.169 | 1.00 | 24.96 |
| ATOM | 1220 | O | GLU | 358 | 21.254 | 18.349 | 21.668 | 1.00 | 24.25 |
| ATOM | 1221 | N | ILE | 359 | 23.371 | 19.113 | 21.542 | 1.00 | 22.67 |
| ATOM | 1222 | CA | ILE | 359 | 23.188 | 19.645 | 20.191 | 1.00 | 22.78 |
| ATOM | 1223 | CB | ILE | 359 | 24.404 | 20.490 | 19.744 | 1.00 | 23.34 |
| ATOM | 1224 | CG2 | ILE | 359 | 24.344 | 20.740 | 18.236 | 1.00 | 22.93 |
| ATOM | 1225 | CG1 | ILE | 359 | 24.430 | 21.803 | 20.538 | 1.00 | 25.31 |
| ATOM | 1226 | CD1 | ILE | 359 | 25.711 | 22.603 | 20.401 | 1.00 | 27.90 |
| ATOM | 1227 | C | ILE | 359 | 23.000 | 18.481 | 19.215 | 1.00 | 21.51 |
| ATOM | 1228 | O | ILE | 359 | 22.223 | 18.570 | 18.272 | 1.00 | 21.39 |
| ATOM | 1229 | N | ALA | 360 | 23.725 | 17.393 | 19.455 | 1.00 | 20.70 |
| ATOM | 1230 | CA | ALA | 360 | 23.629 | 16.208 | 18.617 | 1.00 | 20.02 |
| ATOM | 1231 | CB | ALA | 360 | 24.617 | 15.140 | 19.104 | 1.00 | 19.77 |
| ATOM | 1232 | C | ALA | 360 | 22.198 | 15.675 | 18.688 | 1.00 | 19.38 |
| ATOM | 1233 | O | ALA | 360 | 21.528 | 15.540 | 17.665 | 1.00 | 18.92 |
| ATOM | 1234 | N | LEU | 361 | 21.736 | 15.393 | 19.903 | 1.00 | 18.99 |
| ATOM | 1235 | CA | LEU | 361 | 20.388 | 14.870 | 20.117 | 1.00 | 19.89 |
| ATOM | 1236 | CB | LEU | 361 | 20.184 | 14.533 | 21.600 | 1.00 | 20.98 |
| ATOM | 1237 | CG | LEU | 361 | 21.138 | 13.452 | 22.105 | 1.00 | 22.49 |
| ATOM | 1238 | CD1 | LEU | 361 | 20.951 | 13.265 | 23.592 | 1.00 | 23.19 |
| ATOM | 1239 | CD2 | LEU | 361 | 20.882 | 12.151 | 21.358 | 1.00 | 22.76 |
| ATOM | 1240 | C | LEU | 361 | 19.285 | 15.817 | 19.642 | 1.00 | 19.52 |
| ATOM | 1241 | O | LEU | 361 | 18.374 | 15.402 | 18.933 | 1.00 | 18.57 |
| ATOM | 1242 | N | PHE | 362 | 19.356 | 17.085 | 20.030 | 1.00 | 19.75 |
| ATOM | 1243 | CA | PHE | 362 | 18.335 | 18.030 | 19.595 | 1.00 | 20.87 |
| ATOM | 1244 | CB | PHE | 362 | 18.559 | 19.409 | 20.212 | 1.00 | 19.97 |
| ATOM | 1245 | CG | PHE | 362 | 17.537 | 20.429 | 19.788 | 1.00 | 20.58 |
| ATOM | 1246 | CD1 | PHE | 362 | 16.186 | 20.224 | 20.036 | 1.00 | 22.23 |
| ATOM | 1247 | CD2 | PHE | 362 | 17.925 | 21.590 | 19.127 | 1.00 | 21.76 |
| ATOM | 1248 | CE1 | PHE | 362 | 15.240 | 21.156 | 19.634 | 1.00 | 21.84 |
| ATOM | 1249 | CE2 | PRE | 362 | 16.984 | 22.528 | 18.724 | 1.00 | 23.51 |
| ATOM | 1250 | CZ | PHE | 362 | 15.640 | 22.314 | 18.974 | 1.00 | 21.56 |
| ATOM | 1251 | C | PHE | 362 | 18.306 | 18.161 | 18.067 | 1.00 | 20.26 |
| ATOM | 1252 | O | PHE | 362 | 17.233 | 18.189 | 17.461 | 1.00 | 21.41 |
| ATOM | 1253 | N | SER | 363 | 19.476 | 18.253 | 17.445 | 1.00 | 19.81 |
| ATOM | 1254 | CA | SER | 363 | 19.521 | 18.371 | 15.991 | 1.00 | 20.02 |
| ATOM | 1255 | CB | SER | 363 | 20.963 | 18.572 | 15.498 | 1.00 | 18.56 |
| ATOM | 1256 | OG | SER | 363 | 21.728 | 17.396 | 15.663 | 1.00 | 20.81 |
| ATOM | 1257 | C | SER | 363 | 18.906 | 17.121 | 15.353 | 1.00 | 20.59 |
| ATOM | 1258 | O | SER | 363 | 18.231 | 17.217 | 14.325 | 1.00 | 20.37 |
| ATOM | 1259 | N | SER | 364 | 19.124 | 15.954 | 15.957 | 1.00 | 19.20 |
| ATOM | 1260 | CA | SER | 364 | 18.541 | 14.729 | 15.406 | 1.00 | 20.84 |
| ATOM | 1261 | CB | SER | 364 | 19.108 | 13.474 | 16.099 | 1.00 | 20.81 |
| ATOM | 1262 | OG | SER | 364 | 18.644 | 13.335 | 17.426 | 1.00 | 21.39 |
| ATOM | 1263 | C | SER | 364 | 17.007 | 14.772 | 15.538 | 1.00 | 20.41 |
| ATOM | 1264 | O | SER | 364 | 16.293 | 14.283 | 14.662 | 1.00 | 20.80 |
| ATOM | 1265 | N | ALA | 365 | 16.503 | 15.374 | 16.614 | 1.00 | 20.02 |
| ATOM | 1266 | CA | ALA | 365 | 15.053 | 15.484 | 16.813 | 1.00 | 20.02 |
| ATOM | 1267 | CB | ALA | 365 | 14.734 | 15.950 | 18.227 | 1.00 | 20.30 |
| ATOM | 1268 | C | ALA | 365 | 14.450 | 16.455 | 15.794 | 1.00 | 21.11 |
| ATOM | 1269 | O | ALA | 365 | 13.337 | 16.252 | 15.324 | 1.00 | 19.92 |
| ATOM | 1270 | N | VAL | 366 | 15.189 | 17.509 | 15.458 | 1.00 | 20.57 |
| ATOM | 1271 | CA | VAL | 366 | 14.720 | 18.473 | 14.468 | 1.00 | 19.45 |
| ATOM | 1272 | CB | VAL | 366 | 15.684 | 19.677 | 14.360 | 1.00 | 19.15 |
| ATOM | 1273 | CG1 | VAL | 366 | 15.369 | 20.502 | 13.106 | 1.00 | 19.19 |
| ATOM | 1274 | CG2 | VAL | 366 | 15.567 | 20.542 | 15.618 | 1.00 | 20.02 |
| ATOM | 1275 | C | VAL | 366 | 14.617 | 17.786 | 13.107 | 1.00 | 19.50 |
| ATOM | 1276 | O | VAL | 366 | 13.686 | 18.038 | 12.352 | 1.00 | 19.45 |
| ATOM | 1277 | N | LEU | 367 | 15.576 | 16.911 | 12.811 | 1.00 | 19.31 |
| ATOM | 1278 | CA | LEU | 367 | 15.602 | 16.176 | 11.550 | 1.00 | 21.04 |
| ATOM | 1279 | CB | LEU | 367 | 16.975 | 15.534 | 11.335 | 1.00 | 21.11 |
| ATOM | 1280 | CG | LEU | 367 | 17.117 | 14.679 | 10.070 | 1.00 | 21.76 |
| ATOM | 1281 | CD1 | LEU | 367 | 17.139 | 15.585 | 8.841 | 1.00 | 24.04 |
| ATOM | 1282 | CD2 | LEU | 367 | 18.395 | 13.868 | 10.138 | 1.00 | 19.99 |
| ATOM | 1283 | C | LEU | 367 | 14.540 | 15.073 | 11.520 | 1.00 | 20.91 |
| ATOM | 1284 | O | LEU | 367 | 13.819 | 14.924 | 10.541 | 1.00 | 19.56 |
| ATOM | 1285 | N | ILE | 368 | 14.472 | 14.297 | 12.596 | 1.00 | 20.91 |
| ATOM | 1286 | CA | ILE | 368 | 13.521 | 13.199 | 12.699 | 1.00 | 21.53 |
| ATOM | 1287 | CB | ILE | 368 | 14.047 | 12.095 | 13.649 | 1.00 | 22.94 |
| ATOM | 1288 | CG2 | ILE | 368 | 13.171 | 10.867 | 13.550 | 1.00 | 22.23 |
| ATOM | 1289 | CG1 | ILE | 368 | 15.499 | 11.735 | 13.308 | 1.00 | 24.46 |
| ATOM | 1290 | CD1 | ILE | 368 | 15.719 | 11.309 | 11.896 | 1.00 | 27.21 |
| ATOM | 1291 | C | ILE | 368 | 12.183 | 13.722 | 13.231 | 1.00 | 22.51 |
| ATOM | 1292 | O | ILE | 368 | 11.830 | 13.501 | 14.393 | 1.00 | 21.63 |
| ATOM | 1293 | N | SER | 369 | 11.446 | 14.424 | 12.370 | 1.00 | 22.24 |
| ATOM | 1294 | CA | SER | 369 | 10.155 | 14.986 | 12.750 | 1.00 | 24.12 |
| ATOM | 1295 | CB | SER | 369 | 10.063 | 16.449 | 12.332 | 1.00 | 24.27 |
| ATOM | 1296 | OG | SER | 369 | 8.745 | 16.929 | 12.528 | 1.00 | 26.59 |
| ATOM | 1297 | C | SER | 369 | 9.011 | 14.221 | 12.107 | 1.00 | 25.22 |
| ATOM | 1298 | O | SER | 369 | 8.921 | 14.140 | 10.884 | 1.00 | 23.14 |
| ATOM | 1299 | N | PRO | 370 | 8.117 | 13.646 | 12.929 | 1.00 | 27.45 |
| ATOM | 1300 | CD | PRO | 370 | 8.169 | 13.553 | 14.402 | 1.00 | 28.35 |
| ATOM | 1301 | CA | PRO | 370 | 6.981 | 12.888 | 12.393 | 1.00 | 28.35 |
| ATOM | 1302 | CB | PRO | 370 | 6.488 | 12.105 | 13.607 | 1.00 | 28.25 |
| ATOM | 1303 | CG | PRO | 370 | 6.765 | 13.054 | 14.746 | 1.00 | 29.61 |
| ATOM | 1304 | C | PRO | 370 | 5.906 | 13.791 | 11.812 | 1.00 | 29.35 |
| ATOM | 1305 | O | PRO | 370 | 4.920 | 13.310 | 11.254 | 1.00 | 29.26 |
| ATOM | 1306 | N | ASP | 371 | 6.107 | 15.096 | 11.944 | 1.00 | 29.51 |
| ATOM | 1307 | CA | ASP | 371 | 5.151 | 16.074 | 11.450 | 1.00 | 30.84 |
| ATOM | 1308 | CB | ASP | 371 | 5.132 | 17.296 | 12.377 | 1.00 | 32.99 |
| ATOM | 1309 | CG | ASP | 371 | 4.616 | 16.962 | 13.765 | 1.00 | 35.86 |
| ATOM | 1310 | OD1 | ASP | 371 | 3.508 | 16.390 | 13.853 | 1.00 | 36.71 |
| ATOM | 1311 | OD2 | ASP | 371 | 5.311 | 17.270 | 14.763 | 1.00 | 37.76 |
| ATOM | 1312 | C | ASP | 371 | 5.355 | 16.549 | 10.007 | 1.00 | 29.75 |
| ATOM | 1313 | O | ASP | 371 | 4.515 | 17.276 | 9.484 | 1.00 | 30.14 |
| ATOM | 1314 | N | ARG | 372 | 6.455 | 16.162 | 9.364 | 1.00 | 28.07 |
| ATOM | 1315 | CA | ARG | 372 | 6.694 | 16.597 | 7.988 | 1.00 | 26.59 |
| ATOM | 1316 | CB | ARG | 372 | 8.030 | 16.075 | 7.458 | 1.00 | 25.22 |
| ATOM | 1317 | CG | ARG | 372 | 9.252 | 16.430 | 8.291 | 1.00 | 23.03 |
| ATOM | 1318 | CD | ARG | 372 | 9.412 | 17.926 | 8.486 | 1.00 | 22.25 |
| ATOM | 1319 | NE | ARG | 372 | 10.732 | 18.209 | 9.049 | 1.00 | 21.70 |
| ATOM | 1320 | CZ | ARG | 372 | 11.160 | 19.412 | 9.412 | 1.00 | 21.40 |
| ATOM | 1321 | NH1 | ARG | 372 | 10.374 | 20.472 | 9.278 | 1.00 | 19.74 |
| ATOM | 1322 | NH2 | ARG | 372 | 12.378 | 19.547 | 9.915 | 1.00 | 19.59 |
| ATOM | 1323 | C | ARG | 372 | 5.590 | 16.079 | 7.081 | 1.00 | 27.64 |
| ATOM | 1324 | O | ARG | 372 | 5.186 | 14.917 | 7.172 | 1.00 | 26.49 |
| ATOM | 1325 | N | ALA | 373 | 5.120 | 16.943 | 6.193 | 1.00 | 27.44 |
| ATOM | 1326 | CA | ALA | 373 | 4.074 | 16.563 | 5.259 | 1.00 | 27.38 |
| ATOM | 1327 | CB | ALA | 373 | 3.603 | 17.789 | 4.508 | 1.00 | 28.35 |
| ATOM | 1328 | C | ALA | 373 | 4.585 | 15.504 | 4.276 | 1.00 | 26.33 |
| ATOM | 1329 | O | ALA | 373 | 5.770 | 15.464 | 3.945 | 1.00 | 26.14 |
| ATOM | 1330 | N | TRP | 374 | 3.681 | 14.637 | 3.830 | 1.00 | 26.51 |
| ATOM | 1331 | CA | TRP | 374 | 3.987 | 13.582 | 2.856 | 1.00 | 25.94 |
| ATOM | 1332 | CB | TRP | 374 | 4.795 | 14.153 | 1.674 | 1.00 | 28.23 |
| ATOM | 1333 | CG | TRP | 374 | 4.231 | 15.435 | 1.047 | 1.00 | 30.91 |
| ATOM | 1334 | CD2 | TRP | 374 | 3.112 | 15.543 | 0.146 | 1.00 | 31.01 |
| ATOM | 1335 | CE2 | TRP | 374 | 2.936 | 16.922 | -0.144 | 1.00 | 31.51 |
| ATOM | 1336 | CE3 | TRP | 374 | 2.240 | 14.614 | -0.438 | 1.00 | 30.75 |
| ATOM | 1337 | CD1 | TRP | 374 | 4.676 | 16.718 | 1.252 | 1.00 | 31.80 |
| ATOM | 1338 | NE1 | TRP | 374 | 3.903 | 17.614 | 0.541 | 1.00 | 31.78 |
| ATOM | 1339 | CZ2 | TRP | 374 | 1.920 | 17.390 | -0.998 | 1.00 | 31.60 |
| ATOM | 1340 | CZ3 | TRP | 374 | 1.221 | 16.458 | -1.289 | 1.00 | 32.14 |
| ATOM | 1341 | CH2 | TRP | 374 | 1.075 | 15.082 | -1.556 | 1.00 | 31.37 |
| ATOM | 1342 | C | TRP | 374 | 4.690 | 12.310 | 3.365 | 1.00 | 24.31 |
| ATOM | 1343 | O | TRP | 374 | 4.926 | 11.384 | 2.588 | 1.00 | 23.56 |
| ATOM | 1344 | N | LEU | 375 | 5.039 | 12.258 | 4.648 | 1.00 | 24.62 |
| ATOM | 1345 | CA | LEU | 375 | 5.698 | 11.070 | 5.185 | 1.00 | 24.04 |
| ATOM | 1346 | CB | LEU | 375 | 6.074 | 11.282 | 6.657 | 1.00 | 24.87 |
| ATOM | 1347 | CG | LEU | 375 | 7.256 | 12.194 | 7.022 | 1.00 | 24.99 |
| ATOM | 1348 | CD1 | LEU | 375 | 7.422 | 12.210 | 8.542 | 1.00 | 25.00 |
| ATOM | 1349 | CD2 | LEU | 375 | 8.543 | 11.686 | 6.367 | 1.00 | 25.29 |
| ATOM | 1350 | C | LEU | 375 | 4.769 | 9.853 | 5.065 | 1.00 | 23.50 |
| ATOM | 1351 | O | LEU | 375 | 3.584 | 9.946 | 5.366 | 1.00 | 23.84 |
| ATOM | 1352 | N | LEU | 376 | 5.318 | 8.721 | 4.632 | 1.00 | 23.94 |
| ATOM | 1353 | CA | LEU | 376 | 4.540 | 7.489 | 4.471 | 1.00 | 24.75 |
| ATOM | 1354 | CB | LEU | 376 | 5.202 | 6.595 | 3.423 | 1.00 | 24.43 |
| ATOM | 1355 | CG | LEU | 376 | 5.268 | 7.172 | 2.001 | 1.00 | 26.18 |
| ATOM | 1356 | CD1 | LEU | 376 | 6.160 | 6.296 | 1.124 | 1.00 | 27.91 |
| ATOM | 1357 | CD2 | LEU | 376 | 3.858 | 7.260 | 1.415 | 1.00 | 26.56 |
| ATOM | 1358 | C | LEU | 376 | 4.355 | 6.711 | 5.783 | 1.00 | 25.66 |
| ATOM | 1359 | O | LEU | 376 | 3.317 | 6.079 | 6.002 | 1.00 | 24.55 |
| ATOM | 1360 | N | GLU | 377 | 5.359 | 6.758 | 6.654 | 1.00 | 26.36 |
| ATOM | 1361 | CA | GLU | 377 | 5.284 | 6.063 | 7.938 | 1.00 | 27.47 |
| ATOM | 1362 | CB | GLU | 377 | 6.261 | 4.878 | 7.947 | 1.00 | 29.08 |
| ATOM | 1363 | CG | GLU | 377 | 5.959 | 3.840 | 6.875 | 1.00 | 30.40 |
| ATOM | 1364 | CD | GLU | 377 | 6.773 | 2.574 | 7.010 | 1.00 | 32.62 |
| ATOM | 1365 | OE1 | GLU | 377 | 7.981 | 2.578 | 6.704 | 1.00 | 33.66 |
| ATOM | 1366 | OE2 | GLU | 377 | 6.197 | 1.556 | 7.428 | 1.00 | 33.92 |
| ATOM | 1367 | C | GLU | 377 | 5.602 | 7.037 | 9.077 | 1.00 | 27.62 |
| ATOM | 1368 | O | GLU | 377 | 6.619 | 6.902 | 9.762 | 1.00 | 27.21 |
| ATOM | 1369 | N | PRO | 378 | 4.728 | 8.035 | 9.295 | 1.00 | 27.83 |
| ATOM | 1370 | CD | PRO | 378 | 3.483 | 8.329 | 8.561 | 1.00 | 27.90 |
| ATOM | 1371 | CA | PRO | 378 | 4.951 | 9.020 | 10.358 | 1.00 | 28.17 |
| ATOM | 1372 | CB | PRO | 378 | 3.794 | 10.004 | 10.167 | 1.00 | 29.14 |
| ATOM | 1373 | CG | PRO | 378 | 2.707 | 9.143 | 9.564 | 1.00 | 28.27 |
| ATOM | 1374 | C | PRO | 378 | 5.015 | 8.458 | 11.776 | 1.00 | 29.36 |
| ATOM | 1375 | O | PRO | 378 | 5.655 | 9.050 | 12.649 | 1.00 | 28.11 |
| ATOM | 1376 | N | ARG | 379 | 4.349 | 7.328 | 12.008 | 1.00 | 28.75 |
| ATOM | 1377 | CA | ARG | 379 | 4.339 | 6.714 | 13.331 | 1.00 | 30.40 |
| ATOM | 1378 | CB | ARG | 379 | 3.327 | 5.551 | 13.366 | 1.00 | 32.82 |
| ATOM | 1379 | CG | ARG | 379 | 2.361 | 5.554 | 14.559 | 1.00 | 38.05 |
| ATOM | 1380 | CD | ARG | 379 | 3.009 | 5.102 | 15.888 | 1.00 | 42.49 |
| ATOM | 1381 | NE | ARG | 379 | 3.379 | 6.216 | 16.779 | 1.00 | 45.57 |
| ATOM | 1382 | CZ | ARG | 379 | 3.867 | 6.071 | 18.014 | 1.00 | 47.28 |
| ATOM | 1383 | NH1 | ARG | 379 | 4.062 | 4.858 | 18.530 | 1.00 | 48.40 |
| ATOM | 1384 | NH2 | ARG | 379 | 4.145 | 7.142 | 18.752 | 1.00 | 48.86 |
| ATOM | 1385 | C | ARG | 379 | 5.741 | 6.199 | 13.652 | 1.00 | 29.02 |
| ATOM | 1386 | O | ARG | 379 | 6.230 | 6.334 | 14.773 | 1.00 | 29.03 |
| ATOM | 1387 | N | LYS | 380 | 6.377 | 5.611 | 12.647 | 1.00 | 28.46 |
| ATOM | 1388 | CA | LYS | 380 | 7.706 | 5.058 | 12.791 | 1.00 | 29.03 |
| ATOM | 1389 | CB | LYS | 380 | 8.109 | 4.391 | 11.481 | 1.00 | 30.34 |
| ATOM | 1390 | CG | LYS | 380 | 9.086 | 3.254 | 11.621 | 1.00 | 33.47 |
| ATOM | 1391 | CD | LYS | 380 | 9.080 | 2.353 | 10.379 | 1.00 | 34.27 |
| ATOM | 1392 | CE | LYS | 380 | 7.739 | 1.647 | 10.196 | 1.00 | 35.69 |
| ATOM | 1393 | NZ | LYS | 380 | 7.814 | 0.565 | 9.162 | 1.00 | 37.30 |
| ATOM | 1394 | C | LYS | 380 | 8.657 | 6.196 | 13.137 | 1.00 | 29.26 |
| ATOM | 1395 | O | LYS | 380 | 9.528 | 6.049 | 13.999 | 1.00 | 30.09 |
| ATOM | 1396 | N | VAL | 381 | 8.480 | 7.330 | 12.466 | 1.00 | 27.49 |
| ATOM | 1397 | CA | VAL | 381 | 9.314 | 8.501 | 12.713 | 1.00 | 27.12 |
| ATOM | 1398 | CB | VAL | 381 | 9.054 | 9.617 | 11.663 | 1.00 | 26.02 |
| ATOM | 1399 | CG1 | VAL | 381 | 9.821 | 10.888 | 12.038 | 1.00 | 25.31 |
| ATOM | 1400 | CG2 | VAL | 381 | 9.494 | 9.140 | 10.280 | 1.00 | 25.42 |
| ATOM | 1401 | C | VAL | 381 | 9.014 | 9.047 | 14.104 | 1.00 | 27.48 |
| ATOM | 1402 | O | VAL | 381 | 9.914 | 9.478 | 14.830 | 1.00 | 26.45 |
| ATOM | 1403 | N | GLN | 382 | 7.743 | 9.014 | 14.475 | 1.00 | 28.11 |
| ATOM | 1404 | CA | GLN | 382 | 7.324 | 9.508 | 15.775 | 1.00 | 29.95 |
| ATOM | 1405 | CB | GLN | 382 | 5.803 | 9.505 | 15.873 | 1.00 | 31.75 |
| ATOM | 1406 | CG | GLN | 382 | 5.302 | 10.011 | 17.202 | 1.00 | 35.79 |
| ATOM | 1407 | CD | GLN | 382 | 3.886 | 10.501 | 17.117 | 1.00 | 38.98 |
| ATOM | 1408 | OE1 | GLN | 382 | 2.978 | 9.761 | 16.711 | 1.00 | 40.59 |
| ATOM | 1409 | NE2 | GLN | 382 | 3.677 | 11.765 | 17.487 | 1.00 | 40.58 |
| ATOM | 1410 | C | GLN | 382 | 7.907 | 8.707 | 16.931 | 1.00 | 29.66 |
| ATOM | 1411 | O | GLN | 382 | 8.323 | 9.278 | 17.943 | 1.00 | 28.76 |
| ATOM | 1412 | N | LYS | 383 | 7.922 | 7.385 | 16.776 | 1.00 | 29.16 |
| ATOM | 1413 | CA | LYS | 383 | 8.454 | 6.491 | 17.799 | 1.00 | 29.20 |
| ATOM | 1414 | CB | LYS | 383 | 8.303 | 5.027 | 17.369 | 1.00 | 30.44 |
| ATOM | 1415 | CG | LYS | 383 | 6.860 | 4.503 | 17.326 | 1.00 | 33.92 |
| ATOM | 1416 | CD | LYS | 383 | 6.810 | 3.149 | 16.627 | 1.00 | 37.33 |
| ATOM | 1417 | CE | LYS | 383 | 5.486 | 2.419 | 16.837 | 1.00 | 39.20 |
| ATOM | 1418 | NZ | LYS | 383 | 5.439 | 1.665 | 18.135 | 1.00 | 40.75 |
| ATOM | 1419 | C | LYS | 383 | 9.928 | 6.785 | 18.055 | 1.00 | 28.39 |
| ATOM | 1420 | O | LYS | 383 | 10.370 | 6.781 | 19.202 | 1.00 | 28.89 |
| ATOM | 1421 | N | LEU | 384 | 10.692 | 7.019 | 16.990 | 1.00 | 26.11 |
| ATOM | 1422 | CA | LEU | 384 | 12.117 | 7.324 | 17.138 | 1.00 | 25.71 |
| ATOM | 1423 | CB | LEU | 384 | 12.829 | 7.235 | 15.778 | 1.00 | 23.95 |
| ATOM | 1424 | CG | LEU | 384 | 14.332 | 7.536 | 15.718 | 1.00 | 22.17 |
| ATOM | 1425 | CD1 | LEU | 384 | 15.102 | 6.661 | 16.719 | 1.00 | 24.02 |
| ATOM | 1426 | CD2 | LEU | 384 | 14.833 | 7.285 | 14.299 | 1.00 | 21.00 |
| ATOM | 1427 | C | LEU | 384 | 12.330 | 8.711 | 17.760 | 1.00 | 25.43 |
| ATOM | 1428 | O | LEU | 384 | 13.157 | 8.867 | 18.663 | 1.00 | 25.70 |
| ATOM | 1429 | N | GLN | 385 | 11.591 | 9.714 | 17.294 | 1.00 | 24.38 |
| ATOM | 1430 | CA | GLN | 385 | 11.748 | 11.054 | 17.846 | 1.00 | 24.43 |
| ATOM | 1431 | CB | GLN | 385 | 10.819 | 12.072 | 17.167 | 1.00 | 24.78 |
| ATOM | 1432 | CG | GLN | 385 | 11.260 | 13.503 | 17.459 | 1.00 | 22.10 |
| ATOM | 1433 | CD | GLN | 385 | 10.221 | 14.561 | 17.136 | 1.00 | 23.15 |
| ATOM | 1434 | OB1 | GLN | 385 | 10.528 | 15.569 | 16.498 | 1.00 | 24.07 |
| ATOM | 1435 | NE2 | GLN | 385 | 8.996 | 14.350 | 17.592 | 1.00 | 19.99 |
| ATOM | 1436 | C | GLN | 385 | 11.449 | 11.040 | 19.347 | 1.00 | 24.85 |
| ATOM | 1437 | O | GLN | 385 | 12.101 | 11.731 | 20.125 | 1.00 | 23.88 |
| ATOM | 1438 | N | GLU | 386 | 10.449 | 10.265 | 19.745 | 1.00 | 25.17 |
| ATOM | 1439 | CA | GLU | 386 | 10.093 | 10.170 | 21.159 | 1.00 | 27.06 |
| ATOM | 1440 | CB | GLU | 386 | 8.902 | 9.237 | 21.355 | 1.00 | 30.30 |
| ATOM | 1441 | CG | GLU | 386 | 7.581 | 9.951 | 21.578 | 1.00 | 36.42 |
| ATOM | 1442 | CD | GLU | 386 | 6.470 | 8.966 | 21.878 | 1.00 | 40.38 |
| ATOM | 1443 | OE1 | GLU | 386 | 5.997 | 8.309 | 20.923 | 1.00 | 42.90 |
| ATOM | 1444 | OE2 | GLU | 386 | 6.094 | 8.828 | 23.068 | 1.00 | 43.27 |
| ATOM | 1445 | C | GLU | 386 | 11.271 | 9.661 | 21.978 | 1.00 | 25.28 |
| ATOM | 1446 | O | GLU | 386 | 11.563 | 10.198 | 23.044 | 1.00 | 25.32 |
| ATOM | 1447 | N | LYS | 387 | 11.932 | 8.619 | 21.476 | 1.00 | 23.39 |
| ATOM | 1448 | CA | LYS | 387 | 13.088 | 8.038 | 22.148 | 1.00 | 24.19 |
| ATOM | 1449 | CB | LYS | 387 | 13.532 | 6.769 | 21.434 | 1.00 | 24.48 |
| ATOM | 1450 | CG | LYS | 387 | 12.526 | 5.645 | 21.544 | 1.00 | 25.85 |
| ATOM | 1451 | CD | LYS | 387 | 12.849 | 4.512 | 20.605 | 1.00 | 26.31 |
| ATOM | 1452 | CE | LYS | 387 | 11.751 | 3.477 | 20.656 | 1.00 | 25.95 |
| ATOM | 1453 | NZ | LYS | 387 | 11.950 | 2.448 | 19.620 | 1.00 | 27.79 |
| ATOM | 1454 | C | LYS | 387 | 14.244 | 9.031 | 22.191 | 1.00 | 24.21 |
| ATOM | 1455 | O | LYS | 387 | 14.972 | 9.104 | 23.175 | 1.00 | 21.82 |
| ATOM | 1456 | N | ILE | 388 | 14.410 | 9.783 | 21.109 | 1.00 | 23.57 |
| ATOM | 1457 | CA | ILE | 388 | 15.465 | 10.786 | 21.033 | 1.00 | 23.44 |
| ATOM | 1458 | CB | ILE | 388 | 15.484 | 11.463 | 19.637 | 1.00 | 21.69 |
| ATOM | 1459 | CG2 | ILE | 388 | 16.394 | 12.678 | 19.646 | 1.00 | 22.46 |
| ATOM | 1460 | CG1 | ILE | 388 | 15.936 | 10.449 | 18.588 | 1.00 | 23.21 |
| ATOM | 1461 | CD1 | ILE | 388 | 15.987 | 11.002 | 17.186 | 1.00 | 23.27 |
| ATOM | 1462 | C | ILE | 388 | 15.234 | 11.840 | 22.113 | 1.00 | 22.84 |
| ATOM | 1463 | O | ILE | 388 | 16.145 | 12.173 | 22.872 | 1.00 | 22.21 |
| ATOM | 1464 | N | TYR | 389 | 14.009 | 12.351 | 22.198 | 1.00 | 22.43 |
| ATOM | 1465 | CA | TYR | 389 | 13.695 | 13.363 | 23.197 | 1.00 | 24.16 |
| ATOM | 1466 | CB | TYR | 389 | 12.268 | 13.868 | 23.003 | 1.00 | 25.05 |
| ATOM | 1467 | CG | TYR | 389 | 12.164 | 14.996 | 22.000 | 1.00 | 26.11 |
| ATOM | 1468 | CD1 | TYR | 389 | 11.103 | 15.057 | 21.093 | 1.00 | 26.57 |
| ATOM | 1469 | CE1 | TYR | 389 | 10.971 | 16.124 | 20.203 | 1.00 | 25.71 |
| ATOM | 1470 | CD2 | TYR | 389 | 13.101 | 16.037 | 21.990 | 1.00 | 28.12 |
| ATOM | 1471 | CE2 | TYR | 389 | 12.975 | 17.115 | 21.102 | 1.00 | 27.45 |
| ATOM | 1472 | CZ | TYR | 389 | 11.906 | 17.149 | 20.215 | 1.00 | 26.96 |
| ATOM | 1473 | OH | TYR | 389 | 11.754 | 18.218 | 19.356 | 1.00 | 28.94 |
| ATOM | 1474 | C | TYR | 389 | 13.876 | 12.802 | 24.607 | 1.00 | 24.83 |
| ATOM | 1475 | O | TYR | 389 | 14.337 | 13.502 | 25.503 | 1.00 | 24.78 |
| ATOM | 1476 | N | PHE | 390 | 13.514 | 11.537 | 24.794 | 1.00 | 25.27 |
| ATOM | 1477 | CA | PHE | 390 | 13.672 | 10.908 | 26.100 | 1.00 | 27.07 |
| ATOM | 1478 | CB | PHE | 390 | 13.150 | 9.469 | 26.070 | 1.00 | 29.52 |
| ATOM | 1479 | CG | PHE | 390 | 11.710 | 9.331 | 26.499 | 1.00 | 33.25 |
| ATOM | 1480 | CD1 | PHE | 390 | 10.839 | 8.482 | 25.807 | 1.00 | 34.80 |
| ATOM | 1481 | CD2 | PHE | 390 | 11.230 | 10.029 | 27.609 | 1.00 | 34.21 |
| ATOM | 1482 | CE1 | PHE | 390 | 9.499 | 8.325 | 26.208 | 1.00 | 35.97 |
| ATOM | 1483 | CE2 | PHE | 390 | 9.895 | 9.882 | 28.025 | 1.00 | 37.03 |
| ATOM | 1484 | CZ | PHE | 390 | 9.027 | 9.025 | 27.319 | 1.00 | 35.82 |
| ATOM | 1485 | C | PHE | 390 | 15.156 | 10.929 | 26.451 | 1.00 | 25.45 |
| ATOM | 1486 | O | PHE | 390 | 15.531 | 11.227 | 27.584 | 1.00 | 23.68 |
| ATOM | 1487 | N | ALA | 391 | 15.991 | 10.625 | 25.461 | 1.00 | 24.65 |
| ATOM | 1488 | CA | ALA | 391 | 17.432 | 10.627 | 25.658 | 1.00 | 24.20 |
| ATOM | 1489 | CB | ALA | 391 | 18.141 | 10.116 | 24.414 | 1.00 | 24.05 |
| ATOM | 1490 | C | ALA | 391 | 17.907 | 12.036 | 25.989 | 1.00 | 24.18 |
| ATOM | 1491 | O | ALA | 391 | 18.650 | 12.232 | 26.951 | 1.00 | 25.43 |
| ATOM | 1492 | N | LEU | 392 | 17.465 | 13.014 | 25.201 | 1.00 | 24.17 |
| ATOM | 1493 | CA | LEU | 392 | 17.857 | 14.402 | 25.410 | 1.00 | 23.73 |
| ATOM | 1494 | CB | LEU | 392 | 17.178 | 15.309 | 24.375 | 1.00 | 23.42 |
| ATOM | 1495 | CG | LEU | 392 | 17.432 | 16.815 | 24.510 | 1.00 | 21.24 |
| ATOM | 1496 | CD1 | LEU | 392 | 18.928 | 17.097 | 24.423 | 1.00 | 21.69 |
| ATOM | 1497 | CD2 | LEU | 392 | 16.671 | 17.571 | 23.423 | 1.00 | 22.29 |
| ATOM | 1498 | C | LEU | 392 | 17.506 | 14.868 | 26.819 | 1.00 | 25.25 |
| ATOM | 1499 | O | LEU | 392 | 18.293 | 15.549 | 27.474 | 1.00 | 24.27 |
| ATOM | 1500 | N | GLN | 393 | 16.323 | 14.485 | 27.288 | 1.00 | 25.02 |
| ATOM | 1501 | CA | GLN | 393 | 15.864 | 14.873 | 28.614 | 1.00 | 26.69 |
| ATOM | 1502 | CB | GLN | 393 | 14.439 | 14.366 | 28.818 | 1.00 | 29.39 |
| ATOM | 1503 | CG | GLN | 393 | 13.773 | 14.865 | 30.074 | 1.00 | 34.04 |
| ATOM | 1504 | CD | GLN | 393 | 12.294 | 14.569 | 30.079 | 1.00 | 36.82 |
| ATOM | 1505 | OE1 | GLN | 393 | 11.880 | 13.411 | 29.996 | 1.00 | 37.30 |
| ATOM | 1506 | NE2 | GLN | 393 | 11.484 | 15.617 | 30.167 | 1.00 | 38.82 |
| ATOM | 1507 | C | GLN | 393 | 16.783 | 14.349 | 29.724 | 1.00 | 26.46 |
| ATOM | 1508 | O | GLN | 393 | 17.019 | 15.040 | 30.710 | 1.00 | 25.57 |
| ATOM | 1509 | N | HIS | 394 | 17.287 | 13.128 | 29.572 | 1.00 | 25.89 |
| ATOM | 1510 | CA | HIS | 394 | 18.190 | 12.559 | 30.569 | 1.00 | 26.72 |
| ATOM | 1511 | CB | HIS | 394 | 18.314 | 11.049 | 30.375 | 1.00 | 27.82 |
| ATOM | 1512 | CG | HIS | 394 | 17.087 | 10.289 | 30.776 | 1.00 | 31.60 |
| ATOM | 1513 | CD2 | HIS | 394 | 15.994 | 9.927 | 30.065 | 1.00 | 31.53 |
| ATOM | 1514 | ND1 | HIS | 394 | 16.873 | 9.841 | 32.063 | 1.00 | 33.22 |
| ATOM | 1515 | CE1 | HIS | 394 | 15.702 | 9.238 | 32.127 | 1.00 | 33.60 |
| ATOM | 1516 | NE2 | HIS | 394 | 15.146 | 9.276 | 30.929 | 1.00 | 34.06 |
| ATOM | 1517 | C | HIS | 394 | 19.567 | 13.208 | 30.491 | 1.00 | 25.56 |
| ATOM | 1518 | O | HIS | 394 | 20.155 | 13.552 | 31.513 | 1.00 | 25.72 |
| ATOM | 1519 | N | VAL | 395 | 20.082 | 13.385 | 29.274 | 1.00 | 25.55 |
| ATOM | 1520 | CA | VAL | 395 | 21.402 | 13.993 | 29.094 | 1.00 | 24.89 |
| ATOM | 1521 | CB | VAL | 395 | 21.821 | 13.992 | 27.599 | 1.00 | 25.87 |
| ATOM | 1522 | CG1 | VAL | 395 | 23.094 | 14.815 | 27.399 | 1.00 | 25.30 |
| ATOM | 1523 | CG2 | VAL | 395 | 22.056 | 12.554 | 27.134 | 1.00 | 25.17 |
| ATOM | 1524 | C | VAL | 395 | 21.461 | 15.426 | 29.633 | 1.00 | 25.38 |
| ATOM | 1525 | O | VAL | 395 | 22.476 | 15.847 | 30.185 | 1.00 | 23.83 |
| ATOM | 1526 | N | ILE | 396 | 20.366 | 16.162 | 29.488 | 1.00 | 24.76 |
| ATOM | 1527 | CA | ILE | 396 | 20.310 | 17.542 | 29.956 | 1.00 | 25.56 |
| ATOM | 1528 | CB | ILE | 396 | 18.953 | 18.180 | 29.588 | 1.00 | 25.31 |
| ATOM | 1529 | CG2 | ILE | 396 | 18.725 | 19.465 | 30.382 | 1.00 | 24.55 |
| ATOM | 1530 | CG1 | ILE | 396 | 18.910 | 18.465 | 28.080 | 1.00 | 24.97 |
| ATOM | 1531 | CD1 | ILE | 396 | 17.556 | 18.989 | 27.593 | 1.00 | 24.29 |
| ATOM | 1532 | C | ILE | 396 | 20.531 | 17.635 | 31.471 | 1.00 | 28.65 |
| ATOM | 1533 | O | ILE | 396 | 21.134 | 18.583 | 31.960 | 1.00 | 28.05 |
| ATOM | 1534 | N | GLN | 397 | 20.046 | 16.639 | 32.203 | 1.00 | 31.32 |
| ATOM | 1535 | CA | GLN | 397 | 20.191 | 16.609 | 33.656 | 1.00 | 35.38 |
| ATOM | 1536 | CB | GLN | 397 | 19.472 | 15.393 | 34.221 | 1.00 | 36.34 |
| ATOM | 1537 | CG | GLN | 397 | 18.056 | 15.243 | 33.752 | 1.00 | 39.24 |
| ATOM | 1538 | CD | GLN | 397 | 17.387 | 14.038 | 34.382 | 1.00 | 42.16 |
| ATOM | 1539 | OE1 | GLN | 397 | 17.995 | 12.971 | 34.522 | 1.00 | 42.62 |
| ATOM | 1540 | NE2 | GLN | 397 | 16.125 | 14.195 | 34.755 | 1.00 | 43.91 |
| ATOM | 1541 | C | GLN | 397 | 21.640 | 16.577 | 34.143 | 1.00 | 37.04 |
| ATOM | 1542 | O | GLN | 397 | 21.936 | 17.049 | 35.240 | 1.00 | 37.11 |
| ATOM | 1543 | N | LYS | 398 | 22.533 | 15.993 | 33.350 | 1.00 | 39.23 |
| ATOM | 1544 | CA | LYS | 398 | 23.945 | 15.915 | 33.733 | 1.00 | 42.23 |
| ATOM | 1545 | CB | LYS | 398 | 24.742 | 15.089 | 32.720 | 1.00 | 40.46 |
| ATOM | 1546 | CG | LYS | 398 | 24.193 | 13.689 | 32.476 | 1.00 | 39.92 |
| ATOM | 1547 | CD | LYS | 398 | 25.170 | 12.833 | 31.675 | 1.00 | 39.62 |
| ATOM | 1548 | CE | LYS | 398 | 25.430 | 13.434 | 30.286 | 1.00 | 39.22 |
| ATOM | 1549 | NZ | LYS | 398 | 26.362 | 12.596 | 29.484 | 1.00 | 37.98 |
| ATOM | 1550 | C | LYS | 398 | 24.557 | 17.306 | 33.818 | 1.00 | 44.28 |
| ATOM | 1551 | O | LYS | 398 | 25.543 | 17.535 | 34.529 | 1.00 | 44.44 |
| ATOM | 1552 | N | ASN | 399 | 23.964 | 18.241 | 33.091 | 1.00 | 45.83 |
| ATOM | 1553 | CA | ASN | 399 | 24.469 | 19.595 | 33.063 | 1.00 | 47.39 |
| ATOM | 1554 | CB | ASN | 399 | 24.734 | 19.967 | 31.604 | 1.00 | 47.57 |
| ATOM | 1555 | CG | ASN | 399 | 25.348 | 18.807 | 30.803 | 1.00 | 47.79 |
| ATOM | 1556 | OD1 | ASN | 399 | 26.505 | 18.434 | 31.010 | 1.00 | 47.93 |
| ATOM | 1557 | ND2 | ASN | 399 | 24.564 | 18.226 | 29.896 | 1.00 | 48.24 |
| ATOM | 1558 | C | ASN | 399 | 23.474 | 20.560 | 33.710 | 1.00 | 49.01 |
| ATOM | 1559 | O | ASN | 399 | 23.431 | 20.717 | 34.937 | 1.00 | 49.34 |
| ATOM | 1560 | N | HIS | 400 | 22.671 | 21.186 | 32.861 | 1.00 | 50.43 |
| ATOM | 1561 | CA | HIS | 400 | 21.655 | 22.160 | 33.240 | 1.00 | 52.29 |
| ATOM | 1562 | CB | HIS | 400 | 20.737 | 22.349 | 32.046 | 1.00 | 51.08 |
| ATOM | 1563 | CG | HIS | 400 | 21.475 | 22.344 | 30.750 | 1.00 | 50.35 |
| ATOM | 1564 | CD2 | HIS | 400 | 21.653 | 21.368 | 29.832 | 1.00 | 50.24 |
| ATOM | 1565 | ND1 | HIS | 400 | 22.219 | 23.419 | 30.316 | 1.00 | 50.79 |
| ATOM | 1566 | CE1 | HIS | 400 | 22.823 | 23.102 | 29.183 | 1.00 | 50.91 |
| ATOM | 1567 | NE2 | HIS | 400 | 22.495 | 21.862 | 28.871 | 1.00 | 49.91 |
| ATOM | 1568 | C | HIS | 400 | 20.839 | 21.782 | 34.467 | 1.00 | 54.62 |
| ATOM | 1569 | O | HIS | 400 | 19.881 | 21.006 | 34.369 | 1.00 | 54.65 |
| ATOM | 1570 | N | LEU | 401 | 21.211 | 22.343 | 35.618 | 1.00 | 56.91 |
| ATOM | 1571 | CA | LEU | 401 | 20.487 | 22.061 | 36.854 | 1.00 | 59.10 |
| ATOM | 1572 | CB | LEU | 401 | 21.306 | 22.469 | 38.081 | 1.00 | 59.50 |
| ATOM | 1573 | CG | LEU | 401 | 21.083 | 21.490 | 39.244 | 1.00 | 60.29 |
| ATOM | 1574 | CD1 | LEU | 401 | 19.671 | 21.626 | 39.794 | 1.00 | 60.01 |
| ATOM | 1575. | CD2 | LEU | 401 | 21.317 | 20.062 | 38.748 | 1.00 | 59.79 |
| ATOM | 1576 | C | LEU | 401 | 19.224 | 22.890 | 36.729 | 1.00 | 60.18 |
| ATOM | 1577 | O | LEU | 401 | 19.275 | 24.017 | 36.220 | 1.00 | 61.06 |
| ATOM | 1578 | N | ASP | 402 | 18.097 | 22.341 | 37.188 | 1.00 | 60.81 |
| ATOM | 1579 | CA | ASP | 402 | 16.814 | 23.001 | 37.016 | 1.00 | 61.03 |
| ATOM | 1580 | CB | ASP | 402 | 16.766 | 24.371 | 37.711 | 1.00 | 62.09 |
| ATOM | 1581 | CG | ASP | 402 | 16.409 | 24.256 | 39.205 | 1.00 | 63.42 |
| ATOM | 1582 | OD1 | ASP | 402 | 16.940 | 23.342 | 39.883 | 1.00 | 63.79 |
| ATOM | 1583 | OD2 | ASP | 402 | 15.609 | 25.076 | 39.716 | 1.00 | 63.96 |
| ATOM | 1584 | C | ASP | 402 | 16.836 | 23.099 | 35.499 | 1.00 | 60.63 |
| ATOM | 1585 | O | ASP | 402 | 17.621 | 22.375 | 34.864 | 1.00 | 61.17 |
| ATOM | 1586 | N | ASP | 403 | 16.035 | 23.950 | 34.882 | 1.00 | 59.71 |
| ATOM | 1587 | CA | ASP | 403 | 16.083 | 23.943 | 33.430 | 1.00 | 57.97 |
| ATOM | 1588 | CB | ASP | 403 | 15.969 | 22.502 | 32.944 | 1.00 | 57.91 |
| ATOM | 1589 | CG | ASP | 403 | 14.749 | 21.799 | 33.538 | 1.00 | 58.27 |
| ATOM | 1590 | OD1 | ASP | 403 | 13.789 | 21.500 | 32.785 | 1.00 | 57.61 |
| ATOM | 1591 | OD2 | ASP | 403 | 14.747 | 21.570 | 34.774 | 1.00 | 57.74 |
| ATOM | 1592 | C | ASP | 403 | 15.025 | 24.719 | 32.703 | 1.00 | 56.47 |
| ATOM | 1593 | O | ASP | 403 | 15.042 | 25.947 | 32.631 | 1.00 | 56.67 |
| ATOM | 1594 | N | GLU | 404 | 14.097 | 23.936 | 32.157 | 1.00 | 54.01 |
| ATOM | 1595 | CA | GLU | 404 | 13.021 | 24.422 | 31.319 | 1.00 | 50.92 |
| ATOM | 1596 | CB | GLU | 404 | 12.788 | 25.917 | 31.554 | 1.00 | 52.53 |
| ATOM | 1597 | CG | GLU | 404 | 11.500 | 26.492 | 30.994 | 1.00 | 55.75 |
| ATOM | 1598 | CD | GLU | 404 | 11.230 | 27.889 | 31.537 | 1.00 | 57.67 |
| ATOM | 1599 | OB1 | GLU | 404 | 10.211 | 28.503 | 31.134 | 1.00 | 58.54 |
| ATOM | 1600 | OE2 | GLU | 404 | 12.041 | 28.371 | 32.375 | 1.00 | 58.20 |
| ATOM | 1601 | C | GLU | 404 | 13.714 | 24.171 | 29.977 | 1.00 | 47.15 |
| ATOM | 1602 | O | GLU | 404 | 13.121 | 24.300 | 28.907 | 1.00 | 46.59 |
| ATOM | 1603 | N | THR | 405 | 14.986 | 23.774 | 30.070 | 1.00 | 43.07 |
| ATOM | 1604 | CA | THR | 405 | 15.815 | 23.492 | 28.895 | 1.00 | 38.65 |
| ATOM | 1605 | CB | THR | 405 | 17.122 | 22.739 | 29.288 | 1.00 | 38.43 |
| ATOM | 1606 | OG1 | THR | 405 | 17.950 | 23.598 | 30.083 | 1.00 | 36.75 |
| ATOM | 1607 | CG2 | THR | 405 | 17.915 | 22.330 | 28.036 | 1.00 | 37.77 |
| ATOM | 1608 | C | THR | 405 | 15.101 | 22.712 | 27.791 | 1.00 | 35.86 |
| ATOM | 1609 | O | THR | 405 | 14.859 | 23.258 | 26.721 | 1.00 | 33.56 |
| ATOM | 1610 | N | LEU | 406 | 14.749 | 21.449 | 28.039 | 1.00 | 34.04 |
| ATOM | 1611 | CA | LEU | 406 | 14.083 | 20.668 | 27.000 | 1.00 | 32.51 |
| ATOM | 1612 | CB | LEU | 406 | 13.653 | 19.290 | 27.516 | 1.00 | 32.98 |
| ATOM | 1613 | CG | LEU | 406 | 13.758 | 18.137 | 26.500 | 1.00 | 31.34 |
| ATOM | 1614 | CD1 | LEU | 406 | 12.660 | 17.122 | 26.773 | 1.00 | 32.45 |
| ATOM | 1615 | CD2 | LEU | 406 | 13.645 | 18.644 | 25.077 | 1.00 | 32.35 |
| ATOM | 1616 | C | LEU | 406 | 12.856 | 21.395 | 26.456 | 1.00 | 31.91 |
| ATOM | 1617 | O | LEU | 406 | 12.625 | 21.414 | 25.253 | 1.00 | 30.54 |
| ATOM | 1618 | N | ALA | 407 | 12.070 | 21.988 | 27.348 | 1.00 | 32.12 |
| ATOM | 1619 | CA | ALA | 407 | 10.879 | 22.720 | 26.931 | 1.00 | 31.85 |
| ATOM | 1620 | CB | ALA | 407 | 10.078 | 23.150 | 28.146 | 1.00 | 31.21 |
| ATOM | 1621 | C | ALA | 407 | 11.246 | 23.942 | 26.084 | 1.00 | 31.46 |
| ATOM | 1622 | O | ALA | 407 | 10.584 | 24.221 | 25.093 | 1.00 | 31.62 |
| ATOM | 1623 | N | LYS | 408 | 12.296 | 24.667 | 26.473 | 1.00 | 31.28 |
| ATOM | 1624 | CA | LYS | 408 | 12.723 | 25.849 | 25.718 | 1.00 | 30.77 |
| ATOM | 1625 | CB | LYS | 408 | 13.794 | 26.635 | 26.481 | 1.00 | 33.47 |
| ATOM | 1626 | CG | LYS | 408 | 13.279 | 27.310 | 27.747 | 1.00 | 35.82 |
| ATOM | 1627 | CD | LYS | 408 | 14.332 | 28.218 | 28.353 | 1.00 | 36.77 |
| ATOM | 1628 | CE | LYS | 408 | 13.747 | 29.011 | 29.522 | 1.00 | 38.42 |
| ATOM | 1629 | NZ | LYS | 408 | 14.719 | 29.999 | 30.088 | 1.00 | 39.51 |
| ATOM | 1630 | C | LYS | 408 | 13.263 | 25.462 | 24.351 | 1.00 | 28.18 |
| ATOM | 1631 | O | LYS | 408 | 13.158 | 26.229 | 23.396 | 1.00 | 27.16 |
| ATOM | 1632 | N | LEU | 409 | 13.858 | 24.276 | 24.265 | 1.00 | 27.09 |
| ATOM | 1633 | CA | LEU | 409 | 14.387 | 23.793 | 22.994 | 1.00 | 26.20 |
| ATOM | 1634 | CB | LEU | 409 | 15.246 | 22.546 | 23.210 | 1.00 | 24.61 |
| ATOM | 1635 | CG | LEU | 409 | 16.608 | 22.793 | 23.844 | 1.00 | 25.33 |
| ATOM | 1636 | CD1 | LEU | 409 | 17.347 | 21.465 | 24.018 | 1.00 | 23.27 |
| ATOM | 1637 | CD2 | LEU | 409 | 17.399 | 23.757 | 22.957 | 1.00 | 25.46 |
| ATOM | 1638 | C | LEU | 409 | 13.228 | 23.462 | 22.057 | 1.00 | 26.11 |
| ATOM | 1639 | O | LEU | 409 | 13.184 | 23.928 | 20.916 | 1.00 | 25.38 |
| ATOM | 1640 | N | ILE | 410 | 12.289 | 22.657 | 22.548 | 1.00 | 25.80 |
| ATOM | 1641 | CA | ILE | 410 | 11.130 | 22.263 | 21.750 | 1.00 | 27.11 |
| ATOM | 1642 | CB | ILE | 410 | 10.212 | 21.309 | 22.556 | 1.00 | 27.55 |
| ATOM | 1643 | CG2 | ILE | 410 | 8.831 | 21.225 | 21.915 | 1.00 | 29.14 |
| ATOM | 1644 | CG1 | ILE | 410 | 10.862 | 19.925 | 22.640 | 1.00 | 28.88 |
| ATOM | 1645 | CD1 | ILE | 410 | 10.227 | 18.999 | 23.672 | 1.00 | 29.04 |
| ATOM | 1646 | C | ILE | 410 | 10.324 | 23.477 | 21.271 | 1.00 | 26.54 |
| ATOM | 1647 | O | ILE | 410 | 9.801 | 23.483 | 20.157 | 1.00 | 27.28 |
| ATOM | 1648 | N | ALA | 411 | 10.240 | 24.504 | 22.108 | 1.00 | 26.52 |
| ATOM | 1649 | CA | ALA | 411 | 9.501 | 25.716 | 21.762 | 1.00 | 27.27 |
| ATOM | 1650 | CB | ALA | 411 | 9.388 | 26.618 | 22.982 | 1.00 | 28.31 |
| ATOM | 1651 | C | ALA | 411 | 10.187 | 26.473 | 20.618 | 1.00 | 28.07 |
| ATOM | 1652 | O | ALA | 411 | 9.600 | 27.379 | 20.026 | 1.00 | 27.10 |
| ATOM | 1653 | N | LYS | 412 | 11.426 | 26.095 | 20.310 | 1.00 | 27.07 |
| ATOM | 1654 | CA | LYS | 412 | 12.175 | 26.751 | 19.244 | 1.00 | 28.19 |
| ATOM | 1655 | CB | LYS | 412 | 13.680 | 26.756 | 19.579 | 1.00 | 28.62 |
| ATOM | 1656 | CG | LYS | 412 | 14.067 | 27.694 | 20.728 | 1.00 | 30.34 |
| ATOM | 1657 | CD | LYS | 412 | 15.559 | 27.615 | 21.042 | 1.00 | 31.67 |
| ATOM | 1658 | CB | LYS | 412 | 15.950 | 28.514 | 22.215 | 1.00 | 33.03 |
| ATOM | 1659 | NZ | LYS | 412 | 15.797 | 29.985 | 21.964 | 1.00 | 35.05 |
| ATOM | 1660 | C | LYS | 412 | 11.955 | 26.117 | 17.864 | 1.00 | 27.51 |
| ATOM | 1661 | O | LYS | 412 | 12.316 | 26.708 | 16.847 | 1.00 | 27.32 |
| ATOM | 1662 | N | ILE | 413 | 11.363 | 24.926 | 17.834 | 1.00 | 26.06 |
| ATOM | 1663 | CA | ILE | 413 | 11.105 | 24.202 | 16.588 | 1.00 | 26.80 |
| ATOM | 1664 | CB | ILE | 413 | 10.220 | 22.939 | 16.841 | 1.00 | 27.43 |
| ATOM | 1665 | CG2 | ILE | 413 | 9.881 | 22.247 | 15.523 | 1.00 | 27.29 |
| ATOM | 1666 | CG1 | ILE | 413 | 10.945 | 21.958 | 17.770 | 1.00 | 28.37 |
| ATOM | 1667 | CD1 | ILE | 413 | 12.121 | 21.254 | 17.148 | 1.00 | 29.37 |
| ATOM | 1668 | C | ILE | 413 | 10.433 | 25.057 | 15.519 | 1.00 | 26.56 |
| ATOM | 1669 | O | ILE | 413 | 10.864 | 25.076 | 14.372 | 1.00 | 26.28 |
| ATOM | 1670 | N | PRO | 414 | 9.359 | 25.775 | 15.882 | 1.00 | 27.37 |
| ATOM | 1671 | CD | PRO | 414 | 8.700 | 25.857 | 17.198 | 1.00 | 26.88 |
| ATOM | 1672 | CA | PRO | 414 | 8.669 | 26.614 | 14.897 | 1.00 | 26.65 |
| ATOM | 1673 | CB | PRO | 414 | 7.427 | 27.080 | 15.658 | 1.00 | 27.87 |
| ATOM | 1674 | CG | PRO | 414 | 7.903 | 27.129 | 17.070 | 1.00 | 29.01 |
| ATOM | 1675 | C | PRO | 414 | 9.525 | 27.771 | 14.374 | 1.00 | 25.68 |
| ATOM | 1676 | O | PRO | 414 | 9.448 | 28.126 | 13.201 | 1.00 | 27.18 |
| ATOM | 1677 | N | THR | 415 | 10.352 | 28.342 | 15.241 | 1.00 | 25.08 |
| ATOM | 1678 | CA | THR | 415 | 11.226 | 29.448 | 14.850 | 1.00 | 25.70 |
| ATOM | 1679 | CB | THR | 415 | 11.944 | 30.028 | 16.089 | 1.00 | 27.08 |
| ATOM | 1680 | OG1 | THR | 415 | 10.964 | 30.406 | 17.060 | 1.00 | 27.92 |
| ATOM | 1681 | CG2 | THR | 415 | 12.768 | 31.250 | 15.727 | 1.00 | 26.93 |
| ATOM | 1682 | C | THR | 415 | 12.260 | 28.954 | 13.825 | 1.00 | 23.76 |
| ATOM | 1683 | O | THR | 415 | 12.546 | 29.624 | 12.839 | 1.00 | 23.92 |
| ATOM | 1684 | N | ILE | 416 | 12.801 | 27.768 | 14.072 | 1.00 | 22.76 |
| ATOM | 1685 | CA | ILE | 416 | 13.780 | 27.158 | 13.184 | 1.00 | 21.78 |
| ATOM | 1686 | CB | ILE | 416 | 14.175 | 25.760 | 13.711 | 1.00 | 22.29 |
| ATOM | 1687 | CG2 | ILE | 416 | 14.921 | 24.976 | 12.648 | 1.00 | 21.98 |
| ATOM | 1688 | CG1 | ILE | 416 | 15.012 | 25.918 | 14.979 | 1.00 | 20.99 |
| ATOM | 1689 | CD1 | ILE | 416 | 15.190 | 24.634 | 15.776 | 1.00 | 22.24 |
| ATOM | 1690 | C | ILE | 416 | 13.187 | 27.041 | 11.785 | 1.00 | 20.31 |
| ATOM | 1691 | O | ILE | 416 | 13.790 | 27.454 | 10.797 | 1.00 | 19.18 |
| ATOM | 1692 | N | THR | 417 | 11.988 | 26.480 | 11.710 | 1.00 | 20.67 |
| ATOM | 1693 | CA | THR | 417 | 11.328 | 26.317 | 10.432 | 1.00 | 20.39 |
| ATOM | 1694 | CB | THR | 417 | 10.048 | 25.508 | 10.602 | 1.00 | 21.40 |
| ATOM | 1695 | OG1 | THR | 417 | 10.390 | 24.228 | 11.147 | 1.00 | 20.70 |
| ATOM | 1696 | CG2 | THR | 417 | 9.351 | 25.313 | 9.257 | 1.00 | 19.07 |
| ATOM | 1697 | C | THR | 417 | 11.026 | 27.661 | 9.785 | 1.00 | 20.61 |
| ATOM | 1698 | O | THR | 417 | 11.118 | 27.795 | 8.570 | 1.00 | 21.29 |
| ATOM | 1699 | N | ALA | 418 | 10.674 | 28.651 | 10.605 | 1.00 | 21.09 |
| ATOM | 1700 | CA | ALA | 418 | 10.367 | 29.987 | 10.118 | 1.00 | 20.90 |
| ATOM | 1701 | CB | ALA | 418 | 9.956 | 30.880 | 11.273 | 1.00 | 21.36 |
| ATOM | 1702 | C | ALA | 418 | 11.573 | 30.592 | 9.404 | 1.00 | 21.24 |
| ATOM | 1703 | O | ALA | 418 | 11.441 | 31.157 | 8.321 | 1.00 | 19.90 |
| ATOM | 1704 | N | VAL | 419 | 12.747 | 30.457 | 10.015 | 1.00 | 21.50 |
| ATOM | 1705 | CA | VAL | 419 | 13.975 | 30.995 | 9.440 | 1.00 | 21.76 |
| ATOM | 1706 | CB | VAL | 419 | 15.170 | 30.797 | 10.406 | 1.00 | 20.86 |
| ATOM | 1707 | CG1 | VAL | 419 | 16.491 | 31.146 | 9.713 | 1.00 | 22.17 |
| ATOM | 1708 | CG2 | VAL | 419 | 14.969 | 31.655 | 11.648 | 1.00 | 23.28 |
| ATOM | 1709 | C | VAL | 419 | 14.282 | 30.328 | 8.104 | 1.00 | 21.71 |
| ATOM | 1710 | O | VAL | 419 | 14.586 | 30.997 | 7.120 | 1.00 | 21.28 |
| ATOM | 1711 | N | CYS | 420 | 14.180 | 29.006 | 8.065 | 1.00 | 20.93 |
| ATOM | 1712 | CA | CYS | 420 | 14.467 | 28.282 | 6.840 | 1.00 | 22.24 |
| ATOM | 1713 | CB | CYS | 420 | 14.560 | 26.782 | 7.137 | 1.00 | 20.03 |
| ATOM | 1714 | SG | CYS | 420 | 15.994 | 26.393 | 8.177 | 1.00 | 21.62 |
| ATOM | 1715 | C | CYS | 420 | 13.462 | 28.573 | 5.724 | 1.00 | 22.17 |
| ATOM | 1716 | O | CYS | 420 | 13.815 | 28.560 | 4.545 | 1.00 | 22.39 |
| ATOM | 1717 | N | ASN | 421 | 12.213 | 28.843 | 6.090 | 1.00 | 22.88 |
| ATOM | 1718 | CA | ASN | 421 | 11.213 | 29.165 | 5.083 | 1.00 | 23.16 |
| ATOM | 1719 | CB | ASN | 421 | 9.803 | 29.189 | 5.686 | 1.00 | 24.82 |
| ATOM | 1720 | CG | ASN | 421 | 9.254 | 27.797 | 5.945 | 1.00 | 26.54 |
| ATOM | 1721 | OD1 | ASN | 421 | 9.691 | 26.821 | 5.332 | 1.00 | 28.43 |
| ATOM | 1722 | ND2 | ASN | 421 | 8.274 | 27.703 | 6.841 | 1.00 | 28.17, |
| ATOM | 1723 | C | ASN | 421 | 11.548 | 30.539 | 4.507 | 1.00 | 22.83 |
| ATOM | 1724 | O | ASN | 421 | 11.372 | 30.783 | 3.311 | 1.00 | 23.42 |
| ATOM | 1725 | N | LEU | 422 | 12.026 | 31.438 | 5.362 | 1.00 | 21.51 |
| ATOM | 1726 | CA | LEU | 422 | 12.394 | 32.773 | 4.912 | 1.00 | 21.12 |
| ATOM | 1727 | CB | LEU | 422 | 12.814 | 33.657 | 6.094 | 1.00 | 20.93 |
| ATOM | 1728 | CG | LEU | 422 | 13.106 | 35.117 | 5.724 | 1.00 | 22.12 |
| ATOM | 1729 | CD1 | LEU | 422 | 11.914 | 35.708 | 4.998 | 1.00 | 23.58 |
| ATOM | 1730 | CD2 | LEU | 422 | 13.420 | 35.919 | 6.968 | 1.00 | 21.92 |
| ATOM | 1731 | C | LEU | 422 | 13.539 | 32.651 | 3.909 | 1.00 | 20.79 |
| ATOM | 1732 | O | LEU | 422 | 13.589 | 33.385 | 2.924 | 1.00 | 20.16 |
| ATOM | 1733 | N | HIS | 423 | 14.457 | 31.721 | 4.162 | 1.00 | 20.32 |
| ATOM | 1734 | CA | HIS | 423 | 15.569 | 31.496 | 3.246 | 1.00 | 19.09 |
| ATOM | 1735 | CB | HIS | 423 | 16.480 | 30.377 | 3.774 | 1.00 | 19.51 |
| ATOM | 1736 | CG | HIS | 423 | 17.357 | 29.757 | 2.726 | 1.00 | 20.42 |
| ATOM | 1737 | CD2 | HIS | 423 | 18.560 | 30.132 | 2.233 | 1.00 | 22.15 |
| ATOM | 1738 | ND1 | HIS | 423 | 17.002 | 28.617 | 2.037 | 1.00 | 19.93 |
| ATOM | 1739 | CE1 | HIS | 423 | 17.945 | 28.317 | 1.164 | 1.00 | 21.28 |
| ATOM | 1740 | NE2 | HIS | 423 | 18.903 | 29.221 | 1.261 | 1.00 | 23.18 |
| ATOM | 1741 | C | HIS | 423 | 14.991 | 31.114 | 1.887 | 1.00 | 20.06 |
| ATOM | 1742 | O | HIS | 423 | 15.406 | 31.633 | 0.854 | 1.00 | 19.48 |
| ATOM | 1743 | N | GLY | 424 | 14.025 | 30.202 | 1.897 | 1.00 | 20.89 |
| ATOM | 1744 | CA | GLY | 424 | 13.409 | 29.779 | 0.656 | 1.00 | 21.77 |
| ATOM | 1745 | C | GLY | 424 | 12.747 | 30.948 | -0.045 | 1.00 | 22.46 |
| ATOM | 1746 | O | GLY | 424 | 12.854 | 31.092 | -1.260 | 1.00 | 22.28 |
| ATOM | 1747 | N | GLU | 425 | 12.055 | 31.781 | 0.723 | 1.00 | 23.68 |
| ATOM | 1748 | CA | GLU | 425 | 11.388 | 32.944 | 0.156 | 1.00 | 26.30 |
| ATOM | 1749 | CB | GLU | 425 | 10.654 | 33.714 | 1.254 | 1.00 | 29.71 |
| ATOM | 1750 | CG | GLU | 425 | 9.438 | 32.987 | 1.807 | 1.00 | 34.81 |
| ATOM | 1751 | CD | GLU | 425 | 8.840 | 33.708 | 3.000 | 1.00 | 39.53 |
| ATOM | 1752 | OE1 | GLU | 425 | 8.732 | 34.960 | 2.940 | 1.00 | 40.10 |
| ATOM | 1753 | OE2 | GLU | 425 | 8.471 | 33.026 | 3.992 | 1.00 | 42.79 |
| ATOM | 1754 | C | GLU | 425 | 12.380 | 33.862 | -0.553 | 1.00 | 25.74 |
| ATOM | 1755 | O | GLU | 425 | 12.166 | 34.228 | -1.708 | 1.00 | 26.36 |
| ATOM | 1756 | N | LYS | 426 | 13.468 | 34.221 | 0.125 | 1.00 | 25.26 |
| ATOM | 1757 | CA | LYS | 426 | 14.484 | 35.094 | -0.470 | 1.00 | 25.80 |
| ATOM | 1758 | CB | LYS | 426 | 15.610 | 35.376 | 0.531 | 1.00 | 25.12 |
| ATOM | 1759 | CG | LYS | 426 | 15.150 | 36.115 | 1.775 | 1.00 | 28.49 |
| ATOM | 1760 | CD | LYS | 426 | 14.489 | 37.445 | 1.415 | 1.00 | 29.27 |
| ATOM | 1761 | CE | LYS | 426 | 13.995 | 38.148 | 2.666 | 1.00 | 32.12 |
| ATOM | 1762 | NZ | LYS | 426 | 13.235 | 39.411 | 2.390 | 1.00 | 31.15 |
| ATOM | 1763 | C | LYS | 426 | 15.083 | 34.497 | -1.733 | 1.00 | 25.06 |
| ATOM | 1764 | O | LYS | 426 | 15.363 | 35.209 | -2.703 | 1.00 | 24.27 |
| ATOM | 1765 | N | LEU | 427 | 15.278 | 33.183 | -1.717 | 1.00 | 25.20 |
| ATOM | 1766 | CA | LEU | 427 | 15.848 | 32.480 | -2.857 | 1.00 | 26.06 |
| ATOM | 1767 | CB | LEU | 427 | 16.165 | 31.032 | -2.473 | 1.00 | 25.95 |
| ATOM | 1768 | CG | LEU | 427 | 16.582 | 30.058 | -3.581 | 1.00 | 27.66 |
| ATOM | 1769 | CD1 | LEU | 427 | 17.718 | 30.638 | -4.415 | 1.00 | 26.38 |
| ATOM | 1770 | CD2 | LEU | 427 | 17.002 | 28.740 | -2.940 | 1.00 | 27.07 |
| ATOM | 1771 | C | LEU | 427 | 14.922 | 32.523 | -4.072 | 1.00 | 26.54 |
| ATOM | 1772 | O | LEU | 427 | 15.383 | 32.716 | -5.194 | 1.00 | 26.46 |
| ATOM | 1773 | N | GLN | 428 | 13.622 | 32.369 | -3.847 | 1.00 | 27.45 |
| ATOM | 1774 | CA | GLN | 428 | 12.653 | 32.399 | -4.942 | 1.00 | 29.19 |
| ATOM | 1775 | CB | GLN | 428 | 11.247 | 32.108 | -4.416 | 1.00 | 32.05 |
| ATOM | 1776 | CG | GLN | 428 | 11.172 | 30.852 | -3.561 | 1.00 | 36.92 |
| ATOM | 1777 | CD | GLN | 428 | 12.009 | 29.714 | -4.139 | 1.00 | 39.47 |
| ATOM | 1778 | OE1 | GLN | 428 | 11.946 | 29.442 | -5.345 | 1.00 | 41.41 |
| ATOM | 1779 | NE2 | GLN | 428 | 12.788 | 29.034 | -3.281 | 1.00 | 37.83 |
| ATOM | 1780 | C | GLN | 428 | 12.654 | 33.736 | -5.675 | 1.00 | 28.90 |
| ATOM | 1781 | O | GIN | 428 | 12.466 | 33.780 | -6.889 | 1.00 | 28.50 |
| ATOM | 1782 | N | VAL | 429 | 12.844 | 34.826 | -4.938 | 1.00 | 28.63 |
| ATOM | 1783 | CA | VAL | 429 | 12.893 | 36.145 | -5.551 | 1.00 | 29.18 |
| ATOM | 1784 | CB | VAL | 429 | 12.708 | 37.272 | -4.515 | 1.00 | 29.49 |
| ATOM | 1785 | CG1 | VAL | 429 | 12.789 | 38.632 | -5.215 | 1.00 | 30.49 |
| ATOM | 1786 | CG2 | VAL | 429 | 11.362 | 37.126 | -3.824 | 1.00 | 30.87 |
| ATOM | 1787 | C | VAL | 429 | 14.260 | 36.297 | -6.210 | 1.00 | 28.70 |
| ATOM | 1788 | O | VAL | 429 | 14.398 | 36.924 | -7.267 | 1.00 | 27.69 |
| ATOM | 1789 | N | PHE | 430 | 15.279 | 35.723 | -5.584 | 1.00 | 28.04 |
| ATOM | 1790 | CA | PHE | 430 | 16.601 | 35.793 | -6.177 | 1.00 | 28.41 |
| ATOM | 1791 | CB | PHE | 430 | 17.633 | 35.074 | -5.317 | 1.00 | 27.09 |
| ATOM | 1792 | CG | PHE | 430 | 19.021 | 35.157 | -5.872 | 1.00 | 27.44 |
| ATOM | 1793 | CD1 | PRE | 430 | 19.777 | 36.314 | -5.711 | 1.00 | 27.34 |
| ATOM | 1794 | CD2 | PHE | 430 | 19.553 | 34.096 | -6.602 | 1.00 | 26.42 |
| ATOM | 1795 | CE1 | PHE | 430 | 21.049 | 36.415 | -6.274 | 1.00 | 27.80 |
| ATOM | 1796 | CE2 | PHE | 430 | 20.807 | 34.182 | -7.166 | 1.00 | 27.06 |
| ATOM | 1797 | CZ | PRE | 430 | 21.564 | 35.346 | -7.004 | 1.00 | 27.26 |
| ATOM | 1798 | C | PHE | 430 | 16.549 | 35.120 | -7.553 | 1.00 | 28.91 |
| ATOM | 1799 | O | PHE | 430 | 17.104 | 35.641 | -8.524 | 1.00 | 28.87 |
| ATOM | 1800 | N | LYS | 431 | 15.886 | 33.964 | -7.622 | 1.00 | 29.85 |
| ATOM | 1801 | CA | LYS | 431 | 15.766 | 33.212 | -8.874 | 1.00 | 32.68 |
| ATOM | 1802 | CB | LYS | 431 | 14.976 | 31.906 | -8.670 | 1.00 | 33.56 |
| ATOM | 1803 | CG | LYS | 431 | 14.832 | 31.090 | -9.970 | 1.00 | 37.44 |
| ATOM | 1804 | CD | LYS | 431 | 13.617 | 30.145 | -9.977 | 1.00 | 39.34 |
| ATOM | 1805 | CE | LYS | 431 | 13.997 | 28.714 | -9.618 | 1.00 | 41.84 |
| ATOM | 1806 | NZ | LYS | 431 | 14.937 | 28.127 | -10.621 | 1.00 | 42.78 |
| ATOM | 1807 | C | LYS | 431 | 15.077 | 34.053 | -9.944 | 1.00 | 33.57 |
| ATOM | 1808 | O | LYS | 431 | 15.415 | 33.958 | -11.125 | 1.00 | 33.07 |
| ATOM | 1809 | N | GLN | 432 | 14.103 | 34.865 | -9.536 | 1.00 | 34.34 |
| ATOM | 1810 | CA | GLN | 432 | 13.392 | 35.728 | -10.484 | 1.00 | 35.91 |
| ATOM | 1811 | CB | GLN | 432 | 12.353 | 36.589 | -9.768 | 1.00 | 38.08 |
| ATOM | 1812 | CG | GLN | 432 | 11.091 | 35.901 | -9.303 | 1.00 | 39.88 |
| ATOM | 1813 | CD | GLN | 432 | 10.123 | 36.910 | -8.684 | 1.00 | 41.50 |
| ATOM | 1814 | OE1 | GLN | 432 | 10.388 | 37.471 | -7.617 | 1.00 | 40.92 |
| ATOM | 1815 | NE2 | GLN | 432 | 9.011 | 37.170 | -9.372 | 1.00 | 42.35 |
| ATOM | 1816 | C | GLN | 432 | 14.362 | 36.676 | -11.182 | 1.00 | 35.12 |
| ATOM | 1817 | O | GLN | 432 | 14.358 | 36.804 | -12.408 | 1.00 | 35.26 |
| ATOM | 1818 | N | SER | 433 | 15.180 | 37.347 | -10.377 | 1.00 | 34.32 |
| ATOM | 1819 | CA | SER | 433 | 16.156 | 38.313 | -10.868 | 1.00 | 34.30 |
| ATOM | 1820 | CB | SER | 433 | 16.608 | 39.220 | -9.718 | 1.00 | 33.92 |
| ATOM | 1821 | OG | SER | 433 | 15.605 | 40.165 | -9.392 | 1.00 | 35.35 |
| ATOM | 1822 | C | SER | 433 | 17.386 | 37.707 | -11.536 | 1.00 | 33.58 |
| ATOM | 1823 | O | SER | 433 | 17.948 | 38.298 | -12.458 | 1.00 | 33.32 |
| ATOM | 1824 | N | HIS | 434 | 17.809 | 36.537 | -11.071 | 1.00 | 33.33 |
| ATOM | 1825 | CA | HIS | 434 | 18.990 | 35.890 | -11.635 | 1.00 | 34.01 |
| ATOM | 1826 | CB | HIS | 434 | 20.184 | 36.077 | -10.697 | 1.00 | 34.09 |
| ATOM | 1827 | CG | HIS | 434 | 20.418 | 37.498 | -10.299 | 1.00 | 34.93 |
| ATOM | 1828 | CD2 | HIS | 434 | 21.195 | 38.462 | -10.849 | 1.00 | 35.03 |
| ATOM | 1829 | ND1 | HIS | 434 | 19.776 | 38.087 | -9.232 | 1.00 | 35.41 |
| ATOM | 1830 | CE1 | HIS | 434 | 20.147 | 39.351 | -9.140 | 1.00 | 35.21 |
| ATOM | 1831 | NE2 | HIS | 434 | 21.007 | 39.604 | -10.110 | 1.00 | 36.62 |
| ATOM | 1832 | C | HIS | 434 | 18.771 | 34.405 | -11.883 | 1.00 | 34.38 |
| ATOM | 1833 | O | HIS | 434 | 19.341 | 33.561 | -11.193 | 1.00 | 33.93 |
| ATOM | 1834 | N | PRO | 435 | 17.938 | 34.064 | -12.876 | 1.00 | 34.98 |
| ATOM | 1835 | CD | PRO | 435 | 17.182 | 34.919 | -13.805 | 1.00 | 35.01 |
| ATOM | 1836 | CA | PRO | 435 | 17.689 | 32.650 | -13.158 | 1.00 | 35.44 |
| ATOM | 1837 | CB | PRO | 435 | 16.607 | 32.695 | -14.237 | 1.00 | 35.29 |
| ATOM | 1838 | CG | PRO | 435 | 16.916 | 33.963 | -14.962 | 1.00 | 35.40 |
| ATOM | 1839 | C | PRO | 435 | 18.915 | 31.882 | -13.613 | 1.00 | 36.20 |
| ATOM | 1840 | O | PRO | 435 | 19.122 | 30.758 | -13.172 | 1.00 | 35.55 |
| ATOM | 1841 | N | ASP | 436 | 19.733 | 32.480 | -14.481 | 1.00 | 37.54 |
| ATOM | 1842 | CA | ASP | 436 | 20.91 | 31.785 | -14.980 | 1.00 | 40.08 |
| ATOM | 1843 | CB | ASP | 436 | 21.634 | 32.607 | -16.058 | 1.00 | 42.72 |
| ATOM | 1844 | CG | ASP | 436 | 22.700 | 31.794 | -16.803 | 1.00 | 45.95 |
| ATOM | 1845 | OD1 | ASP | 436 | 22.313 | 30.821 | -17.497 | 1.00 | 46.29 |
| ATOM | 1846 | OD2 | ASP | 436 | 23.914 | 32.119 | -16.695 | 1.00 | 47.67 |
| ATOM | 1847 | C | ASP | 436 | 21.918 | 31.423 | -13.886 | 1.00 | 39.77 |
| ATOM | 1848 | O | ASP | 436 | 22.572 | 30.385 | -13.966 | 1.00 | 39.96 |
| ATOM | 1849 | N | ILE | 437 | 22.042 | 32.270 | -12.867 | 1.00 | 39.31 |
| ATOM | 1850 | CA | ILE | 437 | 22.972 | 31.999 | -11.775 | 1.00 | 38.61 |
| ATOM | 1851 | CB | ILE | 437 | 23.096 | 33.227 | -10.806 | 1.00 | 39.13 |
| ATOM | 1852 | CG2 | ILE | 437 | 23.864 | 32.842 | -9.547 | 1.00 | 39.06 |
| ATOM | 1853 | CG1 | ILE | 437 | 23.848 | 34.378 | -11.491 | 1.00 | 39.49 |
| ATOM | 1854 | CD1 | ILE | 437 | 23.124 | 34.982 | -12.681 | 1.00 | 41.59 |
| ATOM | 1855 | C | ILE | 437 | 22.538 | 30.757 | -10.995 | 1.00 | 38.28 |
| ATOM | 1856 | O | ILE | 437 | 23.350 | 29.858 | -10.745 | 1.00 | 37.65 |
| ATOM | 1857 | N | VAL | 438 | 21.255 | 30.692 | -10.641 | 1.00 | 37.18 |
| ATOM | 1858 | CA | VAL | 438 | 20.727 | 29.566 | -9.885 | 1.00 | 37.56 |
| ATOM | 1859 | CB | VAL | 438 | 19.239 | 29.785 | -9.458 | 1.00 | 37.27 |
| ATOM | 1860 | CG1 | VAL | 438 | 18.716 | 28.546 | -8.751 | 1.00 | 36.25 |
| ATOM | 1861 | CG2 | VAL | 438 | 19.116 | 30.983 | -8.525 | 1.00 | 36.28 |
| ATOM | 1862 | C | VAL | 438 | 20.800 | 28.261 | -10.672 | 1.00 | 38.30 |
| ATOM | 1863 | O | VAL | 438 | 21.265 | 27.246 | -10.157 | 1.00 | 37.66 |
| ATOM | 1864 | N | ASN | 439 | 20.368 | 28.287 | -11.927 | 1.00 | 39.10 |
| ATOM | 1865 | CA | ASN | 439 | 20.370 | 27.060 | -12.716 | 1.00 | 39.91 |
| ATOM | 1866 | CB | ASN | 439 | 19.390 | 27.189 | -13.895 | 1.00 | 41.62 |
| ATOM | 1867 | CG | ASN | 439 | 17.934 | 27.190 | -13.445 | 1.00 | 43.36 |
| ATOM | 1868 | OD1 | ASN | 439 | 17.596 | 26.609 | -12.410 | 1.00 | 44.58 |
| ATOM | 1869 | ND2 | ASN | 439 | 17.064 | 27.827 | -14.225 | 1.00 | 44.62 |
| ATOM | 1870 | C | ASN | 439 | 21.711 | 26.555 | -13.219 | 1.00 | 39.39 |
| ATOM | 1871 | O | ASN | 439 | 21.835 | 25.377 | -13.561 | 1.00 | 40.93 |
| ATOM | 1872 | N | THR | 440 | 22.728 | 27.406 | -13.260 | 1.00 | 39.19 |
| ATOM | 1873 | CA | THR | 440 | 24.017 | 26.950 | -13.777 | 1.00 | 38.65 |
| ATOM | 1874 | CB | THR | 440 | 24.281 | 27.533 | -15.181 | 1.00 | 38.77 |
| ATOM | 1875 | OG1 | THR | 440 | 24.89 | 28.828 | -15.057 | 1.00 | 40.00 |
| ATOM | 1876 | CG2 | THR | 440 | 22.985 | 27.670 | -15.955 | 1.00 | 38.78 |
| ATOM | 1877 | C | THR | 440 | 25.248 | 27.238 | -12.926 | 1.00 | 37.59 |
| ATOM | 1878 | O | THR | 440 | 26.353 | 26.835 | -13.290 | 1.00 | 38.22 |
| ATOM | 1879 | N | LEU | 441 | 25.079 | 27.940 | -11.811 | 1.00 | 35.62 |
| ATOM | 1880 | CA | LEU | 441 | 26.224 | 28.254 | -10.965 | 1.00 | 33.88 |
| ATOM | 1881 | CB | LEU | 441 | 26.494 | 29.762 | -10.971 | 1.00 | 35.57 |
| ATOM | 1882 | CG | LEU | 441 | 27.001 | 30.326 | -12.304 | 1.00 | 37.86 |
| ATOM | 1883 | CD1 | LEU | 441 | 27.177 | 31.837 | -12.174 | 1.00 | 38.20 |
| ATOM | 1884 | CD2 | LEU | 441 | 28.321 | 29.677 | -12.683 | 1.00 | 38.80 |
| ATOM | 1885 | C | LEU | 441 | 26.071 | 27.763 | -9.533 | 1.00 | 31.60 |
| ATOM | 1886 | O | LEU | 441 | 27.064 | 27.532 | -8.839 | 1.00 | 32.48 |
| ATOM | 1887 | N | PHE | 442 | 24.831 | 27.602 | -9.086 | 1.00 | 28.10 |
| ATOM | 1888 | CA | PHE | 442 | 24.587 | 27.122 | -7.731 | 1.00 | 25.29 |
| ATOM | 1889 | CB | PHE | 442 | 23.135 | 27.405 | -7.318 | 1.00 | 25.99 |
| ATOM | 1890 | CG | PHE | 442 | 22.909 | 28.776 | -6.747 | 1.00 | 27.30 |
| ATOM | 1891 | CD1 | PHE | 442 | 23.833 | 29.796 | -6.937 | 1.00 | 26.54 |
| ATOM | 1892 | CD2 | PRE | 442 | 21.748 | 29.047 | -6.026 | 1.00 | 27.97 |
| ATOM | 1893 | CE1 | PRE | 442 | 23.604 | 31.064 | -6.416 | 1.00 | 29.09 |
| ATOM | 1894 | CE2 | PHE | 442 | 21.508 | 30.321 | -5.499 | 1.00 | 28.50 |
| ATOM | 1895 | CZ | PRE | 442 | 22.438 | 31.326 | -5.695 | 1.00 | 27.97 |
| ATOM | 1896 | C | PHE | 442 | 24.830 | 25.615 | -7.676 | 1.00 | 23.49 |
| ATOM | 1897 | O | PHE | 442 | 24.616 | 24.907 | -8.663 | 1.00 | 21.57 |
| ATOM | 1898 | N | PRO | 443 | 25.300 | 25.107 | -6.525 | 1.00 | 21.70 |
| ATOM | 1899 | CD | PRO | 443 | 25.758 | 25.819 | -5.316 | 1.00 | 21.76 |
| ATOM | 1900 | CA | PRO | 443 | 25.539 | 23.665 | -6.414 | 1.00 | 21.30 |
| ATOM | 1901 | CB | PRO | 443 | 25.886 | 23.488 | -4.940 | 1.00 | 20.81 |
| ATOM | 1902 | CG | PRO | 443 | 26.615 | 24.773 | -4.619 | 1.00 | 20.63 |
| ATOM | 1903 | C | PRO | 443 | 24.263 | 22.911 | -6.795 | 1.00 | 22.58 |
| ATOM | 1904 | O | PRO | 443 | 23.179 | 23.243 | -6.326 | 1.00 | 21.97 |
| ATOM | 1905 | N | PRO | 444 | 24.377 | 21.893 | -7.662 | 1.00 | 24.17 |
| ATOM | 1906 | CD | PRO | 444 | 25.574 | 21.463 | -8.404 | 1.00 | 24.11 |
| ATOM | 1907 | CA | PRO | 444 | 23.205 | 21.117 | -8.080 | 1.00 | 24.81 |
| ATOM | 1908 | CB | PRO | 444 | 23.825 | 19.970 | -8.867 | 1.00 | 25.10 |
| ATOM | 1909 | CG | PRO | 444 | 24.976 | 20.652 | -9.543 | 1.00 | 25.51 |
| ATOM | 1910 | C | PRO | 444 | 22.313 | 20.637 | -6.933 | 1.00 | 24.27 |
| ATOM | 1911 | O | PRO | 444 | 21.093 | 20.599 | -7.081 | 1.00 | 24.40 |
| ATOM | 1912 | N | LEU | 445 | 22.905 | 20.272 | -5.797 | 1.00 | 23.78 |
| ATOM | 1913 | CA | LEU | 445 | 22.106 | 19.807 | -4.653 | 1.00 | 23.55 |
| ATOM | 1914 | CB | LEU | 445 | 23.001 | 19.218 | -3.563 | 1.00 | 22.47 |
| ATOM | 1915 | CG | LEU | 445 | 22.273 | 18.815 | -2.274 | 1.00 | 21.23 |
| ATOM | 1916 | CD1 | LEU | 445 | 21.251 | 17.742 | -2.592 | 1.00 | 22.32 |
| ATOM | 1917 | CD2 | LEU | 445 | 23.273 | 18.307 | -1.242 | 1.00 | 20.86 |
| ATOM | 1918 | C | LEU | 445 | 21.278 | 20.943 | -4.055 | 1.00 | 22.92 |
| ATOM | 1919 | O | LEU | 445 | 20.124 | 20.757 | -3.647 | 1.00 | 21.21 |
| ATOM | 1920 | N | TYR | 446 | 21.880 | 22.121 | -3.998 | 1.00 | 23.10 |
| ATOM | 1921 | CA | TYR | 446 | 21.197 | 23.284 | -3.465 | 1.00 | 23.29 |
| ATOM | 1922 | CB | TYR | 446 | 22.171 | 24.458 | -3.418 | 1.00 | 23.30 |
| ATOM | 1923 | CG | TYR | 446 | 21.611 | 25.737 | -2.846 | 1.00 | 23.78 |
| ATOM | 1924 | CD1 | TYR | 446 | 20.891 | 26.617 | -3.647 | 1.00 | 25.71 |
| ATOM | 1925 | CE1 | TYR | 446 | 20.430 | 27.835 | -3.151 | 1.00 | 25.93 |
| ATOM | 1926 | CD2 | TYR | 446 | 21.850 | 26.094 | -1.518 | 1.00 | 23.35 |
| ATOM | 1927 | CE2 | TYR | 446 | 21.394 | 27.311 | -1.002 | 1.00 | 25.61 |
| ATOM | 1928 | CZ | TYR | 446 | 20.688 | 28.179 | -1.829 | 1.00 | 26.53 |
| ATOM | 1929 | OH | TYR | 446 | 20.281 | 29.404 | -1.359 | 1.00 | 26.63 |
| ATOM | 1930 | C | TYR | 446 | 19.986 | 23.592 | -4.346 | 1.00 | 25.01 |
| ATOM | 1931 | O | TYR | 446 | 18.903 | 23.896 | -3.837 | 1.00 | 26.28 |
| ATOM | 1932 | N | LYS | 447 | 20.163 | 23.502 | -5.664 | 1.00 | 24.54 |
| ATOM | 1933 | CA | LYS | 447 | 19.063 | 23.753 | -6.591 | 1.00 | 26.44 |
| ATOM | 1934 | CB | LYS | 447 | 19.566 | 23.733 | -8.042 | 1.00 | 28.07 |
| ATOM | 1935 | CG | LYS | 447 | 18.458 | 23.840 | -9.086 | 1.00 | 33.16 |
| ATOM | 1936 | CD | LYS | 447 | 19.029 | 23.787 | -10.514 | 1.00 | 36.54 |
| ATOM | 1937 | CE | LYS | 447 | 17.920 | 23.595 | -11.542 | 1.00 | 39.14 |
| ATOM | 1938 | NZ | LYS | 447 | 18.494 | 23.447 | -12.910 | 1.00 | 40.17 |
| ATOM | 1939 | C | LYS | 447 | 17.985 | 22.680 | -6.414 | 1.00 | 25.88 |
| ATOM | 1940 | O | LYS | 447 | 16.796 | 22.980 | -6.367 | 1.00 | 26.47 |
| ATOM | 1941 | N | GLU | 448 | 18.417 | 21.430 | -6.306 | 1.00 | 25.85 |
| ATOM | 1942 | CA | GLU | 448 | 17.498 | 20.305 | -6.147 | 1.00 | 26.71 |
| ATOM | 1943 | CB | GLU | 448 | 18.290 | 18.993 | -6.099 | 1.00 | 26.95 |
| ATOM | 1944 | CG | GLU | 448 | 17.448 | 17.775 | -5.804 | 1.00 | 29.45 |
| ATOM | 1945 | CD | GLU | 448 | 18.239 | 16.475 | -5.861 | 1.00 | 29.93 |
| ATOM | 1946 | OE1 | GLU | 448 | 18.785 | 16.140 | -6.935 | 1.00 | 30.55 |
| ATOM | 1947 | OE2 | GLU | 448 | 18.301 | 15.773 | -4.832 | 1.00 | 30.84 |
| ATOM | 1948 | C | GLU | 448 | 16.637 | 20.432 | -4.894 | 1.00 | 26.51 |
| ATOM | 1949 | O | GLU | 448 | 15.430 | 20.187 | -4.923 | 1.00 | 26.27 |
| ATOM | 1950 | N | LEU | 449 | 17.268 | 20.832 | -3.798 | 1.00 | 25.93 |
| ATOM | 1951 | CA | LEU | 449 | 16.589 | 20.981 | -2.523 | 1.00 | 26.71 |
| ATOM | 1952 | CB | LEU | 449 | 17.622 | 21.017 | -1.392 | 1.00 | 26.21 |
| ATOM | 1953 | CG | LEU | 449 | 18.502 | 19.788 | -1.172 | 1.00 | 26.61 |
| ATOM | 1954 | CD1 | LEU | 449 | 19.610 | 20.112 | -0.154 | 1.00 | 25.71 |
| ATOM | 1955 | CD2 | LEU | 449 | 17.633 | 18.623 | -0.697 | 1.00 | 26.71 |
| ATOM | 1956 | C | LEU | 449 | 15.674 | 22.189 | -2.364 | 1.00 | 28.16 |
| ATOM | 1957 | O | LEU | 449 | 14.593 | 22.064 | -1.782 | 1.00 | 28.09 |
| ATOM | 1958 | N | PHE | 450 | 16.098 | 23.348 | -2.876 | 1.00 | 29.00 |
| ATOM | 1959 | CA | PRE | 450 | 15.338 | 24.577 | -2.692 | 1.00 | 31.20 |
| ATOM | 1960 | CB | PRE | 450 | 16.224 | 25.609 | -1.993 | 1.00 | 29.89 |
| ATOM | 1961 | CG | PHE | 450 | 16.886 | 25.084 | -0.757 | 1.00 | 27.85 |
| ATOM | 1962 | CD1 | PHE | 450 | 18.263 | 24.862 | -0.729 | 1.00 | 27.95 |
| ATOM | 1963 | CD2 | PHE | 450 | 16.127 | 24.748 | 0.362 | 1.00 | 28.29 |
| ATOM | 1964 | CE1 | PHE | 450 | 18.877 | 24.305 | 0.402 | 1.00 | 27.72 |
| ATOM | 1965 | CE2 | PHE | 450 | 16.723 | 24.190 | 1.498 | 1.00 | 27.38 |
| ATOM | 1966 | CZ | PHE | 450 | 18.105 | 23.966 | 1.518 | 1.00 | 28.09 |
| ATOM | 1967 | C | PHE | 450 | 14.625 | 25.257 | -3.854 | 1.00 | 33.41 |
| ATOM | 1968 | O | PHE | 450 | 13.844 | 26.169 | -3.615 | 1.00 | 32.89 |
| ATOM | 1969 | N | ASN | 451 | 14.889 | 24.862 | -5.096 | 1.00 | 36.22 |
| ATOM | 1970 | CA | ASN | 451 | 14.185 | 25.493 | -6.219 | 1.00 | 39.47 |
| ATOM | 1971 | CB | ASN | 451 | 15.168 | 26.004 | -7.281 | 1.00 | 41.40 |
| ATOM | 1972 | CG | ASN | 451 | 15.939 | 27.245 | -6.818 | 1.00 | 43.69 |
| ATOM | 1973 | OD1 | ASN | 451 | 15.371 | 28.342 | -6.672 | 1.00 | 44.14 |
| ATOM | 1974 | ND2 | ASN | 451 | 17.238 | 27.071 | -6.567 | 1.00 | 44.76 |
| ATOM | 1975 | C | ASN | 451 | 13.217 | 24.509 | -6.851 | 1.00 | 40.32 |
| ATOM | 1976 | O | ASN | 451 | 11.993 | 24.725 | -6.697 | 1.00 | 41.43 |
| ATOM | 1977 | OXT | ASN | 451 | 13.696 | 23.537 | -7.475 | 1.00 | 40.86 |
| ATOM | 1978 | CB | HIS | 691 | 15.393 | 13.885 | -4.499 | 1.00 | 41.85 |
| ATOM | 1979 | CG | HIS | 691 | 13.918 | 14.001 | -4.283 | 1.00 | 43.67 |
| ATOM | 1980 | CD2 | HIS | 691 | 13.151 | 15.051 | -3.912 | 1.00 | 44.46 |
| ATOM | 1981 | ND1 | HIS | 691 | 13.051 | 12.954 | -4.521 | 1.00 | 44.92 |
| ATOM | 1982 | CE1 | HIS | 691 | 11.810 | 13.358 | -4.305 | 1.00 | 45.35 |
| ATOM | 1983 | NE2 | HIS | 691 | 11.843 | 14.625 | -3.935 | 1.00 | 45.49 |
| ATOM | 1984 | C | HIS | 691 | 16.996 | 13.164 | -6.244 | 1.00 | 38.01 |
| ATOM | 1985 | O | HIS | 691 | 17.471 | 12.469 | -5.361 | 1.00 | 38.13 |
| ATOM | 1986 | N | HIS | 691 | 14.685 | 13.691 | -6.864 | 1.00 | 39.52 |
| ATOM | 1987 | CA | HIS | 691 | 15.813 | 14.063 | -5.956 | 1.00 | 39.14 |
| ATOM | 1988 | N | LYS | 692 | 17.475 | 13.190 | -7.484 | 1.00 | 36.76 |
| ATOM | 1989 | CA | LYS | 692 | 18.589 | 12.350 | -7.903 | 1.00 | 35.59 |
| ATOM | 1990 | CB | LYS | 692 | 18.893 | 12.623 | -9.378 | 1.00 | 37.83 |
| ATOM | 1991 | CG | LYS | 692 | 19.742 | 11.564 | -10.051 | 1.00 | 40.63 |
| ATOM | 1992 | CD | LYS | 692 | 19.728 | 11.714 | -11.575 | 1.00 | 42.76 |
| ATOM | 1993 | CE | LYS | 692 | 20.323 | 10.472 | -12.235 | 1.00 | 43.47 |
| ATOM | 1994 | NZ | LYS | 692 | 21.706 | 10.196 | -11.753 | 1.00 | 43.90 |
| ATOM | 1995 | C | LYS | 692 | 19.873 | 12.498 | -7.080 | 1.00 | 33.87 |
| ATOM | 1996 | O | LYS | 692 | 20.467 | 11.509 | -6.658 | 1.00 | 33.35 |
| ATOM | 1997 | N | ILE | 693 | 20.309 | 13.729 | -6.857 | 1.00 | 32.23 |
| ATOM | 1998 | CA | ILE | 693 | 21.535 | 13.950 | -6.113 | 1.00 | 31.32 |
| ATOM | 1999 | CB | ILE | 693 | 21.930 | 15.427 | -6.149 | 1.00 | 30.80 |
| ATOM | 2000 | CG2 | ILE | 693 | 23.269 | 15.635 | -5.440 | 1.00 | 29.72 |
| ATOM | 2001 | CG1 | ILE | 693 | 22.033 | 15.873 | -7.608 | 1.00 | 31.13 |
| ATOM | 2002 | CD1 | ILE | 693 | 22.230 | 17.371 | -7.780 | 1.00 | 31.56 |
| ATOM | 2003 | C | ILE | 693 | 21.432 | 13.482 | -4.678 | 1.00 | 30.60 |
| ATOM | 2004 | O | ILE | 693 | 22.298 | 12.750 | -4.201 | 1.00 | 30.47 |
| ATOM | 2005 | N | LEU | 694 | 20.373 | 13.894 | -3.988 | 1.00 | 30.60 |
| ATOM | 2006 | CA | LEU | 694 | 20.189 | 13.498 | -2.599 | 1.00 | 29.95 |
| ATOM | 2007 | CB | LEU | 694 | 18.901 | 14.127 | -2.049 | 1.00 | 30.02 |
| ATOM | 2008 | CG | LEU | 694 | 18.575 | 13.956 | -0.565 | 1.00 | 30.42 |
| ATOM | 2009 | CD1 | LEU | 694 | 19.761 | 14.380 | 0.289 | 1.00 | 27.83 |
| ATOM | 2010 | CD2 | LEU | 694 | 17.338 | 14.782 | -0.228 | 1.00 | 29.11 |
| ATOM | 2011 | C | LEU | 694 | 20.133 | 11.973 | -2.530 | 1.00 | 31.03 |
| ATOM | 2012 | O | LEU | 694 | 20.692 | 11.347 | -1.624 | 1.00 | 28.47 |
| ATOM | 2013 | N | HIS | 695 | 19.476 | 11.365 | -3.512 | 1.00 | 31.88 |
| ATOM | 2014 | CA | HIS | 695 | 19.374 | 9.913 | -3.529 | 1.00 | 33.30 |
| ATOM | 2015 | CB | HIS | 695 | 18.477 | 9.459 | -4.670 | 1.00 | 36.30 |
| ATOM | 2016 | CG | HIS | 695 | 17.945 | 8.080 | -4.478 | 1.00 | 40.00 |
| ATOM | 2017 | CD2 | HIS | 695 | 16.683 | 7.629 | -4.299 | 1.00 | 41.54 |
| ATOM | 2018 | ND1 | HIS | 695 | 18.768 | 6.980 | -4.364 | 1.00 | 41.93 |
| ATOM | 2019 | CE1 | HIS | 695 | 18.035 | 5.910 | -4.117 | 1.00 | 42.57 |
| ATOM | 2020 | NE2 | HIS | 695 | 16.766 | 6.277 | -4.071 | 1.00 | 43.22 |
| ATOM | 2021 | C | HIS | 695 | 20.746 | 9.260 | -3.676 | 1.00 | 33.11 |
| ATOM | 2022 | O | HIS | 695 | 21.048 | 8.249 | -3.040 | 1.00 | 32.39 |
| ATOM | 2023 | N | ARG | 696 | 21.579 | 9.849 | -4.521 | 1.00 | 32.29 |
| ATOM | 2024 | CA | ARG | 696 | 22.909 | 9.322 | -4.743 | 1.00 | 30.93 |
| ATOM | 2025 | CB | ARG | 696 | 23.603 | 10.093 | -5.867 | 1.00 | 31.23 |
| ATOM | 2026 | CG | ARG | 696 | 24.984 | 9.543 | -6.182 | 1.00 | 32.75 |
| ATOM | 2027 | CD | ARG | 696 | 25.804 | 10.477 | -7.050 | 1.00 | 33.56 |
| ATOM | 2028 | NE | ARG | 696 | 27.125 | 9.909 | -7.307 | 1.00 | 35.72 |
| ATOM | 2029 | CZ | ARG | 696 | 28.099 | 10.546 | -7.950 | 1.00 | 35.45 |
| ATOM | 2030 | NH1 | ARG | 696 | 27.901 | 11.782 | -8.398 | 1.00 | 35.32 |
| ATOM | 2031 | NH2 | ARG | 696 | 29.269 | 9.943 | -8.151 | 1.00 | 36.04 |
| ATOM | 2032 | C | ARG | 696 | 23.772 | 9.386 | -3.480 | 1.00 | 29.89 |
| ATOM | 2033 | O | ARG | 696 | 24.475 | 8.433 | -3.161 | 1.00 | 29.24 |
| ATOM | 2034 | N | LEU | 697 | 23.716 | 10.509 | -2.768 | 1.00 | 29.17 |
| ATOM | 2035 | CA | LEU | 697 | 24.511 | 10.690 | -1.557 | 1.00 | 28.52 |
| ATOM | 2036 | CB | LEU | 697 | 24.368 | 12.124 | -1.046 | 1.00 | 26.92 |
| ATOM | 2037 | CG | LEU | 697 | 24.699 | 13.228 | -2.053 | 1.00 | 27.27 |
| ATOM | 2038 | CD1 | LEU | 697 | 24.463 | 14.602 | -1.417 | 1.00 | 25.02 |
| ATOM | 2039 | CD2 | LEU | 697 | 26.142 | 13.085 | -2.505 | 1.00 | 26.53 |
| ATOM | 2040 | C | LEU | 697 | 24.140 | 9.712 | -0.448 | 1.00 | 29.70 |
| ATOM | 2041 | O | LEU | 697 | 25.004 | 9.258 | 0.302 | 1.00 | 28.66 |
| ATOM | 2042 | N | LEU | 698 | 22.853 | 9.397 | -0.345 | 1.00 | 31.41 |
| ATOM | 2043 | CA | LEU | 698 | 22.375 | 8.453 | 0.661 | 1.00 | 34.12 |
| ATOM | 2044 | CB | LEU | 698 | 20.846 | 8.472 | 0.718 | 1.00 | 33.23 |
| ATOM | 2045 | CG | LEU | 698 | 20.134 | 9.426 | 1.669 | 1.00 | 32.91 |
| ATOM | 2046 | CD1 | LEU | 698 | 18.680 | 9.463 | 1.307 | 1.00 | 33.90 |
| ATOM | 2047 | CD2 | LEU | 698 | 20.302 | 8.964 | 3.107 | 1.00 | 31.41 |
| ATOM | 2048 | C | LEU | 698 | 22.848 | 7.044 | 0.315 | 1.00 | 36.29 |
| ATOM | 2049 | O | LEU | 698 | 23.159 | 6.242 | 1.194 | 1.00 | 36.31 |
| ATOM | 2050 | N | GLN | 699 | 22.909 | 6.760 | -0.977 | 1.00 | 39.15 |
| ATOM | 2051 | CA | GLN | 699 | 23.328 | 5.450 | -1.471 | 1.00 | 42.12 |
| ATOM | 2052 | CB | GLN | 699 | 22.802 | 5.239 | -2.896 | 1.00 | 43.64 |
| ATOM | 2053 | CG | GLN | 699 | 21.274 | 5.140 | -2.989 | 1.00 | 45.98 |
| ATOM | 2054 | CD | GLN | 699 | 20.750 | 3.781 | -2.547 | 1.00 | 47.08 |
| ATOM | 2055 | OE1 | GLN | 699 | 21.233 | 3.204 | -1.566 | 1.00 | 48.60 |
| ATOM | 2056 | NE2 | GLN | 699 | 19.747 | 3.269 | -3.258 | 1.00 | 48.36 |
| ATOM | 2057 | C | GLN | 699 | 24.836 | 5.245 | -1.465 | 1.00 | 43.37 |
| ATOM | 2058 | O | GLN | 699 | 25.305 | 4.126 | -1.660 | 1.00 | 43.65 |
| ATOM | 2059 | N | GLU | 700 | 25.599 | 6.312 | -1.260 | 1.00 | 44.64 |
| ATOM | 2060 | CA | GLU | 700 | 27.052 | 6.190 | -1.250 | 1.00 | 47.04 |
| ATOM | 2061 | CB | GLU | 700 | 27.700 | 7.568 | -1.095 | 1.00 | 47.40 |
| ATOM | 2062 | CG | GLU | 700 | 29.220 | 7.519 | -1.157 | 1.00 | 49.39 |
| ATOM | 2063 | CD | GLU | 700 | 29.791 | 8.048 | -2.461 | 1.00 | 49.85 |
| ATOM | 2064 | OE1 | GLU | 700 | 29.288 | 7.680 | -3.554 | 1.00 | 49.19 |
| ATOM | 2065 | OE2 | GLU | 700 | 30.763 | 8.836 | -2.382 | 1.00 | 51.58 |
| ATOM | 2066 | C | GLU | 700 | 27.540 | 5.255 | -0.132 | 1.00 | 48.52 |
| ATOM | 2067 | O | GLU | 700 | 28.449 | 4.423 | -0.386 | 1.00 | 48.99 |
| ATOM | 2068 | OXT | GLU | 700 | 27.021 | 5.379 | 1.001 | 1.00 | 49.57 |
| ATOM | 2069 | OH2 | WAT | 801 | 14.817 | 26.937 | 2.587 | 1.00 | 20.16 |
| ATOM | 2070 | OH2 | WAT | 802 | 26.733 | 20.689 | 8.917 | 1.00 | 19.93 |
| ATOM | 2071 | OH2 | WAT | 803 | 26.566 | 41.076 | 13.914 | 1.00 | 25.96 |
| ATOM | 2072 | OH2 | WAT | 804 | 12.901 | 16.692 | 8.722 | 1.00 | 19.78 |
| ATOM | 2073 | OH2 | WAT | 805 | 21.441 | 41.033 | 3.838 | 1.00 | 27.88 |
| ATOM | 2074 | OH2 | WAT | 806 | 11.576 | 19.809 | 13.280 | 1.00 | 21.67 |
| ATOM | 2075 | OH2 | WAT | 807 | 24.269 | 17.527 | 14.566 | 1.00 | 22.47 |
| ATOM | 2076 | OH2 | WAT | 808 | 25.329 | 19.487 | -5.550 | 1.00 | 38.43 |
| ATOM | 2077 | OH2 | WAT | 809 | 28.090 | 41.701 | 1.977 | 1.00 | 27.58 |
| ATOM | 2078 | OH2 | WAT | 810 | 1.405 | 9.268 | 4.014 | 1.00 | 28.13 |
| ATOM | 2079 | OH2 | WAT | 811 | 7.275 | 28.248 | 11.748 | 1.00 | 33.78 |
| ATOM | 2080 | OH2 | WAT | 812 | 24.068 | 26.427 | 23.332 | 1.00 | 29.89 |
| ATOM | 2081 | OH2 | WAT | 813 | 14.772 | 34.264 | 14.684 | 1.00 | 25.08 |
| ATOM | 2082 | OH2 | WAT | 814 | 12.543 | 3.741 | 17.005 | 1.00 | 29.72 |
| ATOM | 2083 | OH2 | WAT | 815 | 30.580 | 18.289 | 2.261 | 1.00 | 31.66 |
| ATOM | 2084 | OH2 | WAT | 816 | 36.688 | 39.061 | 2.238 | 1.00 | 31.61 |
| ATOM | 2085 | OH2 | WAT | 817 | 11.630 | 23.199 | 6.840 | 1.00 | 31.07 |
| ATOM | 2086 | OH2 | WAT | 818 | 2.742 | 5.405 | 10.288 | 1.00 | 30.11 |
| ATOM | 2087 | OH2 | WAT | 819 | 1.368 | 13.915 | 5.083 | 1.00 | 35.24 |
| ATOM | 2088 | OH2 | WAT | 820 | 3.546 | 13.308 | 8.739 | 1.00 | 32.87 |
| ATOM | 2089 | OH2 | WAT | 821 | 0.197 | 11.314 | 7.692 | 1.00 | 31.34 |
| ATOM | 2090 | OH2 | WAT | 822 | -0.265 | 12.324 | 3.836 | 1.00 | 31.56 |
| ATOM | 2091 | OH2 | WAT | 823 | 32.317 | 41.732 | 11.781 | 1.00 | 30.31 |
| ATOM | 2092 | OH2 | WAT | 824 | 31.188 | 22.170 | 11.656 | 1.00 | 30.40 |
| ATOM | 2093 | OH2 | WAT | 825 | 28.708 | 24.054 | 13.003 | 1.00 | 32.74 |
| ATOM | 2094 | OH2 | WAT | 826 | 30.451 | 24.872 | 11.210 | 1.00 | 53.39 |
| ATOM | 2095 | OH2 | WAT | 832 | 14.659 | 19.191 | 30.954 | 1.00 | 33.74 |
| ATOM | 2096 | OH2 | WAT | 835 | 2.458 | 12.055 | 6.762 | 1.00 | 30.04 |
| ATOM | 2097 | OH2 | WAT | 836 | 8.996 | 22.689 | 12.009 | 1.00 | 49.90 |
| ATOM | 2098 | OH2 | WAT | 837 | 14.375 | 24.499 | 4.186 | 1.00 | 30.61 |
| ATOM | 2099 | OH2 | WAT | 839 | 2.130 | 4.386 | 7.857 | 1.00 | 30.22 |
| ATOM | 2100 | OH2 | WAT | 840 | 26.339 | 16.316 | 27.739 | 1.00 | 35.01 |
| ATOM | 2101 | OH2 | WAT | 841 | 8.001 | 21.050 | 7.844 | 1.00 | 25.09 |
| ATOM | 2102 | OH2 | WAT | 842 | 11.218 | 3.956 | 14.667 | 1.00 | 29.78 |
| ATOM | 2103 | OH2 | WAT | 843 | 13.538 | 3.582 | 13.108 | 1.00 | 29.55 |
| ATOM | 2104 | OH2 | WAT | 844 | 12.401 | 22.827 | 13.222 | 1.00 | 30.20 |
| ATOM | 2105 | OH2 | WAT | 846 | 34.979 | 40.443 | 11.829 | 1.00 | 38.06 |
| ATOM | 2106 | OH2 | WAT | 847 | 9.388 | 6.952 | 8.514 | 1.00 | 44.86 |
| ATOM | 2107 | OH2 | WAT | 848 | 9.424 | 32.516 | 7.968 | 1.00 | 34.80 |
| ATOM | 2108 | OH2 | WAT | 849 | 12.152 | 25.422 | 5.217 | 1.00 | 33.49 |
| ATOM | 2109 | OH2 | WAT | 850 | 38.479 | 45.718 | 1.108 | 1.00 | 32.54 |
| ATOM | 2110 | OH2 | WAT | 851 | 32.489 | 27.280 | 7.768 | 1.00 | 52.84 |
| ATOM | 2111 | OH2 | WAT | 852 | 19.651 | 17.055 | -9.301 | 1.00 | 37.88 |
| ATOM | 2112 | OH2 | WAT | 853 | 18.746 | 43.921 | 3.425 | 1.00 | 36.94 |
| ATOM | 2113 | OH2 | WAT | 854 | 25.431 | 10.057 | 28.150 | 1.00 | 38.06 |
| ATOM | 2114 | OH2 | WAT | 855 | 7.134 | 18.540 | 4.931 | 1.00 | 63.81 |
| ATOM | 2115 | OH2 | WAT | 856 | 9.614 | 18.288 | 18.085 | 1.00 | 35.99 |
| ATOM | 2116 | OH2 | WAT | 857 | 9.960 | 11.674 | 24.894 | 1.00 | 34.82 |
| ATOM | 2117 | OH2 | WAT | 858 | 21.673 | 44.846 | 2.726 | 1.00 | 29.09 |
| ATOM | 2118 | OH2 | WAT | 859 | 12.420 | -0.591 | 5.172 | 1.00 | 56.22 |
| ATOM | 2119 | OH2 | WAT | 860 | 2.112 | 7.553 | 21.676 | 1.00 | 63.50 |
| ATOM | 2120 | OH2 | WAT | 861 | 32.871 | 18.504 | 18.445 | 1.00 | 37.53 |
| ATOM | 2121 | OH2 | WAT | 862 | 7.060 | 24.628 | 6.457 | 1.00 | 41.11 |
| ATOM | 2122 | OH2 | WAT | 863 | 30.339 | 19.808 | 24.562 | 1.00 | 35.73 |
| ATOM | 2123 | OH2 | WAT | 865 | 31.408 | 40.456 | -6.981 | 1.00 | 28.87 |
| ATOM | 2124 | OH2 | WAT | 866 | 16.060 | 42.941 | 9.793 | 1.00 | 44.24 |
| ATOM | 2125 | OH2 | WAT | 867 | 12.843 | 26.219 | 0.918 | 1.00 | 35.68 |
| ATOM | 2126 | OH2 | WAT | 868 | 37.364 | 44.144 | -7.616 | 1.00 | 64.48 |
| ATOM | 2127 | OH2 | WAT | 869 | 8.875 | 20.108 | 12.516 | 1.00 | 35.65 |
| ATOM | 2128 | OH2 | WAT | 870 | 15.439 | 22.492 | 5.981 | 1.00 | 34.94 |
| ATOM | 2129 | OH2 | WAT | 871 | 23.795 | 42.644 | 6.558 | 1.00 | 37.44 |
| ATOM | 2130 | OH2 | WAT | 873 | 24.777 | 38.088 | 21.937 | 1.00 | 37.38 |
| ATOM | 2131 | OH2 | WAT | 874 | 19.791 | 35.055 | -15.004 | 1.00 | 49.00 |
| ATOM | 2132 | OH2 | WAT | 875 | 4.885 | 20.278 | 10.170 | 1.00 | 42.95 |
| ATOM | 2133 | OH2 | WAT | 876 | 22.387 | 24.643 | -10.436 | 1.00 | 51.01 |
| ATOM | 2134 | OH2 | WAT | 877 | 12.606 | 26.960 | -1.579 | 1.00 | 38.28 |
| ATOM | 2135 | OH2 | WAT | 878 | 29.020 | 8.186 | 21.464 | 1.00 | 34.65 |
| ATOM | 2136 | OH2 | WAT | 879 | 19.154 | 5.255 | 15.898 | 1.00 | 45.06 |
| ATOM | 2137 | OH2 | WAT | 880 | 23.704 | 47.324 | 18.452 | 1.00 | 68.34 |
| ATOM | 2138 | OH2 | WAT | 881 | 28.057 | 9.062 | 6.603 | 1.00 | 44.32 |
| ATOM | 2139 | OH2 | WAT | 883 | 43.023 | 41.890 | 5.788 | 1.00 | 38.31 |
| ATOM | 2140 | OH2 | WAT | 884 | 7.119 | 28.891 | 9.099 | 1.00 | 32.09 |
| ATOM | 2141 | OH2 | WAT | 885 | 17.021 | 2.408 | 2.764 | 1.00 | 45.47 |
| ATOM | 2142 | OH2 | WAT | 886 | 23.215 | 4.005 | 10.105 | 1.00 | 32.13 |
| ATOM | 2143 | OH2 | WAT | 887 | 15.983 | 38.039 | -2.715 | 1.00 | 54.73 |
| ATOM | 2144 | OH2 | WAT | 889 | 4.205 | 3.441 | 11.079 | 1.00 | 39.06 |
| ATOM | 2145 | OH2 | WAT | 890 | 20.791 | 9.054 | -7.915 | 1.00 | 33.22 |
| ATOM | 2146 | OH2 | WAT | 891 | 25.305 | 7.602 | 26.964 | 1.00 | 51.43 |
| ATOM | 2147 | OH2 | WAT | 892 | 27.413 | 6.919 | 14.503 | 1.00 | 32.31 |
| ATOM | 2148 | OH2 | WAT | 893 | 33.552 | 15.908 | 12.742 | 1.00 | 47.40 |
| ATOM | 2149 | OH2 | WAT | 894 | 27.172 | 23.883 | 29.442 | 1.00 | 51.19 |
| ATOM | 2150 | OH2 | WAT | 895 | 32.219 | 29.350 | 18.302 | 1.00 | 39.52 |
| ATOM | 2151 | OH2 | WAT | 896 | 20.082 | 1.162 | 10.052 | 1.00 | 37.15 |
| ATOM | 2152 | OH2 | WAT | 898 | 23.983 | 4.384 | 5.989 | 1.00 | 35.18 |
| ATOM | 2153 | OH2 | WAT | 899 | 9.782 | 19.780 | 0.976 | 1.00 | 50.23 |
| ATOM | 2154 | OH2 | WAT | 900 | 36.407 | 19.082 | 20.876 | 1.00 | 44.97 |
| ATOM | 2155 | OH2 | WAT | 901 | 7.403 | 19.567 | 14.815 | 1.00 | 37.37 |
| ATOM | 2156 | OH2 | WAT | 902 | 39.710 | 36.772 | 1.207 | 1.00 | 58.27 |
| ATOM | 2157 | OH2 | WAT | 903 | 10.185 | 18.360 | 15.457 | 1.00 | 33.56 |
| ATOM | 2158 | OH2 | WAT | 904 | 10.260 | 38.244 | 11.664 | 1.00 | 51.98 |
| ATOM | 2159 | OH2 | WAT | 905 | 11.584 | 31.849 | -8.614 | 1.00 | 37.52 |
| ATOM | 2160. | OH2 | WAT | 906 | 31.638 | 29.686 | -0.266 | 1.00 | 32.48 |
| ATOM | 2161 | OH2 | WAT | 907 | -0.206 | 4.924 | 11.081 | 1.00 | 36.49 |
| ATOM | 2162 | OH2 | WAT | 908 | 31.665 | 29.237 | 6.272 | 1.00 | 49.19 |
| ATOM | 2163 | OH2 | WAT | 909 | -2.417 | 16.800 | 9.041 | 1.00 | 44.02 |
| ATOM | 2164 | OH2 | WAT | 910 | 10.940 | 39.261 | 6.571 | 1.00 | 43.92 |
| ATOM | 2165 | OH2 | WAT | 911 | 13.036 | 1.866 | 1.304 | 1.00 | 40.13 |
| ATOM | 2166 | OH2 | WAT | 912 | 14.565 | 2.593 | 15.801 | 1.00 | 47.21 |
| ATOM | 2167 | OH2 | WAT | 913 | 15.968 | 17.289 | 31.561 | 1.00 | 33.25 |
| ATOM | 2168 | OH2 | WAT | 914 | 34.332 | 36.096 | 17.436 | 1.00 | 48.32 |
| ATOM | 2169 | OH2 | WAT | 915 | -0.731 | 12.520 | 9.973 | 1.00 | 49.45 |
| ATOM | 2170 | OH2 | WAT | 916 | 19.666 | 1.578 | 18.349 | 1.00 | 44.84 |
| ATOM | 2171 | OH2 | WAT | 917 | 22.978 | 40.125 | 16.179 | 1.00 | 45.10 |
| ATOM | 2172 | OH2 | WAT | 918 | 14.785 | 33.819 | 17.497 | 1.00 | 38.88 |
| ATOM | 2173 | OH2 | WAT | 919 | 25.871 | 13.595 | -7.644 | 1.00 | 45.77 |
| ATOM | 2174 | OH2 | WAT | 920 | 25.570 | 6.591 | -4.596 | 1.00 | 38.57 |
| ATOM | 2175 | OH2 | WAT | 921 | 16.974 | 2.870 | 16.605 | 1.00 | 33.05 |
| ATOM | 2176 | OH2 | WAT | 922 | 33.181 | 45.831 | 10.253 | 1.00 | 42.64 |
| ATOM | 2177 | OH2 | WAT | 923 | 12.975 | 32.550 | -12.651 | 1.00 | 46.09 |
| ATOM | 2178 | OH2 | WAT | 924 | 26.965 | 45.895 | 0.183 | 1.00 | 55.12 |
| ATOM | 2179 | OH2 | WAT | 925 | 7.626 | 22.836 | 18.961 | 1.00 | 41.09 |
| ATOM | 2180 | OH2 | WAT | 926 | 23.414 | 34.472 | 23.517 | 1.00 | 34.89 |
| ATOM | 2181 | OH2 | WAT | 927 | 21.725 | 53.855 | -1.107 | 1.00 | 58.84 |
| ATOM | 2182 | OH2 | WAT | 928 | 30.305 | 48.946 | 2.286 | 1.00 | 45.28 |
| ATOM | 2183 | OH2 | WAT | 929 | 21.547 | 3.556 | 1.853 | 1.00 | 49.41 |
| ATOM | 2184 | OH2 | WAT | 930 | 18.916 | 35.911 | -17.290 | 1.00 | 54.56 |
| ATOM | 2185 | OH2 | WAT | 931 | 7.922 | 23.555 | 24.917 | 1.00 | 41.77 |
| ATOM | 2186 | OH2 | WAT | 932 | 22.938 | 41.070 | 21.176 | 1.00 | 38.69 |
| ATOM | 2187 | OH2 | WAT | 933 | 34.963 | 42.284 | -3.078 | 1.00 | 35.66 |
| ATOM | 2188 | OH2 | WAT | 934 | 10.445 | 29.254 | 25.404 | 1.00 | 53.07 |
| ATOM | 2189 | OH2 | WAT | 935 | 13.385 | 22.747 | 1.929 | 1.00 | 57.68 |
| ATOM | 2190 | OH2 | WAT | 936 | 17.800 | 1.033 | 15.037 | 1.00 | 43.46 |
| ATOM | 2191 | OH2 | WAT | 938 | 29.919 | 23.540 | 7.796 | 1.00 | 38.24 |
| ATOM | 2192 | OH2 | WAT | 939 | 26.837 | 29.236 | -6.134 | 1.00 | 34.00 |
| ATOM | 2193 | OH2 | WAT | 940 | 4.282 | 1.689 | 8.958 | 1.00 | 44.18 |
| ATOM | 2194 | OH2 | WAT | 941 | 16.572 | 41.825 | -1.750 | 1.00 | 64.20 |
| ATOM | 2195 | OH2 | WAT | 942 | 18.212 | 46.711 | 4.803 | 1.00 | 54.44 |
| ATOM | 2196 | OH2 | WAT | 943 | 15.478 | 42.818 | 4.335 | 1.00 | 50.85 |
| ATOM | 2197 | OH2 | WAT | 944 | 26.414 | 23.899 | 9.989 | 1.00 | 39.22 |
| ATOM | 2198 | OH2 | WAT | 945 | 29.440 | 21.150 | 9.611 | 1.00 | 32.92 |
| ATOM | 2199 | OH2 | WAT | 946 | 30.822 | 26.523 | 9.587 | 1.00 | 53.88 |
| ATOM | 2200 | OH2 | WAT | 950 | 8.855 | 22.869 | 6.573 | 1.00 | 38.42 |
| ATOM | 2201 | OH2 | WAT | 951 | 5.112 | 26.325 | 5.735 | 1.00 | 61.02 |
| ATOM | 2202 | OH2 | WAT | 952 | -0.889 | 10.066 | 5.523 | 1.00 | 36.76 |
| ATOM | 2203 | OH2 | WAT | 954 | 34.805 | 28.111 | 7.254 | 1.00 | 38.30 |
| ATOM | 2204 | OH2 | WAT | 955 | 19.872 | 20.234 | -9.431 | 1.00 | 35.47 |
| ATOM | 2205 | OH2 | WAT | 956 | 22.406 | 22.660 | -11.886 | 1.00 | 39.93 |
| ATOM | 2206 | O2 | STE | 1001 | 28.707 | 27.099 | 7.452 | 1.00 | 49.40 |
| ATOM | 2207 | O1 | STE | 1001 | 27.940 | 25.317 | 8.529 | 1.00 | 50.77 |
| ATOM | 2208 | C4 | STE | 1001 | 26.850 | 27.424 | 8.987 | 1.00 | 49.77 |
| ATOM | 2209 | C1 | STE | 1001 | 25.793 | 30.578 | 5.434 | 1.00 | 50.35 |
| ATOM | 2210 | C2 | STE | 1001 | 24.996 | 29.501 | 6.215 | 1.00 | 50.21 |
| ATOM | 2211 | C6 | STE | 1001 | 25.841 | 28.419 | 6.860 | 1.00 | 50.29 |
| ATOM | 2212 | C9 | STE | 1001 | 25.977 | 28.550 | 8.389 | 1.00 | 50.59 |
| ATOM | 2213 | C5 | STE | 1001 | 23.387 | 34.488 | 6.590 | 1.00 | 48.06 |
| ATOM | 2214 | C10 | STE | 1001 | 24.488 | 33.559 | 6.013 | 1.00 | 49.21 |
| ATOM | 2215 | C13 | STE | 1001 | 25.128 | 32.598 | 7.006 | 1.00 | 49.62 |
| ATOM | 2216 | C16 | STE | 1001 | 26.247 | 31.729 | 6.377 | 1.00 | 50.27 |
| ATOM | 2217 | C7 | STE | 1001 | 20.503 | 31.145 | 6.873 | 1.00 | 45.75 |
| ATOM | 2218 | C11 | STE | 1001 | 20.179 | 32.288 | 5.920 | 1.00 | 45.75 |
| ATOM | 2219 | C14 | STE | 1001 | 21.350 | 33.208 | 5.604 | 1.00 | 46.43 |
| ATOM | 2220 | C17 | STE | 1001 | 22.034 | 33.806 | 6.858 | 1.00 | 48.02 |
| ATOM | 2221 | C8 | STE | 1001 | 20.032 | 29.801 | 6.378 | 1.00 | 44.03 |
| ATOM | 2222 | C12 | STE | 1001 | 20.065 | 28.778 | 7.502 | 1.00 | 43.90 |
| ATOM | 2223 | C15 | STE | 1001 | 21.407 | 28.027 | 7.586 | 1.00 | 42.74 |
| ATOM | 2224 | C18 | STE | 1001 | 22.227 | 28.387 | 8.831 | 1.00 | 43.23 |
| ATOM | 2225 | C3 | STE | 1001 | 27.878 | 26.507 | 8.311 | 1.00 | 50.33 |
| END | | | | | | | | | |

**TABLE B: Crystallographic Coordinates of RORbeta LBD/retinoic acid/SRC1 peptide complex**

| | | | | | | | | | |
|---|---|---|---|---|---|---|---|---|---|
| ATOM | 1 | CB | THR | 1 | 14.936 | -1.893 | 26.570 | 1.00 | 44.08 |
| ATOM | 2 | OG1 | THR | 1 | 16.329 | -2.232 | 26.551 | 1.00 | 44.42 |
| ATOM | 3 | CG2 | THR | 1 | 14.475 | -1.471 | 25.180 | 1.00 | 44.84 |
| ATOM | 4 | C | THR | 1 | 15.825 | 0.265 | 27.508 | 1.00 | 44.25 |
| ATOM | 5 | O | THR | 1 | 16.183 | 0.740 | 26.428 | 1.00 | 42.75 |
| ATOM | 6 | N | THR | 1 | 13.347 | -0.138 | 27.331 | 1.00 | 43.63 |
| ATOM | 7 | CA | THR | 1 | 14.685 | -0.761 | 27.580 | 1.00 | 44.30 |
| ATOM | 8 | N | MET | 2 | 16.388 | 0.599 | 28.670 | 1.00 | 44.87 |
| ATOM | 9 | CA | MET | 2 | 17.474 | 1.576 | 28.770 | 1.00 | 45.02 |
| ATOM | 10 | CB | MET | 2 | 17.914 | 1.727 | 30.240 | 1.00 | 48.12 |
| ATOM | 11 | CG | MET | 2 | 18.864 | 2.913 | 30.535 | 1.00 | 52.67 |
| ATOM | 12 | SD | MET | 2 | 18.066 | 4.488 | 31.067 | 1.00 | 58.78 |
| ATOM | 13 | CE | MET | 2 | 18.185 | 4.360 | 32.887 | 1.00 | 53.72 |
| ATOM | 14 | C | MET | 2 | 18.669 | 1.176 | 27.894 | 1.00 | 43.49 |
| ATOM | 15 | O | MET | 2 | 19.405 | 2.034 | 27.406 | 1.00 | 42.50 |
| ATOM | 16 | N | SER | 3 | 18.854 | -0.126 | 27.690 | 1.00 | 41.13 |
| ATOM | 17 | CA | SER | 3 | 19.955 | -0.610 | 26.862 | 1.00 | 40.00 |
| ATOM | 18 | CB | SER | 3 | 19.927 | -2.135 | 26.793 | 1.00 | 40.28 |
| ATOM | 19 | OG | SER | 3 | 20.891 | -2.608 | 25.874 | 1.00 | 41.89 |
| ATOM | 20 | C | SER | 3 | 19.86 | -0.031 | 25.447 | 1.00 | 38.47 |
| ATOM | 21 | O | SER | 3 | 20.860 | 0.429 | 24.883 | 1.00 | 37.74 |
| ATOM | 22 | N | GLU | 4 | 18.666 | -0.056 | 24.876 | 1.00 | 36.43 |
| ATOM | 23 | CA | GLU | 4 | 18.464 | 0.478 | 23.536 | 1.00 | 34.72 |
| ATOM | 24 | CB | GLU | 4 | 17.076 | 0.100 | 23.003 | 1.00 | 34.35 |
| ATOM | 25 | CG | GLU | 4 | 16.711 | 0.808 | 21.697 | 1.00 | 35.50 |
| ATOM | 26 | CD | GLU | 4 | 15.384 | 0.347 | 21.124 | 1.00 | 36.78 |
| ATOM | 27 | OE1 | GLU | 4 | 14.551 | -0.177 | 21.896 | 1.00 | 38.95 |
| ATOM | 28 | OE2 | GLU | 4 | 15.164 | 0.522 | 19.906 | 1.00 | 36.38 |
| ATOM | 29 | C | GLU | 4 | 18.624 | 1.995 | 23.536 | 1.00 | 33.23 |
| ATOM | 30 | O | GLU | 4 | 19.304 | 2.552 | 22.674 | 1.00 | 33.90 |
| ATOM | 31 | N | ILE | 5 | 18.010 | 2.661 | 24.511 | 1.00 | 31.72 |
| ATOM | 32 | CA | ILE | 5 | 18.099 | 4.112 | 24.600 | 1.00 | 30.77 |
| ATOM | 33 | CB | ILE | 5 | 17.390 | 4.654 | 25.856 | 1.00 | 31.04 |
| ATOM | 34 | CG2 | ILE | 5 | 17.525 | 6.169 | 25.921 | 1.00 | 29.17 |
| ATOM | 35 | CG1 | ILE | 5 | 15.907 | 4.272 | 25.828 | 1.00 | 31.73 |
| ATOM | 36 | CD1 | ILE | 5 | 15.143 | 4.871 | 24.668 | 1.00 | 32.36 |
| ATOM | 37 | C | ILE | 5 | 19.554 | 4.547 | 24.640 | 1.00 | 30.75 |
| ATOM | 38 | O | ILE | 5 | 19.947 | 5.467 | 23.939 | 1.00 | 29.87 |
| ATOM | 39 | N | ASP | 6 | 20.358 | 3.876 | 25.458 | 1.00 | 31.69 |
| ATOM | 40 | CA | ASP | 6 | 21.769 | 4.227 | 25.567 | 1.00 | 31.78 |
| ATOM | 41 | CB | ASP | 6 | 22.433 | 3.437 | 26.706 | 1.00 | 35.48 |
| ATOM | 42 | CG | ASP | 6 | 23.900 | 3.807 | 26.898 | 1.00 | 37.43 |
| ATOM | 43 | OD1 | ASP | 6 | 24.241 | 5.002 | 26.755 | 1.00 | 38.83 |
| ATOM | 44 | OD2 | ASP | 6 | 24.711 | 2.907 | 27.203 | 1.00 | 39.59 |
| ATOM | 45 | C | ASP | 6 | 22.524 | 3.999 | 24.256 | 1.00 | 31.34 |
| ATOM | 46 | O | ASP | 6 | 23.301 | 4.855 | 23.832 | 1.00 | 30.34 |
| ATOM | 47 | N | ARG | 7 | 22.298 | 2.860 | 23.605 | 1.00 | 30.65 |
| ATOM | 48 | CA | ARG | 7 | 22.992 | 2.594 | 22.345 | 1.00 | 32.17 |
| ATOM | 49 | CB | ARG | 7 | 22.675 | 1.186 | 21.847 | 1.00 | 34.91 |
| ATOM | 50 | CG | ARG | 7 | 23.169 | 0.086 | 22.788 | 1.00 | 44.22 |
| ATOM | 51 | CD | ARG | 7 | 22.770 | -1.289 | 22.276 | 1.00 | 48.39 |
| ATOM | 52 | NE | ARG | 7 | 21.346 | -1.332 | 21.948 | 1.00 | 52.06 |
| ATOM | 53 | CZ | ARG | 7 | 20.751 | -2.337 | 21.317 | 1.00 | 52.86 |
| ATOM | 54 | NH1 | ARG | 7 | 21.456 | -3.397 | 20.942 | 1.00 | 54.84 |
| ATOM | 55 | NH2 | ARG | 7 | 19.454 | -2.277 | 21.050 | 1.00 | 54.02 |
| ATOM | 56 | C | ARG | 7 | 22.620 | 3.631 | 21.280 | 1.00 | 30.15 |
| ATOM | 57 | O | ARG | 7 | 23.487 | 4.133 | 20.566 | 1.00 | 29.79 |
| ATOM | 58 | N | ILE | 8 | 21.332 | 3.954 | 21.185 | 1.00 | 28.75 |
| ATOM | 59 | CA | ILE | 8 | 20.865 | 4.944 | 20.220 | 1.00 | 27.58 |
| ATOM | 60 | CB | ILE | 8 | 19.330 | 5.161 | 20.324 | 1.00 | 26.92 |
| ATOM | 61 | CG2 | ILE | 8 | 18.917 | 6.408 | 19.534 | 1.00 | 24.05 |
| ATOM | 62 | CG1 | ILE | 8 | 18.589 | 3.924 | 19.803 | 1.00 | 28.50 |
| ATOM | 63 | CD1 | ILE | 8 | 17.065 | 4.003 | 19.947 | 1.00 | 25.10 |
| ATOM | 64 | C | ILE | 8 | 21.571 | 6.271 | 20.494 | 1.00 | 28.49 |
| ATOM | 65 | O | ILE | 8 | 22.081 | 6.908 | 19.579 | 1.00 | 27.22 |
| ATOM | 66 | N | ALA | 9 | 21.600 | 6.677 | 21.760 | 1.00 | 28.38 |
| ATOM | 67 | CA | ALA | 9 | 22.241 | 7.929 | 22.147 | 1.00 | 28.28 |
| ATOM | 68 | CB | ALA | 9 | 22.086 | 8.150 | 23.648 | 1.00 | 26.41 |
| ATOM | 69 | C | ALA | 9 | 23.721 | 7.956 | 21.759 | 1.00 | 29.29 |
| ATOM | 70 | O | ALA | 9 | 24.176 | 8.890 | 21.099 | 1.00 | 27.81 |
| ATOM | 71 | N | GLN | 10 | 24.474 | 6.935 | 22.167 | 1.00 | 30.09 |
| ATOM | 72 | CA | GLN | 10 | 25.897 | 6.878 | 21.844 | 1.00 | 30.31 |
| ATOM | 73 | CB | GLN | 10 | 26.515 | 5.581 | 22.369 | 1.00 | 34.96 |
| ATOM | 74 | CG | GLN | 10 | 26.435 | 5.403 | 23.873 | 1.00 | 41.16 |
| ATOM | 75 | CD | GLN | 10 | 27.020 | 4.076 | 24.329 | 1.00 | 47.78 |
| ATOM | 76 | OE1 | GLN | 10 | 28.217 | 3.809 | 24.162 | 1.00 | 48.59 |
| ATOM | 77 | NE2 | GLN | 10 | 26.171 | 3.229 | 24.905 | 1.00 | 51.11 |
| ATOM | 78 | C | GLN | 10 | 26.141 | 6.967 | 20.338 | 1.00 | 28.54 |
| ATOM | 79 | O | GLN | 10 | 27.031 | 7.684 | 19.887 | 1.00 | 29.98 |
| ATOM | 80 | N | ASN | 11 | 25.350 | 6.228 | 19.568 | 1.00 | 26.39 |
| ATOM | 81 | CA | ASN | 11 | 25.486 | 6.216 | 18.115 | 1.00 | 25.48 |
| ATOM | 82 | CB | ASN | 11 | 24.439 | 5.266 | 17.524 | 1.00 | 25.98 |
| ATOM | 83 | CG | ASN | 11 | 24.439 | 5.251 | 16.014 | 1.00 | 24.40 |
| ATOM | 84 | OD1 | ASN | 11 | 23.422 | 5.527 | 15.394 | 1.00 | 26.44 |
| ATOM | 85 | ND2 | ASN | 11 | 25.578 | 4.920 | 15.413 | 1.00 | 24.88 |
| ATOM | 86 | C | ASN | 11 | 25.329 | 7.627 | 17.533 | 1.00 | 25.80 |
| ATOM | 87 | O | ASN | 11 | 26.106 | 8.047 | 16.674 | 1.00 | 25.13 |
| ATOM | 88 | N | ILE | 12 | 24.326 | 8.360 | 18.008 | 1.00 | 25.44 |
| ATOM | 89 | CA | ILE | 12 | 24.092 | 9.719 | 17.524 | 1.00 | 24.85 |
| ATOM | 90 | CB | ILE | 12 | 22.743 | 10.255 | 18.031 | 1.00 | 23.12 |
| ATOM | 91 | CG2 | ILE | 12 | 22.617 | 11.739 | 17.719 | 1.00 | 22.76 |
| ATOM | 92 | CG1 | ILE | 12 | 21.613 | 9.462 | 17.363 | 1.00 | 23.39 |
| ATOM | 93 | CD1 | ILE | 12 | 20.253 | 9.769 | 17.882 | 1.00 | 20.56 |
| ATOM | 94 | C | ILE | 12 | 25.214 | 10.664 | 17.949 | 1.00 | 25.34 |
| ATOM | 95 | O | ILE | 12 | 25.683 | 11.474 | 17.158 | 1.00 | 25.18 |
| ATOM | 96 | N | ILE | 13 | 25.648 | 10.556 | 19.198 | 1.00 | 25.76 |
| ATOM | 97 | CA | ILE | 13 | 26.728 | 11.403 | 19.686 | 1.00 | 26.18 |
| ATOM | 98 | CB | ILE | 13 | 26.993 | 11.137 | 21.184 | 1.00 | 26.42 |
| ATOM | 99 | CG2 | ILE | 13 | 28.317 | 11.759 | 21.605 | 1.00 | 27.88 |
| ATOM | 100 | CG1 | ILE | 13 | 25.822 | 11.683 | 22.015 | 1.00 | 24.88 |
| ATOM | 101 | CD1 | ILE | 13 | 25.887 | 11.341 | 23.510 | 1.00 | 24.90 |
| ATOM | 102 | C | ILE | 13 | 27.987 | 11.122 | 18.855 | 1.00 | 26.22 |
| ATOM | 103 | O | ILE | 13 | 28.680 | 12.045 | 18.423 | 1.00 | 25.36 |
| ATOM | 104 | N | LYS | 14 | 28.266 | 9.847 | 18.616 | 1.00 | 27.79 |
| ATOM | 105 | CA | LYS | 14 | 29.425 | 9.453 | 17.819 | 1.00 | 28.58 |
| ATOM | 106 | CB | LYS | 14 | 29.504 | 7.927 | 17.734 | 1.00 | 31.83 |
| ATOM | 107 | CG | LYS | 14 | 30.610 | 7.389 | 16.827 | 1.00 | 36.77 |
| ATOM | 108 | CD | LYS | 14 | 30.454 | 5.879 | 16.615 | 1.00 | 41.46 |
| ATOM | 109 | CE | LYS | 14 | 31.523 | 5.320 | 15.681 | 1.00 | 42.19 |
| ATOM | 110 | NZ | LYS | 14 | 32.877 | 5.449 | 16.278 | 1.00 | 44.14 |
| ATOM | 111 | C | LYS | 14 | 29.311 | 10.039 | 16.412 | 1.00 | 28.52 |
| ATOM | 112 | O | LYS | 14 | 30.268 | 10.609 | 15.883 | 1.00 | 29.83 |
| ATOM | 113 | N | SER | 15 | 28.136 | 9.905 | 15.806 | 1.00 | 26.79 |
| ATOM | 114 | CA | SER | 15 | 27.929 | 10.429 | 14.459 | 1.00 | 26.88 |
| ATOM | 115 | CB | SER | 15 | 26.500 | 10.132 | 13.994 | 1.00 | 27.05 |
| ATOM | 116 | OG | SER | 15 | 26.280 | 10.611 | 12.676 | 1.00 | 30.56 |
| ATOM | 117 | C | SER | 15 | 28.193 | 11.932 | 14.414 | 1.00 | 26.19 |
| ATOM | 118 | O | SER | 15 | 28.895 | 12.427 | 13.529 | 1.00 | 25.81 |
| ATOM | 119 | N | HIS | 16 | 27.627 | 12.657 | 15.373 | 1.00 | 25.25 |
| ATOM | 120 | CA | HIS | 16 | 27.807 | 14.104 | 15.448 | 1.00 | 25.35 |
| ATOM | 121 | CB | HIS | 16 | 27.098 | 14.652 | 16.688 | 1.00 | 23.98 |
| ATOM | 122 | CG | HIS | 16 | 27.485 | 16.055 | 17.035 | 1.00 | 22.83 |
| ATOM | 123 | CD2 | HIS | 16 | 28.473 | 16.533 | 17.830 | 1.00 | 22.89 |
| ATOM | 124 | ND1 | HIS | 16 | 26.831 | 17.159 | 16.530 | 1.00 | 21.54 |
| ATOM | 125 | CE1 | HIS | 16 | 27.398 | 18.256 | 16.999 | 1.00 | 23.79 |
| ATOM | 126 | NE2 | HIS | 16 | 28.398 | 17.904 | 17.789 | 1.00 | 24.95 |
| ATOM | 127 | C | HIS | 16 | 29.296 | 14.438 | 15.532 | 1.00 | 27.00 |
| ATOM | 128 | O | HIS | 16 | 29.794 | 15.325 | 14.832 | 1.00 | 25.20 |
| ATOM | 129 | N | LEU | 17 | 29.998 | 13.723 | 16.406 | 1.00 | 27.86 |
| ATOM | 130 | CA | LEU | 17 | 31.423 | 13.940 | 16.591 | 1.00 | 28.80 |
| ATOM | 131 | CB | LEU | 17 | 31.996 | 12.917 | 17.578 | 1.00 | 30.56 |
| ATOM | 132 | CG | LEU | 17 | 33.501 | 13.008 | 17.863 | 1.00 | 33.43 |
| ATOM | 133 | CD1 | LEU | 17 | 33.784 | 14.232 | 18.725 | 1.00 | 35.11 |
| ATOM | 134 | CD2 | LEU | 17 | 33.968 | 11.757 | 18.578 | 1.00 | 35.02 |
| ATOM | 135 | C | LEU | 17 | 32.158 | 13.809 | 15.269 | 1.00 | 28.50 |
| ATOM | 136 | O | LEU | 17 | 33.060 | 14.588 | 14.973 | 1.00 | 29.06 |
| ATOM | 137 | N | GLU | 18 | 31.758 | 12.833 | 14.466 | 1.00 | 27.38 |
| ATOM | 138 | CA | GLU | 18 | 32.430 | 12.584 | 13.197 | 1.00 | 29.19 |
| ATOM | 139 | CB | GLU | 18 | 32.419 | 11.080 | 12.897 | 1.00 | 30.86 |
| ATOM | 140 | CG | GLU | 18 | 32.740 | 10.192 | 14.093 | 1.00 | 35.14 |
| ATOM | 141 | CD | GLU | 18 | 32.508 | 8.718 | 13.807 | 1.00 | 38.27 |
| ATOM | 142 | OE1 | GLU | 18 | 31.604 | 8.399 | 12.997 | 1.00 | 40.84 |
| ATOM | 143 | OE2 | GLU | 18 | 33.215 | 7.877 | 14.407 | 1.00 | 38.21 |
| ATOM | 144 | C | GLU | 18 | 31.884 | 13.311 | 11.977 | 1.00 | 28.77 |
| ATOM | 145 | O | GLU | 18 | 32.495 | 13.242 | 10.909 | 1.00 | 29.35 |
| ATOM | 146 | N | THR | 19 | 30.767 | 14.023 | 12.109 | 1.00 | 26.26 |
| ATOM | 147 | CA | THR | 19 | 30.194 | 14.656 | 10.926 | 1.00 | 25.75 |
| ATOM | 148 | CB | THR | 19 | 28.916 | 13.921 | 10.510 | 1.00 | 24.77 |
| ATOM | 149 | OG1 | THR | 19 | 27.906 | 14.120 | 11.508 | 1.00 | 22.37 |
| ATOM | 150 | CG2 | THR | 19 | 29.194 | 12.428 | 10.366 | 1.00 | 24.32 |
| ATOM | 151 | C | THR | 19 | 29.888 | 16.148 | 10.903 | 1.00 | 26.21 |
| ATOM | 152 | O | THR | 19 | 29.143 | 16.606 | 10.040 | 1.00 | 26.97 |
| ATOM | 153 | N | CYS | 20 | 30.425 | 16.916 | 11.839 | 1.00 | 28.90 |
| ATOM | 154 | CA | CYS | 20 | 30.190 | 18.351 | 11.789 | 1.00 | 31.93 |
| ATOM | 155 | CB | CYS | 20 | 30.306 | 18.979 | 13.175 | 1.00 | 29.90 |
| ATOM | 156 | SG | CYS | 20 | 28.844 | 18.742 | 14.175 | 1.00 | 30.72 |
| ATOM | 157 | C | CYS | 20 | 31.258 | 18.930 | 10.876 | 1.00 | 33.01 |
| ATOM | 158 | O | CYS | 20 | 32.368 | 18.412 | 10.824 | 1.00 | 33.41 |
| ATOM | 159 | N | GLN | 21 | 30.927 | 19.986 | 10.143 | 1.00 | 34.90 |
| ATOM | 160 | CA | GLN | 21 | 31.911 | 20.595 | 9.261 | 1.00 | 36.25 |
| ATOM | 161 | CB | GLN | 21 | 31.314 | 21.782 | 8.509 | 1.00 | 37.23 |
| ATOM | 162 | CG | GLN | 21 | 32.359 | 22.563 | 7.731 | 1.00 | 39.86 |
| ATOM | 163 | CD | GLN | 21 | 31.797 | 23.770 | 7.024 | 1.00 | 39.83 |
| ATOM | 164 | OE1 | GLN | 21 | 32.545 | 24.580 | 6.476 | 1.00 | 43.69 |
| ATOM | 165 | NE2 | GLN | 21 | 30.478 | 23.900 | 7.025 | 1.00 | 41.69 |
| ATOM | 166 | C | GLN | 21 | 33.096 | 21.077 | 10.099 | 1.00 | 36.79 |
| ATOM | 167 | O | GLN | 21 | 34.229 | 21.077 | 9.636 | 1.00 | 36.87 |
| ATOM | 168 | N | TYR | 22 | 32.799 | 21.489 | 11.331 | 1.00 | 35.98 |
| ATOM | 169 | CA | TYR | 22 | 33.818 | 21.974 | 12.262 | 1.00 | 37.06 |
| ATOM | 170 | CB | TYR | 22 | 33.608 | 23.460 | 12.559 | 1.00 | 37.63 |
| ATOM | 171 | CG | TYR | 22 | 34.105 | 24.384 | 11.480 | 1.00 | 37.76 |
| ATOM | 172 | CD1 | TYR | 22 | 35.467 | 24.505 | 11.219 | 1.00 | 39.21 |
| ATOM | 173 | CE1 | TYR | 22 | 35.933 | 25.362 | 10.231 | 1.00 | 41.19 |
| ATOM | 174 | CD2 | TYR | 22 | 33.217 | 25.142 | 10.724 | 1.00 | 38.25 |
| ATOM | 175 | CE2 | TYR | 22 | 33.671 | 26.001 | 9.733 | 1.00 | 40.65 |
| ATOM | 176 | CZ | TYR | 22 | 35.029 | 26.105 | 9.492 | 1.00 | 40.42 |
| ATOM | 177 | OH | TYR | 22 | 35.483 | 26.946 | 8.509 | 1.00 | 43.37 |
| ATOM | 178 | C | TYR | 22 | 33.786 | 21.214 | 13.580 | 1.00 | 35.92 |
| ATOM | 179 | O | TYR | 22 | 32.767 | 21.199 | 14.268 | 1.00 | 35.59 |
| ATOM | 180 | N | THR | 23 | 34.900 | 20.587 | 13.935 | 1.00 | 36.10 |
| ATOM | 181 | CA | THR | 23 | 34.968 | 19.861 | 15.193 | 1.00 | 36.94 |
| ATOM | 182 | CB | THR | 23 | 36.179 | 18.921 | 15.242 | 1.00 | 39.58 |
| ATOM | 183 | OG1 | THR | 23 | 37.380 | 19.700 | 15.216 | 1.00 | 39.59 |
| ATOM | 184 | CG2 | THR | 23 | 36.169 | 17.969 | 14.056 | 1.00 | 40.40 |
| ATOM | 185 | C | THR | 23 | 35.128 | 20.892 | 16.302 | 1.00 | 36.49 |
| ATOM | 186 | O | THR | 23 | 35.459 | 22.052 | 16.041 | 1.00 | 32.29 |
| ATOM | 187 | N | MET | 24 | 34.895 | 20.471 | 17.539 | 1.00 | 37.33 |
| ATOM | 188 | CA | MET | 24 | 35.029 | 21.371 | 18.674 | 1.00 | 38.75 |
| ATOM | 189 | CB | MET | 24 | 34.654 | 20.648 | 19.966 | 1.00 | 39.75 |
| ATOM | 190 | CG | MET | 24 | 33.161 | 20.570 | 20.204 | 1.00 | 42.25 |
| ATOM | 191 | SD | MET | 24 | 32.471 | 22.130 | 20.821 | 1.00 | 43.80 |
| ATOM | 192 | CE | MET | 24 | 32.356 | 23.099 | 19.341 | 1.00 | 43.48 |
| ATOM | 193 | C | MET | 24 | 36.454 | 21.889 | 18.765 | 1.00 | 39.58 |
| ATOM | 194 | O | MET | 24 | 36.688 | 23.054 | 19.083 | 1.00 | 39.75 |
| ATOM | 195 | N | GLU | 25 | 37.409 | 21.017 | 18.469 | 1.00 | 41.04 |
| ATOM | 196 | CA | GLU | 25 | 38.808 | 21.397 | 18.529 | 1.00 | 41.50 |
| ATOM | 197 | CB | GLU | 25 | 39.685 | 20.172 | 18.242 | 1.00 | 45.13 |
| ATOM | 198 | CG | GLU | 25 | 39.356 | 18.989 | 19.165 | 1.00 | 50.86 |
| ATOM | 199 | CD | GLU | 25 | 40.586 | 18.370 | 19.829 | 1.00 | 55.72 |
| ATOM | 200 | OE1 | GLU | 25 | 41.444 | 19.126 | 20.344 | 1.00 | 56.58 |
| ATOM | 201 | OE2 | GLU | 25 | 40.686 | 17.122 | 19.851 | 1.00 | 57.47 |
| ATOM | 202 | C | GLU | 25 | 39.103 | 22.536 | 17.553 | 1.00 | 40.38 |
| ATOM | 203 | O | GLU | 25 | 39.708 | 23.538 | 17.928 | 1.00 | 37.53 |
| ATOM | 204 | N | GLU | 26 | 38.655 | 22.390 | 16.310 | 1.00 | 38.80 |
| ATOM | 205 | CA | GLU | 26 | 38.885 | 23.415 | 15.298 | 1.00 | 38.72 |
| ATOM | 206 | CB | GLU | 26 | 38.353 | 22.948 | 13.943 | 1.00 | 40.80 |
| ATOM | 207 | CG | GLU | 26 | 38.837 | 21.562 | 13.555 | 1.00 | 45.17 |
| ATOM | 208 | CD | GLU | 26 | 38.289 | 21.099 | 12.225 | 1.00 | 47.49 |
| ATOM | 209 | OE1 | GLU | 26 | 37.059 | 21.191 | 12.023 | 1.00 | 50.42 |
| ATOM | 210 | OE2 | GLU | 26 | 39.087 | 20.633 | 11.384 | 1.00 | 50.96 |
| ATOM | 211 | C | GLU | 26 | 38.205 | 24.716 | 15.701 | 1.00 | 37.74 |
| ATOM | 212 | O | GLU | 26 | 38.745 | 25.799 | 15.478 | 1.00 | 35.99 |
| ATOM | 213 | N | LEU | 27 | 37.020 | 24.604 | 16.298 | 1.00 | 37.47 |
| ATOM | 214 | CA | LEU | 27 | 36.278 | 25.776 | 16.744 | 1.00 | 37.81 |
| ATOM | 215 | CB | LEU | 27 | 34.894 | 25.369 | 17.272 | 1.00 | 36.85 |
| ATOM | 216 | CG | LEU | 27 | 33.650 | 25.542 | 16.379 | 1.00 | 36.21 |
| ATOM | 217 | CD1 | LEU | 27 | 34.023 | 26.084 | 15.013 | 1.00 | 34.78 |
| ATOM | 218 | CD2 | LEU | 27 | 32.927 | 24.214 | 16.248 | 1.00 | 34.77 |
| ATOM | 219 | C | LEU | 27 | 37.051 | 26.512 | 17.833 | 1.00 | 39.23 |
| ATOM | 220 | O | LEU | 27 | 37.145 | 27.738 | 17.820 | 1.00 | 37.29 |
| ATOM | 221 | N | HIS | 28 | 37.610 | 25.762 | 18.776 | 1.00 | 42.29 |
| ATOM | 222 | CA | HIS | 28 | 38.369 | 26.378 | 19.852 | 1.00 | 45.83 |
| ATOM | 223 | CB | HIS | 28 | 38.867 | 25.311 | 20.834 | 1.00 | 49.02 |
| ATOM | 224 | CG | HIS | 28 | 37.786 | 24.745 | 21.707 | 1.00 | 53.69 |
| ATOM | 225 | CD2 | HIS | 28 | 36.461 | 25.024 | 21.782 | 1.00 | 54.33 |
| ATOM | 226 | ND1 | HIS | 28 | 38.025 | 23.775 | 22.660 | 1.00 | 54.53 |
| ATOM | 227 | CE1 | HIS | 28 | 36.896 | 23.484 | 23.283 | 1.00 | 53.92 |
| ATOM | 228 | NE2 | HIS | 28 | 35.932 | 24.228 | 22.770 | 1.00 | 54.66 |
| ATOM | 229 | C | HIS | 28 | 39.533 | 27.202 | 19.306 | 1.00 | 46.70 |
| ATOM | 230 | O | HIS | 28 | 39.809 | 28.292 | 19.806 | 1.00 | 46.07 |
| ATOM | 231 | N | GLN | 29 | 40.206 | 26.695 | 18.277 | 1.00 | 47.88 |
| ATOM | 232 | CA | GLN | 29 | 41.321 | 27.432 | 17.686 | 1.00 | 49.79 |
| ATOM | 233 | CB | GLN | 29 | 42.205 | 26.504 | 16.841 | 1.00 | 51.23 |
| ATOM | 234 | CG | GLN | 29 | 43.142 | 25.629 | 17.673 | 1.00 | 55.35 |
| ATOM | 235 | CD | GLN | 29 | 42.632 | 24.210 | 17.843 | 1.00 | 57.34 |
| ATOM | 236 | OE1 | GLN | 29 | 42.896 | 23.556 | 18.856 | 1.00 | 58.45 |
| ATOM | 237 | NE2 | GLN | 29 | 41.913 | 23.717 | 16.839 | 1.00 | 58.43 |
| ATOM | 238 | C | GLN | 29 | 40.842 | 28.609 | 16.835 | 1.00 | 49.61 |
| ATOM | 239 | O | GLN | 29 | 41.532 | 29.621 | 16.725 | 1.00 | 50.02 |
| ATOM | 240 | N | LEU | 30 | 39.659 | 28.472 | 16.245 | 1.00 | 49.60 |
| ATOM | 241 | CA | LEU | 30 | 39.081 | 29.519 | 15.404 | 1.00 | 51.37 |
| ATOM | 242 | CB | LEU | 30 | 38.164 | 28.908 | 14.346 | 1.00 | 51.30 |
| ATOM | 243 | CG | LEU | 30 | 38.817 | 28.187 | 13.172 | 1.00 | 52.22 |
| ATOM | 244 | CD1 | LEU | 30 | 37.770 | 27.391 | 12.407 | 1.00 | 51.55 |
| ATOM | 245 | CD2 | LEU | 30 | 39.491 | 29.212 | 12.275 | 1.00 | 52.87 |
| ATOM | 246 | C | LEU | 30 | 38.266 | 30.493 | 16.233 | 1.00 | 52.89 |
| ATOM | 247 | O | LEU | 30 | 37.645 | 31.409 | 15.694 | 1.00 | 53.39 |
| ATOM | 248 | N | ALA | 31 | 38.271 | 30.283 | 17.544 | 1.00 | 54.40 |
| ATOM | 249 | CA | ALA | 31 | 37.509 | 31.107 | 18.474 | 1.00 | 54.90 |
| ATOM | 250 | CB | ALA | 31 | 37.875 | 30.728 | 19.902 | 1.00 | 55.70 |
| ATOM | 251 | C | ALA | 31 | 37.674 | 32.608 | 18.284 | 1.00 | 54.89 |
| ATOM | 252 | O | ALA | 31 | 36.695 | 33.339 | 18.109 | 1.00 | 52.72 |
| ATOM | 253 | N | TRP | 32 | 38.925 | 33.055 | 18.324 | 1.00 | 56.51 |
| ATOM | 254 | CA | TRP | 32 | 39.254 | 34.468 | 18.197 | 1.00 | 57.22 |
| ATOM | 255 | CB | TRP | 32 | 40.362 | 34.816 | 19.192 | 1.00 | 59.36 |
| ATOM | 256 | CG | TRP | 32 | 39.979 | 34.430 | 20.581 | 1.00 | 62.16 |
| ATOM | 257 | CD2 | TRP | 32 | 39.717 | 35.315 | 21.678 | 1.00 | 63.50 |
| ATOM | 258 | CE2 | TRP | 32 | 39.268 | 34.519 | 22.757 | 1.00 | 64.21 |
| ATOM | 259 | CE3 | TRP | 32 | 39.814 | 36.702 | 21.855 | 1.00 | 63.45 |
| ATOM | 260 | CD1 | TRP | 32 | 39.697 | 33.168 | 21.031 | 1.00 | 62.50 |
| ATOM | 261 | NE1 | TRP | 32 | 39.265 | 33.215 | 22.335 | 1.00 | 63.94 |
| ATOM | 262 | CZ2 | TRP | 32 | 38.913 | 35.068 | 23.995 | 1.00 | 63.81 |
| ATOM | 263 | CZ3 | TRP | 32 | 39.461 | 37.244 | 23.087 | 1.00 | 62.65 |
| ATOM | 264 | CH2 | TRP | 32 | 39.016 | 36.426 | 24.137 | 1.00 | 62.53 |
| ATOM | 265 | C | TRP | 32 | 39.667 | 34.850 | 16.787 | 1.00 | 57.28 |
| ATOM | 266 | O | TRP | 32 | 40.826 | 35.187 | 16.526 | 1.00 | 58.45 |
| ATOM | 267 | N | GLN | 33 | 38.701 | 34.802 | 15.879 | 1.00 | 54.65 |
| ATOM | 268 | CA | GLN | 33 | 38.954 | 35.145 | 14.494 | 1.00 | 52.60 |
| ATOM | 269 | CB | GLN | 33 | 39.326 | 33.892 | 13.697 | 1.00 | 54.99 |
| ATOM | 270 | CG | GLN | 33 | 40.753 | 33.411 | 13.884 | 1.00 | 59.00 |
| ATOM | 271 | CD | GLN | 33 | 41.772 | 34.297 | 13.184 | 1.00 | 61.62 |
| ATOM | 272 | OE1 | GLN | 33 | 42.960 | 33.978 | 13.148 | 1.00 | 63.64 |
| ATOM | 273 | NE2 | GLN | 33 | 41.312 | 35.415 | 12.624 | 1.00 | 63.55 |
| ATOM | 274 | C | GLN | 33 | 37.737 | 35.801 | 13.863 | 1.00 | 49.63 |
| ATOM | 275 | O | GLN | 33 | 36.785 | 35.124 | 13.489 | 1.00 | 47.22 |
| ATOM | 276 | N | THR | 34 | 37.763 | 37.125 | 13.771 | 1.00 | 47.47 |
| ATOM | 277 | CA | THR | 34 | 36.678 | 37.863 | 13.139 | 1.00 | 45.87 |
| ATOM | 278 | CB | THR | 34 | 36.159 | 39.019 | 14.033 | 1.00 | 46.83 |
| ATOM | 279 | OG1 | THR | 34 | 37.237 | 39.911 | 14.346 | 1.00 | 46.09 |
| ATOM | 280 | CG2 | THR | 34 | 35.556 | 38.475 | 15.321 | 1.00 | 47.41 |
| ATOM | 281 | C | THR | 34 | 37.269 | 38.446 | 11.856 | 1.00 | 44.40 |
| ATOM | 282 | O | THR | 34 | 38.469 | 38.731 | 11.797 | 1.00 | 44.57 |
| ATOM | 283 | N | HIS | 35 | 36.451 | 38.605 | 10.822 | 1.00 | 41.42 |
| ATOM | 284 | CA | HIS | 35 | 36.964 | 39.168 | 9.581 | 1.00 | 37.99 |
| ATOM | 285 | CB | HIS | 35 | 35.894 | 39.157 | 8.487 | 1.00 | 34.94 |
| ATOM | 286 | CG | HIS | 35 | 35.570 | 37.792 | 7.967 | 1.00 | 32.18 |
| ATOM | 287 | CD2 | HIS | 35 | 36.167 | 37.037 | 7.015 | 1.00 | 30.88 |
| ATOM | 288 | ND1 | HIS | 35 | 34.510 | 37.047 | 8.438 | 1.00 | 31.87 |
| ATOM | 289 | CE1 | HIS | 35 | 34.467 | 35.893 | 7.797 | 1.00 | 30.43 |
| ATOM | 290 | NE2 | HIS | 35 | 35.463 | 35.862 | 6.929 | 1.00 | 32.44 |
| ATOM | 291 | C | HIS | 35 | 37.414 | 40.601 | 9.844 | 1.00 | 36.88 |
| ATOM | 292 | O | HIS | 35 | 36.856 | 41.287 | 10.697 | 1.00 | 35.24 |
| ATOM | 293 | N | THR | 36 | 38.439 | 41.042 | 9.124 | 1.00 | 38.20 |
| ATOM | 294 | CA | THR | 36 | 38.949 | 42.402 | 9.276 | 1.00 | 39.26 |
| ATOM | 295 | CB | THR | 36 | 40.319 | 42.550 | 8.597 | 1.00 | 39.89 |
| ATOM | 296 | OG1 | THR | 36 | 40.181 | 42.304 | 7.192 | 1.00 | 40.21 |
| ATOM | 297 | CG2 | THR | 36 | 41.312 | 41.548 | 9.178 | 1.00 | 40.05 |
| ATOM | 298 | C | THR | 36 | 37.959 | 43.346 | 8.599 | 1.00 | 39.52 |
| ATOM | 299 | O | THR | 36 | 37.070 | 42.894 | 7.873 | 1.00 | 38.71 |
| ATOM | 300 | N | TYR | 37 | 38.101 | 44.648 | 8.824 | 1.00 | 40.48 |
| ATOM | 301 | CA | TYR | 37 | 37.186 | 45.595 | 8.197 | 1.00 | 41.59 |
| ATOM | 302 | CB | TYR | 37 | 37.474 | 47.026 | 8.653 | 1.00 | 44.12 |
| ATOM | 303 | CG | TYR | 37 | 37.124 | 47.289 | 10.099 | 1.00 | 46.75 |
| ATOM | 304 | CD1 | TYR | 37 | 38.080 | 47.149 | 11.110 | 1.00 | 48.09 |
| ATOM | 305 | CE1 | TYR | 37 | 37.754 | 47.392 | 12.453 | 1.00 | 49.23 |
| ATOM | 306 | CD2 | TYR | 37 | 35.831 | 47.675 | 10.460 | 1.00 | 47.60 |
| ATOM | 307 | CE2 | TYR | 37 | 35.493 | 47.916 | 11.795 | 1.00 | 49.16 |
| ATOM | 308 | CZ | TYR | 37 | 36.456 | 47.775 | 12.786 | 1.00 | 49.69 |
| ATOM | 309 | OH | TYR | 37 | 36.120 | 48.027 | 14.100 | 1.00 | 48.49 |
| ATOM | 310 | C | TYR | 37 | 37.269 | 45.515 | 6.678 | 1.00 | 41.21 |
| ATOM | 311 | O | TYR | 37 | 36.265 | 45.673 | 5.988 | 1.00 | 39.83 |
| ATOM | 312 | N | GLU | 38 | 38.469 | 45.270 | 6.158 | 1.00 | 40.73 |
| ATOM | 313 | CA | GLU | 38 | 38.652 | 45.161 | 4.715 | 1.00 | 41.99 |
| ATOM | 314 | CB | GLU | 38 | 40.136 | 45.020 | 4.358 | 1.00 | 43.80 |
| ATOM | 315 | CG | GLU | 38 | 40.985 | 46.242 | 4.668 | 1.00 | 46.95 |
| ATOM | 316 | CD | GLU | 38 | 41.266 | 46.412 | 6.154 | 1.00 | 48.29 |
| ATOM | 317 | OE1 | GLU | 38 | 41.815 | 47.470 | 6.528 | 1.00 | 50.72 |
| ATOM | 318 | OE2 | GLU | 38 | 40.948 | 45.493 | 6.943 | 1.00 | 48.78 |
| ATOM | 319 | C | GLU | 38 | 37.896 | 43.949 | 4.185 | 1.00 | 41.21 |
| ATOM | 320 | O | GLU | 38 | 37.284 | 44.000 | 3.122 | 1.00 | 39.46 |
| ATOM | 321 | N | GLU | 39 | 37.946 | 42.854 | 4.935 | 1.00 | 41.37 |
| ATOM | 322 | CA | GLU | 39 | 37.265 | 41.632 | 4.527 | 1.00 | 41.22 |
| ATOM | 323 | CB | GLU | 39 | 37.693 | 40.473 | 5.429 | 1.00 | 42.06 |
| ATOM | 324 | CG | GLU | 39 | 39.208 | 40.289 | 5.453 | 1.00 | 46.35 |
| ATOM | 325 | CD | GLU | 39 | 39.668 | 39.164 | 6.363 | 1.00 | 48.63 |
| ATOM | 326 | OE1 | GLU | 39 | 39.234 | 39.126 | 7.534 | 1.00 | 48.96 |
| ATOM | 327 | OE2 | GLU | 39 | 40.476 | 38.324 | 5.909 | 1.00 | 50.59 |
| ATOM | 328 | C | GLU | 39 | 35.756 | 41.844 | 4.581 | 1.00 | 39.62 |
| ATOM | 329 | O | GLU | 39 | 35.023 | 41.339 | 3.728 | 1.00 | 40.20 |
| ATOM | 330 | N | ILE | 40 | 35.296 | 42.601 | 5.575 | 1.00 | 37.13 |
| ATOM | 331 | CA | ILE | 40 | 33.873 | 42.885 | 5.706 | 1.00 | 36.49 |
| ATOM | 332 | CB | ILE | 40 | 33.541 | 43.587 | 7.058 | 1.00 | 35.15 |
| ATOM | 333 | CG2 | ILE | 40 | 32.105 | 44.100 | 7.054 | 1.00 | 34.00 |
| ATOM | 334 | CG1 | ILE | 40 | 33.715 | 42.603 | 8.219 | 1.00 | 34.57 |
| ATOM | 335 | CD1 | ILE | 40 | 32.853 | 41.351 | 8.102 | 1.00 | 32.97 |
| ATOM | 336 | C | ILE | 40 | 33.445 | 43.784 | 4.557 | 1.00 | 36.47 |
| ATOM | 337 | O | ILE | 40 | 32.386 | 43.582 | 3.966 | 1.00 | 35.95 |
| ATOM | 338 | N | LYS | 41 | 34.277 | 44.772 | 4.233 | 1.00 | 37.54 |
| ATOM | 339 | CA | LYS | 41 | 33.955 | 45.688 | 3.141 | 1.00 | 38.47 |
| ATOM | 340 | CB | LYS | 41 | 35.023 | 46.774 | 3.002 | 1.00 | 41.50 |
| ATOM | 341 | CG | LYS | 41 | 34.532 | 48.021 | 2.277 | 1.00 | 45.40 |
| ATOM | 342 | CD | LYS | 41 | 33.917 | 49.022 | 3.257 | 1.00 | 49.51 |
| ATOM | 343 | CE | LYS | 41 | 32.810 | 48.394 | 4.110 | 1.00 | 50.14 |
| ATOM | 344 | NZ | LYS | 41 | 32.365 | 49.280 | 5.217 | 1.00 | 51.19 |
| ATOM | 345 | C | LYS | 41 | 33.876 | 44.899 | 1.846 | 1.00 | 36.39 |
| ATOM | 346 | O | LYS | 41 | 32.987 | 45.121 | 1.023 | 1.00 | 34.38 |
| ATOM | 347 | N | ALA | 42 | 34.813 | 43.972 | 1.679 | 1.00 | 35.77 |
| ATOM | 348 | CA | ALA | 42 | 34.855 | 43.134 | 0.492 | 1.00 | 36.18 |
| ATOM | 349 | CB | ALA | 42 | 35.999 | 42.132 | 0.599 | 1.00 | 37.07 |
| ATOM | 350 | C | ALA | 42 | 33.523 | 42.409 | 0.345 | 1.00 | 35.76 |
| ATOM | 351 | O | ALA | 42 | 32.958 | 42.355 | -0.749 | 1.00 | 35.23 |
| ATOM | 352 | N | TYR | 43 | 33.015 | 41.868 | 1.451 | 1.00 | 34.54 |
| ATOM | 353 | CA | TYR | 43 | 31.740 | 41.155 | 1.423 | 1.00 | 33.59 |
| ATOM | 354 | CB | TYR | 43 | 31.423 | 40.539 | 2.794 | 1.00 | 33.00 |
| ATOM | 355 | CG | TYR | 43 | 32.175 | 39.263 | 3.093 | 1.00 | 31.41 |
| ATOM | 356 | CD1 | TYR | 43 | 32.035 | 38.138 | 2.275 | 1.00 | 31.79 |
| ATOM | 357 | CE1 | TYR | 43 | 32.734 | 36.959 | 2.543 | 1.00 | 32.00 |
| ATOM | 358 | CD2 | TYR | 43 | 33.031 | 39.179 | 4.190 | 1.00 | 32.57 |
| ATOM | 359 | CE2 | TYR | 43 | 33.735 | 38.006 | 4.468 | 1.00 | 32.13 |
| ATOM | 360 | CZ | TYR | 43 | 33.583 | 36.902 | 3.643 | 1.00 | 32.91 |
| ATOM | 361 | OH | TYR | 43 | 34.287 | 35.750 | 3.922 | 1.00 | 32.24 |
| ATOM | 362 | C | TYR | 43 | 30.589 | 42.061 | 1.017 | 1.00 | 33.94 |
| ATOM | 363 | O | TYR | 43 | 29.697 | 41.647 | 0.277 | 1.00 | 33.69 |
| ATOM | 364 | N | GLN | 44 | 30.605 | 43.295 | 1.510 | 1.00 | 34.41 |
| ATOM | 365 | CA | GLN | 44 | 29.540 | 44.244 | 1.206 | 1.00 | 35.55 |
| ATOM | 366 | CB | GLN | 44 | 29.571 | 45.405 | 2.210 | 1.00 | 36.41 |
| ATOM | 367 | CG | GLN | 44 | 29.489 | 44.944 | 3.665 | 1.00 | 38.05 |
| ATOM | 368 | CD | GLN | 44 | 29.509 | 46.090 | 4.667 | 1.00 | 39.51 |
| ATOM | 369 | OE1 | GLN | 44 | 30.353 | 46.984 | 4.592 | 1.00 | 40.40 |
| ATOM | 370 | NE2 | GLN | 44 | 28.586 | 46.056 | 5.621 | 1.00 | 38.09 |
| ATOM | 371 | C | GLN | 44 | 29.635 | 44.774 | -0.220 | 1.00 | 35.22 |
| ATOM | 372 | O | GLN | 44 | 28.657 | 45.279 | -0.769 | 1.00 | 35.57 |
| ATOM | 373 | N | SER | 45 | 30.813 | 44.645 | -0.821 | 1.00 | 36.36 |
| ATOM | 374 | CA | SER | 45 | 31.029 | 45.115 | -2.186 | 1.00 | 37.51 |
| ATOM | 375 | CB | SER | 45 | 32.490 | 45.520 | -2.385 | 1.00 | 36.45 |
| ATOM | 376 | OG | SER | 45 | 32.830 | 46.585 | -1.519 | 1.00 | 37.77 |
| ATOM | 377 | C | SER | 45 | 30.649 | 44.065 | -3.224 | 1.00 | 37.52 |
| ATOM | 378 | O | SER | 45 | 30.433 | 44.390 | -4.394 | 1.00 | 37.81 |
| ATOM | 379 | N | LYS | 46 | 30.581 | 42.805 | -2.804 | 1.00 | 37.01 |
| ATOM | 380 | CA | LYS | 46 | 30.207 | 41.729 | -3.718 | 1.00 | 37.49 |
| ATOM | 381 | CB | LYS | 46 | 30.185 | 40.385 | -2.978 | 1.00 | 38.89 |
| ATOM | 382 | CG | LYS | 46 | 31.554 | 39.830 | -2.619 | 1.00 | 41.39 |
| ATOM | 383 | CD | LYS | 46 | 32.285 | 39.347 | -3.859 | 1.00 | 43.62 |
| ATOM | 384 | CE | LYS | 46 | 33.618 | 38.716 | -3.501 | 1.00 | 45.39 |
| ATOM | 385 | NZ | LYS | 46 | 33.460 | 37.587 | -2.542 | 1.00 | 46.78 |
| ATOM | 386 | C | LYS | 46 | 28.811 | 42.027 | -4.261 | 1.00 | 36.47 |
| ATOM | 387 | O | LYS | 46 | 28.024 | 42.714 | -3.616 | 1.00 | 34.79 |
| ATOM | 388 | N | SER | 47 | 28.505 | 41.526 | -5.450 | 1.00 | 35.94 |
| ATOM | 389 | CA | SER | 47 | 27.181 | 41.741 | -6.011 | 1.00 | 36.64 |
| ATOM | 390 | CB | SER | 47 | 27.149 | 41.341 | -7.486 | 1.00 | 36.69 |
| ATOM | 391 | OG | SER | 47 | 27.443 | 39.964 | -7.636 | 1.00 | 35.57 |
| ATOM | 392 | C | SER | 47 | 26.232 | 40.843 | -5.225 | 1.00 | 37.24 |
| ATOM | 393 | O | SER | 47 | 26.672 | 39.994 | -4.452 | 1.00 | 36.62 |
| ATOM | 394 | N | ARG | 48 | 24.934 | 41.026 | -5.419 | 1.00 | 37.93 |
| ATOM | 395 | CA | ARG | 48 | 23.957 | 40.202 | -4.726 | 1.00 | 37.32 |
| ATOM | 396 | CB | ARG | 48 | 22.551 | 40.762 | -4.966 | 1.00 | 38.56 |
| ATOM | 397 | CG | ARG | 48 | 21.427 | 39.977 | -4.315 | 1.00 | 38.73 |
| ATOM | 398 | CD | ARG | 48 | 20.311 | 40.908 | -3.869 | 1.00 | 39.69 |
| ATOM | 399 | NE | ARG | 48 | 19.091 | 40.181 | -3.530 | 1.00 | 41.75 |
| ATOM | 400 | CZ | ARG | 48 | 18.294 | 39.611 | -4.429 | 1.00 | 43.27 |
| ATOM | 401 | NH1 | ARG | 48 | 18.588 | 39.688 | -5.721 | 1.00 | 43.91 |
| ATOM | 402 | NH2 | ARG | 48 | 17.203 | 38.965 | -4.041 | 1.00 | 41.76 |
| ATOM | 403 | C | ARG | 48 | 24.073 | 38.754 | -5.220 | 1.00 | 36.87 |
| ATOM | 404 | O | ARG | 48 | 23.928 | 37.806 | -4.441 | 1.00 | 35.81 |
| ATOM | 405 | N | GLU | 49 | 24.352 | 38.592 | -6.511 | 1.00 | 34.76 |
| ATOM | 406 | CA | GLU | 49 | 24.501 | 37.268 | -7.113 | 1.00 | 35.71 |
| ATOM | 407 | CB | GLU | 49 | 24.643 | 37.371 | -8.636 | 1.00 | 38.45 |
| ATOM | 408 | CG | GLU | 49 | 23.676 | 38.328 | -9.302 | 1.00 | 44.94 |
| ATOM | 409 | CD | GLU | 49 | 24.015 | 39.782 | -9.028 | 1.00 | 47.01 |
| ATOM | 410 | OE1 | GLU | 49 | 25.099 | 40.231 | -9.460 | 1.00 | 49.86 |
| ATOM | 411 | OE2 | GLU | 49 | 23.201 | 40.476 | -8.380 | 1.00 | 46.92 |
| ATOM | 412 | C | GLU | 49 | 25.751 | 36.591 | -6.568 | 1.00 | 33.86 |
| ATOM | 413 | O | GLU | 49 | 25.731 | 35.414 | -6.213 | 1.00 | 33.86 |
| ATOM | 414 | N | ALA | 50 | 26.843 | 37.344 | -6.511 | 1.00 | 32.28 |
| ATOM | 415 | CA | ALA | 50 | 28.098 | 36.804 | -6.021 | 1.00 | 31.98 |
| ATOM | 416 | CB | ALA | 50 | 29.206 | 37.849 | -6.139 | 1.00 | 30.17 |
| ATOM | 417 | C | ALA | 50 | 27.975 | 36.322 | -4.579 | 1.00 | 31.48 |
| ATOM | 418 | O | ALA | 50 | 28.373 | 35.201 | -4.263 | 1.00 | 29.44 |
| ATOM | 419 | N | LEU | 51 | 27.422 | 37.159 | -3.703 | 1.00 | 30.98 |
| ATOM | 420 | CA | LEU | 51 | 27.289 | 36.762 | -2.305 | 1.00 | 30.66 |
| ATOM | 421 | CB | LEU | 51 | 26.830 | 37.940 | -1.441 | 1.00 | 30.32 |
| ATOM | 422 | CG | LEU | 51 | 26.893 | 37.675 | 0.070 | 1.00 | 32.08 |
| ATOM | 423 | CD1 | LEU | 51 | 28.267 | 37.144 | 0.444 | 1.00 | 32.44 |
| ATOM | 424 | CD2 | LEU | 51 | 26.594 | 38.952 | 0.842 | 1.00 | 32.02 |
| ATOM | 425 | C | LEU | 51 | 26.332 | 35.582 | -2.156 | 1.00 | 29.32 |
| ATOM | 426 | O | LEU | 51 | 26.601 | 34.652 | -1.393 | 1.00 | 28.16 |
| ATOM | 427 | N | TRP | 52 | 25.220 | 35.614 | -2.886 | 1.00 | 28.73 |
| ATOM | 428 | CA | TRP | 52 | 24.261 | 34.520 | -2.835 | 1.00 | 28.59 |
| ATOM | 429 | CB | TRP | 52 | 23.076 | 34.781 | -3.770 | 1.00 | 30.93 |
| ATOM | 430 | CG | TRP | 52 | 21.878 | 35.296 | -3.050 | 1.00 | 35.78 |
| ATOM | 431 | CD2 | TRP | 52 | 20.905 | 34.513 | -2.351 | 1.00 | 39.02 |
| ATOM | 432 | CE2 | TRP | 52 | 19.992 | 35.413 | -1.759 | 1.00 | 39.44 |
| ATOM | 433 | CE3 | TRP | 52 | 20.720 | 33.135 | -2.161 | 1.00 | 40.28 |
| ATOM | 434 | CD1 | TRP | 52 | 21.524 | 36.602 | -2.865 | 1.00 | 35.55 |
| ATOM | 435 | NE1 | TRP | 52 | 20.391 | 36.681 | -2.089 | 1.00 | 38.68 |
| ATOM | 436 | CZ2 | TRP | 52 | 18.905 | 34.980 | -0.991 | 1.00 | 42.34 |
| ATOM | 437 | CZ3 | TRP | 52 | 19.644 | 32.705 | -1.398 | 1.00 | 41.70 |
| ATOM | 438 | CH2 | TRP | 52 | 18.749 | 33.626 | -0.821 | 1.00 | 42.77 |
| ATOM | 439 | C | TRP | 52 | 24.929 | 33.213 | -3.231 | 1.00 | 27.04 |
| ATOM | 440 | O | TRP | 52 | 24.704 | 32.182 | -2.609 | 1.00 | 26.75 |
| ATOM | 441 | N | GLN | 53 | 25.742 | 33.259 | -4.279 | 1.00 | 26.75 |
| ATOM | 442 | CA | GLN | 53 | 26.446 | 32.071 | -4.740 | 1.00 | 27.50 |
| ATOM | 443 | CB | GLN | 53 | 27.209 | 32.391 | -6.031 | 1.00 | 31.81 |
| ATOM | 444 | CG | GLN | 53 | 27.894 | 31.198 | -6.678 | 1.00 | 36.92 |
| ATOM | 445 | CD | GLN | 53 | 28.277 | 31.456 | -8.137 | 1.00 | 42.85 |
| ATOM | 446 | OE1 | GLN | 53 | 28.893 | 30.609 | -8.786 | 1.00 | 43.92 |
| ATOM | 447 | NE2 | GLN | 53 | 27.904 | 32.628 | -8.657 | 1.00 | 42.90 |
| ATOM | 448 | C | GLN | 53 | 27.403 | 31.558 | -3.655 | 1.00 | 26.49 |
| ATOM | 449 | O | GLN | 53 | 27.530 | 30.354 | -3.449 | 1.00 | 24.81 |
| ATOM | 450 | N | GLN | 54 | 28.071 | 32.473 | -2.960 | 1.00 | 25.88 |
| ATOM | 451 | CA | GLN | 54 | 28.992 | 32.094 | -1.890 | 1.00 | 27.32 |
| ATOM | 452 | CB | GLN | 54 | 29.727 | 33.323 | -1.343 | 1.00 | 31.35 |
| ATOM | 453 | CG | GLN | 54 | 30.846 | 33.861 | -2.229 | 1.00 | 37.27 |
| ATOM | 454 | CD | GLN | 54 | 31.493 | 35.112 | -1.645 | 1.00 | 41.33 |
| ATOM | 455 | OE1 | GLN | 54 | 31.105 | 36.239 | -1.969 | 1.00 | 44.63 |
| ATOM | 456 | NE2 | GLN | 54 | 32.474 | 34.916 | -0.766 | 1.00 | 40.57 |
| ATOM | 457 | C | GLN | 54 | 28.210 | 31.445 | -0.754 | 1.00 | 26.99 |
| ATOM | 458 | O | GLN | 54 | 28.621 | 30.423 | -0.201 | 1.00 | 25.42 |
| ATOM | 459 | N | CYS | 55 | 27.084 | 32.060 | -0.402 | 1.00 | 26.01 |
| ATOM | 460 | CA | CYS | 55 | 26.236 | 31.550 | 0.663 | 1.00 | 25.09 |
| ATOM | 461 | CB | CYS | 55 | 25.097 | 32.536 | 0.935 | 1.00 | 26.44 |
| ATOM | 462 | SG | CYS | 55 | 25.637 | 34.013 | 1.827 | 1.00 | 26.26 |
| ATOM | 463 | C | CYS | 55 | 25.673 | 30.175 | 0.304 | 1.00 | 24.90 |
| ATOM | 464 | O | CYS | 55 | 25.603 | 29.284 | 1.151 | 1.00 | 23.90 |
| ATOM | 465 | N | ALA | 56 | 25.287 | 30.011 | -0.958 | 1.00 | 22.85 |
| ATOM | 466 | CA | ALA | 56 | 24.733 | 28.752 | -1.438 | 1.00 | 22.57 |
| ATOM | 467 | CB | ALA | 56 | 24.310 | 28.903 | -2.878 | 1.00 | 18.31 |
| ATOM | 468 | C | ALA | 56 | 25.765 | 27.625 | -1.305 | 1.00 | 23.49 |
| ATOM | 469 | O | ALA | 56 | 25.425 | 26.499 | -0.967 | 1.00 | 24.70 |
| ATOM | 470 | N | ILE | 57 | 27.027 | 27.937 | -1.575 | 1.00 | 24.87 |
| ATOM | 471 | CA | ILE | 57 | 28.094 | 26.945 | -1.471 | 1.00 | 26.21 |
| ATOM | 472 | CB | ILE | 57 | 29.390 | 27.479 | -2.104 | 1.00 | 27.82 |
| ATOM | 473 | CG2 | ILE | 57 | 30.597 | 26.671 | -1.616 | 1.00 | 29.94 |
| ATOM | 474 | CG1 | ILE | 57 | 29.260 | 27.432 | -3.630 | 1.00 | 28.92 |
| ATOM | 475 | CD1 | ILE | 57 | 30.484 | 27.913 | -4.374 | 1.00 | 31.67 |
| ATOM | 476 | C | ILE | 57 | 28.348 | 26.554 | -0.012 | 1.00 | 27.09 |
| ATOM | 477 | O | ILE | 57 | 28.465 | 25.370 | 0.306 | 1.00 | 27.29 |
| ATOM | 478 | N | GLN | 58 | 28.416 | 27.547 | 0.872 | 1.00 | 26.96 |
| ATOM | 479 | CA | GLN | 58 | 28.637 | 27.289 | 2.291 | 1.00 | 28.94 |
| ATOM | 480 | CB | GLN | 58 | 28.761 | 28.613 | 3.056 | 1.00 | 31.47 |
| ATOM | 481 | CG | GLN | 58 | 28.855 | 28.471 | 4.571 | 1.00 | 37.58 |
| ATOM | 482 | CD | GLN | 58 | 29.890 | 27.439 | 5.012 | 1.00 | 43.52 |
| ATOM | 483 | OE1 | GLN | 58 | 31.043 | 27.458 | 4.557 | 1.00 | 45.14 |
| ATOM | 484 | NE2 | GLN | 58 | 29.482 | 26.536 | 5.907 | 1.00 | 40.77 |
| ATOM | 485 | C | GLN | 58 | 27.495 | 26.449 | 2.866 | 1.00 | 25.89 |
| ATOM | 486 | O | GLN | 58 | 27.725 | 25.506 | 3.619 | 1.00 | 25.53 |
| ATOM | 487 | N | ILE | 59 | 26.264 | 26.794 | 2.511 | 1.00 | 24.98 |
| ATOM | 488 | CA | ILE | 59 | 25.104 | 26.049 | 2.985 | 1.00 | 22.92 |
| ATOM | 489 | CB | ILE | 59 | 23.787 | 26.717 | 2.531 | 1.00 | 23.63 |
| ATOM | 490 | CG2 | ILE | 59 | 22.617 | 25.752 | 2.715 | 1.00 | 21.17 |
| ATOM | 491 | CG1 | ILE | 59 | 23.570 | 28.021 | 3.307 | 1.00 | 22.62 |
| ATOM | 492 | CD1 | ILE | 59 | 22.311 | 28.781 | 2.879 | 1.00 | 23.75 |
| ATOM | 493 | C | ILE | 59 | 25.133 | 24.618 | 2.448 | 1.00 | 23.06 |
| ATOM | 494 | O | ILE | 59 | 24.867 | 23.668 | 3.181 | 1.00 | 21.94 |
| ATOM | 495 | N | THR | 60 | 25.445 | 24.470 | 1.163 | 1.00 | 23.15 |
| ATOM | 496 | CA | THR | 60 | 25.510 | 23.149 | 0.552 | 1.00 | 23.53 |
| ATOM | 497 | CB | THR | 60 | 25.837 | 23.243 | -0.956 | 1.00 | 22.65 |
| ATOM | 498 | OG1 | THR | 60 | 24.787 | 23.946 | -1.631 | 1.00 | 24.06 |
| ATOM | 499 | CG2 | THR | 60 | 25.986 | 21.854 | -1.562 | 1.00 | 22.93 |
| ATOM | 500 | C | THR | 60 | 26.596 | 22.333 | 1.244 | 1.00 | 22.48 |
| ATOM | 501 | O | THR | 60 | 26.423 | 21.148 | 1.514 | 1.00 | 24.18 |
| ATOM | 502 | N | HIS | 61 | 27.715 | 22.984 | 1.537 | 1.00 | 23.04 |
| ATOM | 503 | CA | HIS | 61 | 28.837 | 22.324 | 2.194 | 1.00 | 22.43 |
| ATOM | 504 | CB | HIS | 61 | 29.958 | 23.346 | 2.401 | 1.00 | 23.21 |
| ATOM | 505 | CG | HIS | 61 | 31.257 | 22.753 | 2.846 | 1.00 | 26.52 |
| ATOM | 506 | CD2 | HIS | 61 | 32.298 | 23.298 | 3.520 | 1.00 | 28.76 |
| ATOM | 507 | ND1 | HIS | 61 | 31.623 | 21.455 | 2.566 | 1.00 | 28.62 |
| ATOM | 508 | CE1 | HIS | 61 | 32.830 | 21.224 | 3.049 | 1.00 | 27.83 |
| ATOM | 509 | NE2 | HIS | 61 | 33.263 | 22.326 | 3.634 | 1.00 | 29.16 |
| ATOM | 510 | C | HIS | 61 | 28.356 | 21.753 | 3.528 | 1.00 | 22.71 |
| ATOM | 511 | O | HIS | 61 | 28.583 | 20.582 | 3.837 | 1.00 | 22.54 |
| ATOM | 512 | N | ALA | 62 | 27.659 | 22.579 | 4.297 | 1.00 | 21.43 |
| ATOM | 513 | CA | ALA | 62 | 27.141 | 22.168 | 5.596 | 1.00 | 22.12 |
| ATOM | 514 | CB | ALA | 62 | 26.597 | 23.392 | 6.349 | 1.00 | 17.66 |
| ATOM | 515 | C | ALA | 62 | 26.059 | 21.088 | 5.480 | 1.00 | 21.21 |
| ATOM | 516 | O | ALA | 62 | 25.959 | 20.208 | 6.340 | 1.00 | 22.10 |
| ATOM | 517 | N | ILE | 63 | 25.247 | 21.157 | 4.428 | 1.00 | 21.36 |
| ATOM | 518 | CA | ILE | 63 | 24.185 | 20.172 | 4.221 | 1.00 | 21.14 |
| ATOM | 519 | CB | ILE | 63 | 23.239 | 20.582 | 3.075 | 1.00 | 20.43 |
| ATOM | 520 | CG2 | ILE | 63 | 22.333 | 19.429 | 2.711 | 1.00 | 20.13 |
| ATOM | 521 | CG1 | ILE | 63 | 22.402 | 21.795 | 3.493 | 1.00 | 20.25 |
| ATOM | 522 | CD1 | ILE | 63 | 21.416 | 22.260 | 2.425 | 1.00 | 22.13 |
| ATOM | 523 | C | ILE | 63 | 24.773 | 18.804 | 3.901 | 1.00 | 22.25 |
| ATOM | 524 | O | ILE | 63 | 24.238 | 17.779 | 4.323 | 1.00 | 22.39 |
| ATOM | 525 | N | GLN | 64 | 25.875 | 18.787 | 3.155 | 1.00 | 22.82 |
| ATOM | 526 | CA | GLN | 64 | 26.517 | 17.528 | 2.801 | 1.00 | 22.75 |
| ATOM | 527 | CB | GLN | 64 | 27.700 | 17.782 | 1.846 | 1.00 | 21.87 |
| ATOM | 528 | CG | GLN | 64 | 27.200 | 18.212 | 0.465 | 1.00 | 24.19 |
| ATOM | 529 | CD | GLN | 64 | 28.293 | 18.402 | -0.582 | 1.00 | 26.74 |
| ATOM | 530 | OE1 | GLN | 64 | 28.005 | 18.432 | -1.783 | 1.00 | 29.29 |
| ATOM | 531 | NE2 | GLN | 64 | 29.536 | 18.541 | -0.140 | 1.00 | 22.66 |
| ATOM | 532 | C | GLN | 64 | 26.950 | 16.798 | 4.064 | 1.00 | 22.10 |
| ATOM | 533 | O | GLN | 64 | 26.872 | 15.575 | 4.139 | 1.00 | 21.93 |
| ATOM | 534 | N | TYR | 65 | 27.385 | 17.554 | 5.064 | 1.00 | 22.47 |
| ATOM | 535 | CA | TYR | 65 | 27.791 | 16.964 | 6.335 | 1.00 | 23.86 |
| ATOM | 536 | CB | TYR | 65 | 28.572 | 17.980 | 7.171 | 1.00 | 26.62 |
| ATOM | 537 | CG | TYR | 65 | 30.050 | 18.030 | 6.823 | 1.00 | 30.04 |
| ATOM | 538 | CD1 | TYR | 65 | 30.895 | 16.963 | 7.140 | 1.00 | 32.17 |
| ATOM | 539 | CE1 | TYR | 65 | 32.252 | 16.986 | 6.807 | 1.00 | 33.14 |
| ATOM | 540 | CD2 | TYR | 65 | 30.599 | 19.128 | 6.160 | 1.00 | 30.53 |
| ATOM | 541 | CE2 | TYR | 65 | 31.957 | 19.161 | 5.818 | 1.00 | 33.67 |
| ATOM | 542 | CZ | TYR | 65 | 32.777 | 18.086 | 6.147 | 1.00 | 36.28 |
| ATOM | 543 | OH | TYR | 65 | 34.122 | 18.109 | 5.821 | 1.00 | 40.50 |
| ATOM | 544 | C | TYR | 65 | 26.581 | 16.449 | 7.120 | 1.00 | 22.30 |
| ATOM | 545 | O | TYR | 65 | 26.702 | 15.498 | 7.888 | 1.00 | 23.33 |
| ATOM | 546 | N | VAL | 66 | 25.417 | 17.071 | 6.938 | 1.00 | 20.36 |
| ATOM | 547 | CA | VAL | 66 | 24.222 | 16.598 | 7.637 | 1.00 | 20.83 |
| ATOM | 548 | CB | VAL | 66 | 23.020 | 17.574 | 7.502 | 1.00 | 18.86 |
| ATOM | 549 | CG1 | VAL | 66 | 21.784 | 16.965 | 8.176 | 1.00 | 19.22 |
| ATOM | 550 | CG2 | VAL | 66 | 23.348 | 18.914 | 8.159 | 1.00 | 16.99 |
| ATOM | 551 | C | VAL | 66 | 23.830 | 15.246 | 7.040 | 1.00 | 20.81 |
| ATOM | 552 | O | VAL | 66 | 23.338 | 14.370 | 7.740 | 1.00 | 22.04 |
| ATOM | 553 | N | VAL | 67 | 24.048 | 15.085 | 5.739 | 1.00 | 22.31 |
| ATOM | 554 | CA | VAL | 67 | 23.740 | 13.830 | 5.062 | 1.00 | 21.44 |
| ATOM | 555 | CB | VAL | 67 | 23.989 | 13.943 | 3.544 | 1.00 | 22.66 |
| ATOM | 556 | CG1 | VAL | 67 | 23.770 | 12.593 | 2.872 | 1.00 | 22.40 |
| ATOM | 557 | CG2 | VAL | 67 | 23.047 | 14.992 | 2.939 | 1.00 | 23.72 |
| ATOM | 558 | C | VAL | 67 | 24.624 | 12.726 | 5.653 | 1.00 | 22.20 |
| ATOM | 559 | O | VAL | 67 | 24.160 | 11.616 | 5.913 | 1.00 | 22.69 |
| ATOM | 560 | N | GLU | 68 | 25.896 | 13.040 | 5.879 | 1.00 | 23.93 |
| ATOM | 561 | CA | GLU | 68 | 26.825 | 12.072 | 6.463 | 1.00 | 25.73 |
| ATOM | 562 | CB | GLU | 68 | 28.241 | 12.652 | 6.483 | 1.00 | 31.22 |
| ATOM | 563 | CG | GLU | 68 | 28.790 | 12.972 | 5.097 | 1.00 | 35.81 |
| ATOM | 564 | CD | GLU | 68 | 29.333 | 11.745 | 4.375 | 1.00 | 42.48 |
| ATOM | 565 | OE1 | GLU | 68 | 28.652 | 10.692 | 4.362 | 1.00 | 42.24 |
| ATOM | 566 | OE2 | GLU | 68 | 30.449 | 11.841 | 3.808 | 1.00 | 45.88 |
| ATOM | 567 | C | GLU | 68 | 26.366 | 11.749 | 7.882 | 1.00 | 25.40 |
| ATOM | 568 | O | GLU | 68 | 26.432 | 10.597 | 8.322 | 1.00 | 23.68 |
| ATOM | 569 | N | PHE | 69 | 25.896 | 12.776 | 8.590 | 1.00 | 23.61 |
| ATOM | 570 | CA | PHE | 69 | 25.386 | 12.633 | 9.956 | 1.00 | 22.83 |
| ATOM | 571 | CB | PHE | 69 | 24.885 | 14.001 | 10.448 | 1.00 | 21.69 |
| ATOM | 572 | CG | PHE | 69 | 24.252 | 13.989 | 11.822 | 1.00 | 24.10 |
| ATOM | 573 | CD1 | PHE | 69 | 24.903 | 13.412 | 12.912 | 1.00 | 21.29 |
| ATOM | 574 | CD2 | PHE | 69 | 23.018 | 14.613 | 12.034 | 1.00 | 23.32 |
| ATOM | 575 | CE1 | PHE | 69 | 24.341 | 13.459 | 14.184 | 1.00 | 22.08 |
| ATOM | 576 | CE2 | PHE | 69 | 22.444 | 14.667 | 13.308 | 1.00 | 20.43 |
| ATOM | 577 | CZ | PHE | 69 | 23.106 | 14.089 | 14.386 | 1.00 | 22.91 |
| ATOM | 578 | C | PHE | 69 | 24.253 | 11.598 | 9.967 | 1.00 | 23.85 |
| ATOM | 579 | O | PHE | 69 | 24.265 | 10.659 | 10.764 | 1.00 | 25.22 |
| ATOM | 580 | N | ALA | 70 | 23.292 | 11.757 | 9.058 | 1.00 | 23.28 |
| ATOM | 581 | CA | ALA | 70 | 22.151 | 10.851 | 8.962 | 1.00 | 22.31 |
| ATOM | 582 | CB | ALA | 70 | 21.163 | 11.382 | 7.926 | 1.00 | 22.02 |
| ATOM | 583 | C | ALA | 70 | 22.538 | 9.406 | 8.618 | 1.00 | 22.95 |
| ATOM | 584 | O | ALA | 70 | 22.011 | 8.457 | 9.205 | 1.00 | 21.88 |
| ATOM | 585 | N | LYS | 71 | 23.449 | 9.241 | 7.664 | 1.00 | 23.24 |
| ATOM | 586 | CA | LYS | 71 | 23.889 | 7.911 | 7.249 | 1.00 | 26.69 |
| ATOM | 587 | CB | LYS | 71 | 24.853 | 8.026 | 6.065 | 1.00 | 27.02 |
| ATOM | 588 | CG | LYS | 71 | 24.246 | 8.701 | 4.848 | 1.00 | 28.09 |
| ATOM | 589 | CD | LYS | 71 | 25.300 | 9.022 | 3.802 | 1.00 | 29.71 |
| ATOM | 590 | CE | LYS | 71 | 25.970 | 7.770 | 3.257 | 1.00 | 30.06 |
| ATOM | 591 | NZ | LYS | 71 | 26.968 | 8.147 | 2.221 | 1.00 | 33.92 |
| ATOM | 592 | C | LYS | 71 | 24.557 | 7.114 | 8.379 | 1.00 | 26.63 |
| ATOM | 593 | O | LYS | 71 | 24.536 | 5.880 | 8.370 | 1.00 | 26.95 |
| ATOM | 594 | N | ARG | 72 | 25.144 | 7.808 | 9.347 | 1.00 | 24.99 |
| ATOM | 595 | CA | ARG | 72 | 25.804 | 7.120 | 10.449 | 1.00 | 27.31 |
| ATOM | 596 | CB | ARG | 72 | 27.051 | 7.892 | 10.880 | 1.00 | 28.59 |
| ATOM | 597 | CG | ARG | 72 | 28.031 | 8.064 | 9.732 | 1.00 | 29.69 |
| ATOM | 598 | CD | ARG | 72 | 29.365 | 8.593 | 10.180 | 1.00 | 31.03 |
| ATOM | 599 | NE | ARG | 72 | 30.268 | 8.732 | 9.044 | 1.00 | 33.59 |
| ATOM | 600 | CZ | ARG | 72 | 31.546 | 9.081 | 9.145 | 1.00 | 38.36 |
| ATOM | 601 | NH1 | ARG | 72 | 32.076 | 9.326 | 10.338 | 1.00 | 39.73 |
| ATOM | 602 | NH2 | ARG | 72 | 32.291 | 9.194 | 8.054 | 1.00 | 39.15 |
| ATOM | 603 | C | ARG | 72 | 24.891 | 6.874 | 11.643 | 1.00 | 27.40 |
| ATOM | 604 | O | ARG | 72 | 25.333 | 6.367 | 12.675 | 1.00 | 29.05 |
| ATOM | 605 | N | ILE | 73 | 23.618 | 7.243 | 11.504 | 1.00 | 26.22 |
| ATOM | 606 | CA | ILE | 73 | 22.646 | 7.015 | 12.566 | 1.00 | 24.04 |
| ATOM | 607 | CB | ILE | 73 | 21.631 | 8.178 | 12.705 | 1.00 | 22.57 |
| ATOM | 608 | CG2 | ILE | 73 | 20.568 | 7.810 | 13.744 | 1.00 | 23.16 |
| ATOM | 609 | CG1 | ILE | 73 | 22.352 | 9.463 | 13.124 | 1.00 | 23.44 |
| ATOM | 610 | CD1 | ILE | 73 | 21.412 | 10.645 | 13.411 | 1.00 | 23.02 |
| ATOM | 611 | C | ILE | 73 | 21.884 | 5.747 | 12.205 | 1.00 | 25.40 |
| ATOM | 612 | O | ILE | 73 | 21.067 | 5.737 | 11.276 | 1.00 | 23.63 |
| ATOM | 613 | N | THR | 74 | 22.155 | 4.679 | 12.947 | 1.00 | 25.29 |
| ATOM | 614 | CA | THR | 74 | 21.521 | 3.388 | 12.712 | 1.00 | 27.03 |
| ATOM | 615 | CB | THR | 74 | 21.790 | 2.433 | 13.878 | 1.00 | 29.63 |
| ATOM | 616 | OG1 | THR | 74 | 23.157 | 2.555 | 14.286 | 1.00 | 33.21 |
| ATOM | 617 | CG2 | THR | 74 | 21.531 | 0.998 | 13.445 | 1.00 | 33.49 |
| ATOM | 618 | C | THR | 74 | 20.007 | 3.466 | 12.510 | 1.00 | 25.59 |
| ATOM | 619 | O | THR | 74 | 19.479 | 2.985 | 11.508 | 1.00 | 25.68 |
| ATOM | 620 | N | GLY | 75 | 19.315 | 4.063 | 13.472 | 1.00 | 26.41 |
| ATOM | 621 | CA | GLY | 75 | 17.868 | 4.181 | 13.388 | 1.00 | 25.95 |
| ATOM | 622 | C | GLY | 75 | 17.382 | 4.896 | 12.139 | 1.00 | 25.86 |
| ATOM | 623 | O | GLY | 75 | 16.324 | 4.563 | 11.603 | 1.00 | 25.03 |
| ATOM | 624 | N | PHE | 76 | 18.146 | 5.877 | 11.667 | 1.00 | 24.84 |
| ATOM | 625 | CA | PHE | 76 | 17.752 | 6.612 | 10.466 | 1.00 | 25.41 |
| ATOM | 626 | CB | PHE | 76 | 18.643 | 7.847 | 10.259 | 1.00 | 23.08 |
| ATOM | 627 | CG | PHE | 76 | 18.319 | 8.634 | 9.005 | 1.00 | 25.15 |
| ATOM | 628 | CD1 | PHE | 76 | 18.889 | 8.291 | 7.778 | 1.00 | 25.38 |
| ATOM | 629 | CD2 | PHE | 76 | 17.443 | 9.714 | 9.050 | 1.00 | 23.74 |
| ATOM | 630 | CE1 | PHE | 76 | 18.592 | 9.015 | 6.621 | 1.00 | 25.08 |
| ATOM | 631 | CE2 | PHE | 76 | 17.140 | 10.445 | 7.891 | 1.00 | 24.62 |
| ATOM | 632 | CZ | PHE | 76 | 17.717 | 10.092 | 6.680 | 1.00 | 22.76 |
| ATOM | 633 | C | PHE | 76 | 17.828 | 5.717 | 9.240 | 1.00 | 24.88 |
| ATOM | 634 | O | PHE | 76 | 16.888 | 5.665 | 8.447 | 1.00 | 24.05 |
| ATOM | 635 | N | MET | 77 | 18.943 | 5.007 | 9.088 | 1.00 | 25.71 |
| ATOM | 636 | CA | MET | 77 | 19.123 | 4.136 | 7.937 | 1.00 | 26.70 |
| ATOM | 637 | CB | MET | 77 | 20.576 | 3.655 | 7.852 | 1.00 | 28.75 |
| ATOM | 638 | CG | MET | 77 | 21.577 | 4.773 | 7.554 | 1.00 | 30.31 |
| ATOM | 639 | SD | MET | 77 | 21.118 | 5.827 | 6.136 | 1.00 | 31.94 |
| ATOM | 640 | CE | MET | 77 | 21.818 | 4.891 | 4.717 | 1.00 | 35.36 |
| ATOM | 641 | C | MET | 77 | 18.164 | 2.947 | 7.920 | 1.00 | 28.58 |
| ATOM | 642 | O | MET | 77 | 18.071 | 2.233 | 6.919 | 1.00 | 28.47 |
| ATOM | 643 | N | GLU | 78 | 17.449 | 2.737 | 9.022 | 1.00 | 29.72 |
| ATOM | 644 | CA | GLU | 78 | 16.477 | 1.652 | 9.091 | 1.00 | 30.71 |
| ATOM | 645 | CB | GLU | 78 | 16.312 | 1.153 | 10.525 | 1.00 | 32.46 |
| ATOM | 646 | CG | GLU | 78 | 17.393 | 0.187 | 10.966 | 1.00 | 38.31 |
| ATOM | 647 | CD | GLU | 78 | 17.235 | -0.231 | 12.411 | 1.00 | 40.71 |
| ATOM | 648 | OE1 | GLU | 78 | 16.118 | -0.633 | 12.794 | 1.00 | 43.62 |
| ATOM | 649 | OE2 | GLU | 78 | 18.226 | -0.160 | 13.163 | 1.00 | 43.48 |
| ATOM | 650 | C | GLU | 78 | 15.129 | 2.121 | 8.558 | 1.00 | 31.42 |
| ATOM | 651 | O | GLU | 78 | 14.226 | 1.315 | 8.336 | 1.00 | 29.99 |
| ATOM | 652 | N | LEU | 79 | 14.984 | 3.428 | 8.368 | 1.00 | 30.08 |
| ATOM | 653 | CA | LEU | 79 | 13.735 | 3.958 | 7.832 | 1.00 | 29.88 |
| ATOM | 654 | CB | LEU | 79 | 13.658 | 5.476 | 8.044 | 1.00 | 25.44 |
| ATOM | 655 | CG | LEU | 79 | 13.653 | 5.967 | 9.495 | 1.00 | 25.48 |
| ATOM | 656 | CD1 | LEU | 79 | 13.735 | 7.483 | 9.516 | 1.00 | 23.35 |
| ATOM | 657 | CD2 | LEU | 79 | 12.392 | 5.480 | 10.219 | 1.00 | 24.40 |
| ATOM | 658 | C | LEU | 79 | 13.745 | 3.633 | 6.339 | 1.00 | 29.03 |
| ATOM | 659 | O | LEU | 79 | 14.815 | 3.562 | 5.735 | 1.00 | 26.93 |
| ATOM | 660 | N | CYS | 80 | 12.573 | 3.419 | 5.744 | 1.00 | 30.41 |
| ATOM | 661 | CA | CYS | 80 | 12.524 | 3.114 | 4.314 | 1.00 | 32.22 |
| ATOM | 662 | CB | CYS | 80 | 11.081 | 2.931 | 3.830 | 1.00 | 33.77 |
| ATOM | 663 | SG | CYS | 80 | 10.113 | 4.443 | 3.665 | 1.00 | 35.11 |
| ATOM | 664 | C | CYS | 80 | 13.182 | 4.281 | 3.582 | 1.00 | 32.79 |
| ATOM | 665 | O | CYS | 80 | 13.107 | 5.432 | 4.032 | 1.00 | 31.43 |
| ATOM | 666 | N | GLN | 81 | 13.825 | 3.986 | 2.458 | 1.00 | 32.81 |
| ATOM | 667 | CA | GLN | 81 | 14.533 | 5.009 | 1.694 | 1.00 | 33.38 |
| ATOM | 668 | CB | GLN | 81 | 15.144 | 4.401 | 0.433 | 1.00 | 36.20 |
| ATOM | 669 | CG | GLN | 81 | 15.988 | 5.382 | -0.353 | 1.00 | 40.19 |
| ATOM | 670 | CD | GLN | 81 | 16.894 | 4.693 | -1.346 | 1.00 | 42.67 |
| ATOM | 671 | OE1 | GLN | 81 | 16.429 | 4.055 | -2.295 | 1.00 | 44.22 |
| ATOM | 672 | NE2 | GLN | 81 | 18.201 | 4.808 | -1.128 | 1.00 | 42.74 |
| ATOM | 673 | C | GLN | 81 | 13.702 | 6.225 | 1.317 | 1.00 | 32.60 |
| ATOM | 674 | O | GLN | 81 | 14.218 | 7.346 | 1.286 | 1.00 | 31.70 |
| ATOM | 675 | N | ASN | 82 | 12.423 | 6.017 | 1.019 | 1.00 | 30.05 |
| ATOM | 676 | CA | ASN | 82 | 11.571 | 7.139 | 0.655 | 1.00 | 29.42 |
| ATOM | 677 | CB | ASN | 82 | 10.160 | 6.665 | 0.317 | 1.00 | 30.85 |
| ATOM | 678 | CG | ASN | 82 | 9.211 | 7.818 | 0.086 | 1.00 | 33.27 |
| ATOM | 679 | OD1 | ASN | 82 | 8.605 | 8.345 | 1.026 | 1.00 | 35.99 |
| ATOM | 680 | ND2 | ASN | 82 | 9.091 | 8.236 | -1.168 | 1.00 | 34.74 |
| ATOM | 681 | C | ASN | 82 | 11.508 | 8.175 | 1.775 | 1.00 | 27.40 |
| ATOM | 682 | O | ASN | 82 | 11.635 | 9.372 | 1.527 | 1.00 | 26.11 |
| ATOM | 683 | N | ASP | 83 | 11.310 | 7.716 | 3.008 | 1.00 | 26.77 |
| ATOM | 684 | CA | ASP | 83 | 11.236 | 8.635 | 4.140 | 1.00 | 25.04 |
| ATOM | 685 | CB | ASP | 83 | 10.629 | 7.921 | 5.354 | 1.00 | 24.28 |
| ATOM | 686 | CG | ASP | 83 | 9.136 | 7.649 | 5.175 | 1.00 | 25.67 |
| ATOM | 687 | OD1 | ASP | 83 | 8.564 | 8.116 | 4.166 | 1.00 | 26.27 |
| ATOM | 688 | OD2 | ASP | 83 | 8.531 | 6.978 | 6.034 | 1.00 | 25.59 |
| ATOM | 689 | C | ASP | 83 | 12.594 | 9.253 | 4.471 | 1.00 | 23.54 |
| ATOM | 690 | O | ASP | 83 | 12.663 | 10.407 | 4.886 | 1.00 | 24.66 |
| ATOM | 691 | N | GLN | 84 | 13.672 | 8.497 | 4.276 | 1.00 | 22.13 |
| ATOM | 692 | CA | GLN | 84 | 15.008 | 9.029 | 4.518 | 1.00 | 23.18 |
| ATOM | 693 | CB | GLN | 84 | 16.076 | 8.012 | 4.118 | 1.00 | 22.97 |
| ATOM | 694 | CG | GLN | 84 | 16.201 | 6.783 | 5.019 | 1.00 | 24.40 |
| ATOM | 695 | CD | GLN | 84 | 17.276 | 5.826 | 4.511 | 1.00 | 23.49 |
| ATOM | 696 | OE1 | GLN | 84 | 18.170 | 6.233 | 3.777 | 1.00 | 24.96 |
| ATOM | 697 | NE2 | GLN | 84 | 17.202 | 4.563 | 4.916 | 1.00 | 22.35 |
| ATOM | 698 | C | GLN | 84 | 15.176 | 10.290 | 3.663 | 1.00 | 24.86 |
| ATOM | 699 | O | GLN | 84 | 15.624 | 11.331 | 4.147 | 1.00 | 24.41 |
| ATOM | 700 | N | ILE | 85 | 14.819 | 10.175 | 2.384 | 1.00 | 24.70 |
| ATOM | 701 | CA | ILE | 85 | 14.908 | 11.283 | 1.433 | 1.00 | 24.80 |
| ATOM | 702 | CB | ILE | 85 | 14.426 | 10.838 | 0.013 | 1.00 | 26.48 |
| ATOM | 703 | CG2 | ILE | 85 | 14.299 | 12.043 | -0.917 | 1.00 | 23.15 |
| ATOM | 704 | CG1 | ILE | 85 | 15.410 | 9.827 | -0.588 | 1.00 | 26.97 |
| ATOM | 705 | CD1 | ILE | 85 | 16.768 | 10.394 | -0.861 | 1.00 | 29.45 |
| ATOM | 706 | C | ILE | 85 | 14.067 | 12.473 | 1.905 | 1.00 | 24.78 |
| ATOM | 707 | O | ILE | 85 | 14.539 | 13.614 | 1.914 | 1.00 | 25.40 |
| ATOM | 708 | N | LEU | 86 | 12.829 | 12.203 | 2.310 | 1.00 | 23.18 |
| ATOM | 709 | CA | LEU | 86 | 11.935 | 13.259 | 2.784 | 1.00 | 22.88 |
| ATOM | 710 | CB | LEU | 86 | 10.554 | 12.687 | 3.114 | 1.00 | 22.96 |
| ATOM | 711 | CG | LEU | 86 | 9.712 | 12.313 | 1.902 | 1.00 | 28.74 |
| ATOM | 712 | CD1 | LEU | 86 | 8.410 | 11.644 | 2.366 | 1.00 | 27.61 |
| ATOM | 713 | CD2 | LEU | 86 | 9.425 | 13.575 | 1.084 | 1.00 | 29.85 |
| ATOM | 714 | C | LEU | 86 | 12.480 | 13.973 | 4.013 | 1.00 | 21.25 |
| ATOM | 715 | O | LEU | 86 | 12.448 | 15.193 | 4.089 | 1.00 | 21.77 |
| ATOM | 716 | N | LEU | 87 | 12.965 | 13.213 | 4.986 | 1.00 | 22.23 |
| ATOM | 717 | CA | LEU | 87 | 13.510 | 13.821 | 6.196 | 1.00 | 20.76 |
| ATOM | 718 | CB | LEU | 87 | 13.946 | 12.736 | 7.186 | 1.00 | 20.64 |
| ATOM | 719 | CG | LEU | 87 | 12.830 | 11.876 | 7.789 | 1.00 | 20.81 |
| ATOM | 720 | CD1 | LEU | 87 | 13.425 | 10.787 | 8.681 | 1.00 | 21.55 |
| ATOM | 721 | CD2 | LEU | 87 | 11.901 | 12.762 | 8.590 | 1.00 | 21.01 |
| ATOM | 722 | C | LEU | 87 | 14.696 | 14.727 | 5.860 | 1.00 | 21.15 |
| ATOM | 723 | O | LEU | 87 | 14.808 | 15.842 | 6.367 | 1.00 | 21.87 |
| ATOM | 724 | N | LEU | 88 | 15.585 | 14.246 | 5.003 | 1.00 | 20.92 |
| ATOM | 725 | CA | LEU | 88 | 16.744 | 15.039 | 4.624 | 1.00 | 22.07 |
| ATOM | 726 | CB | LEU | 88 | 17.730 | 14.172 | 3.837 | 1.00 | 20.79 |
| ATOM | 727 | CG | LEU | 88 | 18.572 | 13.279 | 4.759 | 1.00 | 24.02 |
| ATOM | 728 | CD1 | LEU | 88 | 19.333 | 12.241 | 3.956 | 1.00 | 21.15 |
| ATOM | 729 | CD2 | LEU | 88 | 19.534 | 14.159 | 5.556 | 1.00 | 20.95 |
| ATOM | 730 | C | LEU | 88 | 16.380 | 16.293 | 3.831 | 1.00 | 22.47 |
| ATOM | 731 | O | LEU | 88 | 16.876 | 17.380 | 4.121 | 1.00 | 22.21 |
| ATOM | 732 | N | LYS | 89 | 15.505 | 16.153 | 2.841 | 1.00 | 24.00 |
| ATOM | 733 | CA | LYS | 89 | 15.106 | 17.303 | 2.028 | 1.00 | 25.58 |
| ATOM | 734 | CB | LYS | 89 | 14.174 | 16.855 | 0.897 | 1.00 | 28.38 |
| ATOM | 735 | CG | LYS | 89 | 13.626 | 18.006 | 0.053 | 1.00 | 33.25 |
| ATOM | 736 | CD | LYS | 89 | 12.677 | 17.499 | -1.032 | 1.00 | 36.96 |
| ATOM | 737 | CE | LYS | 89 | 12.083 | 18.647 | -1.857 | 1.00 | 40.68 |
| ATOM | 738 | NZ | LYS | 89 | 13.067 | 19.285 | -2.776 | 1.00 | 41.21 |
| ATOM | 739 | C | LYS | 89 | 14.416 | 18.384 | 2.861 | 1.00 | 23.76 |
| ATOM | 740 | O | LYS | 89 | 14.693 | 19.572 | 2.716 | 1.00 | 23.44 |
| ATOM | 741 | N | SER | 90 | 13.518 | 17.972 | 3.745 | 1.00 | 24.58 |
| ATOM | 742 | CA | SER | 90 | 12.806 | 18.943 | 4.559 | 1.00 | 24.92 |
| ATOM | 743 | CB | SER | 90 | 11.462 | 18.359 | 5.011 | 1.00 | 25.41 |
| ATOM | 744 | OG | SER | 90 | 11.643 | 17.177 | 5.770 | 1.00 | 25.27 |
| ATOM | 745 | C | SER | 90 | 13.585 | 19.418 | 5.785 | 1.00 | 24.40 |
| ATOM | 746 | O | SER | 90 | 13.377 | 20.536 | 6.255 | 1.00 | 24.10 |
| ATOM | 747 | N | GLY | 91 | 14.493 | 18.587 | 6.290 | 1.00 | 22.98 |
| ATOM | 748 | CA | GLY | 91 | 15.210 | 18.966 | 7.496 | 1.00 | 22.61 |
| ATOM | 749 | C | GLY | 91 | 16.696 | 19.258 | 7.446 | 1.00 | 23.29 |
| ATOM | 750 | O | GLY | 91 | 17.268 | 19.627 | 8.473 | 1.00 | 22.29 |
| ATOM | 751 | N | CYS | 92 | 17.332 | 19.116 | 6.284 | 1.00 | 23.33 |
| ATOM | 752 | CA | CYS | 92 | 18.770 | 19.368 | 6.205 | 1.00 | 23.73 |
| ATOM | 753 | CB | CYS | 92 | 19.291 | 19.140 | 4.781 | 1.00 | 26.18 |
| ATOM | 754 | SG | CYS | 92 | 18.549 | 20.198 | 3.516 | 1.00 | 35.39 |
| ATOM | 755 | C | CYS | 92 | 19.151 | 20.772 | 6.668 | 1.00 | 22.55 |
| ATOM | 756 | O | CYS | 92 | 20.073 | 20.938 | 7.467 | 1.00 | 20.78 |
| ATOM | 757 | N | LEU | 93 | 18.4k2 | 21.782 | 6.176 | 1.00 | 20.74 |
| ATOM | 758 | CA | LEU | 93 | 18.762 | 23.153 | 6.549 | 1.00 | 20.88 |
| ATOM | 759 | CB | LEU | 93 | 18.001 | 24.147 | 5.659 | 1.00 | 19.11 |
| ATOM | 760 | CG | LEU | 93 | 18.457 | 25.615 | 5.791 | 1.00 | 22.77 |
| ATOM | 761 | CD1 | LEU | 93 | 19.968 | 25.732 | 5.556 | 1.00 | 20.31 |
| ATOM | 762 | CD2 | LEU | 93 | 17.689 | 26.483 | 4.787 | 1.00 | 21.80 |
| ATOM | 763 | C | LEU | 93 | 18.478 | 23.427 | 8.024 | 1.00 | 20.09 |
| ATOM | 764 | O | LEU | 93 | 19.190 | 24.205 | 8.667 | 1.00 | 21.56 |
| ATOM | 765 | N | GLU | 94 | 17.452 | 22.788 | 8.568 | 1.00 | 18.63 |
| ATOM | 766 | CA | GLU | 94 | 17.125 | 22.979 | 9.975 | 1.00 | 22.09 |
| ATOM | 767 | CB | GLU | 94 | 15.813 | 22.273 | 10.335 | 1.00 | 20.52 |
| ATOM | 768 | CG | GLU | 94 | 14.643 | 22.753 | 9.497 | 1.00 | 23.52 |
| ATOM | 769 | CD | GLU | 94 | 13.303 | 22.276 | 10.027 | 1.00 | 24.62 |
| ATOM | 770 | OE1 | GLU | 94 | 13.285 | 21.458 | 10.972 | 1.00 | 25.58 |
| ATOM | 771 | OE2 | GLU | 94 | 12.268 | 22.724 | 9.492 | 1.00 | 26.18 |
| ATOM | 772 | C | GLU | 94 | 18.267 | 22.440 | 10.828 | 1.00 | 21.56 |
| ATOM | 773 | O | GLU | 94 | 18.622 | 23.035 | 11.840 | 1.00 | 23.14 |
| ATOM | 774 | N | VAL | 95 | 18.847 | 21.315 | 10.419 | 1.00 | 22.47 |
| ATOM | 775 | CA | VAL | 95 | 19.970 | 20.757 | 11.167 | 1.00 | 21.92 |
| ATOM | 776 | CB | VAL | 95 | 20.358 | 19.348 | 10.660 | 1.00 | 20.19 |
| ATOM | 777 | CG1 | VAL | 95 | 21.640 | 18.884 | 11.347 | 1.00 | 17.76 |
| ATOM | 778 | CG2 | VAL | 95 | 19.230 | 18.359 | 10.954 | 1.00 | 21.38 |
| ATOM | 779 | C | VAL | 95 | 21.179 | 21.690 | 11.043 | 1.00 | 20.91 |
| ATOM | 780 | O | VAL | 95 | 21.891 | 21.921 | 12.022 | 1.00 | 23.34 |
| ATOM | 781 | N | VAL | 96 | 21.419 | 22.219 | 9.843 | 1.00 | 20.72 |
| ATOM | 782 | CA | VAL | 96 | 22.544 | 23.142 | 9.640 | 1.00 | 20.86 |
| ATOM | 783 | CB | VAL | 96 | 22.632 | 23.609 | 8.168 | 1.00 | 20.89 |
| ATOM | 784 | CG1 | VAL | 96 | 23.622 | 24.766 | 8.034 | 1.00 | 21.60 |
| ATOM | 785 | CG2 | VAL | 96 | 23.078 | 22.435 | 7.285 | 1.00 | 22.01 |
| ATOM | 786 | C | VAL | 96 | 22.384 | 24.369 | 10.545 | 1.00 | 20.92 |
| ATOM | 787 | O | VAL | 96 | 23.356 | 24.873 | 11.119 | 1.00 | 20.53 |
| ATOM | 788 | N | LEU | 97 | 21.151 | 24.847 | 10.672 | 1.00 | 21.56 |
| ATOM | 789 | CA | LEU | 97 | 20.879 | 26.008 | 11.507 | 1.00 | 22.41 |
| ATOM | 790 | CB | LEU | 97 | 19.414 | 26.427 | 11.382 | 1.00 | 24.63 |
| ATOM | 791 | CG | LEU | 97 | 19.039 | 27.746 | 12.062 | 1.00 | 27.57 |
| ATOM | 792 | CD1 | LEU | 97 | 19.785 | 28.888 | 11.375 | 1.00 | 27.68 |
| ATOM | 793 | CD2 | LEU | 97 | 17.522 | 27.969 | 11.984 | 1.00 | 27.40 |
| ATOM | 794 | C | LEU | 97 | 21.196 | 25.687 | 12.963 | 1.00 | 23.37 |
| ATOM | 795 | O | LEU | 97 | 21.774 | 26.510 | 13.670 | 1.00 | 24.02 |
| ATOM | 796 | N | VAL | 98 | 20.795 | 24.505 | 13.424 | 1.00 | 22.24 |
| ATOM | 797 | CA | VAL | 98 | 21.083 | 24.124 | 14.801 | 1.00 | 22.66 |
| ATOM | 798 | CB | VAL | 98 | 20.454 | 22.753 | 15.168 | 1.00 | 24.14 |
| ATOM | 799 | CG1 | VAL | 98 | 20.950 | 22.295 | 16.540 | 1.00 | 21.61 |
| ATOM | 800 | CG2 | VAL | 98 | 18.936 | 22.872 | 15.184 | 1.00 | 22.31 |
| ATOM | 801 | C | VAL | 98 | 22.598 | 24.041 | 14.977 | 1.00 | 21.26 |
| ATOM | 802 | O | VAL | 98 | 23.147 | 24.583 | 15.930 | 1.00 | 23.35 |
| ATOM | 803 | N | ARG | 99 | 23.275 | 23.381 | 14.043 | 1.00 | 21.04 |
| ATOM | 804 | CA | ARG | 99 | 24.728 | 23.238 | 14.126 | 1.00 | 22.66 |
| ATOM | 805 | CB | ARG | 99 | 25.241 | 22.338 | 12.997 | 1.00 | 21.34 |
| ATOM | 806 | CG | ARG | 99 | 24.958 | 20.860 | 13.204 | 1.00 | 19.68 |
| ATOM | 807 | CD | ARG | 99 | 25.493 | 20.040 | 12.042 | 1.00 | 20.91 |
| ATOM | 808 | NE | ARG | 99 | 25.596 | 18.628 | 12.392 | 1.00 | 19.97 |
| ATOM | 809 | CZ | ARG | 99 | 26.207 | 17.710 | 11.651 | 1.00 | 21.46 |
| ATOM | 810 | NH1 | ARG | 99 | 26.774 | 18.047 | 10.499 | 1.00 | 19.48 |
| ATOM | 811 | NH2 | ARG | 99 | 26.268 | 16.456 | 12.077 | 1.00 | 19.49 |
| ATOM | 812 | C | ARG | 99 | 25.444 | 24.586 | 14.068 | 1.00 | 23.66 |
| ATOM | 813 | O | ARG | 99 | 26.496 | 24.770 | 14.680 | 1.00 | 22.32 |
| ATOM | 814 | N | MET | 100 | 24.873 | 25.524 | 13.325 | 1.00 | 24.98 |
| ATOM | 815 | CA | MET | 100 | 25.468 | 26.849 | 13.200 | 1.00 | 26.11 |
| ATOM | 816 | CB | MET | 100 | 24.580 | 27.732 | 12.315 | 1.00 | 24.6b |
| ATOM | 817 | CG | MET | 100 | 25.125 | 29.131 | 12.096 | 1.00 | 28.62 |
| ATOM | 818 | SD | MET | 100 | 23.935 | 30.203 | 11.266 | 1.00 | 30.73 |
| ATOM | 819 | CE | MET | 100 | 22.917 | 30.695 | 12.620 | 1.00 | 28.23 |
| ATOM | 820 | C | MET | 100 | 25.647 | 27.500 | 14.580 | 1.00 | 26.32 |
| ATOM | 821 | O | MET | 100 | 26.560 | 28.303 | 14.791 | 1.00 | 25.50 |
| ATOM | 822 | N | CYS | 101 | 24.782 | 27.140 | 15.524 | 1.00 | 26.14 |
| ATOM | 823 | CA | CYS | 101 | 24.857 | 27.716 | 16.861 | 1.00 | 26.26 |
| ATOM | 824 | CB | CYS | 101 | 23.647 | 27.273 | 17.686 | 1.00 | 26.15 |
| ATOM | 825 | SG | CYS | 101 | 22.070 | 27.833 | 16.958 | 1.00 | 28.21 |
| ATOM | 826 | C | CYS | 101 | 26.155 | 27.369 | 17.579 | 1.00 | 26.31 |
| ATOM | 827 | O | CYS | 101 | 26.556 | 28.064 | 18.512 | 1.00 | 27.18 |
| ATOM | 828 | N | ARG | 102 | 26.810 | 26.300 | 17.134 | 1.00 | 26.05 |
| ATOM | 829 | CA | ARG | 102 | 28.080 | 25.863 | 17.715 | 1.00 | 25.81 |
| ATOM | 830 | CB | ARG | 102 | 28.487 | 24.477 | 17.194 | 1.00 | 24.97 |
| ATOM | 831 | CG | ARG | 102 | 27.477 | 23.360 | 17.374 | 1.00 | 24.25 |
| ATOM | 832 | CD | ARG | 102 | 27.899 | 22.131 | 16.567 | 1.00 | 24.77 |
| ATOM | 833 | NE | ARG | 102 | 29.120 | 21.499 | 17.075 | 1.00 | 24.27 |
| ATOM | 834 | CZ | ARG | 102 | 30.251 | 21.376 | 16.384 | 1.00 | 25.69 |
| ATOM | 835 | NH1 | ARG | 102 | 30.334 | 21.847 | 15.146 | 1.00 | 22.95 |
| ATOM | 836 | NH2 | ARG | 102 | 31.300 | 20.763 | 16.924 | 1.00 | 24.83 |
| ATOM | 837 | C | ARG | 102 | 29.167 | 26.837 | 17.290 | 1.00 | 25.16 |
| ATOM | 838 | O | ARG | 102 | 30.189 | 26.965 | 17.956 | 1.00 | 26.46 |
| ATOM | 839 | N | ALA | 103 | 28.949 | 27.499 | 16.158 | 1.00 | 25.50 |
| ATOM | 840 | CA | ALA | 103 | 29.925 | 28.438 | 15.610 | 1.00 | 24.73 |
| ATOM | 841 | CB | ALA | 103 | 30.282 | 28.031 | 14.191 | 1.00 | 24.76 |
| ATOM | 842 | C | ALA | 103 | 29.412 | 29.869 | 15.626 | 1.00 | 25.49 |
| ATOM | 843 | O | ALA | 103 | 29.663 | 30.641 | 14.696 | 1.00 | 26.25 |
| ATOM | 844 | N | PHE | 104 | 28.701 | 30.220 | 16.693 | 1.00 | 24.81 |
| ATOM | 845 | CA | PHE | 104 | 28.138 | 31.552 | 16.837 | 1.00 | 24.87 |
| ATOM | 846 | CB | PHE | 104 | 26.612 | 31.463 | 16.834 | 1.00 | 24.62 |
| ATOM | 847 | CG | PHE | 104 | 25.912 | 32.800 | 16.868 | 1.00 | 27.48 |
| ATOM | 848 | CD1 | PHE | 104 | 25.548 | 33.383 | 18.082 | 1.00 | 27.30 |
| ATOM | 849 | CD2 | PHE | 104 | 25.599 | 33.465 | 15.685 | 1.00 | 25.70 |
| ATOM | 850 | CE1 | PHE | 104 | 24.876 | 34.610 | 18.115 | 1.00 | 27.97 |
| ATOM | 851 | CE2 | PHE | 104 | 24.930 | 34.693 | 15.708 | 1.00 | 26.67 |
| ATOM | 852 | CZ | PHE | 104 | 24.568 | 35.263 | 16.923 | 1.00 | 25.05 |
| ATOM | 853 | C | PHE | 104 | 28.637 | 32.156 | 18.145 | 1.00 | 26.24 |
| ATOM | 854 | O | PHE | 104 | 28.624 | 31.504 | 19.188 | 1.00 | 25.16 |
| ATOM | 855 | N | ASN | 105 | 29.095 | 33.398 | 18.078 | 1.00 | 24.14 |
| ATOM | 856 | CA | ASN | 105 | 29.594 | 34.080 | 19.259 | 1.00 | 25.15 |
| ATOM | 857 | CB | ASN | 105 | 30.833 | 34.889 | 18.884 | 1.00 | 25.27 |
| ATOM | 858 | CG | ASN | 105 | 31.414 | 35.640 | 20.054 | 1.00 | 28.22 |
| ATOM | 859 | OD1 | ASN | 105 | 30.811 | 35.715 | 21.125 | 1.00 | 29.75 |
| ATOM | 860 | ND2 | ASN | 105 | 32.592 | 36.211 | 19.853 | 1.00 | 28.02 |
| ATOM | 861 | C | ASN | 105 | 28.471 | 34.999 | 19.744 | 1.00 | 25.46 |
| ATOM | 862 | O | ASN | 105 | 28.218 | 36.043 | 19.148 | 1.00 | 23.70 |
| ATOM | 863 | N | PRO | 106 | 27.784 | 34.615 | 20.835 | 1.00 | 26.30 |
| ATOM | 864 | CD | PRO | 106 | 27.965 | 33.373 | 21.611 | 1.00 | 27.28 |
| ATOM | 865 | CA | PRO | 106 | 26.680 | 35.414 | 21.382 | 1.00 | 27.94 |
| ATOM | 866 | CB | PRO | 106 | 25.995 | 34.442 | 22.339 | 1.00 | 27.49 |
| ATOM | 867 | CG | PRO | 106 | 27.150 | 33.646 | 22.867 | 1.00 | 27.98 |
| ATOM | 868 | C | PRO | 106 | 27.052 | 36.731 | 22.059 | 1.00 | 28.50 |
| ATOM | 869 | O | PRO | 106 | 26.169 | 37.481 | 22.470 | 1.00 | 29.76 |
| ATOM | 870 | N | LEU | 107 | 28.344 | 37.024 | 22.173 | 1.00 | 28.28 |
| ATOM | 871 | CA | LEU | 107 | 28.761 | 38.277 | 22.800 | 1.00 | 30.25 |
| ATOM | 872 | CB | LEU | 107 | 30.150 | 38.138 | 23.444 | 1.00 | 28.28 |
| ATOM | 873 | CG | LEU | 107 | 30.176 | 37.231 | 24.686 | 1.00 | 31.14 |
| ATOM | 874 | CD1 | LEU | 107 | 31.562 | 37.188 | 25.277 | 1.00 | 29.40 |
| ATOM | 875 | CD2 | LEU | 107 | 29.192 | 37.746 | 25.726 | 1.00 | 32.31 |
| ATOM | 876 | C | LEU | 107 | 28.758 | 39.433 | 21.807 | 1.00 | 29.75 |
| ATOM | 877 | O | LEU | 107 | 28.332 | 40.533 | 22.142 | 1.00 | 31.64 |
| ATOM | 878 | N | ASN | 108 | 29.231 | 39.193 | 20.589 | 1.00 | 28.78 |
| ATOM | 879 | CA | ASN | 108 | 29.249 | 40.246 | 19.580 | 1.00 | 28.18 |
| ATOM | 880 | CB | ASN | 108 | 30.681 | 40.517 | 19.107 | 1.00 | 27.56 |
| ATOM | 881 | CG | ASN | 108 | 31.362 | 39.279 | 18.538 | 1.00 | 28.12 |
| ATOM | 882 | OD1 | ASN | 108 | 30.733 | 38.238 | 18.347 | 1.00 | 26.25 |
| ATOM | 883 | ND2 | ASN | 108 | 32.657 | 39.396 | 18.257 | 1.00 | 24.43 |
| ATOM | 884 | C | ASN | 108 | 28.364 | 39.894 | 18.386 | 1.00 | 27.75 |
| ATOM | 885 | O | ASN | 108 | 28.432 | 40.537 | 17.343 | 1.00 | 27.33 |
| ATOM | 886 | N | ASN | 109 | 27.535 | 38.870 | 18.556 | 1.00 | 27.69 |
| ATOM | 887 | CA | ASN | 109 | 26.627 | 38.418 | 17.512 | 1.00 | 28.83 |
| ATOM | 888 | CB | ASN | 109 | 25.465 | 39.395 | 17.353 | 1.00 | 29.93 |
| ATOM | 889 | CG | ASN | 109 | 24.546 | 39.387 | 18.543 | 1.00 | 32.72 |
| ATOM | 890 | OD1 | ASN | 109 | 24.216 | 38.324 | 19.072 | 1.00 | 32.17 |
| ATOM | 891 | ND2 | ASN | 109 | 24.116 | 40.575 | 18.973 | 1.00 | 31.31 |
| ATOM | 892 | C | ASN | 109 | 27.293 | 38.222 | 16.164 | 1.00 | 27.63 |
| ATOM | 893 | O | ASN | 109 | 26.918 | 38.861 | 15.183 | 1.00 | 27.80 |
| ATOM | 894 | N | THR | 110 | 28.287 | 37.342 | 16.124 | 1.00 | 26.91 |
| ATOM | 895 | CA | THR | 110 | 28.990 | 37.042 | 14.889 | 1.00 | 26.50 |
| ATOM | 896 | CB | THR | 110 | 30.469 | 37.468 | 14.967 | 1.00 | 25.46 |
| ATOM | 897 | OG1 | THR | 110 | 31.071 | 36.887 | 16.130 | 1.00 | 24.60 |
| ATOM | 898 | CG2 | THR | 110 | 30.587 | 38.987 | 15.050 | 1.00 | 26.91 |
| ATOM | 899 | C | THR | 110 | 28.908 | 35.535 | 14.668 | 1.00 | 26.44 |
| ATOM | 900 | O | THR | 110 | 28.813 | 34.765 | 15.625 | 1.00 | 26.49 |
| ATOM | 901 | N | VAL | 111 | 28.928 | 35.124 | 13.407 | 1.00 | 25.62 |
| ATOM | 902 | CA | VAL | 111 | 28.859 | 33.713 | 13.058 | 1.00 | 25.05 |
| ATOM | 903 | CB | VAL | 111 | 27.550 | 33.386 | 12.292 | 1.00 | 26.15 |
| ATOM | 904 | CG1 | VAL | 111 | 27.581 | 34.012 | 10.893 | 1.00 | 25.65 |
| ATOM | 905 | CG2 | VAL | 111 | 27.364 | 31.875 | 12.207 | 1.00 | 25.76 |
| ATOM | 906 | C | VAL | 111 | 30.056 | 33.361 | 12.178 | 1.00 | 25.27 |
| ATOM | 907 | O | VAL | 111 | 30.541 | 34.204 | 11.415 | 1.00 | 24.98 |
| ATOM | 908 | N | LEU | 112 | 30.536 | 32.126 | 12.302 | 1.00 | 23.55 |
| ATOM | 909 | CA | LEU | 112 | 31.677 | 31.660 | 11.520 | 1.00 | 24.51 |
| ATOM | 910 | CB | LEU | 112 | 32.171 | 30.316 | 12.067 | 1.00 | 24.17 |
| ATOM | 911 | CG | LEU | 112 | 33.323 | 29.570 | 11.384 | 1.00 | 26.81 |
| ATOM | 912 | CD1 | LEU | 112 | 34.579 | 30.439 | 11.321 | 1.00 | 24.50 |
| ATOM | 913 | CD2 | LEU | 112 | 33.603 | 28.292 | 12.176 | 1.00 | 26.23 |
| ATOM | 914 | C | LEU | 112 | 31.251 | 31.512 | 10.063 | 1.00 | 25.07 |
| ATOM | 915 | O | LEU | 112 | 30.347 | 30.734 | 9.751 | 1.00 | 23.68 |
| ATOM | 916 | N | PHE | 113 | 31.905 | 32.267 | 9.183 | 1.00 | 25.79 |
| ATOM | 917 | CA | PHE | 113 | 31.595 | 32.240 | 7.759 | 1.00 | 27.26 |
| ATOM | 918 | CB | PHE | 113 | 30.686 | 33.415 | 7.399 | 1.00 | 26.26 |
| ATOM | 919 | CG | PHE | 113 | 30.216 | 33.399 | 5.975 | 1.00 | 26.51 |
| ATOM | 920 | CD1 | PHE | 113 | 29.338 | 32.412 | 5.528 | 1.00 | 27.75 |
| ATOM | 921 | CD2 | PHE | 113 | 30.661 | 34.358 | 5.073 | 1.00 | 26.90 |
| ATOM | 922 | CE1 | PHE | 113 | 28.910 | 32.380 | 4.199 | 1.00 | 27.83 |
| ATOM | 923 | CE2 | PHE | 113 | 30.239 | 34.336 | 3.736 | 1.00 | 27.68 |
| ATOM | 924 | CZ | PHE | 113 | 29.361 | 33.344 | 3.302 | 1.00 | 26.77 |
| ATOM | 925 | C | PHE | 113 | 32.885 | 32.318 | 6.954 | 1.00 | 28.10 |
| ATOM | 926 | O | PHE | 113 | 33.651 | 33.271 | 7.079 | 1.00 | 29.91 |
| ATOM | 927 | N | GLU | 114 | 33.117 | 31.310 | 6.122 | 1.00 | 30.65 |
| ATOM | 928 | CA | GLU | 114 | 34.325 | 31.243 | 5.312 | 1.00 | 31.06 |
| ATOM | 929 | CB | GLU | 114 | 34.296 | 32.295 | 4.188 | 1.00 | 30.36 |
| ATOM | 930 | CG | GLU | 114 | 33.108 | 32.146 | 3.220 | 1.00 | 33.61 |
| ATOM | 931 | CD | GLU | 114 | 33.194 | 33.074 | 2.006 | 1.00 | 36.26 |
| ATOM | 932 | OE1 | GLU | 114 | 33.847 | 34.137 | 2.098 | 1.00 | 37.14 |
| ATOM | 933 | OE2 | GLU | 114 | 32.596 | 32.746 | 0.960 | 1.00 | 36.76 |
| ATOM | 934 | C | GLU | 114 | 35.579 | 31.416 | 6.169 | 1.00 | 31.09 |
| ATOM | 935 | O | GLU | 114 | 36.425 | 32.267 | 5.893 | 1.00 | 29.44 |
| ATOM | 936 | N | GLY | 115 | 35.678 | 30.619 | 7.232 | 1.00 | 31.65 |
| ATOM | 937 | CA | GLY | 115 | 36.862 | 30.653 | 8.077 | 1.00 | 31.02 |
| ATOM | 938 | C | GLY | 115 | 36.987 | 31.636 | 9.227 | 1.00 | 31.03 |
| ATOM | 939 | O | GLY | 115 | 37.850 | 31.453 | 10.081 | 1.00 | 31.02 |
| ATOM | 940 | N | LYS | 116 | 36.165 | 32.678 | 9.266 | 1.00 | 30.47 |
| ATOM | 941 | CA | LYS | 116 | 36.247 | 33.640 | 10.364 | 1.00 | 29.13 |
| ATOM | 942 | CB | LYS | 116 | 37.081 | 34.850 | 9.950 | 1.00 | 31.65 |
| ATOM | 943 | CG | LYS | 116 | 38.518 | 34.542 | 9.546 | 1.00 | 35.17 |
| ATOM | 944 | CD | LYS | 116 | 39.156 | 35.784 | 8.933 | 1.00 | 39.50 |
| ATOM | 945 | CE | LYS | 116 | 40.598 | 35.550 | 8.517 | 1.00 | 41.93 |
| ATOM | 946 | NZ | LYS | 116 | 41.482 | 35.336 | 9.699 | 1.00 | 46.16 |
| ATOM | 947 | C | LYS | 116 | 34.855 | 34.108 | 10.773 | 1.00 | 28.58 |
| ATOM | 948 | O | LYS | 116 | 33.883 | 33.914 | 10.035 | 1.00 | 25.83 |
| ATOM | 949 | N | TYR | 117 | 34.773 | 34.731 | 11.947 | 1.00 | 26.38 |
| ATOM | 950 | CA | TYR | 117 | 33.513 | 35.241 | 12.473 | 1.00 | 26.28 |
| ATOM | 951 | CB | TYR | 117 | 33.557 | 35.311 | 14.006 | 1.00 | 27.82 |
| ATOM | 952 | CG | TYR | 117 | 33.470 | 33.964 | 14.684 | 1.00 | 25.87 |
| ATOM | 953 | CD1 | TYR | 117 | 34.524 | 33.048 | 14.605 | 1.00 | 27.04 |
| ATOM | 954 | CE1 | TYR | 117 | 34.426 | 31.782 | 15.190 | 1.00 | 26.50 |
| ATOM | 955 | CD2 | TYR | 117 | 32.316 | 33.585 | 15.369 | 1.00 | 23.65 |
| ATOM | 956 | CE2 | TYR | 117 | 32.208 | 32.329 | 15.953 | 1.00 | 26.33 |
| ATOM | 957 | CZ | TYR | 117 | 33.263 | 31.433 | 15.858 | 1.00 | 26.31 |
| ATOM | 958 | OH | TYR | 117 | 33.136 | 30.179 | 16.405 | 1.00 | 29.08 |
| ATOM | 959 | C | TYR | 117 | 33.191 | 36.624 | 11.922 | 1.00 | 27.89 |
| ATOM | 960 | O | TYR | 117 | 34.063 | 37.491 | 11.837 | 1.00 | 27.82 |
| ATOM | 961 | N | GLY | 118 | 31.932 | 36.821 | 11.551 | 1.00 | 27.62 |
| ATOM | 962 | CA | GLY | 118 | 31.503 | 38.104 | 11.029 | 1.00 | 27.35 |
| ATOM | 963 | C | GLY | 118 | 30.086 | 38.415 | 11.476 | 1.00 | 27.40 |
| ATOM | 964 | O | GLY | 118 | 29.275 | 37.502 | 11.644 | 1.00 | 26.12 |
| ATOM | 965 | N | GLY | 119 | 29.787 | 39.698 | 11.673 | 1.00 | 27.51 |
| ATOM | 966 | CA | GLY | 119 | 28.455 | 40.092 | 12.104 | 1.00 | 28.25 |
| ATOM | 967 | C | GLY | 119 | 27.465 | 39.994 | 10.964 | 1.00 | 30.48 |
| ATOM | 968 | O | GLY | 119 | 27.845 | 39.624 | 9.852 | 1.00 | 32.66 |
| ATOM | 969 | N | MET | 120 | 26.203 | 40.328 | 11.215 | 1.00 | 30.06 |
| ATOM | 970 | CA | MET | 120 | 25.200 | 40.245 | 10.159 | 1.00 | 33.29 |
| ATOM | 971 | CB | MET | 120 | 23.786 | 40.417 | 10.742 | 1.00 | 35.95 |
| ATOM | 972 | CG | MET | 120 | 23.464 | 41.793 | 11.321 | 1.00 | 40.01 |
| ATOM | 973 | SD | MET | 120 | 23.194 | 43.077 | 10.066 | 1.00 | 44.66 |
| ATOM | 974 | CE | MET | 120 | 21.580 | 42.608 | 9.449 | 1.00 | 38.51 |
| ATOM | 975 | C | MET | 120 | 25.423 | 41.233 | 9.010 | 1.00 | 33.96 |
| ATOM | 976 | O | MET | 120 | 24.889 | 41.036 | 7.916 | 1.00 | 32.84 |
| ATOM | 977 | N | GLN | 121 | 26.218 | 42.280 | 9.245 | 1.00 | 34.68 |
| ATOM | 978 | CA | GLN | 121 | 26.489 | 43.281 | 8.209 | 1.00 | 36.23 |
| ATOM | 979 | CB | GLN | 121 | 27.204 | 44.509 | 8.794 | 1.00 | 37.39 |
| ATOM | 980 | CG | GLN | 121 | 28.683 | 44.292 | 9.088 | 1.00 | 40.21 |
| ATOM | 981 | CD | GLN | 121 | 28.930 | 43.759 | 10.485 | 1.00 | 42.57 |
| ATOM | 982 | OE1 | GLN | 121 | 28.035 | 43.184 | 11.111 | 1.00 | 42.30 |
| ATOM | 983 | NE2 | GLN | 121 | 30.154 | 43.943 | 10.982 | 1.00 | 43.39 |
| ATOM | 984 | C | GLN | 121 | 27.357 | 42.670 | 7.117 | 1.00 | 36.52 |
| ATOM | 985 | O | GLN | 121 | 27.577 | 43.263 | 6.062 | 1.00 | 38.05 |
| ATOM | 986 | N | MET | 122 | 27.852 | 41.474 | 7.397 | 1.00 | 35.28 |
| ATOM | 987 | CA | MET | 122 | 28.686 | 40.731 | 6.474 | 1.00 | 32.99 |
| ATOM | 988 | CB | MET | 122 | 29.324 | 39.562 | 7.239 | 1.00 | 34.32 |
| ATOM | 989 | CG | MET | 122 | 30.068 | 38.536 | 6.417 | 1.00 | 32.66 |
| ATOM | 990 | SD | MET | 122 | 31.016 | 37.409 | 7.491 | 1.00 | 32.03 |
| ATOM | 991 | CE | MET | 122 | 29.704 | 36.572 | 8.406 | 1.00 | 29.49 |
| ATOM | 992 | C | MET | 122 | 27.819 | 40.225 | 5.317 | 1.00 | 33.06 |
| ATOM | 993 | O | MET | 122 | 28.315 | 39.986 | 4.219 | 1.00 | 31.80 |
| ATOM | 994 | N | PHE | 123 | 26.519 | 40.094 | 5.571 | 1.00 | 31.66 |
| ATOM | 995 | CA | PHE | 123 | 25.565 | 39.596 | 4.580 | 1.00 | 32.27 |
| ATOM | 996 | CB | PHE | 123 | 24.640 | 38.567 | 5.235 | 1.00 | 29.78 |
| ATOM | 997 | CG | PHE | 123 | 25.366 | 37.400 | 5.838 | 1.00 | 30.41 |
| ATOM | 998 | CD1 | PHE | 123 | 25.875 | 36.387 | 5.030 | 1.00 | 28.95 |
| ATOM | 999 | CD2 | PHE | 123 | 25.553 | 37.318 | 7.213 | 1.00 | 28.84 |
| ATOM | 1000 | CE1 | PHE | 123 | 26.556 | 35.307 | 5.586 | 1.00 | 28.63 |
| ATOM | 1001 | CE2 | PHE | 123 | 26.234 | 36.243 | 7.779 | 1.00 | 28.70 |
| ATOM | 1002 | CZ | PHE | 123 | 26.736 | 35.235 | 6.965 | 1.00 | 29.76 |
| ATOM | 1003 | C | PHE | 123 | 24.709 | 40.695 | 3.952 | 1.00 | 33.01 |
| ATOM | 1004 | O | PHE | 123 | 23.671 | 40.410 | 3.352 | 1.00 | 32.84 |
| ATOM | 1005 | N | LYS | 124 | 25.149 | 41.941 | 4.082 | 1.00 | 33.57 |
| ATOM | 1006 | CA | LYS | 124 | 24.409 | 43.075 | 3.542 | 1.00 | 35.39 |
| ATOM | 1007 | CB | LYS | 124 | 25.228 | 44.361 | 3.737 | 1.00 | 38.66 |
| ATOM | 1008 | CG | LYS | 124 | 24.439 | 45.645 | 3.501 | 1.00 | 43.12 |
| ATOM | 1009 | CD | LYS | 124 | 23.162 | 45.650 | 4.341 | 1.00 | 46.67 |
| ATOM | 1010 | CE | LYS | 124 | 22.286 | 46.866 | 4.040 | 1.00 | 49.30 |
| ATOM | 1011 | NZ | LYS | 124 | 20.975 | 46.787 | 4.753 | 1.00 | 48.16 |
| ATOM | 1012 | C | LYS | 124 | 24.005 | 42.923 | 2.071 | 1.00 | 33.97 |
| ATOM | 1013 | O | LYS | 124 | 22.858 | 43.169 | 1.719 | 1.00 | 34.76 |
| ATOM | 1014 | N | ALA | 125 | 24.936 | 42.497 | 1.219 | 1.00 | 34.80 |
| ATOM | 1015 | CA | ALA | 125 | 24.666 | 42.347 | -0.215 | 1.00 | 34.52 |
| ATOM | 1016 | CB | ALA | 125 | 25.955 | 41.965 | -0.953 | 1.00 | 33.38 |
| ATOM | 1017 | C | ALA | 125 | 23.548 | 41.366 | -0.583 | 1.00 | 35.75 |
| ATOM | 1018 | O | ALA | 125 | 23.125 | 41.313 | -1.738 | 1.00 | 35.49 |
| ATOM | 1019 | N | LEU | 126 | 23.071 | 40.581 | 0.378 | 1.00 | 35.88 |
| ATOM | 1020 | CA | LEU | 126 | 21.993 | 39.640 | 0.085 | 1.00 | 35.88 |
| ATOM | 1021 | CB | LEU | 126 | 21.877 | 38.583 | 1.181 | 1.00 | 34.67 |
| ATOM | 1022 | CG | LEU | 126 | 23.019 | 37.586 | 1.309 | 1.00 | 33.16 |
| ATOM | 1023 | CD1 | LEU | 126 | 22.701 | 36.629 | 2.442 | 1.00 | 33.20 |
| ATOM | 1024 | CD2 | LEU | 126 | 23.200 | 36.831 | -0.006 | 1.00 | 31.66 |
| ATOM | 1025 | C | LEU | 126 | 20.661 | 40.360 | -0.037 | 1.00 | 35.66 |
| ATOM | 1026 | O | LEU | 126 | 19.745 | 39.881 | -0.699 | 1.00 | 37.30 |
| ATOM | 1027 | N | GLY | 127 | 20.556 | 41.509 | 0.618 | 1.00 | 36.88 |
| ATOM | 1028 | CA | GLY | 127 | 19.319 | 42.261 | 0.577 | 1.00 | 37.45 |
| ATOM | 1029 | C | GLY | 127 | 18.231 | 41.495 | 1.295 | 1.00 | 38.00 |
| ATOM | 1030 | O | GLY | 127 | 17.047 | 41.645 | 0.992 | 1.00 | 37.47 |
| ATOM | 1031 | N | SER | 128 | 18.637 | 40.672 | 2.257 | 1.00 | 38.76 |
| ATOM | 1032 | CA | SER | 128 | 17.696 | 39.862 | 3.024 | 1.00 | 38.85 |
| ATOM | 1033 | CB | SER | 128 | 17.783 | 38.399 | 2.575 | 1.00 | 39.65 |
| ATOM | 1034 | OG | SER | 128 | 17.675 | 38.280 | 1.167 | 1.00 | 40.93 |
| ATOM | 1035 | C | SER | 128 | 18.004 | 39.946 | 4.518 | 1.00 | 38.88 |
| ATOM | 1036 | O | SER | 128 | 18.142 | 38.916 | 5.184 | 1.00 | 38.92 |
| ATOM | 1037 | N | ASP | 129 | 18.104 | 41.160 | 5.050 | 1.00 | 38.31 |
| ATOM | 1038 | CA | ASP | 129 | 18.413 | 41.327 | 6.469 | 1.00 | 37.76 |
| ATOM | 1039 | CB | ASP | 129 | 18.513 | 42.809 | 6.837 | 1.00 | 38.81 |
| ATOM | 1040 | CG | ASP | 129 | 19.785 | 43.458 | 6.322 | 1.00 | 37.85 |
| ATOM | 1041 | OD1 | ASP | 129 | 20.698 | 42.734 | 5.884 | 1.00 | 39.00 |
| ATOM | 1042 | OD2 | ASP | 129 | 19.877 | 44.700 | 6.369 | 1.00 | 40.89 |
| ATOM | 1043 | C | ASP | 129 | 17.399 | 40.651 | 7.382 | 1.00 | 37.23 |
| ATOM | 1044 | O | ASP | 129 | 17.734 | 40.238 | 8.490 | 1.00 | 36.42 |
| ATOM | 1045 | N | ASP | 130 | 16.156 | 40.542 | 6.930 | 1.00 | 36.72 |
| ATOM | 1046 | CA | ASP | 130 | 15.143 | 39.901 | 7.755 | 1.00 | 36.80 |
| ATOM | 1047 | CB | ASP | 130 | 13.760 | 40.044 | 7.110 | 1.00 | 38.13 |
| ATOM | 1048 | CG | ASP | 130 | 13.722 | 39.535 | 5.688 | 1.00 | 40.50 |
| ATOM | 1049 | OD1 | ASP | 130 | 14.726 | 39.717 | 4.969 | 1.00 | 40.76 |
| ATOM | 1050 | OD2 | ASP | 130 | 12.680 | 38.969 | 5.285 | 1.00 | 41.30 |
| ATOM | 1051 | C | ASP | 130 | 15.507 | 38.430 | 7.958 | 1.00 | 34.04 |
| ATOM | 1052 | O | ASP | 130 | 15.376 | 37.899 | 9.058 | 1.00 | 32.59 |
| ATOM | 1053 | N | LEU | 131 | 15.974 | 37.779 | 6.897 | 1.00 | 31.60 |
| ATOM | 1054 | CA | LEU | 131 | 16.368 | 36.380 | 6.994 | 1.00 | 29.28 |
| ATOM | 1055 | CB | LEU | 131 | 16.735 | 35.812 | 5.615 | 1.00 | 28.37 |
| ATOM | 1056 | CG | LEU | 131 | 17.529 | 34.490 | 5.653 | 1.00 | 28.49 |
| ATOM | 1057 | CD1 | LEU | 131 | 16.701 | 33.410 | 6.352 | 1.00 | 26.24 |
| ATOM | 1058 | CD2 | LEU | 131 | 17.895 | 34.046 | 4.249 | 1.00 | 25.43 |
| ATOM | 1059 | C | LEU | 131 | 17.569 | 36.270 | 7.924 | 1.00 | 28.88 |
| ATOM | 1060 | O | LEU | 131 | 17.560 | 35.495 | 8.882 | 1.00 | 28.78 |
| ATOM | 1061 | N | VAL | 132 | 18.603 | 37.058 | 7.646 | 1.00 | 28.88 |
| ATOM | 1062 | CA | VAL | 132 | 19.805 | 37.032 | 8.473 | 1.00 | 28.97 |
| ATOM | 1063 | CB | VAL | 132 | 20.843 | 38.038 | 7.953 | 1.00 | 28.69 |
| ATOM | 1064 | CG1 | VAL | 132 | 22.086 | 37.998 | 8.822 | 1.00 | 27.42 |
| ATOM | 1065 | CG2 | VAL | 132 | 21.195 | 37.706 | 6.512 | 1.00 | 29.10 |
| ATOM | 1066 | C | VAL | 132 | 19.490 | 37.319 | 9.943 | 1.00 | 29.17 |
| ATOM | 1067 | O | VAL | 132 | 19.912 | 36.571 | 10.831 | 1.00 | 29.93 |
| ATOM | 1068 | N | ASN | 133 | 18.748 | 38.395 | 10.198 | 1.00 | 29.09 |
| ATOM | 1069 | CA | ASN | 133 | 18.367 | 38.759 | 11.562 | 1.00 | 28.91 |
| ATOM | 1070 | CB | ASN | 133 | 17.477 | 40.004 | 11.552 | 1.00 | 31.79 |
| ATOM | 1071 | CG | ASN | 133 | 18.275 | 41.297 | 11.466 | 1.00 | 35.19 |
| ATOM | 1072 | OD1 | ASN | 133 | 17.728 | 42.352 | 11.149 | 1.00 | 36.03 |
| ATOM | 1073 | ND2 | ASN | 133 | 19.569 | 41.222 | 11.765 | 1.00 | 34.40 |
| ATOM | 1074 | C | ASN | 133 | 17.634 | 37.620 | 12.266 | 1.00 | 28.71 |
| ATOM | 1075 | O | ASN | 133 | 17.850 | 37.376 | 13.453 | 1.00 | 28.36 |
| ATOM | 1076 | N | GLU | 134 | 16.758 | 36.928 | 11.546 | 1.00 | 27.60 |
| ATOM | 1077 | CA | GLU | 134 | 16.033 | 35.815 | 12.154 | 1.00 | 29.70 |
| ATOM | 1078 | CB | GLU | 134 | 14.904 | 35.340 | 11.235 | 1.00 | 30.25 |
| ATOM | 1079 | CG | GLU | 134 | 13.612 | 36.130 | 11.441 | 1.00 | 37.54 |
| ATOM | 1080 | CD | GLU | 134 | 12.429 | 35.572 | 10.672 | 1.00 | 39.03 |
| ATOM | 1081 | OE1 | GLU | 134 | 12.311 | 34.333 | 10.568 | 1.00 | 42.45 |
| ATOM | 1082 | OE2 | GLU | 134 | 11.604 | 36.376 | 10.186 | 1.00 | 43.40 |
| ATOM | 1083 | C | GLU | 134 | 16.978 | 34.659 | 12.484 | 1.00 | 28.63 |
| ATOM | 1084 | O | GLU | 134 | 16.867 | 34.040 | 13.540 | 1.00 | 27.53 |
| ATOM | 1085 | N | ALA | 135 | 17.916 | 34.385 | 11.583 | 1.00 | 28.23 |
| ATOM | 1086 | CA | ALA | 135 | 18.887 | 33.317 | 11.791 | 1.00 | 27.00 |
| ATOM | 1087 | CB | ALA | 135 | 19.769 | 33.178 | 10.571 | 1.00 | 26.34 |
| ATOM | 1088 | C | ALA | 135 | 19.734 | 33.636 | 13.019 | 1.00 | 27.05 |
| ATOM | 1089 | O | ALA | 135 | 19.955 | 32.774 | 13.876 | 1.00 | 25.93 |
| ATOM | 1090 | N | PHE | 136 | 20.199 | 34.881 | 13.104 | 1.00 | 27.53 |
| ATOM | 1091 | CA | PHE | 136 | 21.018 | 35.322 | 14.229 | 1.00 | 28.38 |
| ATOM | 1092 | CB | PHE | 136 | 21.569 | 36.728 | 13.963 | 1.00 | 29.04 |
| ATOM | 1093 | CG | PHE | 136 | 22.817 | 36.742 | 13.123 | 1.00 | 27.20 |
| ATOM | 1094 | CD1 | PHE | 136 | 22.858 | 36.084 | 11.898 | 1.00 | 28.97 |
| ATOM | 1095 | CD2 | PHE | 136 | 23.954 | 37.422 | 13.555 | 1.00 | 28.84 |
| ATOM | 1096 | CE1 | PHE | 136 | 24.014 | 36.103 | 11.114 | 1.00 | 28.21 |
| ATOM | 1097 | CE2 | PHE | 136 | 25.117 | 37.446 | 12.776 | 1.00 | 27.38 |
| ATOM | 1098 | CZ | PHE | 136 | 25.143 | 36.785 | 11.555 | 1.00 | 27.06 |
| ATOM | 1099 | C | PHE | 136 | 20.278 | 35.306 | 15.566 | 1.00 | 29.12 |
| ATOM | 1100 | O | PHE | 136 | 20.834 | 34.876 | 16.581 | 1.00 | 27.78 |
| ATOM | 1101 | N | ASP | 137 | 19.031 | 35.772 | 15.573 | 1.00 | 30.75 |
| ATOM | 1102 | CA | ASP | 137 | 18.247 | 35.798 | 16.808 | 1.00 | 31.48 |
| ATOM | 1103 | CB | ASP | 137 | 16.888 | 36.463 | 16.570 | 1.00 | 34.88 |
| ATOM | 1104 | CG | ASP | 137 | 17.013 | 37.938 | 16.197 | 1.00 | 39.69 |
| ATOM | 1105 | OD1 | ASP | 137 | 18.118 | 38.513 | 16.338 | 1.00 | 41.72 |
| ATOM | 1106 | OD2 | ASP | 137 | 15.997 | 38.524 | 15.769 | 1.00 | 41.77 |
| ATOM | 1107 | C | ASP | 137 | 18.046 | 34.388 | 17.361 | 1.00 | 30.07 |
| ATOM | 1108 | O | ASP | 137 | 18.094 | 34.173 | 18.572 | 1.00 | 28.12 |
| ATOM | 1109 | N | PHE | 138 | 17.814 | 33.430 | 16.470 | 1.00 | 29.22 |
| ATOM | 1110 | CA | PHE | 138 | 17.635 | 32.043 | 16.888 | 1.00 | 27.56 |
| ATOM | 1111 | CB | PHE | 138 | 17.284 | 31.156 | 15.692 | 1.00 | 26.66 |
| ATOM | 1112 | CG | PHE | 138 | 17.481 | 29.693 | 15.964 | 1.00 | 27.08 |
| ATOM | 1113 | CD1 | PHE | 138 | 16.631 | 29.018 | 16.833 | 1.00 | 27.62 |
| ATOM | 1114 | CD2 | PHE | 138 | 18.562 | 29.010 | 15.414 | 1.00 | 25.94 |
| ATOM | 1115 | CE1 | PHE | 138 | 16.854 | 27.679 | 17.157 | 1.00 | 27.42 |
| ATOM | 1116 | CE2 | PHE | 138 | 18.798 | 27.671 | 15.731 | 1.00 | 26.61 |
| ATOM | 1117 | CZ | PHE | 138 | 17.942 | 27.005 | 16.606 | 1.00 | 27.10 |
| ATOM | 1118 | C | PHE | 138 | 18.931 | 31.524 | 17.520 | 1.00 | 26.65 |
| ATOM | 1119 | O | PHE | 138 | 18.932 | 30.996 | 18.638 | 1.00 | 25.79 |
| ATOM | 1120 | N | ALA | 139 | 20.028 | 31.673 | 16.783 | 1.00 | 26.86 |
| ATOM | 1121 | CA | ALA | 139 | 21.335 | 31.228 | 17.246 | 1.00 | 28.26 |
| ATOM | 1122 | CB | ALA | 139 | 22.409 | 31.620 | 16.246 | 1.00 | 24.42 |
| ATOM | 1123 | C | ALA | 139 | 21.629 | 31.854 | 18.598 | 1.00 | 29.32 |
| ATOM | 1124 | O | ALA | 139 | 22.062 | 31.175 | 19.523 | 1.00 | 29.76 |
| ATOM | 1125 | N | LYS | 140 | 21.388 | 33.154 | 18.705 | 1.00 | 30.35 |
| ATOM | 1126 | CA | LYS | 140 | 21.628 | 33.857 | 19.955 | 1.00 | 33.43 |
| ATOM | 1127 | CB | LYS | 140 | 21.305 | 35.345 | 19.795 | 1.00 | 35.05 |
| ATOM | 1128 | CG | LYS | 140 | 21.512 | 36.156 | 21.056 | 1.00 | 41.64 |
| ATOM | 1129 | CD | LYS | 140 | 21.304 | 37.645 | 20.808 | 1.00 | 45.36 |
| ATOM | 1130 | CE | LYS | 140 | 21.412 | 38.426 | 22.113 | 1.00 | 48.64 |
| ATOM | 1131 | NZ | LYS | 140 | 22.648 | 38.062 | 22.869 | 1.00 | 48.83 |
| ATOM | 1132 | C | LYS | 140 | 20.784 | 33.250 | 21.072 | 1.00 | 32.60 |
| ATOM | 1133 | O | LYS | 140 | 21.271 | 33.018 | 22.173 | 1.00 | 33.54 |
| ATOM | 1134 | N | ASN | 141 | 19.520 | 32.968 | 20.782 | 1.00 | 33.89 |
| ATOM | 1135 | CA | ASN | 141 | 18.645 | 32.397 | 21.794 | 1.00 | 34.26 |
| ATOM | 1136 | CB | ASN | 141 | 17.187 | 32.488 | 21.351 | 1.00 | 35.65 |
| ATOM | 1137 | CG | ASN | 141 | 16.714 | 33.922 | 21.246 | 1.00 | 41.37 |
| ATOM | 1138 | OD1 | ASN | 141 | 17.033 | 34.759 | 22.102 | 1.00 | 41.73 |
| ATOM | 1139 | ND2 | ASN | 141 | 15.944 | 34.218 | 20.203 | 1.00 | 43.55 |
| ATOM | 1140 | C | ASN | 141 | 18.993 | 30.961 | 22.155 | 1.00 | 32.74 |
| ATOM | 1141 | O | ASN | 141 | 18.857 | 30.565 | 23.307 | 1.00 | 32.51 |
| ATOM | 1142 | N | LEU | 142 | 19.439 | 30.178 | 21.180 | 1.00 | 31.43 |
| ATOM | 1143 | CA | LEU | 142 | 19.800 | 28.800 | 21.475 | 1.00 | 29.57 |
| ATOM | 1144 | CB | LEU | 142 | 20.061 | 28.009 | 20.190 | 1.00 | 31.15 |
| ATOM | 1145 | CG | LEU | 142 | 20.302 | 26.512 | 20.415 | 1.00 | 30.62 |
| ATOM | 1146 | CD1 | LEU | 142 | 19.000 | 25.852 | 20.835 | 1.00 | 28.71 |
| ATOM | 1147 | CD2 | LEU | 142 | 20.824 | 25.870 | 19.149 | 1.00 | 30.61 |
| ATOM | 1148 | C | LEU | 142 | 21.058 | 28.809 | 22.329 | 1.00 | 28.67 |
| ATOM | 1149 | O | LEU | 142 | 21.188 | 28.022 | 23.266 | 1.00 | 27.68 |
| ATOM | 1150 | N | CYS | 143 | 21.988 | 29.706 | 22.004 | 1.00 | 28.64 |
| ATOM | 1151 | CA | CYS | 143 | 23.240 | 29.805 | 22.751 | 1.00 | 28.53 |
| ATOM | 1152 | CB | CYS | 143 | 24.164 | 30.857 | 22.118 | 1.00 | 29.40 |
| ATOM | 1153 | SG | CYS | 143 | 25.058 | 30.297 | 20.626 | 1.00 | 30.27 |
| ATOM | 1154 | C | CYS | 143 | 23.011 | 30.141 | 24.225 | 1.00 | 28.51 |
| ATOM | 1155 | O | CYS | 143 | 23.811 | 29.764 | 25.083 | 1.00 | 27.46 |
| ATOM | 1156 | N | SER | 144 | 21.922 | 30.844 | 24.524 | 1.00 | 27.47 |
| ATOM | 1157 | CA | SER | 144 | 21.631 | 31.204 | 25.907 | 1.00 | 30.41 |
| ATOM | 1158 | CB | SER | 144 | 20.407 | 32.123 | 25.978 | 1.00 | 30.97 |
| ATOM | 1159 | OG | SER | 144 | 19.221 | 31.404 | 25.687 | 1.00 | 32.16 |
| ATOM | 1160 | C | SER | 144 | 21.391 | 29.963 | 26.776 | 1.00 | 30.53 |
| ATOM | 1161 | O | SER | 144 | 21.527 | 30.024 | 27.995 | 1.00 | 31.62 |
| ATOM | 1162 | N | LEU | 145 | 21.039 | 28.841 | 26.147 | 1.00 | 29.38 |
| ATOM | 1163 | CA | LEU | 145 | 20.787 | 27.600 | 26.878 | 1.00 | 28.47 |
| ATOM | 1164 | CB | LEU | 145 | 19.886 | 26.664 | 26.062 | 1.00 | 30.06 |
| ATOM | 1165 | CG | LEU | 145 | 18.452 | 27.120 | 25.776 | 1.00 | 30.81 |
| ATOM | 1166 | CD1 | LEU | 145 | 17.736 | 26.074 | 24.929 | 1.00 | 31.42 |
| ATOM | 1167 | CD2 | LEU | 145 | 17.711 | 27.326 | 27.092 | 1.00 | 32.81 |
| ATOM | 1168 | C | LEU | 145 | 22.078 | 26.869 | 27.242 | 1.00 | 27.00 |
| ATOM | 1169 | O | LEU | 145 | 22.060 | 25.936 | 28.041 | 1.00 | 26.93 |
| ATOM | 1170 | N | GLN | 146 | 23.192 | 27.285 | 26.647 | 1.00 | 27.19 |
| ATOM | 1171 | CA | GLN | 146 | 24.481 | 26.669 | 26.938 | 1.00 | 28.35 |
| ATOM | 1172 | CB | GLN | 146 | 24.912 | 27.015 | 28.364 | 1.00 | 31.85 |
| ATOM | 1173 | CG | GLN | 146 | 25.051 | 28.488 | 28.638 | 1.00 | 37.93 |
| ATOM | 1174 | CD | GLN | 146 | 26.153 | 29.120 | 27.823 | 1.00 | 43.03 |
| ATOM | 1175 | OE1 | GLN | 146 | 27.326 | 28.736 | 27.920 | 1.00 | 45.91 |
| ATOM | 1176 | NE2 | GLN | 146 | 25.785 | 30.096 | 27.005 | 1.00 | 46.57 |
| ATOM | 1177 | C | GLN | 146 | 24.451 | 25.147 | 26.797 | 1.00 | 28.31 |
| ATOM | 1178 | O | GLN | 146 | 24.803 | 24.425 | 27.738 | 1.00 | 28.78 |
| ATOM | 1179 | N | LEU | 147 | 24.035 | 24.657 | 25.634 | 1.00 | 24.96 |
| ATOM | 1180 | CA | LEU | 147 | 23.971 | 23.219 | 25.405 | 1.00 | 25.05 |
| ATOM | 1181 | CB | LEU | 147 | 23.129 | 22.919 | 24.166 | 1.00 | 24.70 |
| ATOM | 1182 | CG | LEU | 147 | 21.682 | 23.401 | 24.168 | 1.00 | 25.61 |
| ATOM | 1183 | CD1 | LEU | 147 | 21.020 | 22.977 | 22.858 | 1.00 | 27.01 |
| ATOM | 1184 | CD2 | LEU | 147 | 20.945 | 22.817 | 25.369 | 1.00 | 26.03 |
| ATOM | 1185 | C | LEU | 147 | 25.357 | 22.616 | 25.206 | 1.00 | 25.19 |
| ATOM | 1186 | O | LEU | 147 | 26.253 | 23.262 | 24.664 | 1.00 | 25.02 |
| ATOM | 1187 | N | THR | 148 | 25.533 | 21.377 | 25.650 | 1.00 | 25.71 |
| ATOM | 1188 | CA | THR | 148 | 26.809 | 20.697 | 25.473 | 1.00 | 25.53 |
| ATOM | 1189 | CB | THR | 148 | 27.004 | 19.561 | 26.495 | 1.00 | 25.85 |
| ATOM | 1190 | OG1 | THR | 148 | 25.986 | 18.572 | 26.297 | 1.00 | 25.37 |
| ATOM | 1191 | CG2 | THR | 148 | 26.933 | 20.101 | 27.937 | 1.00 | 25.32 |
| ATOM | 1192 | C | THR | 148 | 26.777 | 20.078, | 24.076 | 1.00 | 26.33 |
| ATOM | 1193 | O | THR | 148 | 25.741 | 20.098 | 23.395 | 1.00 | 23.91 |
| ATOM | 1194 | N | GLU | 149 | 27.906 | 19.528 | 23.647 | 1.00 | 24.54 |
| ATOM | 1195 | CA | GLU | 149 | 27.973 | 18.898 | 22.335 | 1.00 | 25.20 |
| ATOM | 1196 | CB | GLU | 149 | 29.420 | 18.498 | 22.007 | 1.00 | 24.58 |
| ATOM | 1197 | CG | GLU | 149 | 30.257 | 19.657 | 21.475 | 1.00 | 27.87 |
| ATOM | 1198 | CD | GLU | 149 | 29.926 | 20.009 | 20.024 | 1.00 | 28.51 |
| ATOM | 1199 | OE1 | GLU | 149 | 30.532 | 19.410 | 19.113 | 1.00 | 28.15 |
| ATOM | 1200 | OE2 | GLU | 149 | 29.053 | 20.877 | 19.794 | 1.00 | 30.62 |
| ATOM | 1201 | C | GLU | 149 | 27.052 | 17.684 | 22.268 | 1.00 | 24.09 |
| ATOM | 1202 | O | GLU | 149 | 26.472 | 17.403 | 21.225 | 1.00 | 26.05 |
| ATOM | 1203 | N | GLU | 150 | 26.904 | 16.968 | 23.377 | 1.00 | 24.30 |
| ATOM | 1204 | CA | GLU | 150 | 26.034 | 15.789 | 23.393 | 1.00 | 24.51 |
| ATOM | 1205 | CB | GLU | 150 | 26.163 | 15.027 | 24.712 | 1.00 | 24.35 |
| ATOM | 1206 | CG | GLU | 150 | 27.526 | 14.403 | 24.987 | 1.00 | 28.10 |
| ATOM | 1207 | CD | GLU | 150 | 27.522 | 13.586 | 26.280 | 1.00 | 31.06 |
| ATOM | 1208 | OE1 | GLU | 150 | 26.883 | 14.030 | 27.258 | 1.00 | 29.36 |
| ATOM | 1209 | OE2 | GLU | 150 | 28.154 | 12.507 | 26.322 | 1.00 | 31.37 |
| ATOM | 1210 | C | GLU | 150 | 24.571 | 16.189 | 23.211 | 1.00 | 24.19 |
| ATOM | 1211 | O | GLU | 150 | 23.807 | 15.500 | 22.529 | 1.00 | 24.28 |
| ATOM | 1212 | N | GLU | 151 | 24.187 | 17.300 | 23.833 | 1.00 | 23.86 |
| ATOM | 1213 | CA | GLU | 151 | 22.818 | 17.787 | 23.757 | 1.00 | 23.65 |
| ATOM | 1214 | CB | GLU | 151 | 22.595 | 18.871 | 24.816 | 1.00 | 23.47 |
| ATOM | 1215 | CG | GLU | 151 | 22.812 | 18.336 | 26.238 | 1.00 | 23.24 |
| ATOM | 1216 | CD | GLU | 151 | 22.835 | 19.420 | 27.300 | 1.00 | 25.20 |
| ATOM | 1217 | OE1 | GLU | 151 | 23.196 | 20.573 | 26.977 | 1.00 | 25.28 |
| ATOM | 1218 | OE2 | GLU | 151 | 22.505 | 19.111 | 28.466 | 1.00 | 25.81 |
| ATOM | 1219 | C | GLU | 151 | 22.524 | 18.311 | 22.358 | 1.00 | 24.05 |
| ATOM | 1220 | O | GLU | 151 | 21.432 | 18.113 | 21.833 | 1.00 | 22.27 |
| ATOM | 1221 | N | ILE | 152 | 23.510 | 18.968 | 21.751 | 1.00 | 23.71 |
| ATOM | 1222 | CA | ILE | 152 | 23.342 | 19.487 | 20.406 | 1.00 | 24.39 |
| ATOM | 1223 | CB | ILE | 152 | 24.538 | 20.358 | 19.996 | 1.00 | 26.20 |
| ATOM | 1224 | CG2 | ILE | 152 | 24.545 | 20.559 | 18.484 | 1.00 | 27.07 |
| ATOM | 1225 | CG1 | ILE | 152 | 24.461 | 21.699 | 20.732 | 1.00 | 27.68 |
| ATOM | 1226 | CD1 | ILE | 152 | 25.778 | 22.469 | 20.758 | 1.00 | 32.87 |
| ATOM | 1227 | C | ILE | 152 | 23.208 | 18.318 | 19.440 | 1.00 | 24.33 |
| ATOM | 1228 | O | ILE | 152 | 22.394 | 18.346 | 18.515 | 1.00 | 24.36 |
| ATOM | 1229 | N | ALA | 153 | 24.007 | 17.282 | 19.666 | 1.00 | 22.40 |
| ATOM | 1230 | CA | ALA | 153 | 23.968 | 16.097 | 18.821 | 1.00 | 21.95 |
| ATOM | 1231 | CB | ALA | 153 | 25.004 | 15.076 | 19.311 | 1.00 | 20.15 |
| ATOM | 1232 | C | ALA | 153 | 22.578 | 15.471 | 18.836 | 1.00 | 19.88 |
| ATOM | 1233 | O | ALA | 153 | 21.965 | 15.238 | 17.788 | 1.00 | 20.80 |
| ATOM | 1234 | N | LEU | 154 | 22.092 | 15.199 | 20.037 | 1.00 | 19.09 |
| ATOM | 1235 | CA | LEU | 154 | 20.794 | 14.573 | 20.222 | 1.00 | 21.33 |
| ATOM | 1236 | CB | LEU | 154 | 20.604 | 14.206 | 21.699 | 1.00 | 21.40 |
| ATOM | 1237 | CG | LEU | 154 | 21.616 | 13.184 | 22.230 | 1.00 | 24.62 |
| ATOM | 1238 | CD1 | LEU | 154 | 21.415 | 12.961 | 23.728 | 1.00 | 25.79 |
| ATOM | 1239 | CD2 | LEU | 154 | 21.448 | 11.878 | 21.472 | 1.00 | 25.80 |
| ATOM | 1240 | C | LEU | 154 | 19.653 | 15.456 | 19.730 | 1.00 | 21.75 |
| ATOM | 1241 | O | LEU | 154 | 18.742 | 14.979 | 19.044 | 1.00 | 20.76 |
| ATOM | 1242 | N | PHE | 155 | 19.706 | 16.744 | 20.061 | 1.00 | 21.95 |
| ATOM | 1243 | CA | PHE | 155 | 18.662 | 17.654 | 19.621 | 1.00 | 23.38 |
| ATOM | 1244 | CB | PHE | 155 | 18.826 | 19.038 | 20.250 | 1.00 | 22.84 |
| ATOM | 1245 | CG | PHE | 155 | 17.755 | 20.007 | 19.841 | 1.00 | 24.22 |
| ATOM | 1246 | CD1 | PHE | 155 | 16.419 | 19.737 | 20.107 | 1.00 | 25.61 |
| ATOM | 1247 | CD2 | PHE | 155 | 18.077 | 21.187 | 19.186 | 1.00 | 24.20 |
| ATOM | 1248 | CE1 | PHE | 155 | 15.415 | 20.636 | 19.723 | 1.00 | 25.64 |
| ATOM | 1249 | CE2 | PHE | 155 | 17.087 | 22.088 | 18.800 | 1.00 | 24.91 |
| ATOM | 1250 | CZ | PHE | 155 | 15.753 | 21.812 | 19.069 | 1.00 | 24.00 |
| ATOM | 1251 | C | PHE | 155 | 18.654 | 17.792 | 18.104 | 1.00 | 22.70 |
| ATOM | 1252 | O | PHE | 155 | 17.595 | 17.740 | 17.481 | 1.00 | 23.42 |
| ATOM | 1253 | N | SER | 156 | 19.824 | 17.967 | 17.502 | 1.00 | 22.39 |
| ATOM | 1254 | CA | SER | 156 | 19.873 | 18.105 | 16.053 | 1.00 | 21.92 |
| ATOM | 1255 | CB | SER | 156 | 21.308 | 18.330 | 15.565 | 1.00 | 23.31 |
| ATOM | 1256 | OG | SER | 156 | 22.097 | 17.163 | 15.712 | 1.00 | 21.35 |
| ATOM | 1257 | C | SER | 156 | 19.290 | 16.853 | 15.407 | 1.00 | 20.69 |
| ATOM | 1258 | O | SER | 156 | 18.632 | 16.939 | 14.378 | 1.00 | 21.06 |
| ATOM | 1259 | N | SER | 157 | 19.508 | 15.692 | 16.020 | 1.00 | 19.99 |
| ATOM | 1260 | CA | SER | 157 | 18.974 | 14.456 | 15.462 | 1.00 | 20.74 |
| ATOM | 1261 | CB | SER | 157 | 19.609 | 13.232 | 16.139 | 1.00 | 20.43 |
| ATOM | 1262 | OG | SER | 157 | 19.026 | 12.965 | 17.405 | 1.00 | 22.34 |
| ATOM | 1263 | C | SER | 157 | 17.443 | 14.403 | 15.596 | 1.00 | 21.96 |
| ATOM | 1264 | O | SER | 157 | 16.759 | 13.833 | 14.739 | 1.00 | 20.32 |
| ATOM | 1265 | N | ALA | 158 | 16.907 | 14.994 | 16.663 | 1.00 | 21.59 |
| ATOM | 1266 | CA | ALA | 158 | 15.453 | 15.015 | 16.866 | 1.00 | 23.00 |
| ATOM | 1267 | CB | ALA | 158 | 15.111 | 15.494 | 18.284 | 1.00 | 22.43 |
| ATOM | 1268 | C | ALA | 158 | 14.815 | 15.939 | 15.837 | 1.00 | 22.79 |
| ATOM | 1269 | O | ALA | 158 | 13.707 | 15.690 | 15.366 | 1.00 | 24.41 |
| ATOM | 1270 | N | VAL | 159 | 15.519 | 17.012 | 15.494 | 1.00 | 22.53 |
| ATOM | 1271 | CA | VAL | 159 | 15.037 | 17.965 | 14.497 | 1.00 | 21.80 |
| ATOM | 1272 | CB | VAL | 159 | 15.997 | 19.175 | 14.393 | 1.00 | 21.36 |
| ATOM | 1273 | CG1 | VAL | 159 | 15.670 | 20.003 | 13.162 | 1.00 | 23.95 |
| ATOM | 1274 | CG2 | VAL | 159 | 15.877 | 20.036 | 15.649 | 1.00 | 19.81 |
| ATOM | 1275 | C | VAL | 159 | 14.945 | 17.257 | 13.143 | 1.00 | 22.32 |
| ATOM | 1276 | O | VAL | 159 | 13.981 | 17.434 | 12.390 | 1.00 | 22.54 |
| ATOM | 1277 | N | LEU | 160 | 15.946 | 16.429 | 12.861 | 1.00 | 20.50 |
| ATOM | 1278 | CA | LEU | 160 | 16.011 | 15.661 | 11.622 | 1.00 | 21.56 |
| ATOM | 1279 | CB | LEU | 160 | 17.416 | 15.067 | 11.443 | 1.00 | 18.74 |
| ATOM | 1280 | CG | LEU | 160 | 17.565 | 14.114 | 10.252 | 1.00 | 20.46 |
| ATOM | 1281 | CD1 | LEU | 160 | 17.342 | 14.880 | 8.962 | 1.00 | 23.67 |
| ATOM | 1282 | CD2 | LEU | 160 | 18.944 | 13.466 | 10.269 | 1.00 | 22.07 |
| ATOM | 1283 | C | LEU | 160 | 14.988 | 14.523 | 11.574 | 1.00 | 22.22 |
| ATOM | 1284 | O | LEU | 160 | 14.305 | 14.336 | 10.569 | 1.00 | 23.99 |
| ATOM | 1285 | N | ILE | 161 | 14.906 | 13.753 | 12.654 | 1.00 | 24.28 |
| ATOM | 1286 | CA | ILE | 161 | 13.982 | 12.622 | 12.723 | 1.00 | 24.95 |
| ATOM | 1287 | CB | ILE | 161 | 14.492 | 11.534 | 13.712 | 1.00 | 25.36 |
| ATOM | 1288 | CG2 | ILE | 161 | 13.739 | 10.234 | 13.494 | 1.00 | 24.65 |
| ATOM | 1289 | CG1 | ILE | 161 | 15.993 | 11.289 | 13.515 | 1.00 | 29.20 |
| ATOM | 1290 | CD1 | ILE | 161 | 16.372 | 10.830 | 12.152 | 1.00 | 31.88 |
| ATOM | 1291 | C | ILE | 161 | 12.632 | 13.136 | 13.203 | 1.00 | 23.52 |
| ATOM | 1292 | O | ILE | 161 | 12.214 | 12.852 | 14.327 | 1.00 | 22.05 |
| ATOM | 1293 | N | SER | 162 | 11.966 | 13.909 | 12.347 | 1.00 | 23.68 |
| ATOM | 1294 | CA | SER | 162 | 10.671 | 14.482 | 12.681 | 1.00 | 24.76 |
| ATOM | 1295 | CB | SER | 162 | 10.612 | 15.946 | 12.264 | 1.00 | 23.48 |
| ATOM | 1296 | OG | SER | 162 | 9.274 | 16.413 | 12.349 | 1.00 | 27.54 |
| ATOM | 1297 | C | SER | 162 | 9.514 | 13.742 | 12.030 | 1.00 | 25.92 |
| ATOM | 1298 | O | SER | 162 | 9.393 | 13.707 | 10.810 | 1.00 | 23.88 |
| ATOM | 1299 | N | PRO | 163 | 8.630 | 13.154 | 12.848 | 1.00 | 28.81 |
| ATOM | 1300 | CD | PRO | 163 | 8.685 | 13.100 | 14.321 | 1.00 | 28.18 |
| ATOM | 1301 | CA | PRO | 163 | 7.477 | 12.411 | 12.333 | 1.00 | 29.09 |
| ATOM | 1302 | CB | PRO | 163 | 7.018 | 11.612 | 13.546 | 1.00 | 28.39 |
| ATOM | 1303 | CG | PRO | 163 | 7.318 | 12.553 | 14.684 | 1.00 | 29.92 |
| ATOM | 1304 | C | PRO | 163 | 6.375 | 13.312 | 11.786 | 1.00 | 30.74 |
| ATOM | 1305 | O | PRO | 163 | 5.354 | 12.824 | 11.297 | 1.00 | 31.15 |
| ATOM | 1306 | N | ASP | 164 | 6.586 | 14.624 | 11.851 | 1.00 | 31.09 |
| ATOM | 1307 | CA | ASP | 164 | 5.582 | 15.563 | 11.371 | 1.00 | 32.86 |
| ATOM | 1308 | CB | ASP | 164 | 5.494 | 16.768 | 12.309 | 1.00 | 35.68 |
| ATOM | 1309 | CG | ASP | 164 | 5.004 | 16.383 | 13.697 | 1.00 | 41.26 |
| ATOM | 1310 | OD1 | ASP | 164 | 3.928 | 15.754 | 13.790 | 1.00 | 42.32 |
| ATOM | 1311 | OD2 | ASP | 164 | 5.691 | 16.704 | 14.692 | 1.00 | 44.22 |
| ATOM | 1312 | C | ASP | 164 | 5.788 | 16.038 | 9.942 | 1.00 | 31.89 |
| ATOM | 1313 | O | ASP | 164 | 4.929 | 16.727 | 9.394 | 1.00 | 31.96 |
| ATOM | 1314 | N | ARG | 165 | 6.910 | 15.665 | 9.333 | 1.00 | 29.36 |
| ATOM | 1315 | CA | ARG | 165 | 7.184 | 16.073 | 7.963 | 1.00 | 28.58 |
| ATOM | 1316 | CB | ARG | 165 | 8.525 | 15.507 | 7.477 | 1.00 | 25.79 |
| ATOM | 1317 | CG | ARG | 165 | 9.732 | 15.898 | 8.313 | 1.00 | 24.11 |
| ATOM | 1318 | CD | ARG | 165 | 9.861 | 17.404 | 8.460 | 1.00 | 20.61 |
| ATOM | 1319 | NE | ARG | 165 | 11.169 | 17.760 | 8.990 | 1.00 | 22.12 |
| ATOM | 1320 | CZ | ARG | 165 | 11.512 | 18.971 | 9.415 | 1.00 | 22.71 |
| ATOM | 1321 | NH1 | ARG | 165 | 10.641 | 19.972 | 9.383 | 1.00 | 21.83 |
| ATOM | 1322 | NH2 | ARG | 165 | 12.734 | 19.177 | 9.883 | 1.00 | 22.67 |
| ATOM | 1323 | C | ARG | 165 | 6.068 | 15.546 | 7.076 | 1.00 | 30.34 |
| ATOM | 1324 | O | ARG | 165 | 5.655 | 14.396 | 7.207 | 1.00 | 31.07 |
| ATOM | 1325 | N | ALA | 166 | 5.575 | 16.381 | 6.171 | 1.00 | 30.71 |
| ATOM | 1326 | CA | ALA | 166 | 4.507 | 15.945 | 5.287 | 1.00 | 30.26 |
| ATOM | 1327 | CB | ALA | 166 | 3.891 | 17.146 | 4.567 | 1.00 | 31.74 |
| ATOM | 1328 | C | ALA | 166 | 5.051 | 14.946 | 4.276 | 1.00 | 28.73 |
| ATOM | 1329 | O | ALA | 166 | 6.240 | 14.961 | 3.950 | 1.00 | 26.64 |
| ATOM | 1330 | N | TRP | 167 | 4.166 | 14.070 | 3.806 | 1.00 | 28.10 |
| ATOM | 1331 | CA | TRP | 167 | 4.475 | 13.046 | 2.806 | 1.00 | 27.46 |
| ATOM | 1332 | CB | TRP | 167 | 5.309 | 13.631 | 1.650 | 1.00 | 28.13 |
| ATOM | 1333 | CG | TRP | 167 | 4.811 | 14.960 | 1.098 | 1.00 | 32.39 |
| ATOM | 1334 | CD2 | TRP | 167 | 3.672 | 15.177 | 0.245 | 1.00 | 31.61 |
| ATOM | 1335 | CE2 | TRP | 167 | 3.616 | 16.560 | -0.035 | 1.00 | 32.63 |
| ATOM | 1336 | CE3 | TRP | 167 | 2.697 | 14.336 | -0.307 | 1.00 | 31.53 |
| ATOM | 1337 | CD1 | TRP | 167 | 5.378 | 16.192 | 1.292 | 1.00 | 33.04 |
| ATOM | 1338 | NE1 | TRP | 167 | 4.666 | 17.155 | 0.614 | 1.00 | 33.34 |
| ATOM | 1339 | CZ2 | TRP | 167 | 2.620 | 17.123 | -0.847 | 1.00 | 32.95 |
| ATOM | 1340 | CZ3 | TRP | 167 | 1.702 | 14.899 | -1.121 | 1.00 | 31.83 |
| ATOM | 1341 | CH2 | TRP | 167 | 1.675 | 16.277 | -1.379 | 1.00 | 32.36 |
| ATOM | 1342 | C | TRP | 167 | 5.172 | 11.789 | 3.327 | 1.00 | 27.06 |
| ATOM | 1343 | O | TRP | 167 | 5.413 | 10.863 | 2.550 | 1.00 | 27.23 |
| ATOM | 1344 | N | LEU | 168 | 5.507 | 11.737 | 4.617 | 1.00 | 26.83 |
| ATOM | 1345 | CA | LEU | 168 | 6.161 | 10.537 | 5.145 | 1.00 | 26.56 |
| ATOM | 1346 | CB | LEU | 168 | 6.521 | 10.703 | 6.633 | 1.00 | 27.73 |
| ATOM | 1347 | CG | LEU | 168 | 7.684 | 11.644 | 7.009 | 1.00 | 26.59 |
| ATOM | 1348 | CD1 | LEU | 168 | 7.798 | 11.758 | 8.529 | 1.00 | 25.28 |
| ATOM | 1349 | CD2 | LEU | 168 | 8.982 | 11.124 | 6.417 | 1.00 | 25.28 |
| ATOM | 1350 | C | LEU | 168 | 5.217 | 9.347 | 4.973 | 1.00 | 28.20 |
| ATOM | 1351 | O | LEU | 168 | 4.022 | 9.457 | 5.241 | 1.00 | 26.68 |
| ATOM | 1352 | N | LEU | 169 | 5.755 | 8.219 | 4.513 | 1.00 | 28.02 |
| ATOM | 1353 | CA | LEU | 169 | 4.954 | 7.016 | 4.307 | 1.00 | 28.10 |
| ATOM | 1354 | CB | LEU | 169 | 5.588 | 6.132 | 3.231 | 1.00 | 27.55 |
| ATOM | 1355 | CG | LEU | 169 | 5.571 | 6.687 | 1.802 | 1.00 | 29.51 |
| ATOM | 1356 | CD1 | LEU | 169 | 6.406 | 5.802 | 0.879 | 1.00 | 26.49 |
| ATOM | 1357 | CD2 | LEU | 169 | 4.130 | 6.785 | 1.319 | 1.00 | 26.84 |
| ATOM | 1358 | C | LEU | 169 | 4.785 | 6.216 | 5.593 | 1.00 | 29.45 |
| ATOM | 1359 | O | LEU | 169 | 3.754 | 5.582 | 5.800 | 1.00 | 30.01 |
| ATOM | 1360 | N | GLU | 170 | 5.801 | 6.237 | 6.452 | 1.00 | 29.04 |
| ATOM | 1361 | CA | GLU | 170 | 5.736 | 5.518 | 7.721 | 1.00 | 29.15 |
| ATOM | 1362 | CB | GLU | 170 | 6.684 | 4.315 | 7.706 | 1.00 | 29.47 |
| ATOM | 1363 | CG | GLU | 170 | 6.321 | 3.256 | 6.672 | 1.00 | 32.25 |
| ATOM | 1364 | CD | GLU | 170 | 7.296 | 2.095 | 6.659 | 1.00 | 32.48 |
| ATOM | 1365 | OE1 | GLU | 170 | 8.494 | 2.323 | 6.391 | 1.00 | 35.86 |
| ATOM | 1366 | OE2 | GLU | 170 | 6.866 | 0.954 | 6.918 | 1.00 | 33.16 |
| ATOM | 1367 | C | GLU | 170 | 6.106 | 6.453 | 8.863 | 1.00 | 28.40 |
| ATOM | 1368 | O | GLU | 170 | 7.150 | 6.297 | 9.493 | 1.00 | 26.92 |
| ATOM | 1369 | N | PRO | 171 | 5.241 | 7.440 | 9.148 | 1.00 | 29.50 |
| ATOM | 1370 | CD | PRO | 171 | 3.953 | 7.686 | 8.474 | 1.00 | 28.57 |
| ATOM | 1371 | CA | PRO | 171 | 5.467 | 8.416 | 10.217 | 1.00 | 29.67 |
| ATOM | 1372 | CB | PRO | 171 | 4.238 | 9.320 | 10.125 | 1.00 | 30.97 |
| ATOM | 1373 | CG | PRO | 171 | 3.174 | 8.408 | 9.536 | 1.00 | 29.77 |
| ATOM | 1374 | C | PRO | 171 | 5.652 | 7.816 | 11.610 | 1.00 | 31.30 |
| ATOM | 1375 | O | PRO | 171 | 6.520 | 8.259 | 12.371 | 1.00 | 28.88 |
| ATOM | 1376 | N | ARG | 172 | 4.838 | 6.815 | 11.940 | 1.00 | 31.06 |
| ATOM | 1377 | CA | ARG | 172 | 4.915 | 6.153 | 13.240 | 1.00 | 32.55 |
| ATOM | 1378 | CB | ARG | 172 | 3.916 | 4.992 | 13.299 | 1.00 | 36.17 |
| ATOM | 1379 | CG | ARG | 172 | 2.639 | 5.296 | 14.065 | 1.00 | 42.42 |
| ATOM | 1380 | CD | ARG | 172 | 2.802 | 4.986 | 15.561 | 1.00 | 49.54 |
| ATOM | 1381 | NE | ARG | 172 | 3.861 | 5.774 | 16.202 | 1.00 | 53.35 |
| ATOM | 1382 | CZ | ARG | 172 | 4.305 | 5.573 | 17.443 | 1.00 | 55.25 |
| ATOM | 1383 | NH1 | ARG | 172 | 3.788 | 4.604 | 18.192 | 1.00 | 56.19 |
| ATOM | 1384 | NH2 | ARG | 172 | 5.268 | 6.342 | 17.941 | 1.00 | 56.24 |
| ATOM | 1385 | C | ARG | 172 | 6.319 | 5.637 | 13.552 | 1.00 | 31.45 |
| ATOM | 1386 | O | ARG | 172 | 6.764 | 5.702 | 14.702 | 1.00 | 30.68 |
| ATOM | 1387 | N | LYS | 173 | 7.010 | 5.117 | 12.540 | 1.00 | 29.22 |
| ATOM | 1388 | CA | LYS | 173 | 8.363 | 4.615 | 12.743 | 1.00 | 29.66 |
| ATOM | 1389 | CB | LYS | 173 | 8.861 | 3.870 | 11.503 | 1.00 | 32.78 |
| ATOM | 1390 | CG | LYS | 173 | 8.200 | 2.513 | 11.305 | 1.00 | 37.00 |
| ATOM | 1391 | CD | LYS | 173 | 8.782 | 1.776 | 10.116 | 1.00 | 39.09 |
| ATOM | 1392 | CE | LYS | 173 | 8.072 | 0.449 | 9.888 | 1.00 | 41.03 |
| ATOM | 1393 | NZ | LYS | 173 | 8.678 | -0.294 | 8.745 | 1.00 | 43.13 |
| ATOM | 1394 | C | LYS | 173 | 9.300 | 5.768 | 13.076 | 1.00 | 28.29 |
| ATOM | 1395 | O | LYS | 173 | 10.207 | 5.625 | 13.894 | 1.00 | 26.75 |
| ATOM | 1396 | N | VAL | 174 | 9.082 | 6.913 | 12.441 | 1.00 | 26.90 |
| ATOM | 1397 | CA | VAL | 174 | 9.903 | 8.085 | 12.713 | 1.00 | 25.57 |
| ATOM | 1398 | CB | VAL | 174 | 9.616 | 9.215 | 11.709 | 1.00 | 23.33 |
| ATOM | 1399 | CG1 | VAL | 174 | 10.401 | 10.474 | 12.096 | 1.00 | 20.63 |
| ATOM | 1400 | CG2 | VAL | 174 | 9.999 | 8.758 | 10.303 | 1.00 | 22.21 |
| ATOM | 1401 | C | VAL | 174 | 9.587 | 8.575 | 14.122 | 1.00 | 26.24 |
| ATOM | 1402 | O | VAL | 174 | 10.484 | 8.917 | 14.889 | 1.00 | 24.66 |
| ATOM | 1403 | N | GLN | 175 | 8.303 | 8.603 | 14.458 | 1.00 | 27.11 |
| ATOM | 1404 | CA | GLN | 175 | 7.869 | 9.040 | 15.779 | 1.00 | 28.66 |
| ATOM | 1405 | CB | GLN | 175 | 6.339 | 8.967 | 15.878 | 1.00 | 31.02 |
| ATOM | 1406 | CG | GLN | 175 | 5.779 | 9.114 | 17.286 | 1.00 | 38.22 |
| ATOM | 1407 | CD | GLN | 175 | 4.257 | 9.212 | 17.301 | 1.00 | 42.67 |
| ATOM | 1408 | OE1 | GLN | 175 | 3.570 | 8.514 | 16.550 | 1.00 | 45.50 |
| ATOM | 1409 | NE2 | GLN | 175 | 3.723 | 10.073 | 18.164 | 1.00 | 43.62 |
| ATOM | 1410 | C | GLN | 175 | 8.500 | 8.188 | 16.875 | 1.00 | 28.75 |
| ATOM | 1411 | O | GLN | 175 | 8.945 | 8.712 | 17.901 | 1.00 | 26.15 |
| ATOM | 1412 | N | LYS | 176 | 8.541 | 6.876 | 16.654 | 1.00 | 28.86 |
| ATOM | 1413 | CA | LYS | 176 | 9.107 | 5.956 | 17.636 | 1.00 | 28.72 |
| ATOM | 1414 | CB | LYS | 176 | 8.919 | 4.505 | 17.183 | 1.00 | 31.92 |
| ATOM | 1415 | CG | LYS | 176 | 7.473 | 4.016 | 17.228 | 1.00 | 34.30 |
| ATOM | 1416 | CD | LYS | 176 | 7.364 | 2.603 | 16.658 | 1.00 | 37.65 |
| ATOM | 1417 | CE | LYS | 176 | 5.916 | 2.139 | 16.565 | 1.00 | 40.91 |
| ATOM | 1418 | NZ | LYS | 176 | 5.250 | 2.029 | 17.897 | 1.00 | 43.69 |
| ATOM | 1419 | C | LYS | 176 | 10.584 | 6.223 | 17.897 | 1.00 | 28.09 |
| ATOM | 1420 | O | LYS | 176 | 11.030 | 6.193 | 19.046 | 1.00 | 26.98 |
| ATOM | 1421 | N | LEU | 177 | 11.343 | 6.483 | 16.836 | 1.00 | 26.02 |
| ATOM | 1422 | CA | LEU | 177 | 12.765 | 6.759 | 16.994 | 1.00 | 25.81 |
| ATOM | 1423 | CB | LEU | 177 | 13.484 | 6.723 | 15.635 | 1.00 | 24.35 |
| ATOM | 1424 | CG | LEU | 177 | 14.977 | 7.090 | 15.579 | 1.00 | 22.01 |
| ATOM | 1425 | CD1 | LEU | 177 | 15.766 | 6.331 | 16.644 | 1.00 | 22.19 |
| ATOM | 1426 | CD2 | LEU | 177 | 15.518 | 6.775 | 14.192 | 1.00 | 21.74 |
| ATOM | 1427 | C | LEU | 177 | 12.968 | 8.114 | 17.662 | 1.00 | 26.28 |
| ATOM | 1428 | O | LEU | 177 | 13.832 | 8.258 | 18.531 | 1.00 | 25.96 |
| ATOM | 1429 | N | GLN | 178 | 12.173 | 9.107 | 17.272 | 1.00 | 25.47 |
| ATOM | 1430 | CA | GLN | 178 | 12.321 | 10.429 | 17.860 | 1.00 | 25.14 |
| ATOM | 1431 | CB | GLN | 178 | 11.416 | 11.457 | 17.182 | 1.00 | 24.25 |
| ATOM | 1432 | CG | GLN | 178 | 11.846 | 12.882 | 17.499 | 1.00 | 23.94 |
| ATOM | 1433 | CD | GLN | 178 | 10.808 | 13.935 | 17.145 | 1.00 | 25.80 |
| ATOM | 1434 | OE1 | GLN | 178 | 11.140 | 14.977 | 16.579 | 1.00 | 26.74 |
| ATOM | 1435 | NE2 | GLN | 178 | 9.559 | 13.683 | 17.498 | 1.00 | 22.56 |
| ATOM | 1436 | C | GLN | 178 | 12.018 | 10.428 | 19.357 | 1.00 | 26.38 |
| ATOM | 1437 | b | GLN | 178 | 12.634 | 11.181 | 20.117 | 1.00 | 25.08 |
| ATOM | 1438 | N | GLU | 179 | 11.068 | 9.599 | 19.785 | 1.00 | 26.53 |
| ATOM | 1439 | CA | GLU | 179 | 10.722 | 9.544 | 21.205 | 1.00 | 28.84 |
| ATOM | 1440 | CB | GLU | 179 | 9.459 | 8.701 | 21.434 | 1.00 | 31.20 |
| ATOM | 1441 | CG | GLU | 179 | 8.231 | 9.239 | 20.723 | 1.00 | 36.58 |
| ATOM | 1442 | CD | GLU | 179 | 6.954 | 8.494 | 21.087 | 1.00 | 41.47 |
| ATOM | 1443 | OE1 | GLU | 179 | 5.955 | 8.650 | 20.353 | 1.00 | 43.13 |
| ATOM | 1444 | OE2 | GLU | 179 | 6.948 | 7.763 | 22.104 | 1.00 | 43.96 |
| ATOM | 1445 | C | GLU | 179 | 11.880 | 8.974 | 22.015 | 1.00 | 26.71 |
| ATOM | 1446 | O | GLU | 179 | 12.134 | 9.414 | 23.136 | 1.00 | 26.95 |
| ATOM | 1447 | N | LYS | 180 | 12.577 | 7.993 | 21.451 | 1.00 | 25.60 |
| ATOM | 1448 | CA | LYS | 180 | 13.710 | 7.394 | 22.141 | 1.00 | 25.20 |
| ATOM | 1449 | CB | LYS | 180 | 14.201 | 6.153 | 21.391 | 1.00 | 26.31 |
| ATOM | 1450 | CG | LYS | 180 | 13.223 | 4.981 | 21.476 | 1.00 | 26.70 |
| ATOM | 1451 | CD | LYS | 180 | 13.546 | 3.909 | 20.452 | 1.00 | 26.50 |
| ATOM | 1452 | CE | LYS | 180 | 12.394 | 2.906 | 20.308 | 1.00 | 26.04 |
| ATOM | 1453 | NZ | LYS | 180 | 12.689 | 1.913 | 19.246 | 1.00 | 24.63 |
| ATOM | 1454 | C | LYS | 180 | 14.811 | 8.434 | 22.237 | 1.00 | 25.02 |
| ATOM | 1455 | O | LYS | 180 | 15.505 | 8.520 | 23.244 | 1.00 | 23.42 |
| ATOM | 1456 | N | ILE | 181 | 14.955 | 9.242 | 21.189 | 1.00 | 25.84 |
| ATOM | 1457 | CA | ILE | 181 | 15.969 | 10.291 | 21.179 | 1.00 | 23.07 |
| ATOM | 1458 | CB | ILE | 181 | 16.073 | 10.948 | 19.784 | 1.00 | 22.59 |
| ATOM | 1459 | CG2 | ILE | 181 | 16.997 | 12.160 | 19.837 | 1.00 | 21.40 |
| ATOM | 1460 | CG1 | ILE | 181 | 16.612 | 9.926 | 18.773 | 1.00 | 22.13 |
| ATOM | 1461 | CD1 | ILE | 181 | 16.648 | 10.429 | 17.343 | 1.00 | 20.82 |
| ATOM | 1462 | C | ILE | 181 | 15.659 | 11.359 | 22.233 | 1.00 | 24.68 |
| ATOM | 1463 | O | ILE | 181 | 16.544 | 11.768 | 22.988 | 1.00 | 24.27 |
| ATOM | 1464 | N | TYR | 182 | 14.406 | 11.803 | 22.292 | 1.00 | 24.89 |
| ATOM | 1465 | CA | TYR | 182 | 14.010 | 12.821 | 23.263 | 1.00 | 25.67 |
| ATOM | 1466 | CB | TYR | 182 | 12.563 | 13.254 | 23.019 | 1.00 | 27.04 |
| ATOM | 1467 | CG | TYR | 182 | 12.440 | 14.408 | 22.052 | 1.00 | 28.95 |
| ATOM | 1468 | CD1 | TYR | 182 | 11.386 | 14.474 | 21.145 | 1.00 | 29.65 |
| ATOM | 1469 | CE1 | TYR | 182 | 11.270 | 15.540 | 20.250 | 1.00 | 28.75 |
| ATOM | 1470 | CD2 | TYR | 182 | 13.376 | 15.437 | 22.049 | 1.00 | 28.12 |
| ATOM | 1471 | CE2 | TYR | 182 | 13.271 | 16.506 | 21.165 | 1.00 | 30.56 |
| ATOM | 1472 | CZ | TYR | 182 | 12.216 | 16.550 | 20.266 | 1.00 | 28.99 |
| ATOM | 1473 | OH | TYR | 182 | 12.119 | 17.595 | 19.381 | 1.00 | 29.53 |
| ATOM | 1474 | C | TYR | 182 | 14.166 | 12.328 | 24.695 | 1.00 | 26.66 |
| ATOM | 1475 | O | TYR | 182 | 14.539 | 13.093 | 25.586 | 1.00 | 26.29 |
| ATOM | 1476 | N | PHE | 183 | 13.879 | 11.050 | 24.910 | 1.00 | 26.50 |
| ATOM | 1477 | CA | PHE | 183 | 14.008 | 10.457 | 26.232 | 1.00 | 29.79 |
| ATOM | 1478 | CB | PHE | 183 | 13.388 | 9.057 | 26.249 | 1.00 | 34.77 |
| ATOM | 1479 | CG | PHE | 183 | 11.913 | 9.045 | 26.597 | 1.00 | 44.65 |
| ATOM | 1480 | CD1 | PHE | 183 | 11.105 | 7.963 | 26.237 | 1.00 | 48.06 |
| ATOM | 1481 | CD2 | PHE | 183 | 11.337 | 10.097 | 27.321 | 1.00 | 47.29 |
| ATOM | 1482 | CE1 | PHE | 183 | 9.739 | 7.929 | 26.592 | 1.00 | 49.51 |
| ATOM | 1483 | CE2 | PHE | 183 | 9.976 | 10.071 | 27.681 | 1.00 | 48.55 |
| ATOM | 1484 | CZ | PHE | 183 | 9.179 | 8.985 | 27.315 | 1.00 | 48.81 |
| ATOM | 1485 | C | PHE | 183 | 15.483 | 10.397 | 26.619 | 1.00 | 28.13 |
| ATOM | 1486 | O | PHE | 183 | 15.826 | 10.610 | 27.771 | 1.00 | 27.21 |
| ATOM | 1487 | N | ALA | 184 | 16.351 | 10.117 | 25.650 | 1.00 | 28.03 |
| ATOM | 1488 | CA | ALA | 184 | 17.785 | 10.060 | 25.907 | 1.00 | 26.92 |
| ATOM | 1489 | CB | ALA | 184 | 18.519 | 9.527 | 24.684 | 1.00 | 26.34 |
| ATOM | 1490 | C | ALA | 184 | 18.291 | 11.460 | 26.240 | 1.00 | 25.39 |
| ATOM | 1491 | O | ALA | 184 | 19.044 | 11.658 | 27.196 | 1.00 | 25.22 |
| ATOM | 1492 | N | LEU | 185 | 17.862 | 12.429 | 25.441 | 1.00 | 24.98 |
| ATOM | 1493 | CA | LEU | 185 | 18.268 | 13.816 | 25.620 | 1.00 | 24.30 |
| ATOM | 1494 | CB | LEU | 185 | 17.704 | 14.671 | 24.488 | 1.00 | 24.30 |
| ATOM | 1495 | CG | LEU | 185 | 17.940 | 16.182 | 24.573 | 1.00 | 24.55 |
| ATOM | 1496 | CD1 | LEU | 185 | 19.449 | 16.479 | 24.607 | 1.00 | 21.33 |
| ATOM | 1497 | CD2 | LEU | 185 | 17.283 | 16.860 | 23.367 | 1.00 | 22.54 |
| ATOM | 1498 | C | LEU | 185 | 17.821 | 14.392 | 26.957 | 1.00 | 24.99 |
| ATOM | 1499 | O | LEU | 185 | 18.537 | 15.178 | 27.569 | 1.00 | 24.67 |
| ATOM | 1500 | N | GLN | 186 | 16.631 | 14.011 | 27.400 | 1.00 | 25.81 |
| ATOM | 1501 | CA | GLN | 186 | 16.103 | 14.506 | 28.660 | 1.00 | 26.62 |
| ATOM | 1502 | CB | GLN | 186 | 14.694 | 13.966 | 28.876 | 1.00 | 30.90 |
| ATOM | 1503 | CG | GLN | 186 | 13.997 | 14.508 | 30.094 | 1.00 | 36.75 |
| ATOM | 1504 | CD | GLN | 186 | 12.629 | 13.896 | 30.263 | 1.00 | 43.23 |
| ATOM | 1505 | OE1 | GLN | 186 | 12.491 | 12.666 | 30.304 | 1.00 | 46.44 |
| ATOM | 1506 | NE2 | GLN | 186 | 11.602 | 14.744 | 30.354 | 1.00 | 43.40 |
| ATOM | 1507 | C | GLN | 186 | 17.006 | 14.073 | 29.807 | 1.00 | 27.38 |
| ATOM | 1508 | O | GLN | 186 | 17.212 | 14.824 | 30.762 | 1.00 | 27.01 |
| ATOM | 1509 | N | HIS | 187 | 17.544 | 12.860 | 29.715 | 1.00 | 27.90 |
| ATOM | 1510 | CA | HIS | 187 | 18.433 | 12.356 | 30.756 | 1.00 | 27.79 |
| ATOM | 1511 | CB | HIS | 187 | 18.610 | 10.846 | 30.604 | 1.00 | 28.15 |
| ATOM | 1512 | CG | HIS | 187 | 17.409 | 10.064 | 31.028 | 1.00 | 29.94 |
| ATOM | 1513 | CD2 | HIS | 187 | 16.329 | 9.642 | 30.330 | 1.00 | 31.20 |
| ATOM | 1514 | ND1 | HIS | 187 | 17.185 | 9.695 | 32.337 | 1.00 | 32.49 |
| ATOM | 1515 | CE1 | HIS | 187 | 16.019 | 9.083 | 32.428 | 1.00 | 30.69 |
| ATOM | 1516 | NE2 | HIS | 187 | 15.478 | 9.038 | 31.225 | 1.00 | 32.71 |
| ATOM | 1517 | C | HIS | 187 | 19.785 | 13.066 | 30.724 | 1.00 | 27.63 |
| ATOM | 1518 | O | HIS | 187 | 20.312 | 13.450 | 31.771 | 1.00 | 25.57 |
| ATOM | 1519 | N | VAL | 188 | 20.337 | 13.251 | 29.526 | 1.00 | 27.19 |
| ATOM | 1520 | CA | VAL | 188 | 21.623 | 13.932 | 29.371 | 1.00 | 26.76 |
| ATOM | 1521 | CB | VAL | 188 | 22.032 | 14.045 | 27.876 | 1.00 | 27.81 |
| ATOM | 1522 | CG1 | VAL | 188 | 23.281 | 14.923 | 27.734 | 1.00 | 25.30 |
| ATOM | 1523 | CG2 | VAL | 188 | 22.283 | 12.657 | 27.301 | 1.00 | 26.52 |
| ATOM | 1524 | C | VAL | 188 | 21.545 | 15.341 | 29.954 | 1.00 | 27.08 |
| ATOM | 1525 | O | VAL | 188 | 22.460 | 15.801 | 30.639 | 1.00 | 27.95 |
| ATOM | 1526 | N | ILE | 189 | 20.444 | 16.023 | 29.675 | 1.00 | 26.81 |
| ATOM | 1527 | CA | ILE | 189 | 20.243 | 17.382 | 30.156 | 1.00 | 28.56 |
| ATOM | 1528 | CB | ILE | 189 | 18.865 | 17.902 | 29.700 | 1.00 | 27.38 |
| ATOM | 1529 | CG2 | ILE | 189 | 18.428 | 19.099 | 30.549 | 1.00 | 27.87 |
| ATOM | 1530 | CG1 | ILE | 189 | 18.940 | 18.263 | 28.213 | 1.00 | 25.31 |
| ATOM | 1531 | CD1 | ILE | 189 | 17.612 | 18.583 | 27.585 | 1.00 | 23.03 |
| ATOM | 1532 | C | ILE | 189 | 20.381 | 17.482 | 31.675 | 1.00 | 29.59 |
| ATOM | 1533 | O | ILE | 189 | 20.810 | 18.504 | 32.205 | 1.00 | 28.17 |
| ATOM | 1534 | N | GLN | 190 | 20.025 | 16.413 | 32.374 | 1.00 | 31.73 |
| ATOM | 1535 | CA | GLN | 190 | 20.130 | 16.401 | 33.824 | 1.00 | 34.66 |
| ATOM | 1536 | CB | GLN | 190 | 19.517 | 15.115 | 34.383 | 1.00 | 35.71 |
| ATOM | 1537 | CG | GLN | 190 | 18.181 | 14.749 | 33.767 | 1.00 | 36.32 |
| ATOM | 1538 | CD | GLN | 190 | 17.525 | 13.575 | 34.469 | 1.00 | 37.87 |
| ATOM | 1539 | OE1 | GLN | 190 | 18.207 | 12.705 | 35.008 | 1.00 | 37.94 |
| ATOM | 1540 | NE2 | GLN | 190 | 16.195 | 13.537 | 34.453 | 1.00 | 37.69 |
| ATOM | 1541 | C | GLN | 190 | 21.589 | 16.522 | 34.282 | 1.00 | 36.42 |
| ATOM | 1542 | O | GLN | 190 | 21.864 | 17.088 | 35.341 | 1.00 | 37.31 |
| ATOM | 1543 | N | LYS | 191 | 22.522 | 15.995 | 33.491 | 1.00 | 36.35 |
| ATOM | 1544 | CA | LYS | 191 | 23.944 | 16.060 | 33.849 | 1.00 | 38.86 |
| ATOM | 1545 | CB | LYS | 191 | 24.816 | 15.373 | 32.798 | 1.00 | 36.60 |
| ATOM | 1546 | CG | LYS | 191 | 24.892 | 13.887 | 32.917 | 1.00 | 34.28 |
| ATOM | 1547 | CD | LYS | 191 | 26.075 | 13.357 | 32.132 | 1.00 | 33.89 |
| ATOM | 1548 | CE | LYS | 191 | 25.937 | 13.646 | 30.650 | 1.00 | 32.20 |
| ATOM | 1549 | NZ | LYS | 191 | 27.090 | 13.103 | 29.891 | 1.00 | 33.09 |
| ATOM | 1550 | C | LYS | 191 | 24.424 | 17.491 | 33.962 | 1.00 | 40.84 |
| ATOM | 1551 | O | LYS | 191 | 25.233 | 17.835 | 34.822 | 1.00 | 40.86 |
| ATOM | 1552 | N | ASN | 192 | 23.928 | 18.318 | 33.056 | 1.00 | 43.43 |
| ATOM | 1553 | CA | ASN | 192 | 24.297 | 19.714 | 33.003 | 1.00 | 45.38 |
| ATOM | 1554 | CB | ASN | 192 | 24.548 | 20.071 | 31.541 | 1.00 | 44.67 |
| ATOM | 1555 | CG | ASN | 192 | 25.345 | 18.985 | 30.817 | 1.00 | 44.92 |
| ATOM | 1556 | OD1 | ASN | 192 | 26.513 | 18.751 | 31.126 | 1.00 | 42.55 |
| ATOM | 1557 | ND2 | ASN | 192 | 24.705 | 18.301 | 29.870 | 1.00 | 43.86 |
| ATOM | 1558 | C | ASN | 192 | 23.151 | 20.518 | 33.607 | 1.00 | 47.90 |
| ATOM | 1559 | O | ASN | 192 | 22.728 | 20.250 | 34.732 | 1.00 | 50.09 |
| ATOM | 1560 | N | HIS | 193 | 22.653 | 21.490 | 32.859 | 1.00 | 48.81 |
| ATOM | 1561 | CA | HIS | 193 | 21.548 | 22.351 | 33.281 | 1.00 | 51.29 |
| ATOM | 1562 | CB | HIS | 193 | 20.616 | 22.551 | 32.091 | 1.00 | 49.68 |
| ATOM | 1563 | CG | HIS | 193 | 21.321 | 22.468 | 30.777 | 1.00 | 49.19 |
| ATOM | 1564 | CD2 | HIS | 193 | 21.274 | 21.535 | 29.798 | 1.00 | 47.56 |
| ATOM | 1565 | ND1 | HIS | 193 | 22.270 | 23.387 | 30.387 | 1.00 | 47.61 |
| ATOM | 1566 | CE1 | HIS | 193 | 22.780 | 23.022 | 29.224 | 1.00 | 48.67 |
| ATOM | 1567 | NE2 | HIS | 193 | 22.193 | 21.901 | 28.846 | 1.00 | 47.30 |
| ATOM | 1568 | C | HIS | 193 | 20.730 | 21.875 | 34.487 | 1.00 | 53.71 |
| ATOM | 1569 | O | HIS | 193 | 19.715 | 21.195 | 34.328 | 1.00 | 53.57 |
| ATOM | 1570 | N | LEU | 194 | 21.168 | 22.233 | 35.692 | 1.00 | 57.22 |
| ATOM | 1571 | CA | LEU | 194 | 20.433 | 21.852 | 36.897 | 1.00 | 60.04 |
| ATOM | 1572 | CB | LEU | 194 | 21.288 | 22.076 | 38.151 | 1.00 | 60.66 |
| ATOM | 1573 | CG | LEU | 194 | 20.990 | 21.191 | 39.372 | 1.00 | 61.69 |
| ATOM | 1574 | CD1 | LEU | 194 | 19.593 | 21.481 | 39.902 | 1.00 | 61.58 |
| ATOM | 1575 | CD2 | LEU | 194 | 21.127 | 19.716 | 38.987 | 1.00 | 60.81 |
| ATOM | 1576 | C | LEU | 194 | 19.240 | 22.797 | 36.874 | 1.00 | 61.24 |
| ATOM | 1577 | O | LEU | 194 | 19.397 | 23.968 | 36.530 | 1.00 | 61.98 |
| ATOM | 1578 | N | ASP | 195 | 18.059 | 22.304 | 37.242 | 1.00 | 62.55 |
| ATOM | 1579 | CA | ASP | 195 | 16.846 | 23.119 | 37.178 | 1.00 | 63.43 |
| ATOM | 1580 | CB | ASP | 195 | 17.019 | 24.440 | 37.937 | 1.00 | 66.10 |
| ATOM | 1581 | CG | ASP | 195 | 16.716 | 24.303 | 39.424 | 1.00 | 67.60 |
| ATOM | 1582 | OD1 | ASP | 195 | 17.314 | 23.423 | 40.082 | 1.00 | 67.56 |
| ATOM | 1583 | OD2 | ASP | 195 | 15.875 | 25.076 | 39.934 | 1.00 | 68.82 |
| ATOM | 1584 | C | ASP | 195 | 16.696 | 23.351 | 35.681 | 1.00 | 62.91 |
| ATOM | 1585 | O | ASP | 195 | 17.481 | 24.071 | 35.062 | 1.00 | 62.29 |
| ATOM | 1586 | N | ASP | 196 | 15.680 | 22.739 | 35.095 | 1.00 | 62.38 |
| ATOM | 1587 | CA | ASP | 196 | 15.525 | 22.819 | 33.661 | 1.00 | 61.54 |
| ATOM | 1588 | CB | ASP | 196 | 15.581 | 21.397 | 33.106 | 1.00 | 62.97 |
| ATOM | 1589 | CG | ASP | 196 | 14.499 | 20.508 | 33.693 | 1.00 | 63.92 |
| ATOM | 1590 | OD1 | ASP | 196 | 13.365 | 20.512 | 33.165 | 1.00 | 63.95 |
| ATOM | 1591 | OD2 | ASP | 196 | 14.777 | 19.817 | 34.697 | 1.00 | 64.35 |
| ATOM | 1592 | C | ASP | 196 | 14.350 | 23.545 | 33.028 | 1.00 | 60.76 |
| ATOM | 1593 | O | ASP | 196 | 14.171 | 24.758 | 33.190 | 1.00 | 59.34 |
| ATOM | 1594 | N | GLU | 197 | 13.572 | 22.747 | 32.298 | 1.00 | 58.84 |
| ATOM | 1595 | CA | GLU | 197 | 12.429 | 23.157 | 31.494 | 1.00 | 57.17 |
| ATOM | 1596 | CB | GLU | 197 | 11.960 | 24.576 | 31.830 | 1.00 | 59.78 |
| ATOM | 1597 | CG | GLU | 197 | 10.901 | 25.109 | 30.876 | 1.00 | 63.41 |
| ATOM | 1598 | CD | GLU | 197 | 10.666 | 26.601 | 31.023 | 1.00 | 66.17 |
| ATOM | 1599 | OE1 | GLU | 197 | 9.831 | 27.144 | 30.268 | 1.00 | 67.86 |
| ATOM | 1600 | OE2 | GLU | 197 | 11.313 | 27.231 | 31.888 | 1.00 | 68.19 |
| ATOM | 1601 | C | GLU | 197 | 13.138 | 23.163 | 30.147 | 1.00 | 53.54 |
| ATOM | 1602 | O | GLU | 197 | 12.537 | 23.364 | 29.092 | 1.00 | 54.00 |
| ATOM | 1603 | N | THR | 198 | 14.446 | 22.924 | 30.229 | 1.00 | 48.95 |
| ATOM | 1604 | CA | THR | 198 | 15.345 | 22.910 | 29.088 | 1.00 | 43.99 |
| ATOM | 1605 | CB | THR | 198 | 16.731 | 22.376 | 29.499 | 1.00 | 43.60 |
| ATOM | 1606 | OG1 | THR | 198 | 17.251 | 23.185 | 30.559 | 1.00 | 42.61 |
| ATOM | 1607 | CG2 | THR | 198 | 17.699 | 22.422 | 28.318 | 1.00 | 43.36 |
| ATOM | 1608 | C | THR | 198 | 14.842 | 22.125 | 27.889 | 1.00 | 39.98 |
| ATOM | 1609 | O | THR | 198 | 14.772 | 22.665 | 26.790 | 1.00 | 39.06 |
| ATOM | 1610 | N | LEU | 199 | 14.497 | 20.857 | 28.084 | 1.00 | 37.03 |
| ATOM | 1611 | CA | LEU | 199 | 14.009 | 20.057 | 26.966 | 1.00 | 35.00 |
| ATOM | 1612 | CB | LEU | 199 | 13.650 | 18.639 | 27.414 | 1.00 | 34.87 |
| ATOM | 1613 | CG | LEU | 199 | 13.972 | 17.518 | 26.418 | 1.00 | 34.97 |
| ATOM | 1614 | CD1 | LEU | 199 | 13.114 | 16.299 | 26.728 | 1.00 | 34.01 |
| ATOM | 1615 | CD2 | LEU | 199 | 13.729 | 17.980 | 25.004 | 1.00 | 33.06 |
| ATOM | 1616 | C | LEU | 199 | 12.774 | 20.725 | 26.362 | 1.00 | 33.27 |
| ATOM | 1617 | O | LEU | 199 | 12.668 | 20.860 | 25.148 | 1.00 | 31.34 |
| ATOM | 1618 | N | ALA | 200 | 11.849 | 21.147 | 27.219 | 1.00 | 32.76 |
| ATOM | 1619 | CA | ALA | 200 | 10.625 | 21.804 | 26.763 | 1.00 | 33.19 |
| ATOM | 1620 | CB | ALA | 200 | 9.706 | 22.120 | 27.963 | 1.00 | 32.22 |
| ATOM | 1621 | C | ALA | 200 | 10.956 | 23.084 | 26.002 | 1.00 | 32.43 |
| ATOM | 1622 | O | ALA | 200 | 10.341 | 23.380 | 24.977 | 1.00 | 31.79 |
| ATOM | 1623 | N | LYS | 201 | 11.931 | 23.841 | 26.497 | 1.00 | 32.28 |
| ATOM | 1624 | CA | LYS | 201 | 12.327 | 25.084 | 25.838 | 1.00 | 32.01 |
| ATOM | 1625 | CB | LYS | 201 | 13.342 | 25.854 | 26.682 | 1.00 | 35.09 |
| ATOM | 1626 | CG | LYS | 201 | 12.853 | 26.322 | 28.036 | 1.00 | 36.56 |
| ATOM | 1627 | CD | LYS | 201 | 13.903 | 27.242 | 28.652 | 1.00 | 40.79 |
| ATOM | 1628 | CE | LYS | 201 | 13.616 | 27.553 | 30.107 | 1.00 | 43.94 |
| ATOM | 1629 | NZ | LYS | 201 | 14.677 | 28.430 | 30.671 | 1.00 | 47.03 |
| ATOM | 1630 | C | LYS | 201 | 12.946 | 24.787 | 24.480 | 1.00 | 30.91 |
| ATOM | 1631 | O | LYS | 201 | 12.775 | 25.552 | 23.531 | 1.00 | 30.11 |
| ATOM | 1632 | N | LEU | 202 | 13.683 | 23.683 | 24.391 | 1.00 | 30.46 |
| ATOM | 1633 | CA | LEU | 202 | 14.307 | 23.297 | 23.129 | 1.00 | 29.29 |
| ATOM | 1634 | CB | LEU | 202 | 15.258 | 22.117 | 23.331 | 1.00 | 28.23 |
| ATOM | 1635 | CG | LEU | 202 | 16.633 | 22.426 | 23.923 | 1.00 | 29.50 |
| ATOM | 1636 | CD1 | LEU | 202 | 17.429 | 21.126 | 24.055 | 1.00 | 27.57 |
| ATOM | 1637 | CD2 | LEU | 202 | 17.367 | 23.417 | 23.022 | 1.00 | 27.80 |
| ATOM | 1638 | C | LEU | 202 | 13.224 | 22.906 | 22.138 | 1.00 | 28.85 |
| ATOM | 1639 | O | LEU | 202 | 13.181 | 23.399 | 21.011 | 1.00 | 30.02 |
| ATOM | 1640 | N | ILE | 203 | 12.348 | 22.011 | 22.567 | 1.00 | 28.20 |
| ATOM | 1641 | CA | ILE | 203 | 11.261 | 21.552 | 21.722 | 1.00 | 29.72 |
| ATOM | 1642 | CB | ILE | 203 | 10.370 | 20.553 | 22.498 | 1.00 | 30.66 |
| ATOM | 1643 | CG2 | ILE | 203 | 9.101 | 20.248 | 21.709 | 1.00 | 31.62 |
| ATOM | 1644 | CG1 | ILE | 203 | 11.165 | 19.271 | 22.774 | 1.00 | 33.22 |
| ATOM | 1645 | CD1 | ILE | 203 | 10.477 | 18.296 | 23.714 | 1.00 | 33.21 |
| ATOM | 1646 | C | ILE | 203 | 10.415 | 22.729 | 21.217 | 1.00 | 28.90 |
| ATOM | 1647 | O | ILE | 203 | 9.962 | 22.734 | 20.073 | 1.00 | 26.56 |
| ATOM | 1648 | N | ALA | 204 | 10.228 | 23.733 | 22.070 | 1.00 | 28.33 |
| ATOM | 1649 | CA | ALA | 204 | 9.434 | 24.904 | 21.716 | 1.00 | 28.52 |
| ATOM | 1650 | CB | ALA | 204 | 9.188 | 25.761 | 22.948 | 1.00 | 29.61 |
| ATOM | 1651 | C | ALA | 204 | 10.076 | 25.754 | 20.629 | 1.00 | 29.35 |
| ATOM | 1652 | O | ALA | 204 | 9.423 | 26.622 | 20.054 | 1.00 | 28.72 |
| ATOM | 1653 | N | LYS | 205 | 11.354 | 25.520 | 20.351 | 1.00 | 27.27 |
| ATOM | 1654 | CA | LYS | 205 | 12.039 | 26.293 | 19.326 | 1.00 | 27.82 |
| ATOM | 1655 | CB | LYS | 205 | 13.525 | 26.443 | 19.674 | 1.00 | 28.06 |
| ATOM | 1656 | CG | LYS | 205 | 13.779 | 27.246 | 20.941 | 1.00 | 31.35 |
| ATOM | 1657 | CD | LYS | 205 | 15.270 | 27.343 | 21.249 | 1.00 | 32.78 |
| ATOM | 1658 | CE | LYS | 205 | 15.505 | 27.938 | 22.624 | 1.00 | 36.42 |
| ATOM | 1659 | NZ | LYS | 205 | 14.840 | 29.262 | 22.784 | 1.00 | 40.34 |
| ATOM | 1660 | C | LYS | 205 | 11.894 | 25.657 | 17.945 | 1.00 | 27.11 |
| ATOM | 1661 | O | LYS | 205 | 12.205 | 26.284 | 16.938 | 1.00 | 28.06 |
| ATOM | 1662 | N | ILE | 206 | 11.417 | 24.418 | 17.897 | 1.00 | 27.34 |
| ATOM | 1663 | CA | ILE | 206 | 11.256 | 23.725 | 16.621 | 1.00 | 28.94 |
| ATOM | 1664 | CB | ILE | 206 | 10.453 | 22.417 | 16.794 | 1.00 | 29.50 |
| ATOM | 1665 | CG2 | ILE | 206 | 10.159 | 21.801 | 15.429 | 1.00 | 31.00 |
| ATOM | 1666 | CG1 | ILE | 206 | 11.214 | 21.447 | 17.703 | 1.00 | 28.15 |
| ATOM | 1667 | CD1 | ILE | 206 | 12.423 | 20.830 | 17.088 | 1.00 | 28.61 |
| ATOM | 1668 | C | ILE | 206 | 10.562 | 24.590 | 15.559 | 1.00 | 29.28 |
| ATOM | 1669 | O | ILE | 206 | 11.094 | 24.783 | 14.462 | 1.00 | 30.10 |
| ATOM | 1670 | N | PRO | 207 | 9.366 | 25.125 | 15.869 | 1.00 | 30.04 |
| ATOM | 1671 | CD | PRO | 207 | 8.554 | 24.973 | 17.089 | 1.00 | 29.62 |
| ATOM | 1672 | CA | PRO | 207 | 8.673 | 25.957 | 14.877 | 1.00 | 30.42 |
| ATOM | 1673 | CB | PRO | 207 | 7.365 | 26.337 | 15.582 | 1.00 | 31.19 |
| ATOM | 1674 | CG | PRO | 207 | 7.695 | 26.204 | 17.041 | 1.00 | 33.14 |
| ATOM | 1675 | C | PRO | 207 | 9.480 | 27.165 | 14.417 | 1.00 | 29.62 |
| ATOM | 1676 | O | PRO | 207 | 9.339 | 27.616 | 13.281 | 1.00 | 29.83 |
| ATOM | 1677 | N | THR | 208 | 10.339 | 27.675 | 15.290 | 1.00 | 26.85 |
| ATOM | 1678 | CA | THR | 208 | 11.162 | 28.819 | 14.935 | 1.00 | 28.12 |
| ATOM | 1679 | CB | THR | 208 | 11.856 | 29.396 | 16.159 | 1.00 | 29.03 |
| ATOM | 1680 | OG1 | THR | 208 | 10.864 | 29.918 | 17.052 | 1.00 | 31.90 |
| ATOM | 1681 | CG2 | THR | 208 | 12.821 | 30.506 | 15.751 | 1.00 | 29.68 |
| ATOM | 1682 | C | THR | 208 | 12.216 | 28.407 | 13.915 | 1.00 | 28.22 |
| ATOM | 1683 | O | THR | 208 | 12.463 | 29.113 | 12.944 | 1.00 | 28.12 |
| ATOM | 1684 | N | ILE | 209 | 12.830 | 27.252 | 14.142 | 1.00 | 26.44 |
| ATOM | 1685 | CA | ILE | 209 | 13.850 | 26.746 | 13.240 | 1.00 | 25.22 |
| ATOM | 1686 | CB | ILE | 209 | 14.375 | 25.383 | 13.732 | 1.00 | 23.83 |
| ATOM | 1687 | CG2 | ILE | 209 | 15.308 | 24.776 | 12.693 | 1.00 | 23.71 |
| ATOM | 1688 | CG1 | ILE | 209 | 15.079 | 25.568 | 15.081 | 1.00 | 21.30 |
| ATOM | 1689 | CD1 | ILE | 209 | 15.644 | 24.294 | 15.685 | 1.00 | 21.88 |
| ATOM | 1690 | C | ILE | 209 | 13.256 | 26.602 | 11.842 | 1.00 | 23.64 |
| ATOM | 1691 | O | ILE | 209 | 13.884 | 26.965 | 10.847 | 1.00 | 24.10 |
| ATOM | 1692 | N | THR | 210 | 12.035 | 26.083 | 11.781 | 1.00 | 23.76 |
| ATOM | 1693 | CA | THR | 210 | 11.334 | 25.888 | 10.520 | 1.00 | 21.79 |
| ATOM | 1694 | CB | THR | 210 | 10.047 | 25.064 | 10.737 | 1.00 | 22.51 |
| ATOM | 1695 | OG1 | THR | 210 | 10.397 | 23.748 | 11.173 | 1.00 | 21.60 |
| ATOM | 1696 | CG2 | THR | 210 | 9.248 | 24.958 | 9.448 | 1.00 | 24.33 |
| ATOM | 1697 | C | THR | 210 | 10.974 | 27.202 | 9.825 | 1.00 | 22.08 |
| ATOM | 1698 | O | THR | 210 | 11.033 | 27.289 | 8.603 | 1.00 | 22.39 |
| ATOM | 1699 | N | ALA | 211 | 10.604 | 28.220 | 10.600 | 1.00 | 22.88 |
| ATOM | 1700 | CA | ALA | 211 | 10.239 | 29.526 | 10.037 | 1.00 | 22.29 |
| ATOM | 1701 | CB | ALA | 211 | 9.678 | 30.435 | 11.127 | 1.00 | 23.28 |
| ATOM | 1702 | C | ALA | 211 | 11.451 | 30.186 | 9.384 | 1.00 | 23.47 |
| ATOM | 1703 | O | ALA | 211 | 11.334 | 30.822 | 8.335 | 1.00 | 23.25 |
| ATOM | 1704 | N | VAL | 212 | 12.617 | 30.045 | 10.006 | 1.00 | 22.80 |
| ATOM | 1705 | CA | VAL | 212 | 13.824 | 30.627 | 9.433 | 1.00 | 23.37 |
| ATOM | 1706 | CB | VAL | 212 | 15.049 | 30.433 | 10.353 | 1.00 | 23.62 |
| ATOM | 1707 | CG1 | VAL | 212 | 16.298 | 30.998 | 9.684 | 1.00 | 22.67 |
| ATOM | 1708 | CG2 | VAL | 212 | 14.811 | 31.125 | 11.682 | 1.00 | 24.14 |
| ATOM | 1709 | C | VAL | 212 | 14.115 | 29.970 | 8.088 | 1.00 | 23.99 |
| ATOM | 1710 | O | VAL | 212 | 14.409 | 30.649 | 7.106 | 1.00 | 25.86 |
| ATOM | 1711 | N | CYS | 213 | 14.012 | 28.645 | 8.041 | 1.00 | 23.57 |
| ATOM | 1712 | CA | CYS | 213 | 14.284 | 27.910 | 6.814 | 1.00 | 25.76 |
| ATOM | 1713 | CB | CYS | 213 | 14.404 | 26.411 | 7.123 | 1.00 | 25.45 |
| ATOM | 1714 | SG | CYS | 213 | 15.842 | 26.040 | 8.202 | 1.00 | 29.19 |
| ATOM | 1715 | C | CYS | 213 | 13.259 | 28.167 | 5.704 | 1.00 | 27.48 |
| ATOM | 1716 | O | CYS | 213 | 13.613 | 28.194 | 4.521 | 1.00 | 26.56 |
| ATOM | 1717 | N | ASN | 214 | 11.994 | 28.358 | 6.063 | 1.00 | 28.96 |
| ATOM | 1718 | CA | ASN | 214 | 10.998 | 28.640 | 5.031 | 1.00 | 30.22 |
| ATOM | 1719 | CB | ASN | 214 | 9.573 | 28.612 | 5.592 | 1.00 | 32.61 |
| ATOM | 1720 | CG | ASN | 214 | 9.129 | 27.218 | 5.987 | 1.00 | 34.72 |
| ATOM | 1721 | OD1 | ASN | 214 | 9.481 | 26.237 | 5.337 | 1.00 | 35.64 |
| ATOM | 1722 | ND2 | ASN | 214 | 8.335 | 27.128 | 7.043 | 1.00 | 36.05 |
| ATOM | 1723 | C | ASN | 214 | 11.297 | 30.021 | 4.473 | 1.00 | 29.75 |
| ATOM | 1724 | O | ASN | 214 | 11.165 | 30.260 | 3.275 | 1.00 | 30.40 |
| ATOM | 1725 | N | LEU | 215 | 11.712 | 30.930 | 5.348 | 1.00 | 28.89 |
| ATOM | 1726 | CA | LEU | 215 | 12.033 | 32.278 | 4.909 | 1.00 | 27.73 |
| ATOM | 1727 | CB | LEU | 215 | 12.411 | 33.151 | 6.104 | 1.00 | 26.66 |
| ATOM | 1728 | CG | LEU | 215 | 12.531 | 34.649 | 5.816 | 1.00 | 28.15 |
| ATOM | 1729 | CD1 | LEU | 215 | 11.296 | 35.147 | 5.064 | 1.00 | 29.37 |
| ATOM | 1730 | CD2 | LEU | 215 | 12.699 | 35.393 | 7.124 | 1.00 | 29.37 |
| ATOM | 1731 | C | LEU | 215 | 13.179 | 32.197 | 3.904 | 1.00 | 27.61 |
| ATOM | 1732 | O | LEU | 215 | 13.206 | 32.939 | 2.918 | 1.00 | 27.88 |
| ATOM | 1733 | N | HIS | 216 | 14.119 | 31.284 | 4.150 | 1.00 | 26.67 |
| ATOM | 1734 | CA | HIS | 216 | 15.246 | 31.081 | 3.244 | 1.00 | 25.98 |
| ATOM | 1735 | CB | HIS | 216 | 16.177 | 29.982 | 3.777 | 1.00 | 26.15 |
| ATOM | 1736 | CG | HIS | 216 | 17.116 | 29.420 | 2.748 | 1.00 | 25.88 |
| ATOM | 1737 | CD2 | HIS | 216 | 18.361 | 29.799 | 2.372 | 1.00 | 25.25 |
| ATOM | 1738 | ND1 | HIS | 216 | 16.785 | 28.351 | 1.942 | 1.00 | 27.09 |
| ATOM | 1739 | CE1 | HIS | 216 | 17.784 | 28.098 | 1.115 | 1.00 | 26.92 |
| ATOM | 1740 | NE2 | HIS | 216 | 18.753 | 28.964 | 1.355 | 1.00 | 24.89 |
| ATOM | 1741 | C | HIS | 216 | 14.692 | 30.674 | 1.884 | 1.00 | 26.04 |
| ATOM | 1742 | O | HIS | 216 | 15.052 | 31.247 | 0.860 | 1.00 | 26.79 |
| ATOM | 1743 | N | GLY | 217 | 13.810 | 29.681 | 1.885 | 1.00 | 26.73 |
| ATOM | 1744 | CA | GLY | 217 | 13.220 | 29.223 | 0.641 | 1.00 | 28.62 |
| ATOM | 1745 | C | GLY | 217 | 12.537 | 30.359 | -0.096 | 1.00 | 30.50 |
| ATOM | 1746 | O | GLY | 217 | 12.648 | 30.471 | -1.314 | 1.00 | 30.23 |
| ATOM | 1747 | N | GLU | 218 | 11.829 | 31.209 | 0.644 | 1.00 | 33.00 |
| ATOM | 1748 | CA | GLU | 218 | 11.128 | 32.344 | 0.050 | 1.00 | 34.40 |
| ATOM | 1749 | CB | GLU | 218 | 10.279 | 33.050 | 1.110 | 1.00 | 37.30 |
| ATOM | 1750 | CG | GLU | 218 | 9.078 | 32.243 | 1.568 | 1.00 | 43.62 |
| ATOM | 1751 | CD | GLU | 218 | 8.375 | 32.862 | 2.765 | 1.00 | 48.13 |
| ATOM | 1752 | OE1 | GLU | 218 | 8.082 | 34.077 | 2.718 | 1.00 | 50.14 |
| ATOM | 1753 | OE2 | GLU | 218 | 8.108 | 32.131 | 3.750 | 1.00 | 50.72 |
| ATOM | 1754 | C | GLU | 218 | 12.092 | 33.338 | -0.590 | 1.00 | 33.43 |
| ATOM | 1755 | O | GLU | 218 | 11.906 | 33.735 | -1.735 | 1.00 | 33.17 |
| ATOM | 1756 | N | LYS | 219 | 13.120 | 33.742 | 0.151 | 1.00 | 33.47 |
| ATOM | 1757 | CA | LYS | 219 | 14.100 | 34.686 | -0.368 | 1.00 | 33.64 |
| ATOM | 1758 | CB | LYS | 219 | 15.188 | 34.972 | 0.677 | 1.00 | 33.37 |
| ATOM | 1759 | CG | LYS | 219 | 14.707 | 35.722 | 1.911 | 1.00 | 34.09 |
| ATOM | 1760 | CD | LYS | 219 | 14.125 | 37.076 | 1.537 | 1.00 | 35.69 |
| ATOM | 1761 | CE | LYS | 219 | 13.682 | 37.852 | 2.765 | 1.00 | 39.13 |
| ATOM | 1762 | NZ | LYS | 219 | 13.047 | 39.158 | 2.399 | 1.00 | 40.00 |
| ATOM | 1763 | C | LYS | 219 | 14.745 | 34.118 | -1.625 | 1.00 | 35.52 |
| ATOM | 1764 | O | LYS | 219 | 15.051 | 34.847 | -2.573 | 1.00 | 34.96 |
| ATOM | 1765 | N | LEU | 220 | 14.950 | 32.807 | -1.626 | 1.00 | 35.22 |
| ATOM | 1766 | CA | LEU | 220 | 15.566 | 32.138 | -2.759 | 1.00 | 36.55 |
| ATOM | 1767 | CB | LEU | 220 | 15.877 | 30.690 | -2.389 | 1.00 | 37.39 |
| ATOM | 1768 | CG | LEU | 220 | 16.647 | 29.830 | -3.383 | 1.00 | 38.60 |
| ATOM | 1769 | CD1 | LEU | 220 | 17.945 | 30.519 | -3.792 | 1.00 | 38.29 |
| ATOM | 1770 | CD2 | LEU | 220 | 16.936 | 28.489 | -2.729 | 1.00 | 39.42 |
| ATOM | 1771 | C | LEU | 220 | 14.652 | 32.186 | -3.979 | 1.00 | 37.19 |
| ATOM | 1772 | O | LEU | 220 | 15.102 | 32.465 | -5.086 | 1.00 | 37.42 |
| ATOM | 1773 | N | GLN | 221 | 13.369 | 31.916 | -3.773 | 1.00 | 38.14 |
| ATOM | 1774 | CA | GLN | 221 | 12.409 | 31.930 | -4.870 | 1.00 | 39.61 |
| ATOM | 1775 | CB | GLN | 221 | 11.002 | 31.632 | -4.350 | 1.00 | 43.16 |
| ATOM | 1776 | CG | GLN | 221 | 10.849 | 30.214 | -3.829 | 1.00 | 47.61 |
| ATOM | 1777 | CD | GLN | 221 | 11.363 | 29.177 | -4.820 | 1.00 | 51.15 |
| ATOM | 1778 | OE1 | GLN | 221 | 10.772 | 28.966 | -5.881 | 1.00 | 52.70 |
| ATOM | 1779 | NE2 | GLN | 221 | 12.480 | 28.534 | -4.479 | 1.00 | 51.82 |
| ATOM | 1780 | C | GLN | 221 | 12.420 | 33.261 | -5.607 | 1.00 | 38.21 |
| ATOM | 1781 | O | GLN | 221 | 12.365 | 33.296 | -6.835 | 1.00 | 37.75 |
| ATOM | 1782 | N | VAL | 222 | 12.497 | 34.354 | -4.857 | 1.00 | 36.97 |
| ATOM | 1783 | CA | VAL | 222 | 12.529 | 35.676 | -5.467 | 1.00 | 37.21 |
| ATOM | 1784 | CB | VAL | 222 | 12.377 | 36.795 | -4.411 | 1.00 | 36.85 |
| ATOM | 1785 | CG1 | VAL | 222 | 12.444 | 38.154 | -5.086 | 1.00 | 37.24 |
| ATOM | 1786 | CG2 | VAL | 222 | 11.050 | 36.645 | -3.678 | 1.00 | 37.77 |
| ATOM | 1787 | C | VAL | 222 | 13.846 | 35.867 | -6.217 | 1.00 | 37.47 |
| ATOM | 1788 | O | VAL | 222 | 13.869 | 36.424 | -7.316 | 1.00 | 37.13 |
| ATOM | 1789 | N | PHE | 223 | 14.943 | 35.400 | -5.624 | 1.00 | 37.83 |
| ATOM | 1790 | CA | PHE | 223 | 16.248 | 35.521 | -6.268 | 1.00 | 39.20 |
| ATOM | 1791 | CB | PHE | 223 | 17.349 | 34.906 | -5.392 | 1.00 | 37.95 |
| ATOM | 1792 | CG | PHE | 223 | 18.731 | 35.023 | -5.984 | 1.00 | 39.28 |
| ATOM | 1793 | CD1 | PHE | 223 | 19.405 | 36.238 | -5.975 | 1.00 | 39.67 |
| ATOM | 1794 | CD2 | PHE | 223 | 19.339 | 33.927 | -6.589 | 1.00 | 39.69 |
| ATOM | 1795 | CE1 | PHE | 223 | 20.666 | 36.362 | -6.565 | 1.00 | 41.73 |
| ATOM | 1796 | CE2 | PHE | 223 | 20.600 | 34.042 | -7.180 | 1.00 | 40.32 |
| ATOM | 1797 | CZ | PHE | 223 | 21.262 | 35.263 | -7.168 | 1.00 | 39.45 |
| ATOM | 1798 | C | PHE | 223 | 16.201 | 34.789 | -7.612 | 1.00 | 40.64 |
| ATOM | 1799 | O | PHE | 223 | 16.767 | 35.249 | -8.603 | 1.00 | 40.48 |
| ATOM | 1800 | N | LYS | 224 | 15.520 | 33.647 | -7.634 | 1.00 | 42.33 |
| ATOM | 1801 | CA | LYS | 224 | 15.399 | 32.857 | -8.851 | 1.00 | 45.23 |
| ATOM | 1802 | CB | LYS | 224 | 14.629 | 31.560 | -8.571 | 1.00 | 47.13 |
| ATOM | 1803 | CG | LYS | 224 | 14.588 | 30.593 | -9.749 | 1.00 | 50.45 |
| ATOM | 1804 | CD | LYS | 224 | 13.866 | 29.303 | -9.383 | 1.00 | 52.28 |
| ATOM | 1805 | CE | LYS | 224 | 13.785 | 28.351 | -10.572 | 1.00 | 54.02 |
| ATOM | 1806 | NZ | LYS | 224 | 13.051 | 27.088 | -10.246 | 1.00 | 54.04 |
| ATOM | 1807 | C | LYS | 224 | 14.688 | 33.677 | -9.925 | 1.00 | 45.85 |
| ATOM | 1808 | O | LYS | 224 | 14.990 | 33.545 | -11.108 | 1.00 | 45.85 |
| ATOM | 1809 | N | GLN | 225 | 13.748 | 34.525 | -9.509 | 1.00 | 47.18 |
| ATOM | 1810 | CA | GLN | 225 | 13.020 | 35.378 | -10.448 | 1.00 | 48.45 |
| ATOM | 1811 | CB | GLN | 225 | 11.936 | 36.196 | -9.732 | 1.00 | 49.96 |
| ATOM | 1812 | CG | GLN | 225 | 10.784 | 35.397 | -9.136 | 1.00 | 51.68 |
| ATOM | 1813 | CD | GLN | 225 | 9.678 | 36.298 | -8.600 | 1.00 | 53.23 |
| ATOM | 1814 | OE1 | GLN | 225 | 9.911 | 37.141 | -7.732 | 1.00 | 52.56 |
| ATOM | 1815 | NE2 | GLN | 225 | 8.470 | 36.127 | -9.123 | 1.00 | 53.37 |
| ATOM | 1816 | C | GLN | 225 | 13.990 | 36.352 | -11.106 | 1.00 | 47.94 |
| ATOM | 1817 | O | GLN | 225 | 14.094 | 36.417 | -12.331 | 1.00 | 48.26 |
| ATOM | 1818 | N | SER | 226 | 14.698 | 37.107 | -10.272 | 1.00 | 48.06 |
| ATOM | 1819 | CA | SER | 226 | 15.658 | 38.104 | -10.731 | 1.00 | 47.80 |
| ATOM | 1820 | CB | SER | 226 | 16.139 | 38.940 | -9.542 | 1.00 | 47.91 |
| ATOM | 1821 | OG | SER | 226 | 15.055 | 39.594 | -8.906 | 1.00 | 49.47 |
| ATOM | 1822 | C | SER | 226 | 16.871 | 37.533 | -11.467 | 1.00 | 47.59 |
| ATOM | 1823 | O | SER | 226 | 17.314 | 38.099 | -12.464 | 1.00 | 46.97 |
| ATOM | 1824 | N | HIS | 227 | 17.414 | 36.422 | -10.979 | 1.00 | 47.93 |
| ATOM | 1825 | CA | HIS | 227 | 18.586 | 35.827 | -11.614 | 1.00 | 47.70 |
| ATOM | 1826 | CB | HIS | 227 | 19.831 | 36.103 | -10.769 | 1.00 | 48.42 |
| ATOM | 1827 | CG | HIS | 227 | 19.920 | 37.513 | -10.273 | 1.00 | 48.29 |
| ATOM | 1828 | CD2 | HIS | 227 | 20.711 | 38.543 | -10.651 | 1.00 | 48.92 |
| ATOM | 1829 | ND1 | HIS | 227 | 19.120 | 37.996 | -9.260 | 1.00 | 48.46 |
| ATOM | 1830 | CE1 | HIS | 227 | 19.418 | 39.263 | -9.033 | 1.00 | 49.03 |
| ATOM | 1831 | NE2 | HIS | 227 | 20.381 | 39.620 | -9.864 | 1.00 | 50.21 |
| ATOM | 1832 | C | HIS | 227 | 18.439 | 34.324 | -11.826 | 1.00 | 48.58 |
| ATOM | 1833 | O | HIS | 227 | 19.133 | 33.523 | -11.189 | 1.00 | 47.32 |
| ATOM | 1834 | N | PRO | 228 | 17.543 | 33.921 | -12.741 | 1.00 | 48.73 |
| ATOM | 1835 | CD | PRO | 228 | 16.807 | 34.782 | -13.684 | 1.00 | 49.28 |
| ATOM | 1836 | CA | PRO | 228 | 17.303 | 32.504 | -13.039 | 1.00 | 50.04 |
| ATOM | 1837 | CB | PRO | 228 | 16.245 | 32.563 | -14.143 | 1.00 | 49.26 |
| ATOM | 1838 | CG | PRO | 228 | 16.574 | 33.846 | -14.849 | 1.00 | 48.18 |
| ATOM | 1839 | C | PRO | 228 | 18.554 | 31.735 | -13.471 | 1.00 | 50.55 |
| ATOM | 1840 | O | PRO | 228 | 18.823 | 30.641 | -12.974 | 1.00 | 49.03 |
| ATOM | 1841 | N | ASP | 229 | 19.317 | 32.315 | -14.392 | 1.00 | 51.87 |
| ATOM | 1842 | CA | ASP | 229 | 20.525 | 31.674 | -14.901 | 1.00 | 53.58 |
| ATOM | 1843 | CB | ASP | 229 | 21.177 | 32.564 | -15.964 | 1.00 | 55.87 |
| ATOM | 1844 | CG | ASP | 229 | 22.285 | 31.852 | -16.723 | 1.00 | 58.49 |
| ATOM | 1845 | OD1 | ASP | 229 | 22.014 | 30.790 | -17.328 | 1.00 | 58.29 |
| ATOM | 1846 | OD2 | ASP | 229 | 23.429 | 32.356 | -16.719 | 1.00 | 61.04 |
| ATOM | 1847 | C | ASP | 229 | 21.543 | 31.343 | -13.806 | 1.00 | 53.73 |
| ATOM | 1848 | O | ASP | 229 | 22.073 | 30.232 | -13.765 | 1.00 | 53.65 |
| ATOM | 1849 | N | ILE | 230 | 21.817 | 32.304 | -12.925 | 1.00 | 52.95 |
| ATOM | 1850 | CA | ILE | 230 | 22.776 | 32.094 | -11.838 | 1.00 | 52.12 |
| ATOM | 1851 | CB | ILE | 230 | 22.850 | 33.326 | -10.891 | 1.00 | 52.89 |
| ATOM | 1852 | CG2 | ILE | 230 | 23.663 | 32.984 | -9.644 | 1.00 | 51.95 |
| ATOM | 1853 | CG1 | ILE | 230 | 23.479 | 34.520 | -11.616 | 1.00 | 53.24 |
| ATOM | 1854 | CD1 | ILE | 230 | 22.607 | 35.126 | -12.699 | 1.00 | 55.39 |
| ATOM | 1855 | C | ILE | 230 | 22.405 | 30.868 | -11.002 | 1.00 | 51.37 |
| ATOM | 1856 | O | ILE | 230 | 23.277 | 30.119 | -10.553 | 1.00 | 50.26 |
| ATOM | 1857 | N | VAL | 231 | 21.105 | 30.672 | -10.803 | 1.00 | 49.90 |
| ATOM | 1858 | CA | VAL | 231 | 20.601 | 29.554 | -10.015 | 1.00 | 48.50 |
| ATOM | 1859 | CB | VAL | 231 | 19.103 | 29.753 | -9.683 | 1.00 | 48.35 |
| ATOM | 1860 | CG1 | VAL | 231 | 18.587 | 28.582 | -8.857 | 1.00 | 47.45 |
| ATOM | 1861 | CG2 | VAL | 231 | 18.908 | 31.063 | -8.933 | 1.00 | 48.33 |
| ATOM | 1862 | C | VAL | 231 | 20.769 | 28.200 | -10.701 | 1.00 | 48.45 |
| ATOM | 1863 | O | VAL | 231 | 21.269 | 27.245 | -10.105 | 1.00 | 46.75 |
| ATOM | 1864 | N | ASN | 232 | 20.353 | 28.124 | -11.959 | 1.00 | 48.93 |
| ATOM | 1865 | CA | ASN | 232 | 20.432 | 26.880 | -12.708 | 1.00 | 49.80 |
| ATOM | 1866 | CB | ASN | 232 | 19.459 | 26.920 | -13.889 | 1.00 | 53.19 |
| ATOM | 1867 | CG | ASN | 232 | 18.019 | 27.092 | -13.448 | 1.00 | 56.24 |
| ATOM | 1868 | OD1 | ASN | 232 | 17.513 | 26.322 | -12.630 | 1.00 | 57.63 |
| ATOM | 1869 | ND2 | ASN | 232 | 17.347 | 28.105 | -13.991 | 1.00 | 57.88 |
| ATOM | 1870 | C | ASN | 232 | 21.816 | 26.527 | -13.222 | 1.00 | 48.52 |
| ATOM | 1871 | O | ASN | 232 | 22.077 | 25.366 | -13.534 | 1.00 | 48.20 |
| ATOM | 1872 | N | THR | 233 | 22.710 | 27.508 | -13.300 | 1.00 | 47.20 |
| ATOM | 1873 | CA | THR | 233 | 24.042 | 27.241 | -13.831 | 1.00 | 46.67 |
| ATOM | 1874 | CB | THR | 233 | 24.295 | 28.057 | -15.122 | 1.00 | 46.77 |
| ATOM | 1875 | OG1 | THR | 233 | 24.612 | 29.414 | -14.787 | 1.00 | 47.29 |
| ATOM | 1876 | CG2 | THR | 233 | 23.055 | 28.045 | -16.001 | 1.00 | 46.89 |
| ATOM | 1877 | C | THR | 233 | 25.209 | 27.492 | -12.887 | 1.00 | 45.74 |
| ATOM | 1878 | O | THR | 233 | 26.313 | 26.999 | -13.121 | 1.00 | 46.15 |
| ATOM | 1879 | N | LEU | 234 | 24.987 | 28.256 | -11.825 | 1.00 | 44.27 |
| ATOM | 1880 | CA | LEU | 234 | 26.078 | 28.536 | -10.906 | 1.00 | 42.05 |
| ATOM | 1881 | CB | LEU | 234 | 26.278 | 30.049 | -10.789 | 1.00 | 43.57 |
| ATOM | 1882 | CG | LEU | 234 | 26.870 | 30.673 | -12.058 | 1.00 | 44.97 |
| ATOM | 1883 | CD1 | LEU | 234 | 26.979 | 32.180 | -11.909 | 1.00 | 46.57 |
| ATOM | 1884 | CD2 | LEU | 234 | 28.240 | 30.068 | -12.318 | 1.00 | 45.46 |
| ATOM | 1885 | C | LEU | 234 | 25.950 | 27.899 | -9.523 | 1.00 | 40.08 |
| ATOM | 1886 | O | LEU | 234 | 26.941 | 27.410 | -8.978 | 1.00 | 41.11 |
| ATOM | 1887 | N | PHE | 235 | 24.745 | 27.889 | -8.960 | 1.00 | 36.54 |
| ATOM | 1888 | CA | PHE | 235 | 24.535 | 27.295 | -7.637 | 1.00 | 33.39 |
| ATOM | 1889 | CB | PHE | 235 | 23.080 | 27.480 | -7.190 | 1.00 | 33.46 |
| ATOM | 1890 | CG | PHE | 235 | 22.786 | 28.827 | -6.591 | 1.00 | 31.78 |
| ATOM | 1891 | CD1 | PHE | 235 | 23.700 | 29.867 | -6.683 | 1.00 | 33.45 |
| ATOM | 1892 | CD2 | PHE | 235 | 21.579 | 29.057 | -5.942 | 1.00 | 33.88 |
| ATOM | 1893 | CE1 | PHE | 235 | 23.416 | 31.120 | -6.137 | 1.00 | 34.74 |
| ATOM | 1894 | CE2 | PHE | 235 | 21.285 | 30.308 | -5.393 | 1.00 | 33.31 |
| ATOM | 1895 | CZ | PHE | 235 | 22.205 | 31.338 | -5.492 | 1.00 | 33.08 |
| ATOM | 1896 | C | PHE | 235 | 24.856 | 25.807 | -7.651 | 1.00 | 30.87 |
| ATOM | 1897 | O | PHE | 235 | 24.744 | 25.154 | -8.687 | 1.00 | 29.90 |
| ATOM | 1898 | N | PRO | 236 | 25.259 | 25.251 | -6.498 | 1.00 | 29.01 |
| ATOM | 1899 | CD | PRO | 236 | 25.543 | 25.924 | -5.220 | 1.00 | 27.51 |
| ATOM | 1900 | CA | PRO | 236 | 25.586 | 23.825 | -6.408 | 1.00 | 28.71 |
| ATOM | 1901 | CB | PRO | 236 | 25.967 | 23.652 | -4.941 | 1.00 | 27.24 |
| ATOM | 1902 | CG | PRO | 236 | 26.543 | 24.992 | -4.598 | 1.00 | 28.61 |
| ATOM | 1903 | C | PRO | 236 | 24.372 | 22.985 | -6.788 | 1.00 | 29.15 |
| ATOM | 1904 | O | PRO | 236 | 23.257 | 23.245 | -6.336 | 1.00 | 27.23 |
| ATOM | 1905 | N | PRO | 237 | 24.573 | 21.966 | -7.630 | 1.00 | 29.03 |
| ATOM | 1906 | CD | PRO | 237 | 25.811 | 21.603 | -8.341 | 1.00 | 28.29 |
| ATOM | 1907 | CA | PRO | 237 | 23.460 | 21.113 | -8.047 | 1.00 | 29.54 |
| ATOM | 1908 | CB | PRO | 237 | 24.166 | 19.971 | -8.765 | 1.00 | 29.15 |
| ATOM | 1909 | CG | PRO | 237 | 25.285 | 20.697 | -9.457 | 1.00 | 30.03 |
| ATOM | 1910 | C | PRO | 237 | 22.561 | 20.642 | -6.898 | 1.00 | 30.20 |
| ATOM | 1911 | O | PRO | 237 | 21.334 | 20.703 | -7.011 | 1.00 | 28.77 |
| ATOM | 1912 | N | LEU | 238 | 23.159 | 20.193 | -5.795 | 1.00 | 29.38 |
| ATOM | 1913 | CA | LEU | 238 | 22.371 | 19.720 | -4.652 | 1.00 | 29.57 |
| ATOM | 1914 | CB | LEU | 238 | 23.280 | 19.148 | -3.558 | 1.00 | 28.08 |
| ATOM | 1915 | CG | LEU | 238 | 22.562 | 18.712 | -2.271 | 1.00 | 27.84 |
| ATOM | 1916 | CD1 | LEU | 238 | 21.542 | 17.633 | -2.597 | 1.00 | 28.16 |
| ATOM | 1917 | CD2 | LEU | 238 | 23.573 | 18.196 | -1.250 | 1.00 | 25.13 |
| ATOM | 1918 | C | LEU | 238 | 21.504 | 20.828 | -4.060 | 1.00 | 31.04 |
| ATOM | 1919 | O | LEU | 238 | 20.385 | 20.579 | -3.608 | 1.00 | 31.07 |
| ATOM | 1920 | N | TYR | 239 | 22.022 | 22.051 | -4.062 | 1.00 | 30.34 |
| ATOM | 1921 | CA | TYR | 239 | 21.290 | 23.189 | -3.524 | 1.00 | 31.47 |
| ATOM | 1922 | CB | TYR | 239 | 22.196 | 24.419 | -3.509 | 1.00 | 30.35 |
| ATOM | 1923 | CG | TYR | 239 | 21.607 | 25.640 | -2.836 | 1.00 | 29.41 |
| ATOM | 1924 | CD1 | TYR | 239 | 20.775 | 26.513 | -3.535 | 1.00 | 30.76 |
| ATOM | 1925 | CE1 | TYR | 239 | 20.284 | 27.672 | -2.934 | 1.00 | 30.38 |
| ATOM | 1926 | CD2 | TYR | 239 | 21.926 | 25.950 | -1.514 | 1.00 | 28.97 |
| ATOM | 1927 | CE2 | TYR | 239 | 21.442 | 27.101 | -0.904 | 1.00 | 30.43 |
| ATOM | 1928 | CZ | TYR | 239 | 20.625 | 27.960 | -1.624 | 1.00 | 30.74 |
| ATOM | 1929 | OH | TYR | 239 | 20.177 | 29.117 | -1.037 | 1.00 | 35.16 |
| ATOM | 1930 | C | TYR | 239 | 20.056 | 23.442 | -4.384 | 1.00 | 33.26 |
| ATOM | 1931 | O | TYR | 239 | 18.969 | 23.714 | -3.870 | 1.00 | 32.84 |
| ATOM | 1932 | N | LYS | 240 | 20.223 | 23.348 | -5.699 | 1.00 | 33.87 |
| ATOM | 1933 | CA | LYS | 240 | 19.100 | 23.546 | -6.605 | 1.00 | 35.74 |
| ATOM | 1934 | CB | LYS | 240 | 19.580 | 23.506 | -8.058 | 1.00 | 37.63 |
| ATOM | 1935 | CG | LYS | 240 | 18.468 | 23.659 | -9.083 | 1.00 | 43.97 |
| ATOM | 1936 | CD | LYS | 240 | 19.026 | 23.682 | -10.503 | 1.00 | 47.75 |
| ATOM | 1937 | CE | LYS | 240 | 17.906 | 23.676 | -11.535 | 1.00 | 51.07 |
| ATOM | 1938 | NZ | LYS | 240 | 18.426 | 23.651 | -12.941 | 1.00 | 52.39 |
| ATOM | 1939 | C | LYS | 240 | 18.059 | 22.447 | -6.370 | 1.00 | 35.10 |
| ATOM | 1940 | O | LYS | 240 | 16.862 | 22.721 | -6.270 | 1.00 | 35.12 |
| ATOM | 1941 | N | GLU | 241 | 18.529 | 21.209 | -6.259 | 1.00 | 33.16 |
| ATOM | 1942 | CA | GLU | 241 | 17.657 | 20.060 | -6.053 | 1.00 | 33.21 |
| ATOM | 1943 | CB | GLU | 241 | 18.483 | 18.767 | -6.055 | 1.00 | 33.06 |
| ATOM | 1944 | CG | GLU | 241 | 17.684 | 17.505 | -5.711 | 1.00 | 33.33 |
| ATOM | 1945 | CD | GLU | 241 | 18.533 | 16.243 | -5.731 | 1.00 | 32.45 |
| ATOM | 1946 | OE1 | GLU | 241 | 19.105 | 15.919 | -6.792 | 1.00 | 35.20 |
| ATOM | 1947 | OE2 | GLU | 241 | 18.629 | 15.569 | -4.687 | 1.00 | 33.93 |
| ATOM | 1948 | C | GLU | 241 | 16.832 | 20.125 | -4.776 | 1.00 | 33.04 |
| ATOM | 1949 | O | GLU | 241 | 15.664 | 19.747 | -4.771 | 1.00 | 34.27 |
| ATOM | 1950 | N | LEU | 242 | 17.434 | 20.607 | -3.695 | 1.00 | 33.22 |
| ATOM | 1951 | CA | LEU | 242 | 16.740 | 20.682 | -2.416 | 1.00 | 34.25 |
| ATOM | 1952 | CB | LEU | 242 | 17.754 | 20.729 | -1.271 | 1.00 | 32.75 |
| ATOM | 1953 | CG | LEU | 242 | 18.705 | 19.539 | -1.138 | 1.00 | 34.23 |
| ATOM | 1954 | CD1 | LEU | 242 | 19.752 | 19.841 | -0.083 | 1.00 | 35.05 |
| ATOM | 1955 | CD2 | LEU | 242 | 17.926 | 18.292 | -0.779 | 1.00 | 35.31 |
| ATOM | 1956 | C | LEU | 242 | 15.782 | 21.853 | -2.256 | 1.00 | 35.37 |
| ATOM | 1957 | O | LEU | 242 | 14.755 | 21.721 | -1.597 | 1.00 | 34.72 |
| ATOM | 1958 | N | PHE | 243 | 16.113 | 22.992 | -2.854 | 1.00 | 37.53 |
| ATOM | 1959 | CA | PHE | 243 | 15.286 | 24.182 | -2.710 | 1.00 | 41.05 |
| ATOM | 1960 | CB | PHE | 243 | 16.129 | 25.297 | -2.090 | 1.00 | 38.64 |
| ATOM | 1961 | CG | PHE | 243 | 16.939 | 24.850 | -0.897 | 1.00 | 37.15 |
| ATOM | 1962 | CD1 | PHE | 243 | 18.329 | 24.797 | -0.962 | 1.00 | 34.33 |
| ATOM | 1963 | CD2 | PHE | 243 | 16.309 | 24.446 | 0.277 | 1.00 | 34.27 |
| ATOM | 1964 | CE1 | PHE | 243 | 19.082 | 24.346 | 0.123 | 1.00 | 34.36 |
| ATOM | 1965 | CE2 | PHE | 243 | 17.051 | 23.992 | 1.369 | 1.00 | 36.44 |
| ATOM | 1966 | CZ | PHE | 243 | 18.442 | 23.941 | 1.291 | 1.00 | 34.07 |
| ATOM | 1967 | C | PHE | 243 | 14.594 | 24.690 | -3.975 | 1.00 | 45.43 |
| ATOM | 1968 | O | PHE | 243 | 14.154 | 25.842 | -4.021 | 1.00 | 45.60 |
| ATOM | 1969 | N | ASN | 244 | 14.496 | 23.835 | -4.992 | 1.00 | 50.27 |
| ATOM | 1970 | CA | ASN | 244 | 13.827 | 24.187 | -6.248 | 1.00 | 54.32 |
| ATOM | 1971 | CB | ASN | 244 | 14.845 | 24.575 | -7.327 | 1.00 | 55.30 |
| ATOM | 1972 | CG | ASN | 244 | 15.432 | 25.954 | -7.105 | 1.00 | 58.57 |
| ATOM | 1973 | OD1 | ASN | 244 | 14.720 | 26.961 | -7.151 | 1.00 | 59.86 |
| ATOM | 1974 | ND2 | ASN | 244 | 16.737 | 26.010 | -6.861 | 1.00 | 60.33 |
| ATOM | 1975 | C | ASN | 244 | 12.977 | 23.028 | -6.749 | 1.00 | 55.97 |
| ATOM | 1976 | O | ASN | 244 | 11.823 | 23.215 | -7.137 | 1.00 | 58.40 |
| ATOM | 1977 | N | HIS | 691 | 15.075 | 13.032 | -6.848 | 1.00 | 41.98 |
| ATOM | 1978 | CA | HIS | 691 | 16.238 | 13.605 | -6.167 | 1.00 | 42.61 |
| ATOM | 1979 | CB | HIS | 691 | 15.999 | 13.671 | -4.658 | 1.00 | 45.30 |
| ATOM | 1980 | CG | HIS | 691 | 14.679 | 14.268 | -4.285 | 1.00 | 47.14 |
| ATOM | 1981 | CD2 | HIS | 691 | 14.328 | 15.542 | -3.992 | 1.00 | 47.32 |
| ATOM | 1982 | ND1 | HIS | 691 | 13.518 | 13.527 | -4.227 | 1.00 | 48.40 |
| ATOM | 1983 | CE1 | HIS | 691 | 12.509 | 14.318 | -3.913 | 1.00 | 49.67 |
| ATOM | 1984 | NE2 | HIS | 691 | 12.973 | 15.547 | -3.765 | 1.00 | 50.69 |
| ATOM | 1985 | C | HIS | 691 | 17.466 | 12.756 | -6.442 | 1.00 | 41.10 |
| ATOM | 1986 | O | HIS | 691 | 17.940 | 12.027 | -5.570 | 1.00 | 39.31 |
| ATOM | 1987 | N | LYS | 692 | 17.977 | 12.862 | -7.661 | 1.00 | 40.46 |
| ATOM | 1988 | CA | LYS | 692 | 19.136 | 12.092 | -8.079 | 1.00 | 40.37 |
| ATOM | 1989 | CB | LYS | 692 | 19.497 | 12.451 | -9.523 | 1.00 | 42.93 |
| ATOM | 1990 | CG | LYS | 692 | 20.567 | 11.554 | -10.137 | 1.00 | 47.97 |
| ATOM | 1991 | CD | LYS | 692 | 20.901 | 11.967 | -11.569 | 1.00 | 51.10 |
| ATOM | 1992 | CE | LYS | 692 | 21.771 | 10.913 | -12.257 | 1.00 | 52.83 |
| ATOM | 1993 | NZ | LYS | 692 | 23.026 | 10.630 | -11.497 | 1.00 | 54.26 |
| ATOM | 1994 | C | LYS | 692 | 20.354 | 12.299 | -7.177 | 1.00 | 38.35 |
| ATOM | 1995 | O | LYS | 692 | 20.951 | 11.336 | -6.697 | 1.00 | 37.83 |
| ATOM | 1996 | N | ILE | 693 | 20.714 | 13.553 | -6.941 | 1.00 | 35.38 |
| ATOM | 1997 | CA | ILE | 693 | 21.882 | 13.854 | -6.122 | 1.00 | 35.12 |
| ATOM | 1998 | CB | ILE | 693 | 22.166 | 15.363 | -6.123 | 1.00 | 32.22 |
| ATOM | 1999 | CG2 | ILE | 693 | 23.426 | 15.656 | -5.328 | 1.00 | 33.60 |
| ATOM | 2000 | CG1 | ILE | 693 | 22.336 | 15.844 | -7.568 | 1.00 | 34.55 |
| ATOM | 2001 | CD1 | ILE | 693 | 22.439 | 17.342 | -7.716 | 1.00 | 32.01 |
| ATOM | 2002 | C | ILE | 693 | 21.788 | 13.355 | -4.678 | 1.00 | 33.70 |
| ATOM | 2003 | O | ILE | 693 | 22.664 | 12.636 | -4.205 | 1.00 | 31.97 |
| ATOM | 2004 | N | LEU | 694 | 20.730 | 13.742 | -3.978 | 1.00 | 33.87 |
| ATOM | 2005 | CA | LEU | 694 | 20.560 | 13.328 | -2.592 | 1.00 | 35.23 |
| ATOM | 2006 | CB | LEU | 694 | 19.232 | 13.866 | -2.050 | 1.00 | 36.03 |
| ATOM | 2007 | CG | LEU | 694 | 18.964 | 13.754 | -0.549 | 1.00 | 36.23 |
| ATOM | 2008 | CD1 | LEU | 694 | 20.078 | 14.436 | 0.243 | 1.00 | 33.63 |
| ATOM | 2009 | CD2 | LEU | 694 | 17.622 | 14.395 | -0.241 | 1.00 | 34.69 |
| ATOM | 2010 | C | LEU | 694 | 20.587 | 11.804 | -2.520 | 1.00 | 35.61 |
| ATOM | 2011 | O | LEU | 694 | 21.159 | 11.212 | -1.602 | 1.00 | 33.23 |
| ATOM | 2012 | N | HIS | 695 | 19.982 | 11.178 | -3.520 | 1.00 | 36.63 |
| ATOM | 2013 | CA | HIS | 695 | 19.913 | 9.729 | -3.595 | 1.00 | 39.34 |
| ATOM | 2014 | CB | HIS | 695 | 19.071 | 9.324 | -4.805 | 1.00 | 43.87 |
| ATOM | 2015 | CG | HIS | 695 | 18.316 | 8.049 | -4.616 | 1.00 | 50.18 |
| ATOM | 2016 | CD2 | HIS | 695 | 16.985 | 7.800 | -4.582 | 1.00 | 51.51 |
| ATOM | 2017 | ND1 | HIS | 695 | 18.939 | 6.831 | -4.437 | 1.00 | 52.83 |
| ATOM | 2018 | CE1 | HIS | 695 | 18.024 | 5.887 | -4.305 | 1.00 | 52.75 |
| ATOM | 2019 | NE2 | HIS | 695 | 16.830 | 6.448 | -4.389 | 1.00 | 53.18 |
| ATOM | 2020 | C | HIS | 695 | 21.317 | 9.153 | -3.717 | 1.00 | 39.12 |
| ATOM | 2021 | O | HIS | 695 | 21.658 | 8.158 | -3.068 | 1.00 | 40.09 |
| ATOM | 2022 | N | ARG | 696 | 22.139 | 9.784 | -4.545 | 1.00 | 37.19 |
| ATOM | 2023 | CA | ARG | 696 | 23.496 | 9.307 | -4.730 | 1.00 | 36.26 |
| ATOM | 2024 | CB | ARG | 696 | 24.199 | 10.085 | -5.835 | 1.00 | 37.76 |
| ATOM | 2025 | CG | ARG | 696 | 25.596 | 9.562 | -6.085 | 1.00 | 39.24 |
| ATOM | 2026 | CD | ARG | 696 | 26.427 | 10.510 | -6.899 | 1.00 | 40.46 |
| ATOM | 2027 | NE | ARG | 696 | 27.767 | 9.968 | -7.097 | 1.00 | 43.23 |
| ATOM | 2028 | CZ | ARG | 696 | 28.741 | 10.610 | -7.730 | 1.00 | 44.16 |
| ATOM | 2029 | NH1 | ARG | 696 | 28.520 | 11.823 | -8.221 | 1.00 | 42.50 |
| ATOM | 2030 | NH2 | ARG | 696 | 29.927 | 10.033 | -7.884 | 1.00 | 43.92 |
| ATOM | 2031 | C | ARG | 696 | 24.308 | 9.431 | -3.447 | 1.00 | 34.63 |
| ATOM | 2032 | O | ARG | 696 | 25.043 | 8.517 | -3.081 | 1.00 | 32.87 |
| ATOM | 2033 | N | LEU | 697 | 24.174 | 10.567 | -2.769 | 1.00 | 32.92 |
| ATOM | 2034 | CA | LEU | 697 | 24.918 | 10.800 | -1.538 | 1.00 | 32.40 |
| ATOM | 2035 | CB | LEU | 697 | 24.693 | 12.235 | -1.047 | 1.00 | 29.94 |
| ATOM | 2036 | CG | LEU | 697 | 25.210 | 13.332 | -1.989 | 1.00 | 28.77 |
| ATOM | 2037 | CD1 | LEU | 697 | 24.983 | 14.716 | -1.377 | 1.00 | 27.97 |
| ATOM | 2038 | CD2 | LEU | 697 | 26.686 | 13.116 | -2.257 | 1.00 | 28.17 |
| ATOM | 2039 | C | LEU | 697 | 24.567 | 9.793 | -0.446 | 1.00 | 33.91 |
| ATOM | 2040 | O | LEU | 697 | 25.447 | 9.321 | 0.282 | 1.00 | 33.09 |
| ATOM | 2041 | N | LEU | 698 | 23.287 | 9.455 | -0.338 | 1.00 | 35.43 |
| ATOM | 2042 | CA | LEU | 698 | 22.836 | 8.494 | 0.662 | 1.00 | 39.05 |
| ATOM | 2043 | CB | LEU | 698 | 21.317 | 8.332 | 0.605 | 1.00 | 36.77 |
| ATOM | 2044 | CG | LEU | 698 | 20.470 | 9.189 | 1.534 | 1.00 | 35.45 |
| ATOM | 2045 | CD1 | LEU | 698 | 19.002 | 8.875 | 1.295 | 1.00 | 33.66 |
| ATOM | 2046 | CD2 | LEU | 698 | 20.857 | 8.902 | 2.977 | 1.00 | 34.33 |
| ATOM | 2047 | C | LEU | 698 | 23.464 | 7.119 | 0.473 | 1.00 | 41.14 |
| ATOM | 2048 | O | LEU | 698 | 23.649 | 6.374 | 1.434 | 1.00 | 41.09 |
| ATOM | 2049 | N | GLN | 699 | 23.790 | 6.786 | -0.769 | 1.00 | 44.86 |
| ATOM | 2050 | CA | GLN | 699 | 24.355 | 5.478 | -1.064 | 1.00 | 48.97 |
| ATOM | 2051 | CB | GLN | 699 | 23.629 | 4.895 | -2.278 | 1.00 | 50.91 |
| ATOM | 2052 | CG | GLN | 699 | 22.142 | 5.248 | -2.281 | 1.00 | 54.28 |
| ATOM | 2053 | CD | GLN | 699 | 21.316 | 4.356 | -3.180 | 1.00 | 56.50 |
| ATOM | 2054 | OE1 | GLN | 699 | 21.080 | 3.187 | -2.866 | 1.00 | 57.96 |
| ATOM | 2055 | NE2 | GLN | 699 | 20.870 | 4.901 | -4.309 | 1.00 | 57.46 |
| ATOM | 2056 | C | GLN | 699 | 25.866 | 5.479 | -1.285 | 1.00 | 49.73 |
| ATOM | 2057 | O | GLN | 699 | 26.471 | 4.426 | -1.486 | 1.00 | 50.77 |
| ATOM | 2058 | N | GLU | 700 | 26.475 | 6.658 | -1.237 | 1.00 | 50.70 |
| ATOM | 2059 | CA | GLU | 700 | 27.917 | 6.773 | -1.426 | 1.00 | 51.84 |
| ATOM | 2060 | CB | GLU | 700 | 28.326 | 8.248 | -1.470 | 1.00 | 52.07 |
| ATOM | 2061 | CG | GLU | 700 | 29.708 | 8.509 | -2.060 | 1.00 | 53.82 |
| ATOM | 2062 | CD | GLU | 700 | 29.815 | 8.077 | -3.516 | 1.00 | 53.97 |
| ATOM | 2063 | OE1 | GLU | 700 | 28.904 | 8.404 | -4.307 | 1.00 | 54.46 |
| ATOM | 2064 | OE2 | GLU | 700 | 30.815 | 7.419 | -3.871 | 1.00 | 55.26 |
| ATOM | 2065 | C | GLU | 700 | 28.627 | 6.070 | -0.269 | 1.00 | 52.20 |
| ATOM | 2066 | O | GLU | 700 | 29.459 | 5.175 | -0.527 | 1.00 | 52.35 |
| ATOM | 2067 | OXT | GLU | 700 | 28.335 | 6.428 | 0.891 | 1.00 | 52.40 |
| ATOM | 2068 | OH2 | WAT | 801 | 14.711 | 26.653 | 2.728 | 1.00 | 24.40 |
| ATOM | 2069 | OH2 | WAT | 802 | 26.414 | 20.512 | 8.977 | 1.00 | 20.53 |
| ATOM | 2070 | OH2 | WAT | 803 | 25.635 | 40.975 | 13.967 | 1.00 | 28.74 |
| ATOM | 2071 | OH2 | WAT | 804 | 13.552 | 16.269 | 8.746 | 1.00 | 21.45 |
| ATOM | 2072 | OH2 | WAT | 805 | 21.059 | 40.743 | 3.993 | 1.00 | 34.61 |
| ATOM | 2073 | OH2 | WAT | 806 | 11.847 | 19.482 | 13.154 | 1.00 | 27.86 |
| ATOM | 2074 | OH2 | WAT | 807 | 24.620 | 17.326 | 14.726 | 1.00 | 20.00 |
| ATOM | 2075 | OH2 | WAT | 808 | 25.763 | 19.395 | -5.509 | 1.00 | 24.65 |
| ATOM | 2076 | OH2 | WAT | 809 | 27.566 | 41.817 | 2.053 | 1.00 | 34.09 |
| ATOM | 2077 | OH2 | WAT | 810 | 1.729 | 8.907 | 4.075 | 1.00 | 31.53 |
| ATOM | 2078 | OH2 | WAT | 811 | 7.205 | 27.241 | 11.662 | 1.00 | 35.98 |
| ATOM | 2079 | OH2 | WAT | 812 | 23.647 | 26.541 | 23.470 | 1.00 | 28.06 |
| ATOM | 2080 | OH2 | WAT | 813 | 14.409 | 33.457 | 14.799 | 1.00 | 33.72 |
| ATOM | 2081 | OH2 | WAT | 814 | 13.065 | 3.124 | 16.758 | 1.00 | 28.22 |
| ATOM | 2082 | OH2 | WAT | 815 | 30.885 | 19.043 | 2.598 | 1.00 | 33.40 |
| ATOM | 2083 | OH2 | WAT | 816 | 36.157 | 39.234 | 1.950 | 1.00 | 43.36 |
| ATOM | 2084 | OH2 | WAT | 817 | 12.139 | 22.822 | 7.031 | 1.00 | 30.28 |
| ATOM | 2085 | OH2 | WAT | 818 | 3.298 | 4.825 | 10.176 | 1.00 | 23.53 |
| ATOM | 2086 | OH2 | WAT | 819 | 1.704 | 13.633 | 4.812 | 1.00 | 22.44 |
| ATOM | 2087 | OH2 | WAT | 820 | 4.214 | 13.018 | 8.864 | 1.00 | 29.86 |
| ATOM | 2088 | OH2 | WAT | 821 | 0.762 | 10.736 | 7.722 | 1.00 | 36.08 |
| ATOM | 2089 | OH2 | WAT | 822 | 0.166 | 11.985 | 3.762 | 1.00 | 37.47 |
| ATOM | 2090 | OH2 | WAT | 823 | 31.803 | 41.991 | 11.509 | 1.00 | 38.01 |
| ATOM | 2091 | OH2 | WAT | 825 | 29.323 | 24.136 | 13.813 | 1.00 | 31.41 |
| ATOM | 2092 | OH2 | WAT | 832 | 15.159 | 19.303 | 30.516 | 1.00 | 39.31 |
| ATOM | 2093 | OH2 | WAT | 835 | 3.146 | 11.579 | 6.786 | 1.00 | 34.28 |
| ATOM | 2094 | OH2 | WAT | 836 | 8.412 | 22.351 | 12.154 | 1.00 | 37.30 |
| ATOM | 2095 | OH2 | WAT | 837 | 14.427 | 24.078 | 4.226 | 1.00 | 33.47 |
| ATOM | 2096 | OH2 | WAT | 839 | 2.395 | 4.154 | 7.605 | 1.00 | 29.06 |
| ATOM | 2097 | OH2 | WAT | 840 | 26.537 | 16.496 | 27.832 | 1.00 | 31.09 |
| ATOM | 2098 | OH2 | WAT | 841 | 8.106 | 20.585 | 7.958 | 1.00 | 33.63 |
| ATOM | 2099 | OH2 | WAT | 842 | 11.326 | 3.463 | 14.716 | 1.00 | 31.28 |
| ATOM | 2100 | OH2 | WAT | 843 | 14.267 | 3.376 | 12.820 | 1.00 | 30.09 |
| ATOM | 2101 | OH2 | WAT | 844 | 12.746 | 22.210 | 13.480 | 1.00 | 53.73 |
| ATOM | 2102 | OH2 | WAT | 846 | 34.204 | 40.570 | 11.521 | 1.00 | 54.26 |
| ATOM | 2103 | OH2 | WAT | 847 | 9.625 | 5.990 | 8.120 | 1.00 | 29.28 |
| ATOM | 2104 | OH2 | WAT | 848 | 9.201 | 31.722 | 7.032 | 1.00 | 45.91 |
| ATOM | 2105 | OH2 | WAT | 849 | 12.004 | 24.958 | 5.330 | 1.00 | 32.20 |
| ATOM | 2106 | OH2 | WAT | 850 | 37.886 | 45.476 | 1.031 | 1.00 | 31.54 |
| ATOM | 2107 | OH2 | WAT | 851 | 31.059 | 28.266 | 8.430 | 1.00 | 44.76 |
| ATOM | 2108 | OH2 | WAT | 852 | 19.435 | 16.697 | -9.090 | 1.00 | 46.89 |
| ATOM | 2109 | OH2 | WAT | 853 | 18.131 | 44.059 | 3.829 | 1.00 | 54.92 |
| ATOM | 2110 | OH2 | WAT | 854 | 26.481 | 10.672 | 28.939 | 1.00 | 48.07 |
| ATOM | 2111 | OH2 | WAT | 855 | 6.654 | 19.232 | 5.941 | 1.00 | 41.68 |
| ATOM | 2112 | OH2 | WAT | 856 | 9.668 | 17.943 | 18.268 | 1.00 | 32.81 |
| ATOM | 2113 | OH2 | WAT | 857 | 10.512 | 10.846 | 24.734 | 1.00 | 38.33 |
| ATOM | 2114 | OH2 | WAT | 858 | 20.864 | 44.415 | 2.676 | 1.00 | 50.37 |
| ATOM | 2115 | OH2 | WAT | 859 | 10.258 | -0.353 | 5.697 | 1.00 | 56.40 |
| ATOM | 2116 | OH2 | WAT | 860 | 2.560 | 9.064 | 21.518 | 1.00 | 69.57 |
| ATOM | 2117 | OH2 | WAT | 861 | 33.537 | 17.935 | 17.817 | 1.00 | 38.63 |
| ATOM | 2118 | OH2 | WAT | 862 | 7.370 | 24.146 | 6.448 | 1.00 | 36.09 |
| ATOM | 2119 | OH2 | WAT | 863 | 30.158 | 20.333 | 25.058 | 1.00 | 30.28 |
| ATOM | 2120 | OH2 | WAT | 865 | 30.909 | 40.546 | -7.045 | 1.00 | 44.78 |
| ATOM | 2121 | OH2 | WAT | 866 | 15.610 | 43.031 | 8.646 | 1.00 | 47.88 |
| ATOM | 2122 | OH2 | WAT | 867 | 12.882 | 25.860 | 0.818 | 1.00 | 36.53 |
| ATOM | 2123 | OH2 | WAT | 868 | 36.979 | 45.229 | -7.650 | 1.00 | 75.27 |
| ATOM | 2124 | OH2 | WAT | 869 | 9.261 | 19.297 | 12.679 | 1.00 | 36.84 |
| ATOM | 2125 | OH2 | WAT | 870 | 15.360 | 22.134 | 6.145 | 1.00 | 36.08 |
| ATOM | 2126 | OH2 | WAT | 871 | 23.204 | 42.943 | 6.553 | 1.00 | 55.50 |
| ATOM | 2127 | OH2 | WAT | 873 | 24.254 | 39.737 | 21.553 | 1.00 | 69.04 |
| ATOM | 2128 | OH2 | WAT | 874 | 20.154 | 35.236 | -14.461 | 1.00 | 40.58 |
| ATOM | 2129 | OH2 | WAT | 875 | 5.710 | 19.528 | 10.717 | 1.00 | 51.87 |
| ATOM | 2130 | OH2 | WAT | 876 | 22.924 | 24.573 | -10.275 | 1.00 | 59.21 |
| ATOM | 2131 | OH2 | WAT | 877 | 12.716 | 26.906 | -1.662 | 1.00 | 46.87 |
| ATOM | 2132 | OH2 | WAT | 878 | 29.438 | 8.222 | 21.664 | 1.00 | 31.21 |
| ATOM | 2133 | OH2 | WAT | 879 | 20.109 | 5.113 | 16.032 | 1.00 | 39.95 |
| ATOM | 2134 | OH2 | WAT | 880 | 24.412 | 46.283 | 17.227 | 1.00 | 55.64 |
| ATOM | 2135 | OH2 | WAT | 881 | 28.720 | 9.423 | 6.651 | 1.00 | 32.54 |
| ATOM | 2136 | OH2 | WAT | 883 | 42.162 | 41.811 | 5.003 | 1.00 | 55.81 |
| ATOM | 2137 | OH2 | WAT | 884 | 7.066 | 29.019 | 8.902 | 1.00 | 52.17 |
| ATOM | 2138 | OH2 | WAT | 885 | 18.047 | 2.153 | 2.538 | 1.00 | 50.39 |
| ATOM | 2139 | OH2 | WAT | 886 | 24.362 | 3.892 | 9.915 | 1.00 | 41.25 |
| ATOM | 2140 | OH2 | WAT | 887 | 15.604 | 37.471 | -2.711 | 1.00 | 42.93 |
| ATOM | 2141 | OH2 | WAT | 889 | 4.907 | 3.035 | 10.619 | 1.00 | 41.58 |
| ATOM | 2142 | OH2 | WAT | 890 | 21.088 | 8.951 | -7.950 | 1.00 | 37.07 |
| ATOM | 2143 | OH2 | WAT | 891 | 26.883 | 7.714 | 24.992 | 1.00 | 38.03 |
| ATOM | 2144 | OH2 | WAT | 892 | 27.672 | 6.863 | 14.497 | 1.00 | 33.97 |
| ATOM | 2145 | OH2 | WAT | 893 | 32.830 | 16.371 | 13.351 | 1.00 | 39.04 |
| ATOM | 2146 | OH2 | WAT | 894 | 27.935 | 24.454 | 28.849 | 1.00 | 58.27 |
| ATOM | 2147 | OH2 | WAT | 895 | 31.271 | 29.479 | 18.350 | 1.00 | 35.40 |
| ATOM | 2148 | OH2 | WAT | 896 | 20.663 | 0.959 | 10.098 | 1.00 | 29.75 |
| ATOM | 2149 | OH2 | WAT | 898 | 25.086 | 4.474 | 5.856 | 1.00 | 58.91 |
| ATOM | 2150 | OH2 | WAT | 899 | 10.823 | 19.991 | 1.639 | 1.00 | 50.34 |
| ATOM | 2151 | OH2 | WAT | 900 | 36.503 | 19.016 | 22.377 | 1.00 | 47.47 |
| ATOM | 2152 | OH2 | WAT | 901 | 7.753 | 18.823 | 14.805 | 1.00 | 63.11 |
| ATOM | 2153 | OH2 | WAT | 902 | 39.898 | 36.941 | 0.917 | 1.00 | 58.76 |
| ATOM | 2154 | OH2 | WAT | 903 | 9.902 | 17.486 | 15.681 | 1.00 | 43.86 |
| ATOM | 2155 | OH2 | WAT | 904 | 10.082 | 39.246 | 11.235 | 1.00 | 61.89 |
| ATOM | 2156 | OH2 | WAT | 905 | 11.163 | 31.359 | -8.260 | 1.00 | 56.41 |
| ATOM | 2157 | OH2 | WAT | 906 | 31.409 | 30.077 | 0.054 | 1.00 | 38.17 |
| ATOM | 2158 | OH2 | WAT | 907 | -0.262 | 4.591 | 11.602 | 1.00 | 40.33 |
| ATOM | 2159 | OH2 | WAT | 908 | 31.641 | 28.926 | 2.593 | 1.00 | 66.15 |
| ATOM | 2160 | OH2 | WAT | 909 | -1.843 | 16.272 | 8.893 | 1.00 | 44.52 |
| ATOM | 2161 | OH2 | WAT | 910 | 10.182 | 38.951 | 7.471 | 1.00 | 67.08 |
| ATOM | 2162 | OH2 | WAT | 911 | 13.297 | 1.372 | 1.110 | 1.00 | 53.70 |
| ATOM | 2163 | OH2 | WAT | 912 | 15.299 | 2.429 | 15.304 | 1.00 | 39.81 |
| ATOM | 2164 | OH2 | WAT | 913 | 16.202 | 17.257 | 31.878 | 1.00 | 39.68 |
| ATOM | 2165 | OH2 | WAT | 914 | 33.600 | 36.849 | 17.451 | 1.00 | 35.02 |
| ATOM | 2166 | OH2 | WAT | 915 | -0.283 | 12.217 | 10.059 | 1.00 | 33.29 |
| ATOM | 2167 | OH2 | WAT | 916 | 20.496 | 1.221 | 17.973 | 1.00 | 45.64 |
| ATOM | 2168 | OH2 | WAT | 917 | 22.207 | 39.746 | 15.987 | 1.00 | 51.55 |
| ATOM | 2169 | OH2 | WAT | 918 | 14.385 | 33.613 | 17.595 | 1.00 | 42.92 |
| ATOM | 2170 | OH2 | WAT | 919 | 26.248 | 13.606 | -7.378 | 1.00 | 37.16 |
| ATOM | 2171 | OH2 | WAT | 920 | 25.959 | 6.408 | -4.445 | 1.00 | 48.88 |
| ATOM | 2172 | OH2 | WAT | 921 | 17.591 | 2.898 | 16.434 | 1.00 | 39.25 |
| ATOM | 2173 | OH2 | WAT | 922 | 33.239 | 45.203 | 11.047 | 1.00 | 49.84 |
| ATOM | 2174 | OH2 | WAT | 923 | 12.608 | 31.888 | -12.406 | 1.00 | 50.03 |
| ATOM | 2175 | OH2 | WAT | 924 | 26.381 | 45.820 | 0.731 | 1.00 | 44.95 |
| ATOM | 2176 | OH2 | WAT | 925 | 7.350 | 22.130 | 18.821 | 1.00 | 48.55 |
| ATOM | 2177 | OH2 | WAT | 926 | 23.505 | 34.031 | 25.189 | 1.00 | 52.64 |
| ATOM | 2178 | OH2 | WAT | 927 | 23.404 | 53.128 | -2.298 | 1.00 | 76.10 |
| ATOM | 2179 | OH2 | WAT | 928 | 29.823 | 49.147 | 1.695 | 1.00 | 48.44 |
| ATOM | 2180 | OH2 | WAT | 929 | 21.489 | 3.727 | 1.417 | 1.00 | 61.20 |
| ATOM | 2181 | OH2 | WAT | 930 | 18.898 | 35.011 | -16.838 | 1.00 | 67.70 |
| ATOM | 2182 | OH2 | WAT | 931 | 8.048 | 22.678 | 24.638 | 1.00 | 35.53 |
| ATOM | 2183 | OH2 | WAT | 933 | 33.982 | 42.818 | -3.037 | 1.00 | 40.39 |
| ATOM | 2184 | OH2 | WAT | 934 | 9.979 | 28.055 | 25.701 | 1.00 | 46.53 |
| ATOM | 2185 | OH2 | WAT | 935 | 13.628 | 22.137 | 2.420 | 1.00 | 44.85 |
| ATOM | 2186 | OH2 | WAT | 936 | 19.171 | 1.332 | 15.385 | 1.00 | 37.36 |
| ATOM | 2187 | OH2 | WAT | 939 | 27.207 | 28.433 | -6.434 | 1.00 | 68.57 |
| ATOM | 2188 | OH2 | WAT | 940 | 3.857 | 1.418 | 8.795 | 1.00 | 68.25 |
| ATOM | 2189 | OH2 | WAT | 941 | 16.520 | 42.036 | -2.158 | 1.00 | 79.07 |
| ATOM | 2190 | OH2 | WAT | 942 | 17.605 | 46.558 | 2.927 | 1.00 | 47.55 |
| ATOM | 2191 | OH2 | WAT | 943 | 15.096 | 42.405 | 3.989 | 1.00 | 53.50 |
| ATOM | 2192 | OH2 | WAT | 950 | 9.314 | 22.415 | 6.528 | 1.00 | 40.77 |
| ATOM | 2193 | OH2 | WAT | 951 | 5.922 | 25.754 | 4.200 | 1.00 | 58.05 |
| ATOM | 2194 | OH2 | WAT | 952 | -0.116 | 13.000 | 6.780 | 1.00 | 96.22 |
| ATOM | 2195 | OH2 | WAT | 954 | 33.641 | 28.462 | 7.707 | 1.00 | 38.33 |
| ATOM | 2196 | OH2 | WAT | 955 | 20.020 | 19.781 | -9.186 | 1.00 | 46.18 |
| ATOM | 2197 | OH2 | WAT | 956 | 22.350 | 22.390 | -11.831 | 1.00 | 44.39 |
| ATOM | 2198 | C1 | REA | 500 | 22.676 | 32.386 | 7.585 | 1.00 | 44.19 |
| ATOM | 2199 | C2 | REA | 500 | 21.906 | 33.574 | 6.875 | 1.00 | 44.67 |
| ATOM | 2200 | C3 | REA | 500 | 21.637 | 33.455 | 5.374 | 1.00 | 44.62 |
| ATOM | 2201 | C4 | REA | 500 | 22.790 | 32.926 | 4.506 | 1.00 | 44.15 |
| ATOM | 2202 | C5 | REA | 500 | 23.891 | 32.096 | 5.255 | 1.00 | 43.36 |
| ATOM | 2203 | C6 | REA | 500 | 23.862 | 31.835 | 6.638 | 1.00 | 42.89 |
| ATOM | 2204 | C7 | REA | 500 | 24.919 | 31.045 | 7.334 | 1.00 | 41.43 |
| ATOM | 2205 | C8 | REA | 500 | 25.046 | 29.710 | 7.573 | 1.00 | 38.85 |
| ATOM | 2206 | C9 | REA | 500 | 26.080 | 29.094 | 8.397 | 1.00 | 39.63 |
| ATOM | 2207 | C10 | REA | 500 | 26.066 | 27.725 | 8.531 | 1.00 | 37.78 |
| ATOM | 2208 | C11 | REA | 500 | 27.051 | 27.018 | 9.329 | 1.00 | 37.42 |
| ATOM. | 2209 | C12 | REA | 500 | 27.070 | 25.694 | 9.440 | 1.00 | 37.89 |
| ATOM | 2210 | C13 | REA | 500 | 28.057 | 24.977 | 10.242 | 1.00 | 38.53 |
| ATOM | 2211 | C14 | REA | 500 | 27.951 | 23.626 | 10.224 | 1.00 | 36.87 |
| ATOM | 2212 | C15 | REA | 500 | 28.705 | 22.564 | 10.870 | 1.00 | 35.41 |
| ATOM | 2213 | C16 | REA | 500 | 21.650 | 31.254 | 7.888 | 1.00 | 44.23 |
| ATOM | 2214 | C17 | REA | 500 | 23.235 | 32.990 | 8.910 | 1.00 | 45.49 |
| ATOM | 2215 | C18 | REA | 500 | 24.948 | 31.651 | 4.234 | 1.00 | 43.96 |
| ATOM | 2216 | C19 | REA | 500 | 27.157 | 29.991 | 9.084 | 1.00 | 37.79 |
| ATOM | 2217 | C20 | REA | 500 | 29.159 | 25.776 | 11.043 | 1.00 | 39.49 |
| ATOM | 2218 | O1 | REA | 500 | 28.377 | 21.424 | 10.626 | 1.00 | 36.24 |
| ATOM | 2219 | O2 | REA | 500 | 29.641 | 22.779 | 11.628 | 1.00 | 31.52 |
| END | | | | | | | | | |

### References

Becker-André, M., André E., and DeLamarter, J. F. (1993) Identification of nuclear receptor mRNAs by RT-PCR amplification of conserved zinc-finger motif sequences. Biochem. Biophys. Res. Commun. 194, 1371-1379.
Brünger, A.T. et al. Crystallography & NMR system: A new software suite for macromolecular structure determination. Acta Crystallogr. A47, 110-119, (1998).
Hirose, T., Smith, R. J., and Jetten, A. M. (1994) RORg: the third member of ROR/RZR orphan receptor subfamily that is highly expressed in skeletal muscle. Biochem. Biophys. Res. Commun. 205, 1976-1983.
Jones, T. A., Zou, J. Y., Cowan, S. W. et Kjeldgaard, M. (1991) Improved methods for building protein models in electron density maps and the location of errors in theses models. Acta crystallogr. A47, 110-119
Kurebayashi S, and Hirose T. (1998) Novel orphan receptor: ROR gamma expressed during adipocyte differenciation. Nippon Rinsho, 56, 1729-1733.
Lau P., Bailey P., Dowhan D. H., Muscat G.E. (1999) Exogenous expression of a dominant negative RORalphal vector in muscle cells impairs differenciation: RORalphal directly interacts with p300 and myoD. Nucleic Acids Research, 27, 411-420.
Koibuchi N., and Chin W., (1998) RORα gene expression in the perinatal rat cerebellum: ontogeny and thyroid hormone regulation. Endocrinology 139, 2335-2341.
Matysiak-Scholze U., and Nehls M. (1997) The structural integrity of RORα isoforms is mutated in staggerer mice: cerebellar coexpression of RORα1 and RORα4. Genomics, 43, 78-84.
Navaza, J. (1994) AMoRe: an automated package for molecular replacement. Acta Crystallog. A50, 157-163.
Nolte, R. T., Wisely G.B. , Westin B., Cobbs J. E., Lambert M. H., Kurokawa R./, Rosenfeld M. G., Willson T., Glass C. K., and Millburn M. V. (1998) Ligand binding and co-activator assembly of the peroxisome proliferator-activated receptor-γ.
Otwinowski, Z., and Minor, W. (1997) Processing of X-ray diffraction data collected in oscillation mode. Methods Enzymol. 276,307-326.
Perrakis, A., Morris, R., and Lamzin V. S. (1999) Automated protein model building combined with iterative structure refinement. Nature Struct. Biol. 6, 458-463.
Renaud, J-P, Rochel N., RuffM., Vivat V., Chambon P., Gronemeyer H., and Moras D. (1995) Crystal structure of the RAR-γ ligand-binding domain bound to all-trans retinoic acid. Nature, 378, 681-689.
Rochel N., Wurtz J. M., Mitschler A., Klaholz B, and D. Moras. (2000) The crystal structure of the nuclear receptor for vitamin D bound to its natural ligand. Mol. Cell 5, 173-179.
Sirlin, J. L. (1956) Vacillans, a neurological mutant in the house mouse linked with brown. J. Genet., 54, 42-48.

## Claims

1. Polypeptides from the retinoic acid-related orphan receptor (ROR) in mammals, **characterized in that** they are fragments of rat, human, or murine RORβ, α, or γ, delimited in their N-terminal extremity by the amino acid located in one of the positions 201 to 209 of the ROR sequences represented on figure 3, and in their C-terminal extremity by the amino acid located in one of the positions 451 or 452, of the RORsequences represented on figure 3.

2. Polypeptides according to claim 1, chosen among:
- the fragment delimited by the amino acids located in positions 209 to 452 of:
. the sequence of the rat RORβ SEQ ID NO : 2,
. the sequence of the human RORβ SEQ ID NO : 3,
. the sequence of the human RORγ SEQ ID NO : 4,
. the sequence of the murine RORγ SEQ ID NO : 5,
. the sequence of the human RORα SEQ ID NO : 6,
. the sequence of the murine RORα SEQ ID NO : 7,
- the fragment delimited by the amino acids located in positions 208 to 452 of:
. the sequence of the rat RORβ SEQ ID NO : 8,
. the sequence of the human RORβ SEQ ID NO : 9,
. the sequence of the human RORγ SEQ ID NO : 10,
. the sequence of the murine RORγ SEQ ID NO : 11,
. the sequence of the human RORα SEQ ID NO : 12,
. the sequence of the murine RORα SEQ ID NO : 13,
- the fragment delimited by the amino acids located in positions 208 to 451 of:
. the sequence of the rat RORβ SEQ ID NO : 14,
. the sequence of the human RORβ SEQ ID NO : 15,
. the sequence of the human RORγ SEQ ID NO : 16,
. the sequence of the murine RORγ SEQ ID NO : 17,
. the sequence of the human RORα SEQ ID NO : 18,
. the sequence of the murine RORα SEQ ID NO : 19,
- the fragment delimited by the amino acids located in positions 209 to 451 of:
. the sequence of the rat RORβ SEQ ID NO : 20,
. the sequence of the human RORβ SEQ ID NO : 21,
. the sequence of the human RORγ SEQ ID NO : 22,
. the sequence of the murine RORγ SEQ ID NO : 23,
. the sequence of the human RORα SEQ ID NO : 24,
. the sequence of the murine RORα SEQ ID NO : 25,
- the fragment delimited by the amino acids located in positions 201 to 451 of:
. the sequence of the rat RORβ SEQ ID NO : 26,
. the sequence of the human RORβ SEQ ID NO : 27,
. the sequence of the human RORγ SEQ ID NO : 28,
. the sequence of the murine RORγ SEQ ID NO : 29,
. the sequence of the human RORα SEQ ID NO : 30,
. the sequence of the murine RORα SEQ ID NO : 31,
- the fragment delimited by the amino acids located in positions 201 to 452 of:
. the sequence of the rat RORβ SEQ ID NO : 32,
. the sequence of the human RORβ SEQ ID NO : 33,
. the sequence of the human RORγ SEQ ID NO : 34,
. the sequence of the murine RORγ SEQ ID NO : 35,
. the sequence of the human RORα SEQ ID NO : 36,
. the sequence of the murine RORα SEQ ID NO : 37.

3. Polypeptides according to claim 1 or 2, **characterized by** the following characteristics:
- they have the properties of binding a ligand and of transactivation of the LBD of the receptor ROR,
- they are soluble in aqueous solvents,
- they are crystallisable in aqueous solvents, especially by the hanging drop vapour diffusion method, more particularly at approximately 4°C.

4. Molecular complexes comprising a polypeptide according to any one of claims 1 to 3, said polypeptide being in association with:
- a ROR-LBD ligand which is an agonist, such as stearic acid, or an antagonist of the ROR-LBD, such as retinoic acid,
- and/or with a co-peptide having a sequence of approximately 15-20 amino-acids and comprising the co-activator motif LXXLL or a co-repressor motif (I/L)XX(V/I)I or LXX(H/I)IXXX(I/L) wherein X represents any amino acid, natural or not, such as co-peptides chosen among fragments of co-activators of transcription, especially those of the p160 family, and more particularly among fragments of the co-activators SRC1, such as the fragment 686-700 of SRC1, or among fragments of co-repressors of transcription.

5. Nucleotide sequence coding for a polypeptide according to any one of claims 1 to 3.

6. Nucleotide sequence according to claim 5, associated to elements necessary for the transcription of this sequence, particularly a promoter and a terminator of transcription.

7. Vector, particularly plasmid, comprising a nucleotide sequence according to claim 5 or 6.

8. Host cells, such as *E.coli,* transformed with a vector according to claim 7.

9. Process for obtaining a polypeptide according to any one of claims 1 to 3, or a molecular complex according to claim 4, **characterized in that** it comprises:
- a step of transforming host cells with a nucleotide sequence according to claim 5 or 6, using a vector according to claim 7,
- a step of cultivating the transformed host cell according to claim 8 thus obtained, in an appropriate culture medium,
- and the recovery, and if necessary, the purification of the recombinant polypeptide or molecular complex obtained.

10. A crystal comprising a polypeptide according to any one of claims 1 to 3, or a molecular complex according to claim 4.

11. A crystal according to claim 10, **characterized in that** said crystal diffracts to at least 3 angstrom resolution and has a crystal stability within 5% of its unit cell dimensions.

12. A crystal according to claim 10 or 11, wherein the ROR-LBD has the following unit cell dimensions in angstroms: a = 52.302 Å, b = 58.490 Å and c = 106.036 Å, α=β=χ=90°, and an orthorhombic space group P212121.

13. A crystal according to any one of claims 10 to 12, such as obtained by carrying out a process according to claim 9, and comprising a step of crystallisation in aqueous solvents of the polypeptides any one of claims 1 to 3, or the molecular complexes according to claim 4, especially at 4°C by the hanging drop vapour diffusion method.

14. Use of the polypeptides according to any one of claims 1 to 3, or of the molecular complexes according to claim 4, or of the crystals according to any one of claims 10 to 13, for carrying out:
- a process for the screening of a ROR-LBD ligand which is an agonist, or an antagonist of said receptor, or for the screening of ligands that perturb the structure of the receptor and having an effect on the recruitment of cofactors (co-activators and co-repressors) and hence on gene regulation,
- or a process for the analysis of the tridimensional structure of the complexes formed with said polypeptides, molecular complexes or crystals and a particular compound.

15. Use according to claim 14, for the screening of compounds acting as agonists or antagonists of ROR, said compounds being useful in the frame of the treatment of pathologies related to the central nervous system, the retinal organisation, the sensorial signal integration, the motricity, and sterility.

16. Process for the screening of a ROR-LBD ligand which is an agonist, or an antagonist of said receptor, said process comprising the following steps:
contacting a polypeptide according to any one of claims 1 to 3, or a molecular complex according to claim 4, or a crystal according to any one of claims 10 to 13, advantageously linked to a solid support, with the particular compound susceptible to be a ROR-LBD ligand, preferably one of the said polypeptide, or molecular complex, or crystal, or tested ligand, being labelled, such as with a fluorescent, radioactive or enzymatic label,
- detection of the possible association between the said polypeptide, or molecular complex, or crystal, and the tested ligand, by measuring the used label, especially after rinsing the support used in the preceding step, or by mass spectrometry under non denaturing conditions.

17. Process for the analysis of the tridimensional structure of the complexes formed with a polypeptide according to any one of claims 1 to 3, or a molecular complex according to claim 4, or a crystal according to any one of claims 10 to 13, and a particular compound susceptible to be a ROR-LBD ligand, said process comprising the following steps:
- contacting the said polypeptide, or molecular complex, or crystal, with said particular compound,
- crystallisation of the complex formed between the said polypeptide, or molecular complex, or crystal, and the tested ligand, especially with the vapour diffusion method, and tridimensional analysis of said complex, especially with the molecular replacement method,
- or tridimensional analysis of said complex in soluble state, by using an appropriate method such as NMR.

## Patentansprüche

1. Polypeptide aus dem Retinoic-acid-related-orphan-Rezeptor (ROR) in Säugern, **dadurch gekennzeichnet, dass** sie Fragmente des RORβ, RORα oder RORγ von Ratte, Mensch oder Maus sind, die an ihrem N-terminalen Ende durch die Aminosäure begrenzt sind, die an einer der Positionen 201 bis 209 der ROR-Sequenzen, die in Figur 3 dargestellt sind, liegt, und an ihrem C-terminalen Ende durch eine Aminosäure begrenzt ist, die an einer der Positionen 451 oder 452, der ROR-Sequenzen, die in Figur 3 dargestellt sind, liegt.

2. Polypeptide nach Anspruch 1, ausgewählt aus:
- dem Fragment, das durch die Aminosäuren begrenzt wird, die an den Positionen 209 bis 452:
• der Sequenz des RORβ der Ratte, SEQ ID NO: 2,
• der Sequenz des RORβ des Menschen, SEQ ID NO: 3,
• der Sequenz des RORγ des Menschen, SEQ ID NO: 4,
• der Sequenz des RORγ SEQ der Maus, ID NO: 5,
• der Sequenz des RORα des Menschen, SEQ ID NO: 6,
• der Sequenz des RORα der Maus, SEQ ID NO: 7 liegen,
- dem Fragment, das durch die Aminosäuren begrenzt wird, die an den Positionen 208 bis 452:
• der Sequenz des RORβ der Ratte, SEQ ID NO: 8,
• der Sequenz des RORβ des Menschen, SEQ ID NO: 9,
• der Sequenz des RORγ des Menschen, SEQ ID NO: 10,
• der Sequenz des RORγ der Maus, SEQ ID NO: 11,
• der Sequenz des RORα des Menschen, SEQ ID NO: 12,
• der Sequenz des RORα der Maus, SEQ ID NO: 13 liegen,
- dem Fragment, das durch die Aminosäuren begrenzt wird, die an den Positionen 208 bis 451:
• der Sequenz des RORβ der Ratte, SEQ ID NO: 14,
• der Sequenz des RORβ des Menschen, SEQ ID NO: 15,
• der Sequenz des RORγ des Menschen, SEQ ID NO: 16,
• der Sequenz des RORγ der Maus, SEQ ID NO: 17,
• der Sequenz des RORα des Menschen, SEQ ID NO: 18,
• der Sequenz des RORα der Maus, SEQ ID NO: 19 liegen,
- dem Fragment, das durch die Aminosäuren begrenzt wird, die an den Positionen 209 bis 451:
• der Sequenz des RORβ der Ratte, SEQ ID NO: 20,
• der Sequenz des RORβ des Menschen, SEQ ID NO: 21,
• der Sequenz des RORγ des Menschen, SEQ ID NO: 22,
• der Sequenz des RORγ der Maus, SEQ ID NO: 23,
• der Sequenz des RORα des Menschen, SEQ ID NO: 24,
• der Sequenz des RORα der Maus, SEQ ID NO: 25 liegen,
- dem Fragment, das durch die Aminosäuren begrenzt wird, die an den Positionen 201 bis 451:
• der Sequenz des RORβ der Ratte, SEQ ID NO: 26,
• der Sequenz des RORβ des Menschen, SEQ ID NO: 27,
• der Sequenz des RORγ des Menschen, SEQ ID NO: 28,
• der Sequenz des RORγ der Maus, SEQ ID NO: 29,
• der Sequenz des RORα des Menschen, SEQ ID NO: 30,
• der Sequenz des RORα der Maus, SEQ ID NO: 31 liegen,
- dem Fragment, das durch die Aminosäuren begrenzt wird, die an den Positionen 201 bis 452:
• der Sequenz des RORβ der Ratte, SEQ ID NO: 32,
• der Sequenz des RORβ des Menschen, SEQ ID NO: 33,
• der Sequenz des RORγ des Menschen, SEQ ID NO: 34,
• der Sequenz des RORγ der Maus, SEQ ID NO: 35,
• der Sequenz des RORα des Menschen, SEQ ID NO: 36,
• der Sequenz des RORα der Maus, SEQ ID NO: 37 liegen.

3. Polypeptide nach Anspruch 1 oder 2, die durch die folgenden Merkmale **gekennzeichnet** sind:
- sie haben das Vermögen, einen Liganden zu binden und die LBD des Rezeptors ROR zu transaktivieren,
- sie sind in wässrigen Lösemitteln löslich,
- sie sind in wässrigen Lösemitteln, speziell durch die Hanging-drop-Dampfdiffusionsmethode kristallisierbar, insbesondere bei ungefähr 4 °C.

4. Molekularkomplexe umfassend ein Polypeptid nach einem der Ansprüche 1 bis 3, wobei das Polypeptid assoziiert ist mit:
- einem ROR-LBD-Liganden, der ein Agonist, wie etwa Stearinsäure, oder ein Antagonist der ROR-LBD ist, wie etwa Retinsäure,
- und/oder mit einem Co-Peptid, das eine Sequenz von ungefähr 15 bis 20 Aminosäuren aufweist und das Co-Aktivatormotiv LXXLL oder ein Co-Repressormotiv (I/L)XX(V/I)I oder LXX(H/I)IXXX(I/L) umfasst, worin X für jede Aminosäure, natürlich oder nicht, steht, wie etwa Co-Peptide, ausgewählt aus Fragmenten von transkriptionellen Co-Aktivatoren, speziell solche der p160-Familie, und insbesondere aus Fragmenten des Co-Aktivators SRC1, wie etwa das Fragment 686-700 von SRC1, oder aus Fragmenten von transkriptionellen Co-Repressoren.

5. Nukleotidsequenz, die für ein Polypeptid nach einem der Ansprüche 1 bis 3 kodiert.

6. Nukleotidsequenz nach Anspruch 5, die mit Elementen assoziiert ist, die für die Transkription dieser Sequenz notwendig sind, insbesondere ein Promoter und ein Terminator der Transkription.

7. Vektor, insbesondere Plasmid, umfassend eine Nukleotidsequenz nach Anspruch 5 oder 6.

8. Wirtszellen, wie etwa *E*. *coli,* die mit einem Vektor nach Anspruch 7 transformiert wurden.

9. Verfahren zum Erhalten eines Polypeptids nach einem der Ansprüche 1 bis 3 oder eines Molekularkomplex nach Anspruch 4, **dadurch gekennzeichnet, dass** es umfasst:
- einen Schritt des Transformierens der Wirtszellen mit einer Nukleotidsequenz nach Anspruch 5 oder 6 unter Verwendung eines Vektors nach Anspruch 7,
- einen Schritt des Kultivierens der so erhaltenen transformierten Wirtszelle nach Anspruch 8 in einem geeigneten Kulturmedium,
- und das Gewinnen, und gegebenenfalls die Aufreinigung des erhaltenen rekombinanten Polypeptids oder Molekularkomplexes.

10. Kristall, der ein Polypeptid nach einem der Ansprüche 1 bis 3 oder einen Molekularkomplex nach Anspruch 4 umfasst.

11. Kristall nach Anspruch 10, **dadurch gekennzeichnet, dass** der Kristall mit einer Auflösung von mindestens 3 Angstrom beugt und eine Kristallstabilität innerhalb 5 % seiner Elementarzelldimensionen aufweist.

12. Kristall nach Anspruch 10 oder 11, worin die ROR-LBD die folgenden Elementarzelldimensionen in Angstrom aufweist: a = 52,302 Å, b = 58,490 Å und c = 106,036 Å, α = β = χ = 90 °, und eine orthorhombische Raumgruppe P212121.

13. Kristall nach einem der Ansprüche 10 bis 12, wie er erhalten wird, indem ein Verfahren nach Anspruch 9 durchgeführt wird, und umfassend einen Schritt der Kristallisierung der Polypeptide aus einem der Ansprüche 1 bis 3 oder der Molekularkomplexe nach Anspruch 4 in wässrigen Lösemitteln, speziell bei 4 °C, durch die Hanging-drop-Dampfdiffusionsmethode.

14. Verwendung der Polypeptide nach einem der Ansprüche 1 bis 3 oder der Molekularkomplexe nach Anspruch 4 oder der Kristalle nach einem der Ansprüche 10 bis 13 zum Durchführen:
- eines Verfahrens zum Screenen eines ROR-LBD-Liganden, der ein Agonist oder ein Antagonist des Rezeptors ist, oder zum Screenen von Liganden, die die Struktur des Rezeptors stören und eine Wirkung auf die Rekrutierung von Co-Faktoren (Co-Aktivatoren und Co-Repressoren), und somit auf die Genregulation haben,
- oder eines Verfahrens zur Analyse der dreidimensionalen Struktur der Komplexes, die mit den Polypeptiden, Molekülkomplexen oder Kristallen und einer besonderen Verbindung gebildet werden.

15. Verwendung nach Anspruch 14 zum Screenen von Verbindungen, die als Agonisten oder Antagonisten von ROR wirken, wobei die Verbindungen im Rahmen der Behandlung von Pathologien nützlich sind, die mit dem zentralen Nervensystem, der retinalen Organisation, der sensoriellen Signalintegration, der Beweglichkeit und der Sterilität zusammenhängen.

16. Verfahren zum Screenen eines ROR-LBD-Liganden, der ein Agonist oder ein Antagonist des Rezeptors ist, wobei das Verfahren die folgenden Schritte umfasst:
- Kontaktieren eines Polypeptids nach einem der Ansprüche 1 bis 3 oder eines Molekülkomplexes nach Anspruch 4 oder eines Kristalls nach einem der Ansprüche 10 bis 13, das vorteilhafterweise mit einem festen Träger verbunden ist, mit der besonderen Verbindung, die vermutlich ein ROR-LBD-Ligand ist, wobei entweder das Polypeptid oder der Molekularkomplex oder der Kristall oder der getestete Ligand markiert ist, etwa mit einem fluoreszierenden, radioaktiven oder enzymatischen Marker,
- Nachweisen der möglichen Assoziation zwischen dem Polypeptid oder dem Molekularkomplex oder dem Kristall und dem getesteten Liganden durch Messen des verwendeten Markers, speziell nach dem Spülen des Trägers, der in dem vorhergehenden Schritt verwendet wurde, oder durch Massenspektrometrie unter nichtdenaturierenden Bedingungen.

17. Verfahren zur Analyse der dreidimensionalen Struktur der Komplexe, die mit einem Polypeptid nach Anspruch 1 bis 3, oder einem Molekularkomplex nach Anspruch 4 oder einem Kristall gemäß jedem der Ansprüche 10 bis 13 und einer bestimmten Verbindung gebildet wurden, die vermutlich ein ROR-LBD-Ligand ist, wobei das Verfahren die folgenden Schritte umfasst:
- Kontaktieren des Polypeptids oder des Molekülkomplexes oder des Kristalls mit der besonderen Verbindung,
- Kristallisierung des Komplexes, der zwischen dem Polypeptid oder dem Molekülkomplex oder dem Kristall und dem getesteten Liganden gebildet wurde, speziell mit der Dampfdiffusionsmethode, und dreidimensionale Analyse des Komplexes, speziell mit der Methode des molekularen Ersatzes,
- oder dreidimensionale Analyse des Komplexes in löslichem Zustand unter Verwendung einer geeigneten Methode, wie etwa NMR.

## Revendications

1. Polypeptides du récepteur orphelin apparenté au récepteur de l'acide rétinoïque (ROR) des mammifères, **caractérisés en ce qu'**ils sont des fragments de RORβ, α, ou γ de rat, humain ou murin, délimités dans leur extrémité N-terminale par les acides aminés situés dans une des positions 2001 à 209 des séquences de ROR représentées dans la figure 3, et dans leur extrémité C-terminale par les acides aminés situés dans l'une des positions 451 ou 452, des séquences ROR représentées dans la figure 3.

2. Polypeptides selon la revendication 1, choisis parmi:
- le fragment délimité par les acides aminés situés en position 209 à 452 de :
. la séquence de rat RORβ SEQ ID NO : 2,
. la séquence humaine RORβ SEQ ID NO : 3,
. la séquence humaine RORγ SEQ ID NO : 4,
. la séquence murine RORγ SEQ ID NO : 5,
. la séquence humaine RORα SEQ ID NO : 6,
. la séquence murine RORα SEQ ID NO : 7,
- le fragment délimité par les acides aminés situés en position 208 à 452 de :
. la séquence de rat RORβ SEQ ID NO : 8,
. la séquence humaine RORβ SEQ ID NO : 9,
. la séquence humaine RORγ SEQ ID NO : 10,
. la séquence murine RORγ SEQ ID NO : 11,
. la séquence humaine RORα SEQ ID NO : 12,
. la séquence murine RORα SEQ ID NO : 13,
- le fragment délimité par les acides aminés situés en position 208 à 451 de :
. la séquence de rat RORβ SEQ ID NO : 14,
. la séquence humaine RORβ SEQ ID NO : 15,
. la séquence humaine RORγ SEQ ID NO : 16,
. la séquence murine RORγ SEQ ID NO : 17,
. la séquence humaine RORα SEQ ID NO : 18,
. la séquence murine RORα SEQ ID NO : 19,
- le fragment délimité par les acides aminés situés en position 209 à 451 de :
. la séquence de rat RORβ SEQ ID NO : 20,
. la séquence humaine RORβ SEQ ID NO : 21,
. la séquence humaine RORγ SEQ ID NO : 22,
. la séquence murine RORγ SEQ ID NO : 23,
. la séquence humaine RORα SEQ ID NO : 24,
. la séquence murine RORα SEQ ID NO : 25,
- le fragment délimité par les acides aminés situés en position 201 à 451 de :
. la séquence de rat RORβ SEQ ID NO : 26,
. la séquence humaine RORβ SEQ ID NO : 27,
. la séquence humaine RORγ SEQ ID NO : 28,
. la séquence murine RORγ SEQ ID NO : 29,
. la séquence humaine RORα SEQ ID NO : 30,
. la séquence murine RORα SEQ ID NO : 31,
- le fragment délimité par les acides aminés situés en position 201 à 452 de :
. la séquence de rat RORβ SEQ ID NO : 32,
. la séquence humaine RORβ SEQ ID NO : 33,
. la séquence humaine RORγ SEQ ID NO : 34,
. la séquence murine RORγ SEQ ID NO : 35,
. la séquence humaine RORα SEQ ID NO : 36,
. la séquence murine RORα SEQ ID NO : 37.

3. Polypeptides selon la revendication 1 ou 2, **caractérisés par** les caractéristiques suivantes :
- ils possèdent les propriétés de liaison à un ligand et de transactivation du LBD du récepteur ROR,
- ils sont solubles dans un solvant aqueux,
- ils sont cristallisables dans un solvant aqueux, spécifiquement par la méthode de diffusion de vapeur en gouttes suspendues, plus particulièrement à environ 4°C.

4. Complexes moléculaires comprenant un polypeptide selon l'une quelconque des revendications 1 à 3, ledit polypeptide étant en association avec :
- un ligand de ROR-LBD qui est un agoniste, tel que l'acide stéarique, ou un antagoniste de ROR-LBD, tel que l'acide rétinoïque,
- et/ou avec un co-peptide ayant une séquence d'approximativement 15-20 acides aminés et comprenant le motif co-activateur LXXLL ou un motif co-répresseur (I/L)XX(V/I)I ou LXX(H/I)IXXX(I/L) où X représente n'importe quel acide aminé, naturel ou non, tel que des co-peptides choisis parmi des fragments co-activateur de transcription, spécialement ceux de la famille p160, et plus particulièrement parmi des fragments des co-activateurs SRC1, tel que le fragment 686-700 de SRC1, ou parmi des fragments de co-répresseurs de la transcription.

5. Séquence nucléotidique codant pour un polypeptide selon n'importe laquelle des revendications 1 à 3.

6. Séquence nucléotidique selon la revendication 5, associée à des éléments nécessaires pour la transcription de cette séquence, particulièrement un promoteur et un terminateur de la transcription.

7. Vecteur, particulièrement un plasmide, comprenant une séquence nucléotidique selon les revendications 5 ou 6.

8. Cellules hôtes, tel que *E. coli,* transformées par un vecteur selon la revendication 7.

9. Procédé pour obtenir un polypeptide selon l'une quelconque des revendications 1 à 3, ou un complexe moléculaire selon la revendication 4, **caractérisé en ce qu'**il comprend :
- une étape de transformation des cellules hôtes avec une séquence nucléotidique selon les revendications 5 ou 6, en utilisant un vecteur selon la revendication 7,
- une étape de culture des cellules hôtes ainsi obtenues selon la revendication 8, dans un milieu de culture approprié,
- et la récupération, et si nécessaire, la purification du polypeptide recombinant ou du complexe moléculaire obtenu.

10. Cristal comprenant un polypeptide selon l'une quelconque des revendications 1 à 3, ou un complexe moléculaire selon la revendication 4.

11. Cristal selon la revendication 10, **caractérisé en ce que** ledit cristal diffracte à une résolution d'au moins 3 angströms et a une stabilité cristalline dans 5% de ses dimensions cellulaires unitaires.

12. Cristal selon les revendications 10 ou 11, ou le ROR-LBD a les dimensions d'unité cellulaire suivantes : a = 52,302 Å, b = 58,490 Å et c = 106,036 Å, α=β=χ=90°, et un groupe d'espace orthorhombique P212121.

13. Cristal selon les revendications 10 à 12, tel qu'obtenu par la mise en oeuvre d'un procédé selon la revendication 9, et comprenant une étape de cristallisation dans un solvant aqueux des peptides de l'une quelconque des revendications 1 à 3, ou les complexe moléculaires selon la revendication 4, spécialement à 4°C par la méthode de diffusion de vapeur en gouttes suspendues.

14. Utilisation des peptides selon l'une quelconque des revendications 1 à 3, ou des complexes moléculaires selon la revendication 4, ou les cristaux selon l'une quelconque des revendications 10 à 13, pour la mise en oeuvre :
- d'un procédé de criblage d'un ligand de RPR-LBD qui est un agoniste, ou un antagoniste dudit récepteur, ou pour le criblage de ligands qui perturbent la structure du récepteur et ayant un effet sur le recrutement de cofacteurs (co-activateurs ou co-represseurs) et donc sur la régulation génique,
- ou un procédé pour l'analyse de la structure tridimensionnelle des complexes formés avec lesdits polypeptides, complexes moléculaires ou cristaux et un composé particulier.

15. Utilisation selon la revendication 14, pour le criblage de composés agissant comme agonistes ou antagonistes du ROR, lesdits composés étant utiles dans le cadre du traitement de pathologies associées au système nerveux central, à l'organisation rétinienne, à l'intégration du signal sensoriel, à la motricité, et à la stérilité.

16. Procédé pour le criblage d'un ligand de ROR-LBD qui est un agoniste, ou un antagoniste dudit récepteur, ledit procédé comprenant les étapes suivantes :
- la mise en contact d'un polypeptide selon l'une quelconque des revendications 1 à 3, ou un complexe moléculaire selon la revendication 4, ou un cristal selon l'une quelconque des revendications 10 à 13, avantageusement lié à un support solide, avec un composé particulier susceptible d'être un ligand de ROR-LBD, préférentiellement un desdits polypeptide, ou complexe moléculaire ou cristal, ou ligand testé, étant marqué, tel que avec un agent fluorescent, radioactif ou enzymatique,
- la détection de la possible association entre ledit polypeptide, ou complexe moléculaire ou cristal, et un ligand testé, en mesurant le marqueur utilisé, spécialement après rinçage du support utilisé dans les étapes précédentes, ou par spectrométrie de masse dans des conditions non dénaturantes.

17. Procédé pour l'analyse de la structure tridimensionnelle des complexes moléculaires formés avec un polypeptide selon l'une quelconque des revendications 1 à 3, ou un complexe moléculaire selon la revendication 4, ou un cristal selon l'une quelconque des revendications 10 à 13, et un composé particulier susceptible d'être un ligand de ROR-LBD, ledit procédé comprenant les étapes suivantes :
- la mise en contact dudit polypeptide, ou complexe moléculaire ou cristal avec ledit composé particulier,
- la cristallisation du complexe formé entre ledit polypeptide, ou complexe moléculaire, ou cristal, et le ligand testé, en particulier par la méthode de diffusion de vapeur en gouttes suspendues, et une analyse tridimensionnelle dudit complexe, spécialement avec la méthode de remplacement moléculaire,
- ou l'analyse tridimensionnelle dudit complexe dans un état soluble, en utilisant une méthode appropriée telle que la RMN.
